(19) Europäisches Patentamt
European Patent Office
Office européen des brevets

(11) EP 4 129 345 A1

(12) **EUROPEAN PATENT APPLICATION**
published in accordance with Art. 153(4) EPC

(43) Date of publication:
**08.02.2023 Bulletin 2023/06**

(21) Application number: **21775069.4**

(22) Date of filing: **25.03.2021**

(51) International Patent Classification (IPC):
***A61K 47/68*** (2017.01) ***A61K 47/65*** (2017.01)
***A61K 31/4745*** (2000.01) ***A61P 35/00*** (2000.01)
***A61P 35/02*** (2000.01) ***C07D 491/22*** (1974.07)

(52) Cooperative Patent Classification (CPC):
**A61K 47/6803; A61K 47/6851; A61K 47/6855; A61K 47/6889; A61P 35/00; A61P 35/02; C07D 491/22**

(86) International application number:
**PCT/CN2021/082854**

(87) International publication number:
**WO 2021/190581 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.03.2020 CN 202010219601**
**17.03.2021 CN 202110287012**

(71) Applicants:
- **Jiangsu Hengrui Pharmaceuticals Co., Ltd.
Lianyungang, Jiangsu 222047 (CN)**
- **Shanghai Hengrui Pharmaceutical Co., Ltd.
Shanghai 200245 (CN)**

(72) Inventors:
- **YUE, Zhanlong
Shanghai 200245 (CN)**
- **YAN, Zhen
Shanghai 200245 (CN)**
- **LIU, Xun
Shanghai 200245 (CN)**

(74) Representative: **Potter Clarkson
The Belgrave Centre
Talbot Street
Nottingham NG1 5GG (GB)**

Remarks:
The complete document including Reference Table(s) and the Sequence Listing(s) can be downloaded from the EPO website

(54) **PHARMACEUTICAL COMPOSITION COMPRISING ANTIBODY DRUG CONJUGATE AND USE THEREOF**

(57) A pharmaceutical composition, comprising an antibody drug conjugate in a buffer solution. The antibody drug conjugate has a structure represented by the general formula (Pc-L-Y-D). The pharmaceutical composition further comprises sugar and a surfactant.

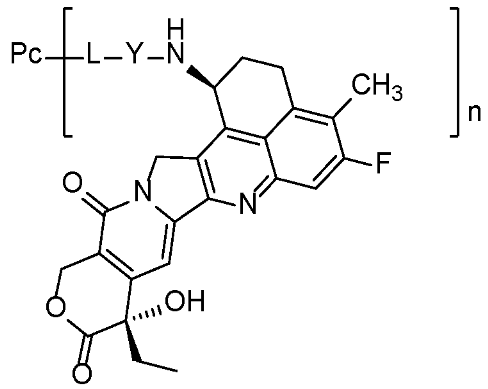

(Pc-L-Y-D)

EP 4 129 345 A1

## Description

The present application claims priority to Chinese Patent Application (Application No. CN202010219601.7) filed on Mar. 25, 2020 and Chinese Patent Application (Application No. CN202110287012.7) filed on Mar. 17, 2021.

## TECHNICAL FIELD

The present disclosure belongs to the field of pharmaceutical formulations, and in particular relates to a pharmaceutical composition comprising an antibody drug conjugate and use thereof as an anti-cancer medicament.

## BACKGROUND

The statements herein merely provide background information related to the present disclosure and may not necessarily constitute the prior art.

Antibody drug conjugate (ADC) links a monoclonal antibody or an antibody fragment to a biologically active cytotoxin via a stable chemical linker compound, fully exploiting the binding specificity of the antibody to surface antigens of normal cells and tumor cells and the high-efficiency of the cytotoxin, and also avoiding the former's disadvantage of having a poor therapeutic effect, the latter's disadvantage of having serious toxic side effects, and the like. This means that the antibody drug conjugate can bind to tumor cells more precisely and has a reduced effect on normal cells compared to conventional chemotherapeutic drugs in the past (Mullard A, (2013) Nature Reviews Drug Discovery, 12:329-332; DiJoseph JF, Armellino DC, (2004) Blood, 103:1807-1814).

Mylotarg® (gemtuzumab ozogamicin, Wyeth Pharmaceutical Co., Ltd.), the first antibody drug conjugate, was approved by U.S. FDA in 2000 for the treatment of acute myelocytic leukemia (Drugs of the Future (2000) 25(7):686; US4970198; US5079233; US5585089; US5606040; US5693762; US5739116; US5767285; US5773001).

Adcetris® (brentuximab vedotin, Seattle Genetics) was approved by fast track review designed by U.S. FDA in August 2011 for the treatment of Hodgkin lymphoma and recurrent anaplastic large cell lymphoma (Nat. Biotechnol (2003) 21(7):778-784; WO2004010957; WO2005001038; US7090843A; US7659241; WO2008025020). Adcetris® is a novel ADC-targeted drug that can enable the drug to directly act on target CD30 on lymphoma cells and then carry out endocytosis so as to induce apoptosis of the tumor cells.

Both Mylotarg® and Adcetris® are targeted therapies against hematological tumors which are relatively simple in tissue structure compared to solid tumors. Kadcyla® (ado-trastuzumab emtansine, T-DM1) was approved by U.S. FDA in February 2013 for the treatment of HER2-positive patients with advanced or metastatic breast cancer and having drug resistance to tratuzumab (trade name: Herceptin) and paclitaxel (WO2005037992; US8088387). Kadcyla® is the first ADC drug approved by U.S. FDA for the treatment of solid tumors.

There are several classes of small molecules with cytotoxicity for antibody drug conjugates, one of which is camptothecin derivatives having an antitumor effect by inhibiting topoisomerase I. In WO2014057687; Clinical Cancer Research (2016) 22(20):5097-5108; and Cancer Sci (2016) 107:1039-1046, it was reported that camptothecin derivative exatecan (chemical name: (1*S*,9*S*)-1-amino-9-ethyl-5-fluoro-2,3-dihydro-9-hydroxy-4-methyl-1*H*,12*H*-benzo[*de*]pyrano[3',4':6,7]imidazo[1,2-*b*]quinoline-10,13(9*H*,15*H*)-dione) was applied to antibody drug conjugates (ADCs). There is still a need to further develop ADC drugs with better therapeutic effects. However, ADCs are of more complicated heterostructures than antibodies, and therefore, a greater challenge has been posed to ADC formulations for therapeutic purposes.

## SUMMARY

The present disclosure provides a pharmaceutical composition comprising an antibody drug conjugate and a buffer, wherein the antibody drug conjugate has a structure of general formula (Pc-L-Y-D):

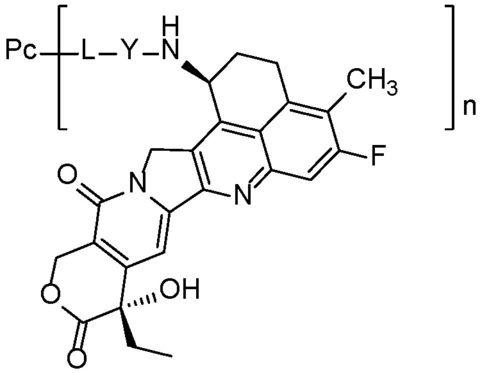

(Pc-L-Y-D) ;

wherein:

Y is selected from the group consisting of $-O-(CR^aR^b)_m-CR^1R^2-C(O)-$, $-O-CR^1R^2-(CR^aR^b)_m-$, $-O-CR^1R^2-$, $-NH-(CR^aR^b)_m-CR^1R^2-C(O)-$ and $-S-(CR^aR^b)_m-CR^1R^2-C(O)-$;

$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl;

or, $R^a$ and $R^b$, together with carbon atoms linked thereto, form cycloalkyl or heterocyclyl; $R^1$ is selected from the group consisting of halogen, haloalkyl, deuterated alkyl, cycloalkyl, cycloalkylalkyl, alkoxyalkyl, heterocyclyl, aryl and heteroaryl;

$R^2$ is selected from the group consisting of hydrogen, halogen, haloalkyl, deuterated alkyl, cycloalkyl, cycloalkylalkyl, alkoxyalkyl, heterocyclyl, aryl and heteroaryl;

or, $R^1$ and $R^2$, together with carbon atoms linked thereto, form cycloalkyl or heterocyclyl;

or, $R^a$ and $R^2$, together with carbon atoms connected thereto, form cycloalkyl or heterocyclyl;

m is an integer from 0 to 4;

n is a decimal or an integer from 1 to 10;

L is a linker unit;

Pc is an antibody or an antigen-binding fragment thereof;

the composition is at a pH of about 4.5 to about 6.0, preferably a pH of about 4.8 to about 5.3, and more preferably a pH of about 5.0 to about 5.1.

In an alternative embodiment, the buffer in the pharmaceutical composition is at a pH of about 4.5 to about 6.0; non-limiting examples include about 4.5, about 4.6, about 4.7, about 4.8, about 4.9, about 5.0, about 5.1, about 5.2, about 5.3, about 5.4, about 5.5, about 5.6, about 5.7, about 5.8, about 5.9 and about 6.0; about 4.8 to about 5.3 is preferred, and about 5.0 to about 5.1 is more preferred. In some embodiments, the pharmaceutical composition is at a pH of 4.5 to 5.2, preferably a pH of 4.8 to 5.2, and more preferably a pH of 5.0 to 5.1. In some embodiments, the pharmaceutical composition is at a pH of 5.0. In an alternative embodiment, the pharmaceutical composition further comprises a surfactant, which may be selected from the group consisting of polysorbate, polysorbate 20, polysorbate 80, poloxamer, Triton, sodium dodecyl sulfonate, sodium lauryl sulfonate, sodium octyl glycoside, lauryl-sulfobetaine, myristyl-sulfobetaine, linoleyl-sulfobetaine, stearyl-sulfobetaine, lauryl-sarcosine, myristyl-sarcosine, linoleyl-sarcosine, stearyl-sarcosine, linoleyl-betaine, myristyl-betaine, cetyl-betaine, lauramido propyl-betaine, cocaramide propyl-betaine, linoleinamide propyl-betaine, myristylamide propyl-betaine, palmitamide propyl-betaine, isostearamide propyl-betaine, myristylamide propyl-dimethylamine, palmitamide propyl-dimethylamine, isostearamide propyl-dimethylamine, sodium methyl cocoyl, sodium methyl oleyl taurate, polyethylene glycol, polypropylene glycol, copolymer of ethylene and propylene glycol, and the like. The surfactant is preferably polysorbate 80 or polysorbate 20, more preferably polysorbate 80.

In an alternative embodiment, the surfactant in the pharmaceutical composition is at a concentration of about 0.01 mg/mL to about 1.0 mg/mL. In an alternative embodiment, the surfactant in the pharmaceutical composition is at a concentration of about 0.05 mg/mL to about 0.5 mg/mL, preferably about 0.1 mg/mL to about 0.3 mg/mL, about 0.2 mg/mL to about 0.6 mg/mL, about 0.2 mg/mL to about 0.5 mg/mL, or about 0.2 mg/mL to about 0.3 mg/mL, and more preferably about 0.2 mg/mL; non-limiting examples include 0.1 mg/mL, 0.15 mg/mL, 0.2 mg/mL, 0.25 mg/mL, 0.3 mg/mL, 0.35 mg/mL, 0.4 mg/mL, 0.45 mg/mL, 0.5 mg/mL and 0.6 mg/mL.

In an alternative embodiment, the aforementioned pharmaceutical composition further comprises a saccharide. "Saccharide" of the present disclosure includes the general composition $(CH_2O)_n$ and derivatives thereof, including

monosaccharides, disaccharides, trisaccharides, polysaccharides, sugar alcohols, reducing sugars, non-reducing sugars, etc. The saccharide may be selected from the group consisting of glucose, sucrose, trehalose, lactose, fructose, maltose, dextran, glycerin, erythritol, glycerol, arabitol, sylitol, sorbitol, mannitol, mellibiose, melezitose, raffinose, mannotriose, stachyose, maltose, lactulose, maltulose, glucitol, maltitol, lactitol, iso-maltulose, etc. The saccharide is preferably non-reducing disaccharide, more preferably trehalose or sucrose, and most preferably sucrose.

In an alternative embodiment, the saccharide in the aforementioned pharmaceutical compositions is at a concentration of about 60 mg/mL to about 90 mg/mL; non-limiting examples include 60 mg/mL, 65 mg/mL, 70 mg/mL, 75 mg/mL, 80 mg/mL, 85 mg/mL and 90 mg/mL; 80 mg/mL is preferred. In some embodiments, the saccharide is at a concentration of 70 mg/mL to 90 mg/mL.

In an alternative embodiment, the antibody drug conjugate in the pharmaceutical composition is at a concentration of about 1 mg/mL to about 100 mg/mL; non-limiting examples include 1 mg/mL, 10 mg/mL, 11 mg/mL, 12 mg/mL, 13 mg/mL, 14 mg/mL, 15 mg/mL, 16 mg/mL, 17 mg/mL, 18 mg/mL, 19 mg/mL, 20 mg/mL, 21 mg/mL, 22 mg/mL, 23 mg/mL, 24 mg/mL, 25 mg/mL, 26 mg/mL, 27 mg/mL, 28 mg/mL, 29 mg/mL, 30 mg/mL, 40 mg/mL, 50 mg/mL, 60 mg/mL, 70 mg/mL, 80 mg/mL, 90 mg/mL and 100 mg/mL; about 10 mg/mL to about 30 mg/mL is preferred, and about 20 mg/mL to about 22 mg/mL is more preferred. Specifically, non-limiting examples include 20.1 mg/mL, 20.2 mg/mL, 20.3 mg/mL, 20.4 mg/mL, 20.5 mg/mL, 20.6 mg/mL, 20.7 mg/mL, 20.8 mg/mL, 20.81 mg/mL, 20.82 mg/mL, 20.83 mg/mL, 20.84 mg/mL, 20.85 mg/mL, 20.86 mg/mL, 20.87 mg/mL, 20.88 mg/mL, 20.89 mg/mL, 20.9 mg/mL, 20.9 mg/mL, 20.91 mg/mL, 20.92 mg/mL, 20.93 mg/mL, 20.94 mg/mL, 20.95 mg/mL, 20.96 mg/mL, 20.97 mg/mL, 20.98 mg/mL, 20.99 mg/mL and 21 mg/mL. In an alternative embodiment, the antibody drug conjugate in the pharmaceutical composition is at a concentration of about 10 mg/mL to about 30 mg/mL, preferably about 20 mg/mL, based on the naked antibody (i.e., the antibody portion of the ADC).

In an alternative embodiment, the buffer in the aforementioned pharmaceutical composition is selected from the group consisting of a histidine salt buffer, a succinate buffer and a citrate buffer, preferably a succinate buffer, and more preferably succinic acid-sodium succinate buffer.

In an alternative embodiment, the buffer in the pharmaceutical composition is at a concentration of about 5 mM to about 50 mM; non-limiting examples include 1 mM, 5 mM, 6 mM, 7 mM, 8 mM, 9 mM, 10 mM, 11 mM, 12 mM, 13 mM, 14 mM, 15 mM, 16 mM, 17 mM, 18 mM, 19 mM, 20 mM, 30 mM, 40 mM and 50 mM; about 5 mM to about 20 mM is preferred, and about 10 mM is most preferred.

In an alternative embodiment, the drug loading (n) may range from 3 to 8, 4 to 8, 5 to 7, more preferably 5.3 to 6.1, 5.7 cytotoxic drugs binding to each antibody or an antigen-binding fragment (Pc) thereof. n is a decimal or an integer.

In an alternative embodiment, the pharmaceutical composition comprises:
(a) the antibody drug conjugate at about 10 mg/mL to about 30 mg/mL, (b) a polysorbate at about 0.05 mg/mL to about 0.5 mg/mL, (c) a saccharide at about 60 mg/mL to about 90 mg/mL, and (d) a buffer at about 5 mM to about 20 mM; the pharmaceutical composition being at a pH of about 4.8 to about 5.3.

In an alternative embodiment, the pharmaceutical composition comprises:
(a) the antibody drug conjugate at about 10 mg/mL to about 30 mg/mL, (b) a polysorbate at about 0.05 mg/mL to about 0.5 mg/mL, (c) a saccharide at about 60 mg/mL to about 90 mg/mL, and (d) a buffer at about 5 mM to about 20 mM; the pharmaceutical composition being at a pH of 4.8 to 5.2.

In an alternative embodiment, the pharmaceutical composition comprises:
(a) the antibody drug conjugate at about 20 mg/mL to about 22 mg/mL, (b) polysorbate 80 at about 0.2 mg/mL, (c) sucrose at 80 mg/mL, and (d) a succinate buffer at 10 mM; the pharmaceutical composition being at a pH of about 5.0 to about 5.1. In some embodiments, the pharmaceutical composition is at a pH of 5.0 to 5.1.

In an alternative embodiment, in the aforementioned antibody drug conjugate,

-Y- is $-O-(CR^aR^b)m-CR^1R^2-C(O)-$;

$R^a$ and $R^b$ are identical or different and are each independently selected from the group consisting of hydrogen, deuterium, halogen and alkyl;
$R^1$ is $C_{3-7}$ cycloalkylalkyl or $C_{3-7}$ cycloalkyl;
$R^2$ is selected from the group consisting of hydrogen, haloalkyl and $C_{3-7}$ cycloalkyl, preferably hydrogen;
or, $R^1$ and $R^2$, together with carbon atoms linked thereto, form $C_{3-7}$ cycloalkyl;
m is 0 or 1.

In an alternative embodiment, in the aforementioned antibody drug conjugate,

-Y- is $-O-(CH_2)m-CR^1R^2-C(O)-$;

$R^1$ is $C_{3-7}$ cycloalkylalkyl or $C_{3-7}$ cycloalkyl;
$R^2$ is selected from the group consisting of hydrogen, haloalkyl and $C_{3-7}$ cycloalkyl;
or, $R^1$ and $R^2$, together with carbon atoms linked thereto, form $C_{3-7}$ cycloalkyl;
m is 0 or 1.

In an alternative embodiment, in the aforementioned antibody drug conjugate,

-Y- is $-O-(CH_2)m-CR^1R^2-C(O)-$;

$R^1$ is $C_{3-7}$ cycloalkylalkyl or $C_{3-7}$ cycloalkyl;
$R^2$ is hydrogen;
or, $R^1$ and $R^2$, together with carbon atoms linked thereto, form $C_{3-7}$ cycloalkyl;
m is 0 or 1.

In an alternative embodiment, in the aforementioned antibody drug conjugate,

-Y- is $-O-(CH_2)m-CR^1R^2-C(O)-$;

$R^1$ is $C_{3-7}$ cycloalkylalkyl or $C_{3-7}$ cycloalkyl;
$R^2$ is hydrogen;
or, $R^1$ and $R^2$, together with carbon atoms linked thereto, form $C_{3-7}$ cycloalkyl;
m is 0.

In an alternative embodiment, in the aforementioned antibody drug conjugate,

- Y- is selected from the group consisting of:

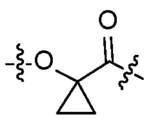 , 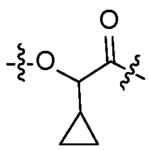 , 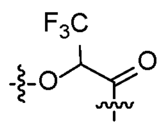 , 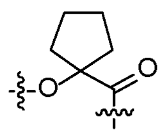 , 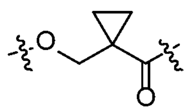 , 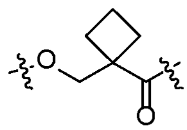 ,

and

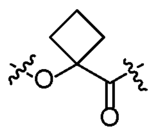 .

In an alternative embodiment, in the aforementioned antibody drug conjugate, a O end of Y is linked to the linker unit L.

In an alternative embodiment, in the aforementioned antibody drug conjugate,

- Y- is selected from the group consisting of:

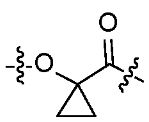 , 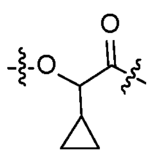 , 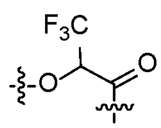 , 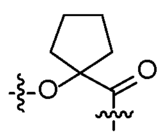 , 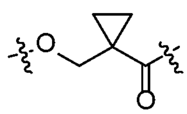 , 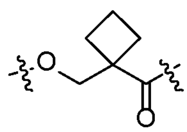 ,

and

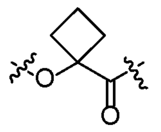

In an alternative embodiment, the aforementioned antibody drug conjugate has a structure of general formula ($Pc-L-D_1$):

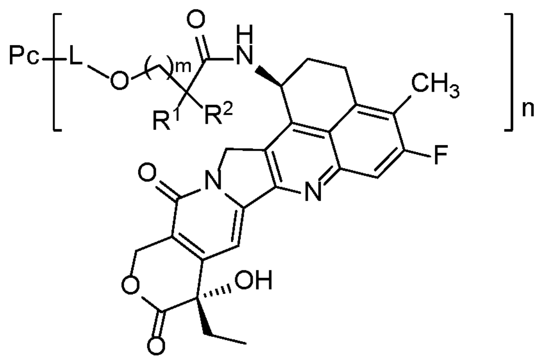

(Pc-L-D1)

wherein:

$R^1$ is cycloalkylalkyl or cycloalkyl, preferably $C_{3-7}$ cycloalkylalkyl or $C_{3-7}$ cycloalkyl;
$R^2$ is selected from the group consisting of hydrogen, haloalkyl and $C_{3-7}$ cycloalkyl, preferably hydrogen;
or, $R^1$ and $R^2$, together with carbon atoms linked thereto, form $C_{3-7}$ cycloalkyl;
m is 0 or 1;
n may be an integer or a decimal from 1 to 10;
Pc is an antibody or an antigen-binding fragment thereof; and L is a linker unit.

In an alternative embodiment, in the aforementioned antibody drug conjugate, n may be an integer or a decimal from 2 to 8, preferably an integer or a decimal from 3 to 8.

In an alternative embodiment, in the aforementioned antibody drug conjugate, the linker unit -L- is $-L^1-L^2-L^3-L^4-$, wherein:

$L^1$ is selected from the group consisting of -(succinimidyl-3-yl-*N*)-W-C(O)-, $-CH_2-C(O)-NR^3-W-C(O)-$ and -C(O)-W-C(O)-, wherein W is selected from the group consisting of $C_{1-8}$ alkyl, $C_{1-8}$ alkyl-cycloalkyl and linear heteroalkyl of 1 to 8 atoms, and the heteroalkyl comprises 1 to 3 heteroatoms selected from the group consisting of N, O and S, wherein the $C_{1-8}$ alkyl, cycloalkyl and linear heteroalkyl are each independently optionally further substituted with one or more substituents selected from the group consisting of halogen, hydroxy, cyano, amino, alkyl, chloroalkyl, deuterated alkyl, alkoxy and cycloalkyl;
$L^2$ is selected from the group consisting of $-NR^4(CH_2CH_2O)p^1CH_2CH_2C(O)-$, - $NR^4(CH_2CH_2O)p^1CH_2C(O)-$, $-S(CH_2)p^1C(O)-$ and a chemical bond, wherein $p^1$ is an integer from 1 to 20; $L^2$ is preferably a chemical bond;
$L^3$ is a peptide residue consisting of 2 to 7 amino acids, wherein the amino acids are optionally further substituted with one or more substituents selected from the group consisting of halogen, hydroxy, cyano, amino, alkyl, chloroalkyl, deuterated alkyl, alkoxy and cycloalkyl;
$L^4$ is selected from the group consisting of $-NR^5(CR^6R^7)_t-$, $-C(O)NR^5$, $-C(O)NR^5(CH_2)t-$ and a chemical bond, wherein t is an integer from 1 to 6; $L^4$ is preferably $-NR^5(CR^6R^7)t-$; $R^3$, $R^4$ and $R^5$ are identical or different and are each independently selected from the group consisting of hydrogen, alkyl, haloalkyl, deuterated alkyl and hydroxyalkyl; $R^6$ and $R^7$ are identical or different and are each independently selected from the group consisting of hydrogen, halogen, alkyl, haloalkyl, deuterated alkyl and hydroxyalkyl.

In an alternative embodiment, in the aforementioned antibody drug conjugate, the linker unit $L^1$ is selected from the group consisting of $-(succinimidin-3-yl-N)-(CH_2)s^1-C(O)-$, - $(succinimidin-3-yl-N)-CH_2-cyclohexyl-C(O)-$, $-(succinimidin-3-yl-N)-(CH_2CH_2O)s^2-CH_2CH_2-C(O)-$, $-CH_2-C(O)-NR^3-(CH_2)s^3-C(O)-$ and $-C(O)-(CH_2)s^4C(O)-$, wherein $s^1$ is an integer from 2 to 8, $s^2$ is an integer from 1 to 3, $s^3$ is an integer from 1 to 8, and $s^4$ is an integer from 1 to 8; $s^1$ is preferably 5.

In an alternative embodiment, in the aforementioned antibody drug conjugate, the linker unit $L^2$ is $-NR^4(CH_2CH_2O)p^1CH_2C(O)-$ or a chemical bond, and $p^1$ is an integer from 6 to 12.

In an alternative embodiment, in the aforementioned antibody drug conjugate, $L^4$ is $-NR^5(CR^6R^7)t-$, $R^5$ is hydrogen or alkyl, $R^6$ and $R^7$ are identical or different and are each independently hydrogen or alkyl, and t is 1 or 2, preferably 2; $L^4$ is preferably $-NR^5CR^6R^7-$, more preferably $-NHCH_2-$.

In an alternative embodiment, in the aforementioned antibody drug conjugate, the linker unit -L- is $-L^1-L^2-L^3-L^4-$, wherein:

$L^1$ is

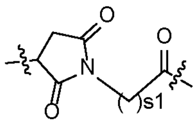

,

and $s^1$ is an integer from 2 to 8;
$L^2$ is a chemical bond;
$L^3$ is a tetrapeptide residue;
$L^4$ is $-NR^5(CR^6R^7)t-$, $R^5$ is hydrogen or alkyl, $R^6$ and $R^7$ are identical or different and are each independently hydrogen or alkyl, and t is 1 or 2.

In an alternative embodiment, in the aforementioned antibody drug conjugate, the linker unit -L- is $-L^1-L^2-L^3-L^4-$, wherein:

$L^1$ is -(succinimidin-3-yl-*N*)-$CH_2$-cyclohexyl-C(O)-;
$L^2$ is $-NR^4(CH_2CH_2O)_9CH_2C(O)-$;
$L^3$ is a tetrapeptide residue;
$L^4$ is $-NR^5(CR^6R^7)t-$, $R^5$ is hydrogen or alkyl, $R^6$ and $R^7$ are identical or different and are each independently hydrogen or alkyl, and t is 1 or 2.

In an alternative embodiment, in the aforementioned antibody drug conjugate, the peptide residue of $L^3$ is an amino acid residue formed from one, two or more amino acids selected from the group consisting of phenylalanine (E), glycine (G), valine (V), lysine (K), citrulline, serine (S), glutamic acid (E) and aspartic acid (N), preferably an amino acid residue formed from one, two or more amino acids selected from the group consisting of phenylalanine and glycine, more preferably a tetrapeptide residue, and most preferably a tetrapeptide residue of GGFG (glycine-glycine-phenylalanine-glycine).

In an alternative embodiment, in the aforementioned antibody drug conjugate, the linker unit -L- is $-L^1-L^2-L^3-L^4-$, wherein the $L^1$ end is linked to the antibody or the antigen-binding fragment thereof, and the $L^4$ end is linked to Y.

In an alternative embodiment, in the aforementioned antibody drug conjugate, the -L-Y-is:

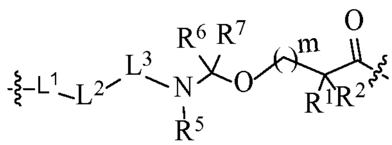

$L^1$ is -(succinimidin-3-yl-*N*)-$(CH_2)s^1$-C(O)- or -(succinimidin-3-yl-*N*)-$CH_2$-cyclohexyl-C(O)-;
$L^2$ is $-NR^4(CH_2CH_2O)p^1CH_2C(O)-$ or a chemical bond, and $p^1$ is an integer from 6 to 12;
$R^4$ is selected from the group consisting of hydrogen, alkyl, haloalkyl, deuterated alkyl and hydroxyalkyl;
$L^3$ is a tetrapeptide residue of GGFG;
$R^1$ is cycloalkylalkyl or cycloalkyl, preferably $C_{3-7}$ cycloalkylalkyl or $C_{3-7}$ cycloalkyl;
$R^2$ is selected from the group consisting of hydrogen, haloalkyl and $C_{3-7}$ cycloalkyl, preferably hydrogen;
or, $R^1$ and $R^2$, together with carbon atoms linked thereto, form $C_{3-7}$ cycloalkyl;
$R^5$ is selected from the group consisting of hydrogen and alkyl, and $R^6$ and $R^7$ are identical or different and are each independently hydrogen or alkyl;
$s^1$ is an integer from 2 to 8, preferably 5;
m is an integer from 0 to 4.

In an alternative embodiment, in the aforementioned antibody drug conjugate, the -L-Y-is:

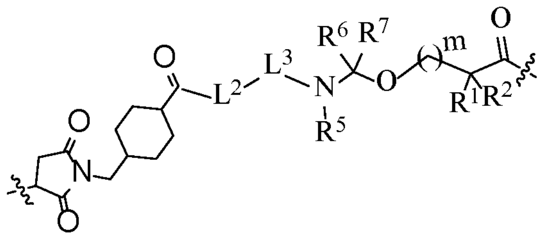

preferably:

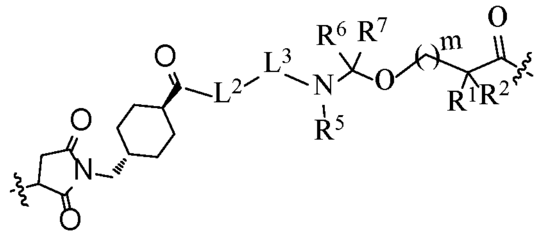

$L^2$ is $-NR^4(CH_2CH_2O)_9CH_2C(O)-$; $R^4$ is selected from the group consisting of hydrogen, alkyl, haloalkyl, deuterated alkyl and hydroxyalkyl;
$L^3$ is a tetrapeptide residue of GGFG;
$R^1$ is cycloalkylalkyl or cycloalkyl, preferably $C_{3-7}$ cycloalkylalkyl or $C_{3-7}$ cycloalkyl;
$R^2$ is selected from the group consisting of hydrogen, haloalkyl and $C_{3-7}$ cycloalkyl, preferably hydrogen;
or, $R^1$ and $R^2$, together with carbon atoms linked thereto, form $C_{3-7}$ cycloalkyl;
$R^5$ is selected from the group consisting of hydrogen and alkyl, and $R^6$ and $R^7$ are identical or different and are each independently hydrogen or alkyl;
m is an integer from 0 to 4.

In an alternative embodiment, in the aforementioned antibody drug conjugate, the -L-Y-is:

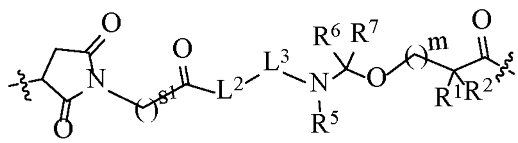

$L^2$ is a chemical bond;
$L^3$ is a tetrapeptide residue of GGFG;
$R^1$ is cycloalkylalkyl or cycloalkyl, preferably $C_{3-7}$ cycloalkylalkyl or $C_{3-7}$ cycloalkyl;
$R^2$ is selected from the group consisting of hydrogen, haloalkyl and $C_{3-7}$ cycloalkyl, preferably hydrogen;
or, $R^1$ and $R^2$, together with carbon atoms linked thereto, form $C_{3-7}$ cycloalkyl;
$R^5$ is selected from the group consisting of hydrogen and alkyl, and $R^6$ and $R^7$ are identical or different and are each independently hydrogen or alkyl;
$s^1$ is an integer from 2 to 8, preferably 5;
m is an integer from 0 to 4.

In an alternative embodiment, in the aforementioned antibody drug conjugate, the -L-Y-is:

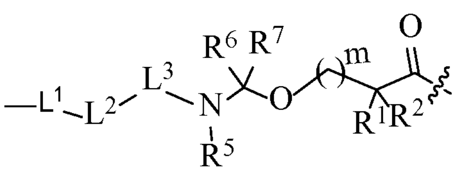

wherein:

$L^1$ is -(succinimidin-3-yl-*N*)-$(CH_2)s^1$-C(O)- or -(succinimidin-3-yl-*N*)-$CH_2$-cyclohexyl-C(O)-;
$L^2$ is -$NR^4(CH_2CH_2O)p^1CH_2C(O)$- or a chemical bond, and $p^1$ is an integer from 1 to 20;
$R^4$ is selected from the group consisting of hydrogen, alkyl, haloalkyl, deuterated alkyl and hydroxyalkyl;
$L^3$ is a tetrapeptide residue of GGFG;
$R^1$ is cycloalkylalkyl or cycloalkyl, preferably $C_{3-7}$ cycloalkylalkyl or $C_{3-7}$ cycloalkyl;
$R^2$ is selected from the group consisting of hydrogen, haloalkyl and $C_{3-7}$ cycloalkyl, preferably hydrogen;
or, $R^1$ and $R^2$, together with carbon atoms linked thereto, form $C_{3-7}$ cycloalkyl;
$R^5$, $R^6$ and $R^7$ are identical or different and are each independently hydrogen or alkyl;
$s^1$ is an integer from 2 to 8;
m is an integer from 0 to 4.

In an alternative embodiment, in the aforementioned antibody drug conjugate, the -L-Y-is:

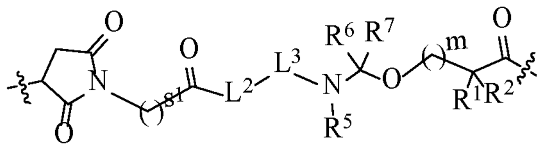

(-L-Y-)

wherein:

$L^2$ is a chemical bond;
$L^3$ is a tetrapeptide residue of GGFG;
$R^1$ is cycloalkylalkyl or cycloalkyl, preferably $C_{3-7}$ cycloalkylalkyl or $C_{3-7}$ cycloalkyl;
$R^2$ is selected from the group consisting of hydrogen, haloalkyl and $C_{3-7}$ cycloalkyl, preferably hydrogen;
or, $R^1$ and $R^2$, together with carbon atoms linked thereto, form $C_{3-7}$ cycloalkyl;
$R^5$ is selected from the group consisting of hydrogen and alkyl, and $R^6$ and $R^7$ are identical or different and are each independently hydrogen or alkyl;
$s^1$ is an integer from 2 to 8;
m is an integer from 0 to 4.

In an alternative embodiment, the antibody drug conjugate has a structure of general formula (Pc-$L_a$-Y-D):

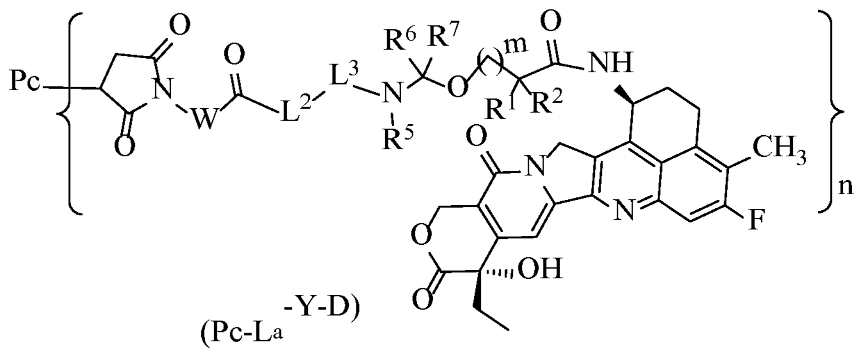

(Pc-La-Y-D)

;

wherein,

W is selected from the group consisting of $C_{1-8}$ alkyl, $C_{1-8}$ alkyl-$C_{3-7}$ cycloalkyl, and a linear heteroalkyl of 1 to 8 atoms, and the heteroalkyl comprises 1 to 3 heteroatoms selected from the group consisting of N, O and S, wherein the $C_{1-8}$ alkyl, $C_{3-7}$ cycloalkyl and linear heteroalkyl are each independently optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy, and $C_{3-7}$ cycloalkyl;
$L^2$ is selected from the group consisting of -$NR^4(CH_2CH_2O)p^1CH_2CH_2C(O)$-, - $NR^4(CH_2CH_2O)p^1CH_2C(O)$-,

$-S(CH_2)p^1C(O)-$ and a chemical bond, wherein $p^1$ is an integer from 1 to 20; $R^4$ is selected from the group consisting of hydrogen, alkyl, haloalkyl, deuterated alkyl, and hydroxyalkyl;

$L^3$ is a peptide residue consisting of 2 to 7 amino acid residues, wherein the amino acid residues are selected from the group consisting of amino acid residues formed from amino acids of phenylalanine (F), glycine (G), valine (V), lysine (K), citrulline, serine (S), glutamic acid (Q) and aspartic acid (D), and are optionally further substituted with one or more substituents selected from the group consisting of halogen, hydroxy, cyano, amino, $C_{1-6}$ alkyl, $C_{1-6}$ chloroalkyl, deuterated $C_{1-6}$ alkyl, $C_{1-6}$ alkoxy and $C_{3-7}$ cycloalkyl;

$R^1$ is $C_{1-6}$ haloalkyl or $C_{3-7}$ cycloalkyl;

$R^2$ is selected from the group consisting of hydrogen, $C_{1-6}$ haloalkyl and $C_{3-7}$ cycloalkyl; or, $R^1$ and $R^2$, together with carbon atoms linked thereto, form $C_{3-7}$ cycloalkyl;

$R^5$ is selected from the group consisting of hydrogen, $C_{1-6}$ alkyl, $C_{1-6}$ haloalkyl, deuterated $C_{1-6}$ alkyl and hydroxy $C_{1-6}$ alkyl;

$R^6$ and $R^7$ are identical or different and are each independently selected from the group consisting of hydrogen, halogen, $C_{1-6}$ alkyl, $C_{1-6}$ haloalkyl, deuterated $C_{1-6}$ alkyl and hydroxy $C_{1-6}$ alkyl;

m is 0 or 1;

n is a decimal or an integer from 3 to 8;

Pc is an antibody or an antigen-binding fragment thereof.

In an alternative embodiment, the antibody drug conjugate has a structure of general formula ($Pc-L_b-Y-D$):

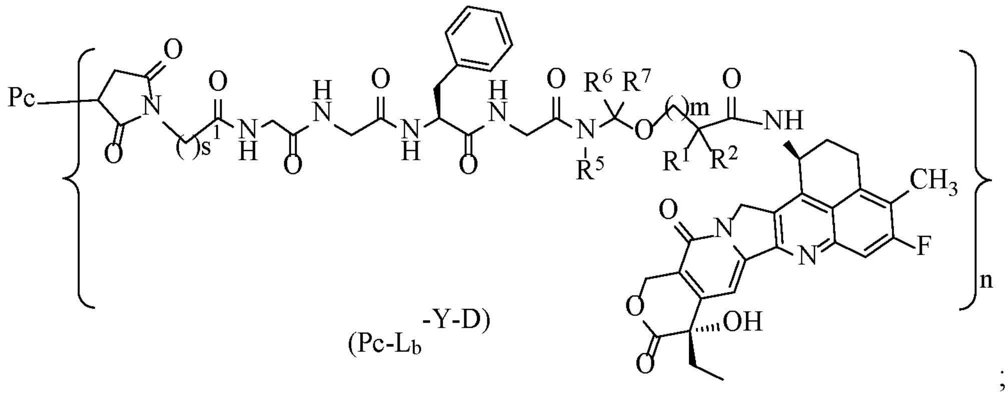

($Pc-L_b-Y-D$)

;

wherein:

$s^1$ is an integer from 2 to 8;

Pc, $R^1$, $R^2$, $R^5$, $R^6$, $R^7$, m and n are as defined in general formula ($Pc-L_a-Y-D$).

In an alternative embodiment, in the aforementioned antibody drug conjugate, the -L-Y-includes, but is not limited to:

| No. | Structure |
|---|---|
| $-L-Y^2-$ | 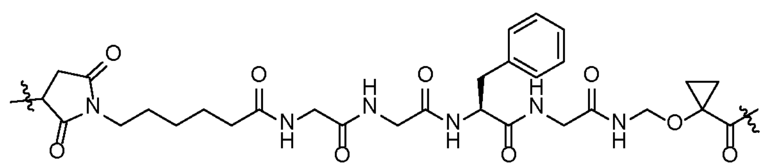 |
| $-L-Y^3-$ | 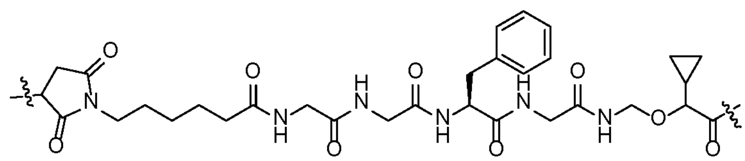 |

(continued)

| No. | Structure |
|---|---|
| -L-Y3-A- | 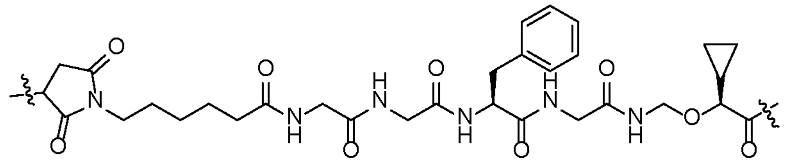 |
| -L-Y3-B- | 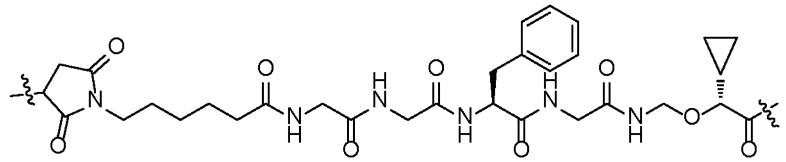 |
| -L-Y6- | 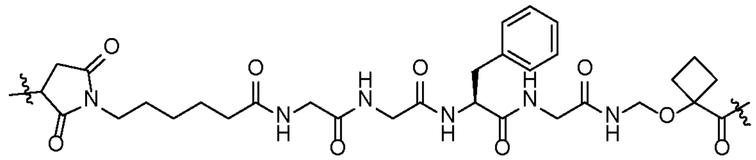 |
| -L-Y7- | 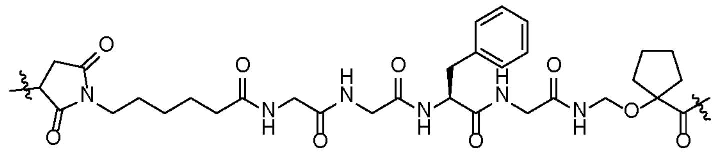 |
| -L-Y8- | 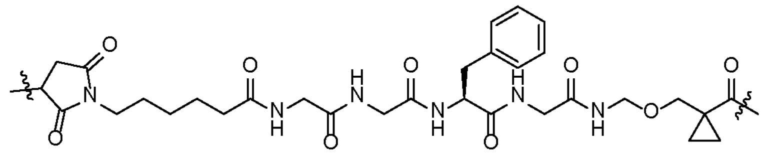 |
| -L-Y9- | 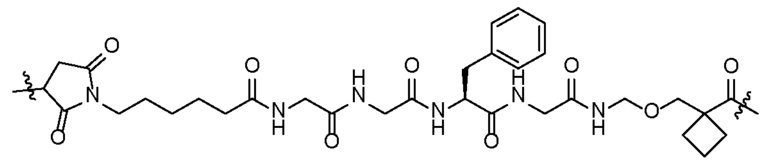 |
| -L-Y10- | 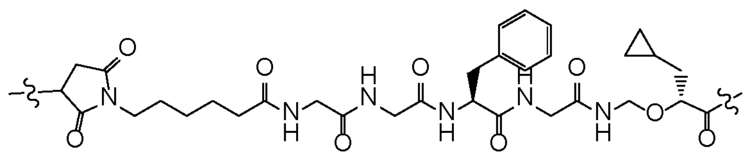 |
| -L-Y11- | 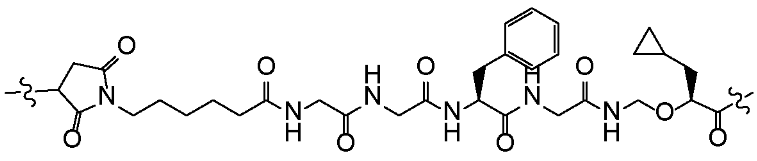 |
| -L-Y12- | 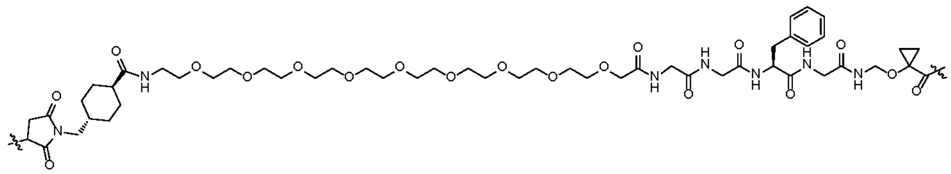 |

(continued)

| No. | Structure |
|---|---|
| -L-Y13- | 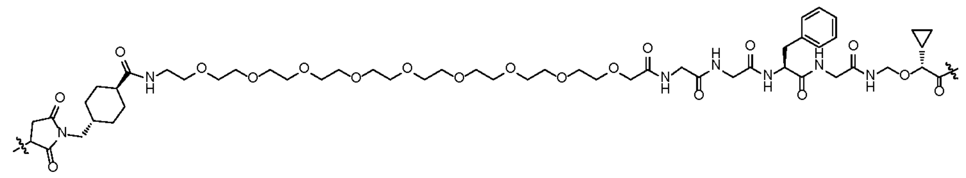 |
| -L-Y14- | 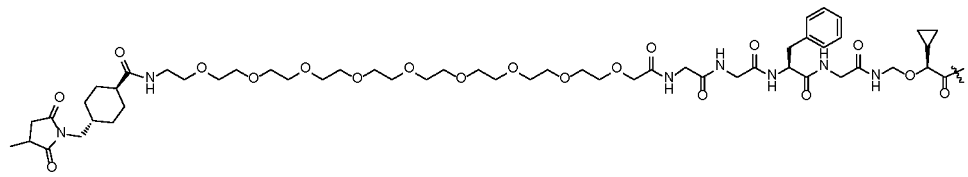 |

In an alternative embodiment, the aforementioned antibody drug conjugate has the following structure:

| Structure |
|---|
| 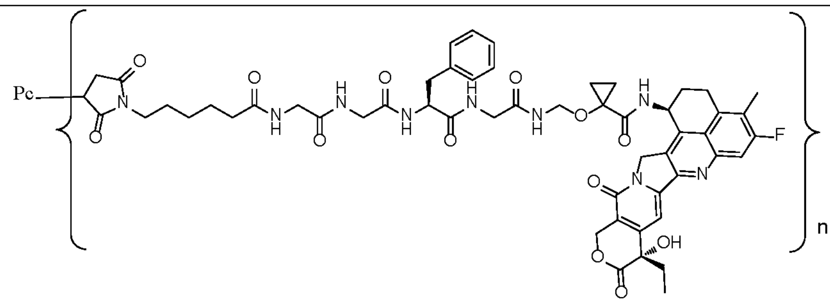 |
| 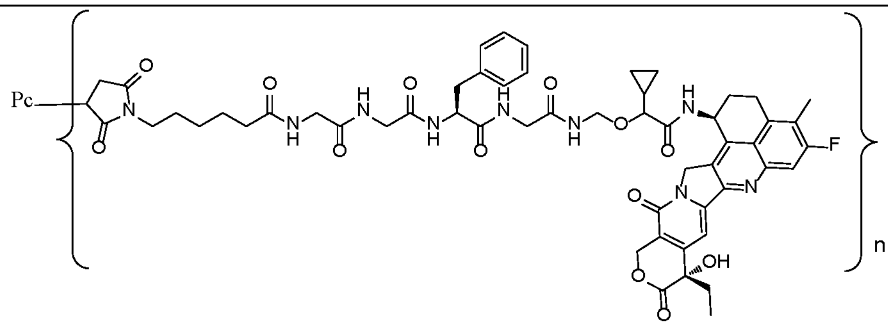 |

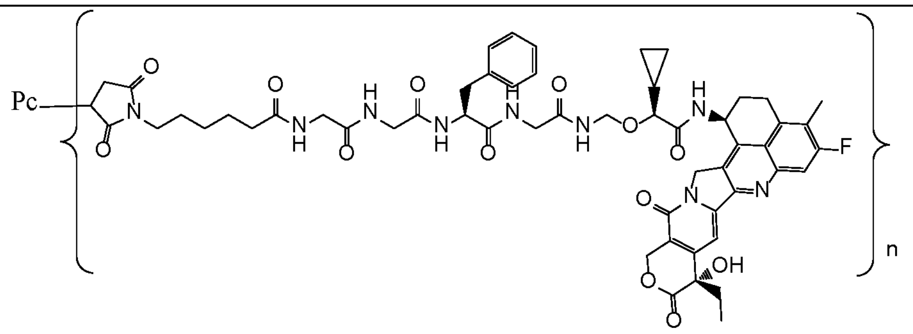

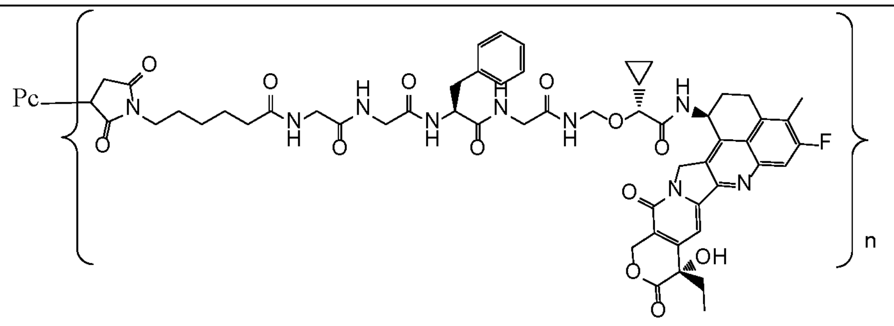

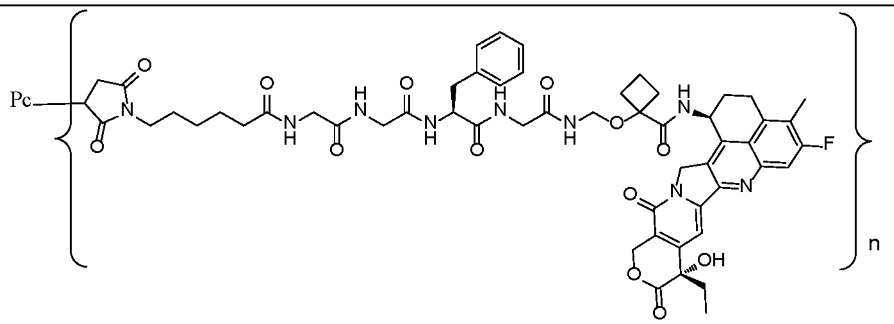

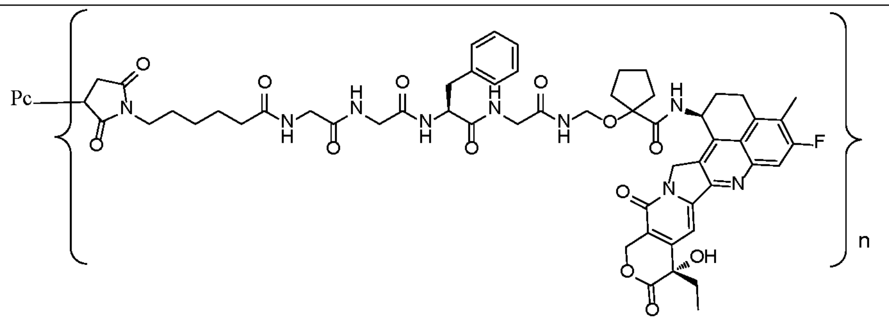

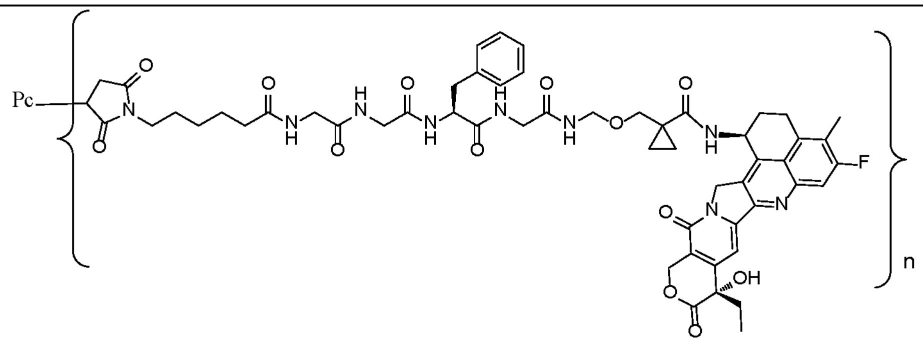

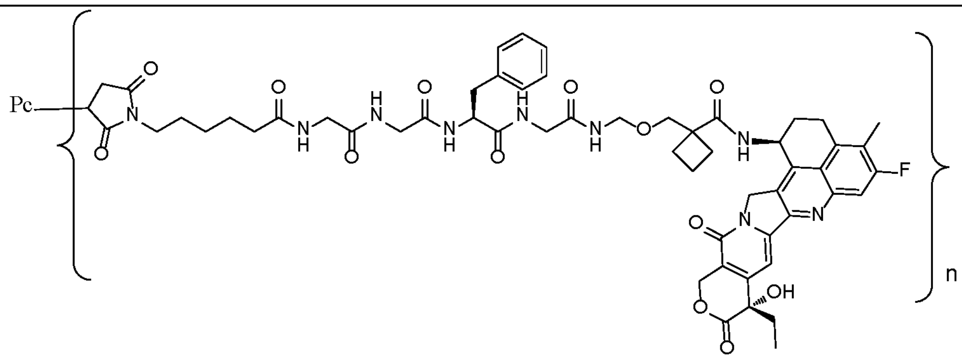

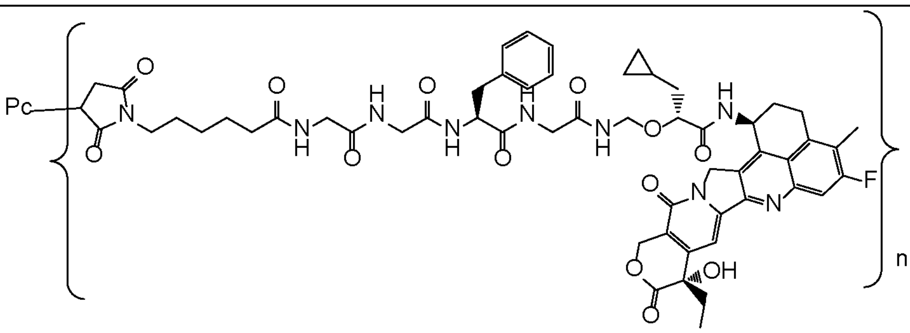

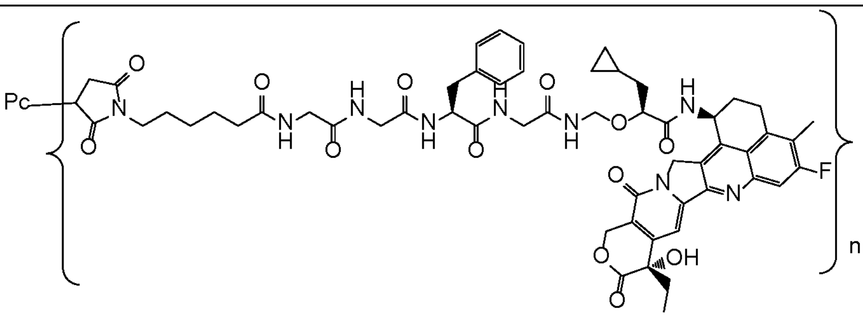

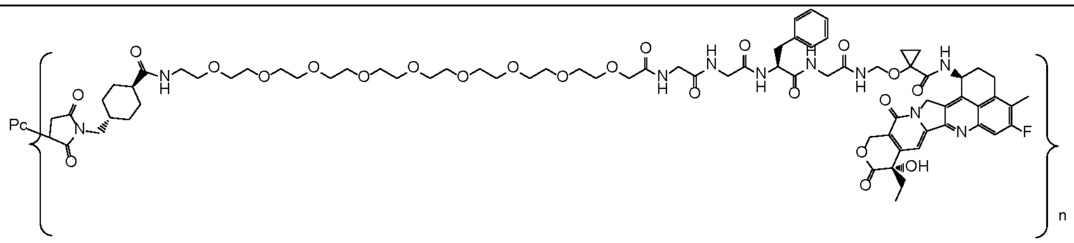

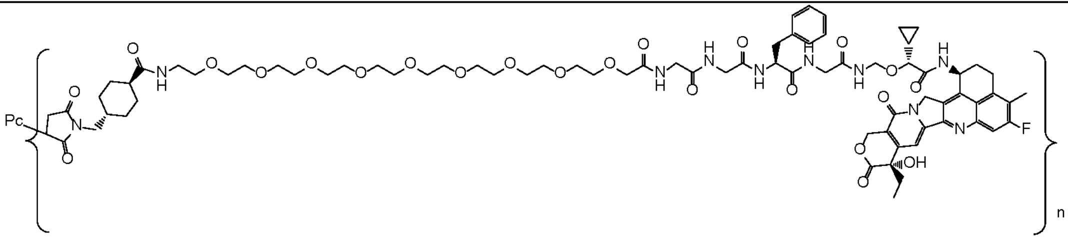

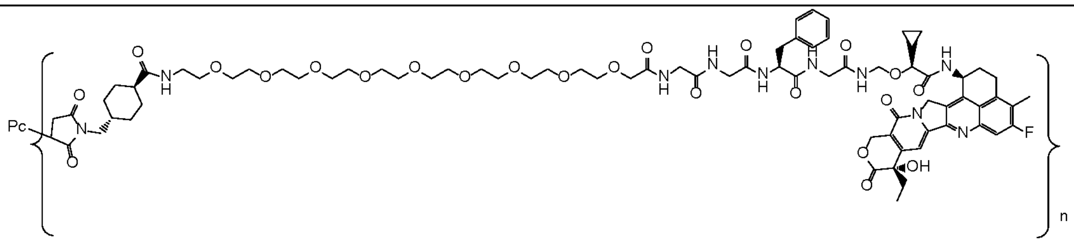

wherein Pc and n are as defined in general formula ($Pc-L_a-Y-D$).

In an alternative embodiment, the antibody drug conjugate has the following structure:

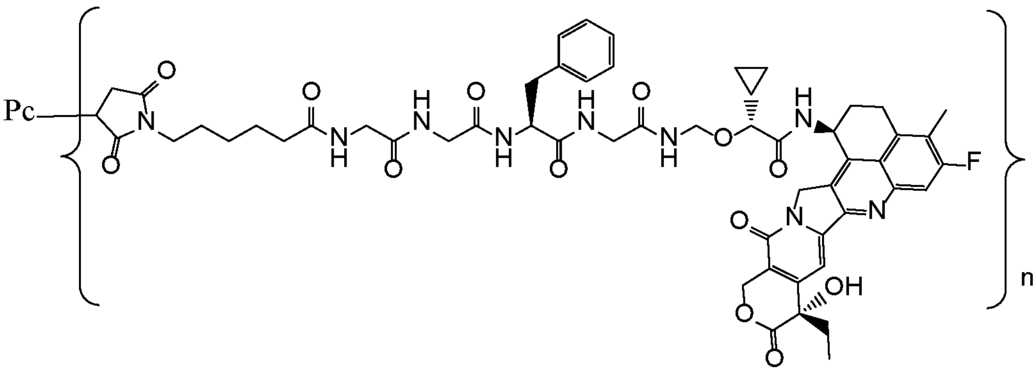

;

wherein:

n is a decimal or an integer from 3 to 8;
Pc is an antibody or an antigen-binding fragment thereof.

In an alternative embodiment, the Pc is an antibody or an antigen-binding fragment thereof, wherein the antibody is selected from the group consisting of a chimeric antibody, a humanized antibody or a fully human-derived antibody, preferably a monoclonal antibody.

In an alternative embodiment, the Pc is selected from the group consisting of an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-integrin antibody, an anti-PSMA antibody, an anti-tenascin-C antibody, an anti-SLC44A4 antibody and an anti-mesothelin antibody, or antigen-binding fragments thereof.

In an alternative embodiment, the antibody or the antigen-binding fragment thereof in the antibody drug conjugate is selected from the group consisting of trastuzumab, pertuzumab, nimotuzumab, enoblituzumab, emibetuzumab, inotuzumab, pinatuzumab, brentuximab, gemtuzumab, bivatuzumab, lorvotuzumab, cBR96 and glematumamab, or antigen-binding fragments thereof.

In an alternative embodiment, the antibody drug conjugate has the following structure:

| Exemplary ADC | Structural formula |
|---|---|
| ADC-3 | 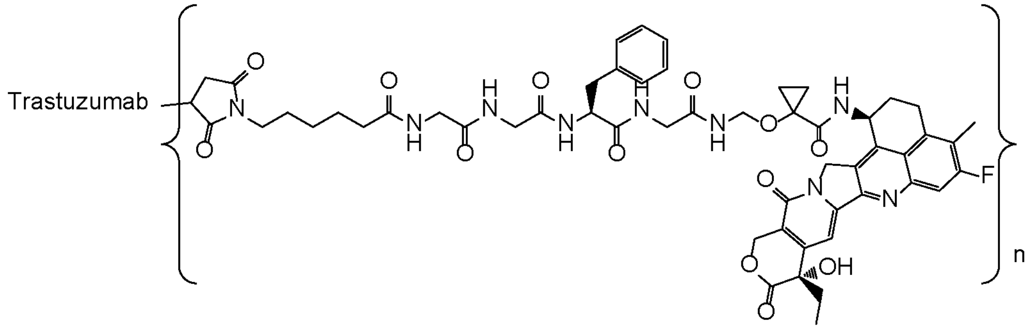 |
|  | 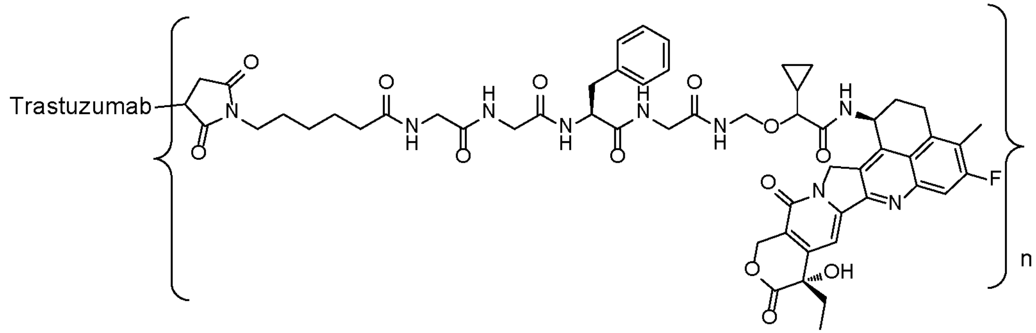 |
| ADC-6 | 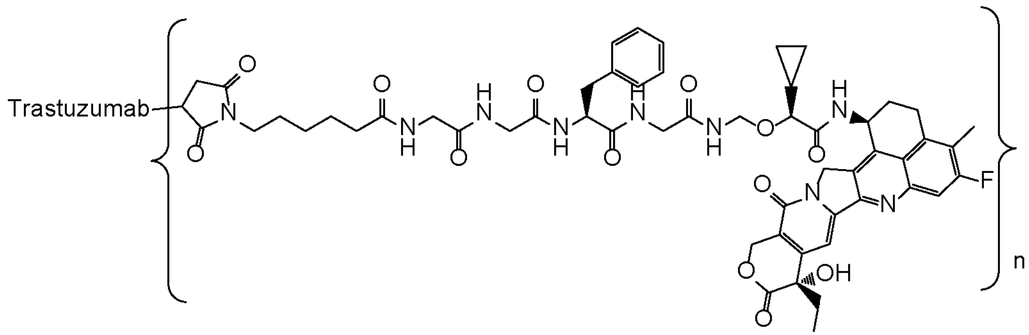 |

(continued)

| Exemplary ADC | Structural formula |
|---|---|
| ADC-4/ ADC-5/ ADC-17/ ADC-19/ ADC-22/ ADC-24/ ADC-32/ ADC-33 | 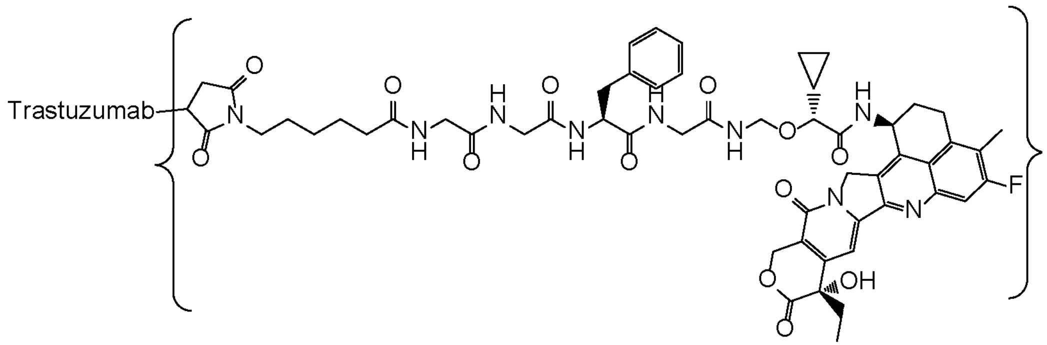 |
| ADC-7 | 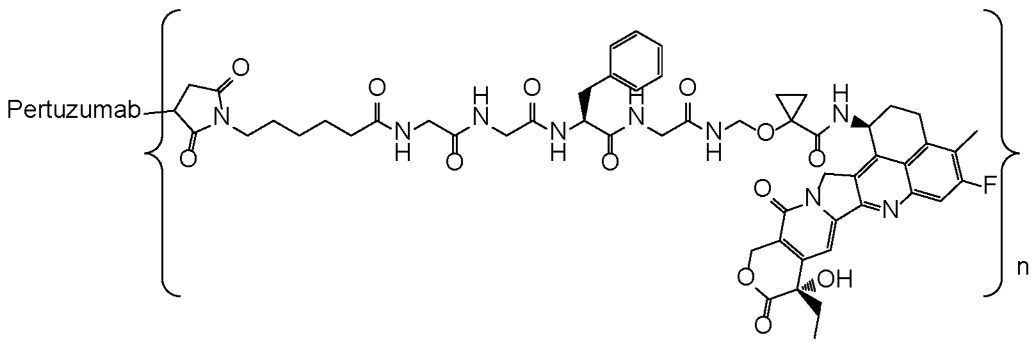 |
| | 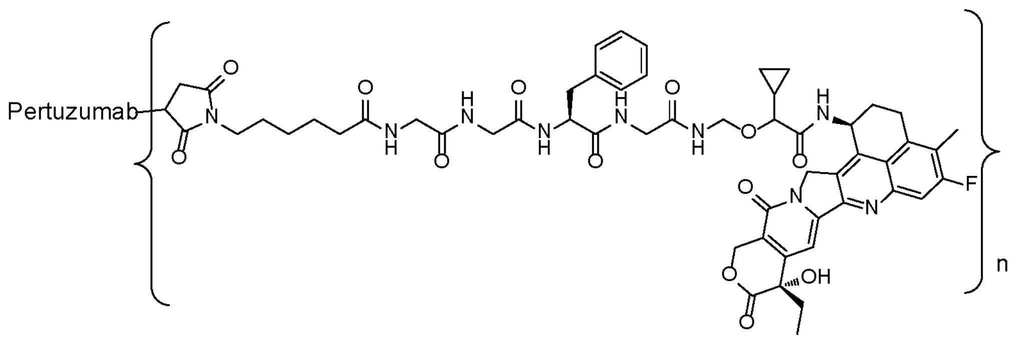 |

(continued)

| Exemplary ADC | Structural formula |
|---|---|
| | 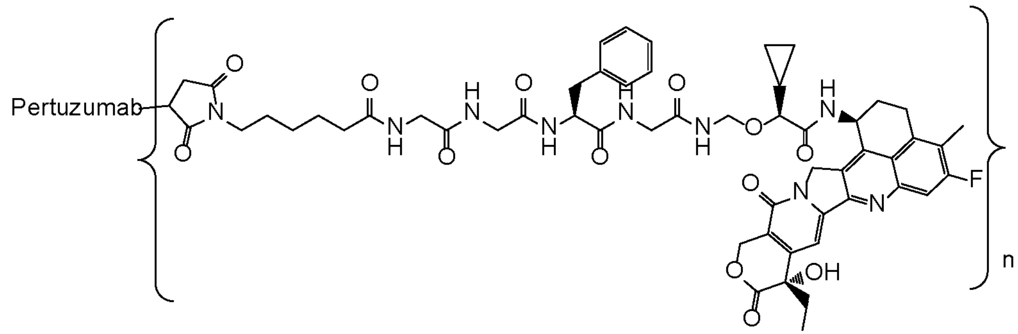 |
| | 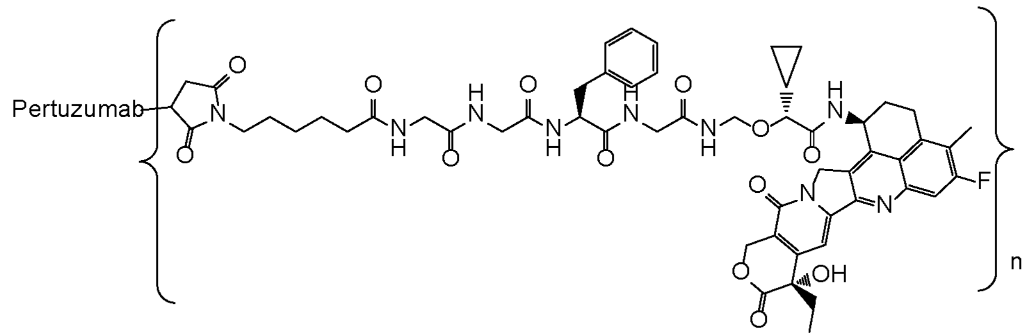 |
| ADC-10/ ADC-11 | 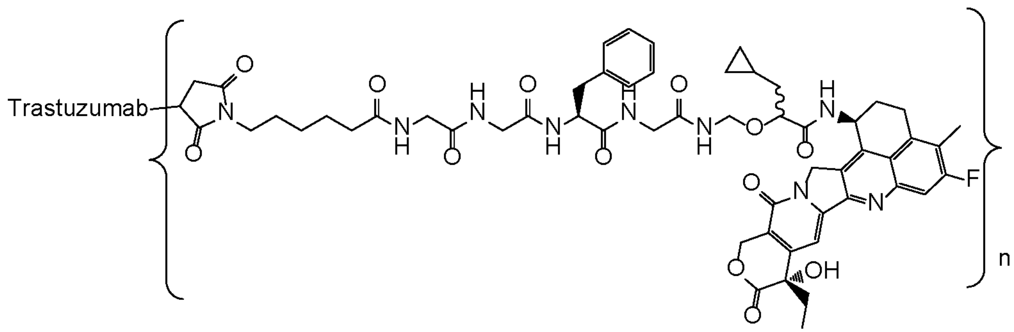 |

(continued)

| Exemplary ADC | Structural formula |
|---|---|
| | 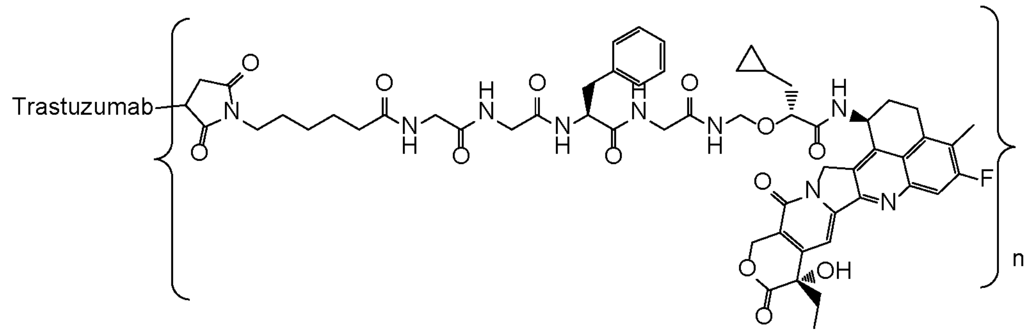 |
| | 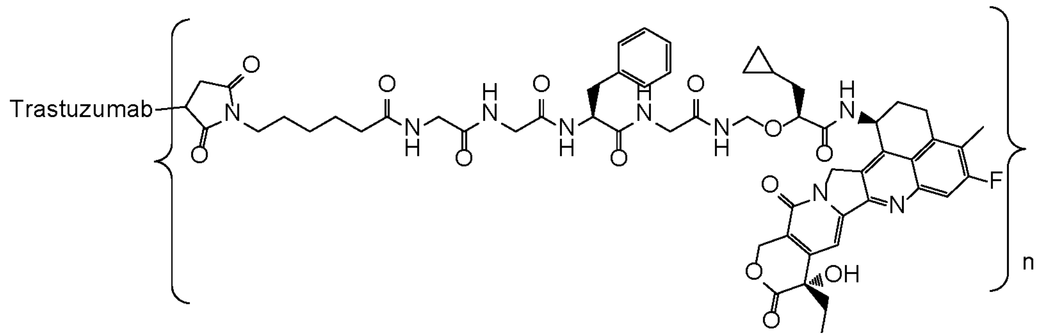 |
| ADC-12 | 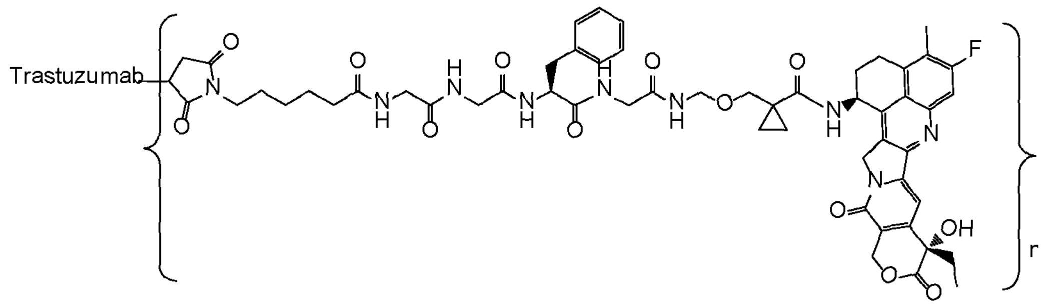 |

(continued)

| Exemplary ADC | Structural formula |
| --- | --- |
| ADC-13 | 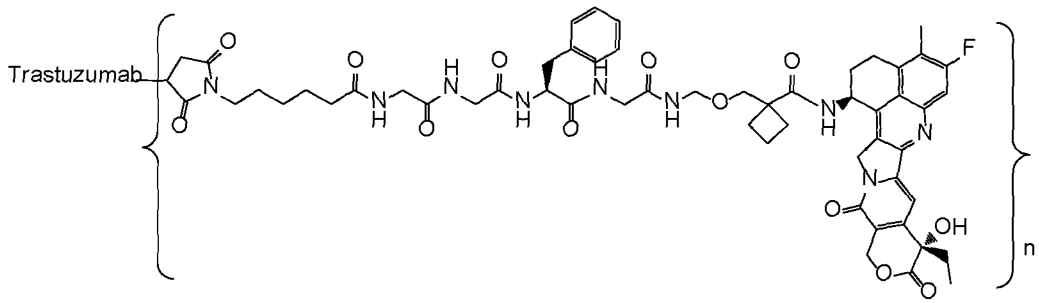 |
| ADC-14 | 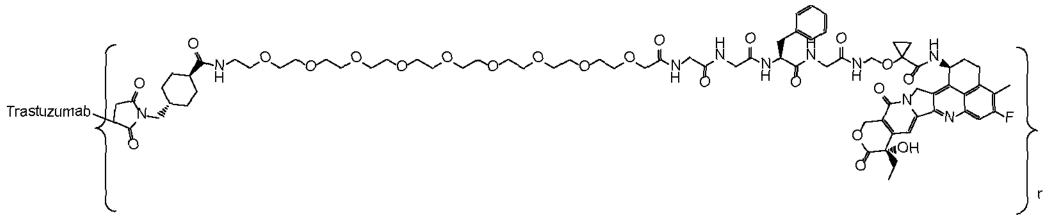 |
| ADC-15 | 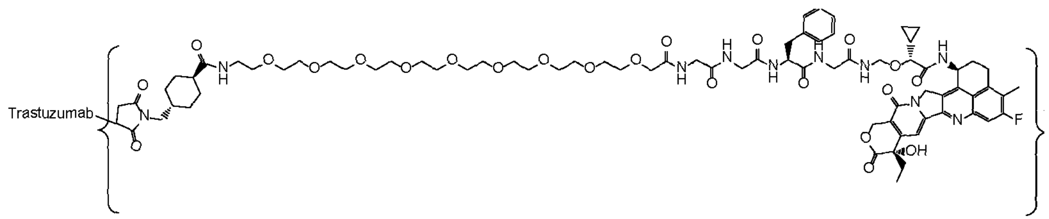 |
| ADC-16 | 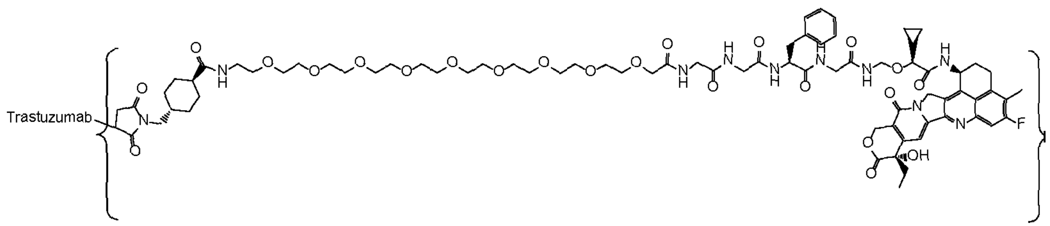 |

(continued)

| Exemplary ADC | Structural formula |
|---|---|
| ADC-25/ ADC-27/ ADC-29 | 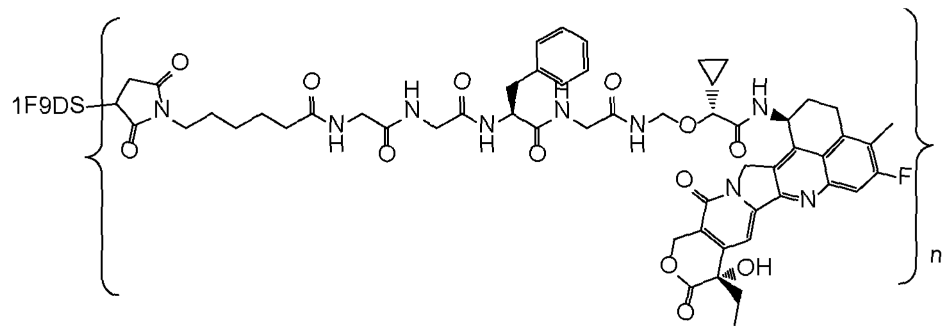 |
| ADC-31 | 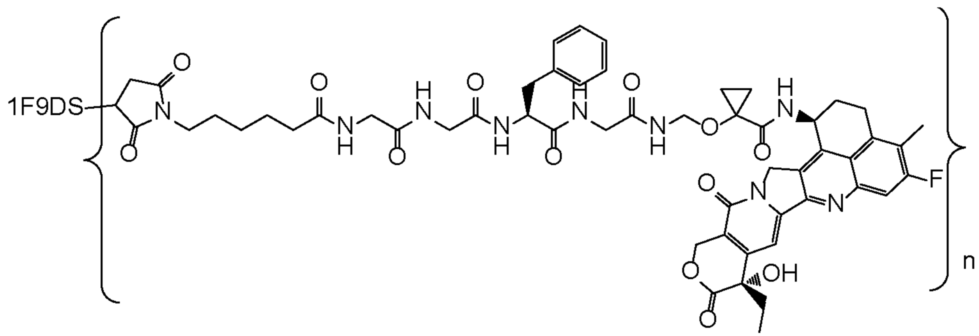 |

wherein n is a non-zero integer or a decimal from 0 to 10, preferably an integer or a decimal between 1 and 10, more preferably an integer or a decimal from 2 to 8, and most preferably an integer or a decimal from 3 to 8.

In an alternative embodiment, the antibody drug conjugate in the pharmaceutical composition has the following structure:

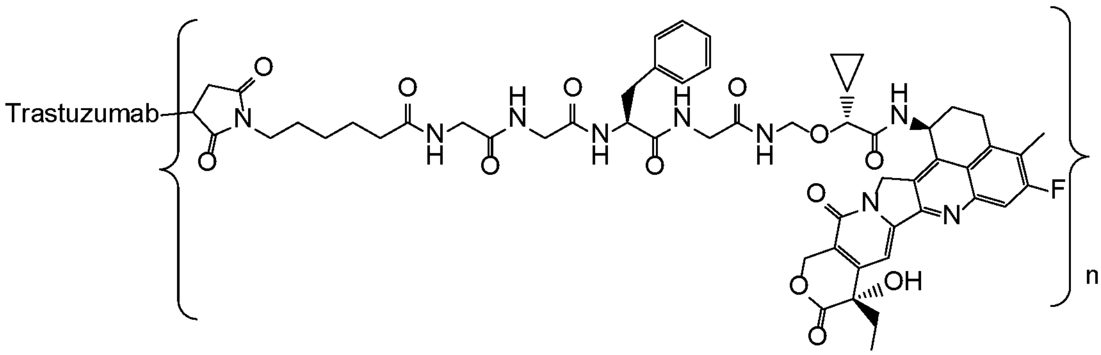

;

wherein n is a decimal or an integer from 3 to 8.

The present disclosure provides a pharmaceutical composition comprising: (a) the antibody drug conjugate at about 10 mg/mL to about 30 mg/mL, (b) a polysorbate at about 0.05 mg/mL to about 0.5 mg/mL, (c) a saccharide at about 60 mg/mL to about 90 mg/mL, and (d) a buffer at about 5 mM to about 20 mM; the composition being at a pH of 4.8 to 5.2;

wherein the antibody drug conjugate has the following structure:

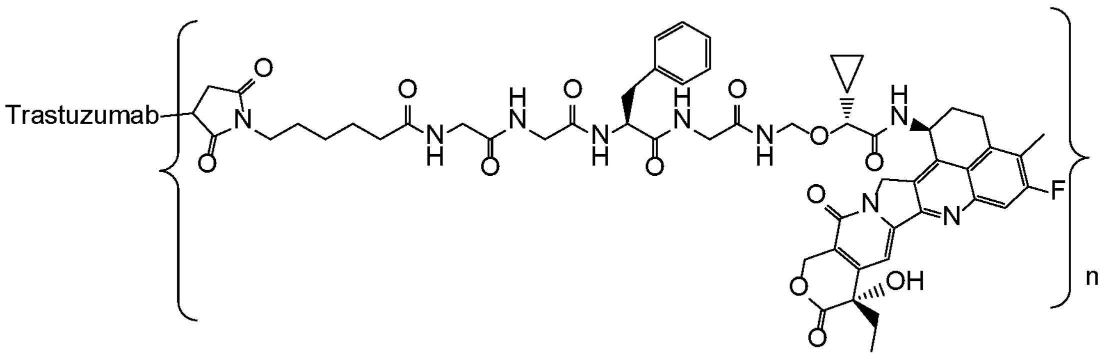

;

wherein n is a decimal or an integer from 3 to 8.

The present disclosure provides a pharmaceutical composition comprising: (a) an antibody drug conjugate at about 20 mg/mL to about 22 mg/mL, (b) polysorbate 80 at about 0.2 mg/mL, (c) sucrose at about 80 mg/mL, and (d) a succinate buffer at about 10 mM; the pharmaceutical composition being at a pH of 5.0 to 5.1;

wherein the antibody drug conjugate has the following structure:

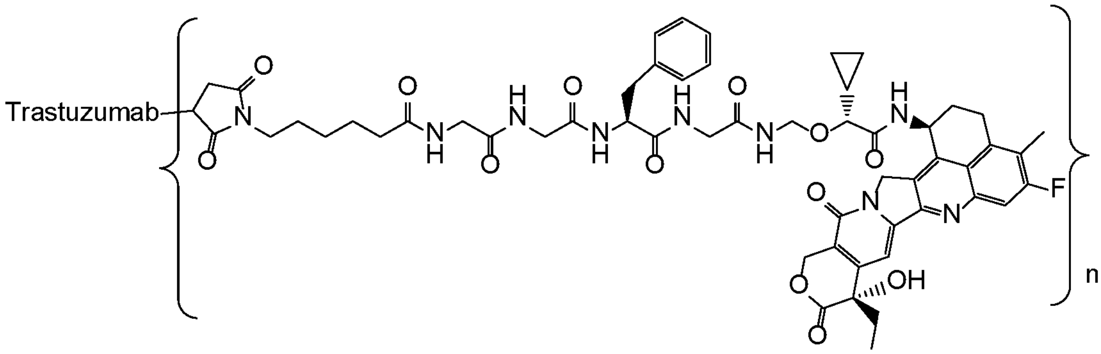

;

wherein n is a decimal or an integer from 3 to 8.

The present disclosure also provides a lyophilized formulation comprising an antibody drug conjugate, wherein the formulation can be reconstituted to form the pharmaceutical composition described above.

The present disclosure also provides a method for preparing a lyophilized formulation comprising an antibody drug conjugate, which comprises a step of lyophilizing the pharmaceutical composition described above.

In an alternative embodiment, the lyophilizing in the method for preparing a lyophilized formulation comprising an antibody drug conjugate comprises steps of pre-freezing, primary drying and secondary drying. Lyophilization is carried out by freezing the formulation and then subliming water at a temperature suitable for the primary drying. Under these conditions, the product is at a temperature lower than the eutectic point or the collapse temperature of the formulation. Typically, the temperature for the primary drying ranges from about -30 °C to 25 °C (assuming the product remains frozen during the primary drying). The formulation, the size and type of the container (e.g., glass vial) containing the sample, and the liquid volume determine the time required for drying, which may range from a few hours to several days (e.g., 40-60 hours). The secondary drying may be carried out at about 0-40 °C, depending primarily on the type and size of the container and the type of the protein used. The time needed for the secondary drying is determined by the desired residual water content in the product, and it typically takes at least about 5 hours. Typically, the water content of the formulation lyophilized at low pressure is less than about 5%, preferably less than about 3%. The pressure may be the same as that applied to the step of primary drying; preferably, the pressure of the secondary drying is lower than that of the primary drying. The lyophilization conditions may vary with the formulation and vial size.

In an alternative embodiment of the present disclosure, 5 mL of a stock solution of the pharmaceutical composition is lyophilized, the lyophilization program being as follows: for the pre-freezing, the temperature is -5 °C or -45 °C; for the primary drying, the temperature is -20 °C, and the degree of vacuum is 10 Pa; for the secondary drying, the temperature is 25 °C, and the degree of vacuum is 1 Pa.

In some embodiments, the lyophilized formulation is stable at 2-8 °C for at least 3 months, at least 6 months, at least 12 months, at least 18 months, or at least 24 months. In some embodiments, the lyophilized formulation is stable at 40 °C for at least 7 days, at least 14 days, or at least 28 days.

The present disclosure also provides a lyophilized formulation comprising an antibody drug conjugate obtained by lyophilizing the pharmaceutical composition comprising an anti-HER2 antibody drug conjugate described above.

The present disclosure also provides a reconstituted solution comprising an antibody drug conjugate obtained by reconstituting the lyophilized formulation described above.

The present disclosure also provides a method for preparing the reconstituted solution described above comprising a step of reconstituting the aforementioned lyophilized formulation with a solution selected from the group consisting of, but not limited to, water for injection, normal saline or glucose solution.

In an alternative embodiment, the reconstituted solution comprises the following components:

(a) the antibody drug conjugate at about 10 mg/mL to about 30 mg/mL, (b) a polysorbate at about 0.05 mg/mL to about 0.5 mg/mL, (c) a saccharide at about 60 mg/mL to about 90 mg/mL, and (d) a buffer at about 5 mM to about 20 mM; the reconstituted solution being at a pH of 4.8 to 5.2.

In an alternative embodiment, the reconstituted solution comprises the following components:

(a) an antibody drug conjugate at about 20 mg/mL to 22 mg/mL, (b) polysorbate 80 at about 0.2 mg/mL, (c) sucrose at about 80 mg/mL, and (d) a succinate buffer at about 10 mM; the reconstituted solution being at a pH of 5.0 to 5.1.

The present disclosure also provides an article of manufacture comprising a container containing the pharmaceutical composition, the lyophilized formulation or the reconstituted solution described above. In some embodiments, the container is a tubular injection vial made of neutral borosilicate glass.

The disclosure also provides use of the aforementioned pharmaceutical composition or the lyophilized formulation or the reconstituted solution or the article of manufacture in preparing a medicament for treating or preventing tumors.

The present disclosure also provides a method for treating a disease comprising providing the aforementioned pharmaceutical composition or lyophilized formulation or reconstituted solution or article of manufacture.

The present disclosure also provides the aforementioned pharmaceutical composition, or lyophilized formulation, or reconstituted solution, or article of manufacture, as a medicament, preferably for treating or preventing a tumor disease.

In an alternative embodiment, the disease or tumor is cancer associated with HER2, HER3, B7H3 or EGFR expression.

In an alternative embodiment, the cancer is selected from the group consisting of breast cancer, ovarian cancer, cervical cancer, uterine cancer, prostate cancer, kidney cancer, urinary tract cancer, bladder cancer, liver cancer, stomach cancer, endometrial cancer, salivary gland carcinoma, esophageal cancer, melanoma, neuroglioma, neuroblastoma, sarcoma, lung cancer, colon cancer, rectal cancer, colorectal cancer, leukemia, bone cancer, skin cancer, thyroid cancer, pancreatic cancer and lymphoma.

As is well known to those skilled in the art, one, some or all of the features of the various embodiments described in the present disclosure may be further combined to form other embodiments of the present disclosure. The above

embodiments of the present disclosure and other embodiments obtained by combination are further illustrated through the following detailed description.

The present disclosure provides a pharmaceutical composition that favors production and administration and is stable in properties. In particular, the pharmaceutical composition described in the present disclosure comprise an antibody drug conjugate and a buffer.

**Terms**

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure belongs. The application PCT/CN2019/107873 (WO2020/063676) is incorporated herein by reference in its entirety.

"Antibody drug conjugate (ADC)" is formed by connecting an antibody or an antibody fragment with cytotoxin with biological activity or a small-molecule drug with cell killing activity by a stable chemical linker compound, in which case the binding specificity of the antibody to tumor cell-specific or highly expressed antigens and the high-efficiency of the cytotoxin are fully exploited, and toxic side effects on normal cells are avoided. The antibody drug conjugate can bind to tumor cells precisely and has a reduced effect on normal cells compared to conventional chemotherapeutic drugs in the past.

"Buffer" refers to a buffer that resists changes in pH by the action of its acid-base conjugate components. Examples of buffers that control the pH in an appropriate range include acetate, succinate, gluconate, histidine salt, oxalate, lactate, phosphate, citrate, tartrate, fumarate, glycylglycine and other organic acid buffers.

"Histidine salt buffer" is a buffer comprising histidine ions. Examples of histidine salt buffers include histidine-hydrochloride buffer, histidine-acetate buffer, histidine-phosphate buffer, histidine-sulfate buffer, and the like, and the histidine-acetate buffer is preferred. The histidine-acetate buffer is prepared from histidine and acetic acid, and the histidine hydrochloride buffer is prepared from histidine and hydrochloric acid.

"Citrate buffer" is a buffer comprising citrate ions. Examples of citrate buffers include citric acid-sodium citrate, citric acid-potassium citrate, citric acid-calcium citrate, citric acid-magnesium citrate, and the like. The preferred citrate buffer is citric acid-sodium citrate.

"Succinate buffer" is a buffer comprising succinate ions. Examples of succinate buffers include succinic acid-sodium succinate, succinic acid-potassium succinate, succinic acid-calcium succinate, and the like. The preferred succinate buffer is succinic acid-sodium succinate. Illustratively, the succinic acid-sodium succinate may be prepared from

succinic acid and sodium hydroxide, or from succinic acid and sodium succinate. "Phosphate buffer" is a buffer comprising phosphate ions. Examples of phosphate buffers include disodium hydrogen phosphate-sodium dihydrogen phosphate, disodium hydrogen phosphate-potassium dihydrogen phosphate, disodium hydrogen phosphate-citric acid, and the like. The preferred phosphate buffer is disodium hydrogen phosphate-sodium dihydrogen phosphate.

"Acetate buffer" is a buffer comprising acetate ions. Examples of acetate buffers include acetic acid-sodium acetate, acetic acid histidine salt, acetic acid-potassium acetate, acetic acid-calcium acetate, acetic acid-magnesium acetate, and the like. The preferred acetate buffer is acetic acid-sodium acetate.

"Pharmaceutical composition" refers to a mixture containing one or more of the antibody drug conjugates described herein or physiologically/pharmaceutically acceptable salts or prodrugs thereof, and other chemical components, and the other components are, for example, physiologically/pharmaceutically acceptable carriers and excipients. The purpose of the pharmaceutical composition is to maintain the stability of the active ingredient of the antibody and promote the administration to an organism, which facilitates the absorption of the active ingredient, thereby exerting biological activity.

As used herein, "pharmaceutical composition" and "formulation" are not mutually exclusive.

The pharmaceutical composition described in the present disclosure is in solution form, and unless otherwise specified, the solvent is water.

"Lyophilized formulation" refers to a formulation or a pharmaceutical composition obtained by vacuum lyophilization of a pharmaceutical composition or a formulation in liquid or solution form.

The terms "about" and "approximately" as used herein mean that a numerical value is within an acceptable error range for the particular value determined by one of ordinary skill in the art, and the numerical value depends in part on how the value is measured or determined (i.e., the limits of the measurement system). For example, "about" can mean within 1 or more than 1 standard deviation per the practice in the art. Or, "about" or "substantially comprising" may mean a range of up to 20%. Furthermore, particularly for biological systems or processes, the term can mean up to an order of magnitude or up to 5-fold of a numerical value. When a particular value is provided in the present application and claims, unless otherwise stated, the meaning of "about" or "substantially comprising" should be assumed to be within an acceptable error range for that particular value.

The pharmaceutical composition of the present disclosure can achieve a stable effect: a pharmaceutical composition in which the antibody drug conjugate substantially retains its physical and/or chemical stability and/or biological activity after storage; preferably, the pharmaceutical composition substantially retains its physical and chemical stability as well as its biological activity after storage. The storage period is generally selected based on a predetermined shelf life of the pharmaceutical composition. There are a variety of analytical techniques currently available for measuring protein stability, and the stability after storage for a selected period of time at a selected temperature can be measured.

A stable formulation is one in which no significant change is observed under the following conditions: stored at refrigeration temperature (2-8 °C) for at least 3 months, preferably 6 months, more preferably 1 year, and even more preferably up to 2 years. In addition, stable liquid formulations include liquid formulations that exhibit desirable features after storage at temperatures including 25 °C for periods including 1 month, 3 months and 6 months. Typical examples for stability are as follows: typically, no more than about 10%, preferably no more than about 5%, of antibody monomers aggregate or are degraded as measured by SEC-HPLC. The formulation is a pale yellow, nearly colorless and transparent liquid, or colorless, or transparent to slightly opalescent, by visual analysis. The concentration, pH and osmotic pressure of the formulation have no more than ±10% change. Typically, no more than about 10%, preferably no more than about 5%, of decrease is observed. Typically, no more than about 10%, preferably no more than about 5%, of aggregation is formed.

An antibody drug conjugate "retains its physical stability" in a pharmaceutical formulation if it shows no significant increase in aggregation, precipitation and/or denaturation upon visual examination of color and/or clarity, or as measured by UV light scattering, size exclusion chromatography (SEC) and dynamic light scattering (DLS). Changes of protein conformation can be evaluated by fluorescence spectroscopy (which determines the protein tertiary structure) and by FTIR spectroscopy (which determines the protein secondary structure).

An antibody drug conjugate "retains its chemical stability" in a pharmaceutical formulation if it shows no significant chemical change. Chemical stability can be assessed by detecting and quantifying chemically changed forms of the protein. Degradation processes that often change the chemical structure of proteins include hydrolysis or clipping (evaluated by methods such as size exclusion chromatography and CE-SDS), oxidation (evaluated by methods such as peptide mapping in conjunction with mass spectroscopy or MALDI/TOF/MS), deamidation (evaluated by methods such as ionexchange chromatography, capillary isoelectric focusing, peptide mapping, and isoaspartic acid measurement), and isomerization (evaluated by measuring the isoaspartic acid content, peptide mapping, etc.).

An antibody drug conjugate "retains its biological activity" in a pharmaceutical formulation if the biological activity of the antibody drug conjugate at a given time is within a predetermined range of the biological activity exhibited at the time the pharmaceutical formulation was prepared.

The three-letter and single-letter codes for amino acids used in the present disclosure are described as in J. Biol. Chem., 243, p3558 (1968).

"Antibody" described herein refers to an immunoglobulin, and an intact antibody is of a tetrapeptide chain structure formed by connection between two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, otherwise called isotypes of immunoglobulins, namely IgM, IgD, IgG, IgA and IgE, with their corresponding heavy chains being μ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG may be divided into IgG1, IgG2, IgG3 and IgG4. Light chains are classified into κ or λ chains by the differences in the constant regions. Each of the five classes of Ig may have a κ chain or λ chain. The antibody described in the present disclosure is preferably specific antibodies against cell surface antigens on target cells, non-limiting examples of the antibodies being one or more of an anti-HER2 (ErbB2) antibody, an anti-EGFR antibody, an anti-B7-H3 antibody, an anti-c-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCI antibody, an anti-Lewis Y antibody, an anti-VEGFR antibody, an anti-GPNMB antibody, an anti-integrin antibody, an anti-PSMA antibody, an anti-tenascin-C antibody, an anti-SLC44A4 antibody and an anti-mesothelin antibody, preferably trastuzumab (under trade name Herceptin), pertuzumab (also known as 2C4, under trade name Perjeta), nimotuzumab (under trade name Taixinsheng), enoblituzumab, emibetuzumab, inotuzumab, pinatuzumab, brentuximab, gemtuzumab, bivatuzumab, lorvotuzumab, cBR96 and glematumamab.

In the heavy and light chains of the antibody, the sequences of about 110 amino acids near the N-terminus vary considerably and thus are referred to as variable regions (Fv regions); the remaining amino acid sequences near the C-terminus are relatively stable and thus are referred to as constant regions. The variable regions comprise 3 hypervariable regions (HVRs) and 4 framework regions (FRs) with relatively conservative sequences. The 3 hypervariable regions determine the specificity of the antibody and thus are also known as complementarity determining regions (CDRs). Each light chain variable region (LCVR) or heavy chain variable region (HCVR) consists of 3 CDRs and 4 FRs

arranged from the amino-terminus to the carboxy-terminus in the following order: FR1, CDR1, FR2, CDR2, FR3, CDR3 and FR4. The 3 CDRs of the light chain refer to LCDR1, LCDR2 and LCDR3, and the 3 CDRs of the heavy chain refer to HCDR1, HCDR2 and HCDR3. CDR amino acid residues of the LCVR and HCVR regions of the antibodies or antigen-binding fragments described in the present disclosure correspond with known Kabat numbering scheme (LCDRs 1-3, HCDRs 1-3) in terms of number and positions. In the present disclosure, the antibody light chain of the present disclosure may further comprise a light chain constant region comprising a human or murine $\kappa$ and $\lambda$ chains or variants thereof.

In the present disclosure, the antibody heavy chain of the present disclosure may further comprise a heavy chain constant region comprising human or murine IgG1, IgG2, IgG3 and IgG4 or variants thereof.

The antibody of the present disclosure includes a murine antibody, a chimeric antibody and a humanized antibody, and preferably a humanized antibody.

The term "murine antibody" used herein refers to an antibody prepared from mice according to the knowledge and skill in the art. During the preparation, a test subject is injected with a specific antigen, and then hybridomas expressing antibodies with desired sequence or functional properties are isolated.

The term "chimeric antibody" refers to an antibody obtained by fusing a variable region of a murine antibody and a constant region of a human antibody, which can reduce an immune response induced by the murine antibody. The chimeric antibody is established by firstly establishing hybridoma secreting murine specific monoclonal antibody, then cloning a variable region gene from the mouse hybridoma cells, cloning a constant region gene of human antibody as required, linking the mouse variable region gene and the human constant region gene into a chimeric gene, inserting the chimeric gene into a human vector, and finally expressing chimeric antibody molecules in a eukaryotic industrial system or prokaryotic industrial system.

The term "humanized antibody", also known as a CDR-grafted antibody, refers to an antibody produced by grafting murine CDR sequences into a human antibody variable region framework, i.e., a different type of human species antibody framework sequence. Such antibody can overcome the strong heterogeneous reaction induced by the chimeric antibody because of carrying a large amount of mouse protein components. Such framework sequences can be obtained from public DNA databases or published references that include germline antibody gene sequences. For example, germline DNA sequences of genes of the human heavy and light chain variable regions can be found in the "VBase" human species sequence database, as well as in Kabat, E. A. et al., 1991 Sequences of Proteins of Immunological Interest, 5th edition. To avoid the decrease in activity caused by the decrease in immunogenicity, the FR sequence in human antibody variable region can be subjected to minimum reverse mutation or back mutation to maintain activity. The humanized antibody of the present disclosure also include humanized antibodies which were further subjected to CDR affinity maturation by phage display.

The term "naked antibody" refers to an antibody that is not conjugated to a heterologous module (e.g., a cytotoxic module) or a radioactive label.

"Antigen-binding fragment of an antibody" described herein may refer to an Fab fragment, an Fab' fragment, an $F(ab')_2$ fragment, or an scFv fragment, an Fv fragment that binds to an antigen, which has antigen-binding activity. The Fv fragment comprises the heavy chain variable region and the light chain variable region of the antibody, but no the constant region, and has the smallest antibody fragment of the entire antigen-binding sites. Generally, the Fv antibody also comprises a polypeptide linker between the VH and VL domains, and is capable of forming the structure required for antigen binding. Two antibody variable regions can also be linked into a single polypeptide chain using different linkers, known as single chain antibody or single chain fv (sFv).

The term "antigen-binding site" disclosed herein refers to a continuous or discontinuous three-dimensional spatial site on an antigen that is recognized by an antibody or an antigen-binding fragment of the present disclosure.

"ADCC", i.e., antibody-dependent cell-mediated cytotoxicity, described herein means that the Fc receptor-expressing cells directly kill antibody-coated target cells by recognition of the Fc segment of the antibody. The ADCC effector function of the antibody may be reduced or eliminated by modification of the Fc segment of the IgG. The modification refers to a mutation in the heavy chain constant region of the antibody, such as a mutation selected from the group consisting of N297A, L234A and L235A of IgG1; IgG2/4 chimera, and F234A/L235A of IgG4.

The "mutations" in the mutant sequences described herein include, but are not limited to, "back mutation", "conservative modification" or "conservative replacement or substitution". The "conservative modification" or "conservative replacement or substitution" described herein refers to replacement of amino acids in a protein with other amino acids having similar characteristics (e.g., charge, side-chain size, hydrophobicity/hydrophilicity, or backbone conformation and rigidity), so that changes can be frequently made without changing the biological activity of the protein. Those skilled in the art know that, generally speaking, a single amino acid replacement in a non-essential region of a polypeptide does not substantially change the biological activity (see, e.g., Watson et al. (1987) Molecular Biology of the Gene, The Benjamin/Cummings Pub. Co., p224, (4th edition)). In addition, the replacement of amino acids with similar structure or function is unlikely to disrupt the biological activity.

"Mutant sequence" described herein refers to a nucleotide sequence and/or amino acid sequence having a different degree of percent sequence identity to the nucleotide sequence and/or amino acid sequence of the present

disclosure, when mutational modifications such as replacements, insertions or deletions are appropriately made to the nucleotide sequence and/or amino acid sequence of the present disclosure. The sequence identity described herein may be at least 85%, 90% or 95%, and preferably at least 95%. Non-limiting examples include 85%, 86%, 87%, 88%, 89%, 90%, 91%, 92%, 93%, 94%, 95%, 96%, 97%, 98%, 99% and 100%. Sequence comparison and percent identity determination between two sequences can be performed by the default settings for the BLASTN/BLASTP algorithm available on the website National Center For Biotechnology Institute.

The term "linker unit" or "linker fragment" refers to a chemical structure fragment or bond, which is linked to an antibody or antigen-binding fragment thereof at one end and to a drug at the other end, and also may be linked to an antibody or drug after being linked to another linker. In preferred embodiments of the present disclosure, they are indicated as L and $L^1$ through $L^4$, wherein the $L^1$ end is linked to an antibody, and the $L^4$ end is linked to a structure unit Y and then to a compound or toxin.

The linker includes stretcher units, spacer units and amino acid units, and may be synthesized using methods known in the art, such as those described in US2005-0238649A1. The linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research 52: 127-131(1992); U.S. Patent No. 5,208,020). The engineered antibody or antigen-binding fragment of the present disclosure can be prepared and purified using conventional methods. For example, cDNA sequences encoding the heavy and light chains can be cloned and recombined into a GS expression vector. Recombinant immunoglobulin expression vectors can be stably transfected into CHO cells. As a more recommended prior art, mammalian expression systems may result in glycosylation of antibodies, particularly at the highly conserved N-terminal site of the Fc region. Positive clones are expanded in a serum-free medium of a bioreactor to produce antibodies. The culture with the secreted antibody can be purified using conventional techniques. For example, purification is carried out on an A or G Sepharose FF column containing an adjusted buffer. Non-specifically bound fractions are washed away. The bound antibody is eluted using pH gradient method, and the antibody fragments are detected by SDS-PAGE and collected. The antibody can be filtered and concentrated using conventional methods. Soluble mixtures and polymers can also be removed using conventional methods, such as molecular sieves and ion exchange. The resulting product needs to be immediately frozen, e.g., at -70 °C, or lyophilized.

The term "alkyl" refers to a saturated aliphatic hydrocarbon group that is a linear or branched group containing 1 to 20 carbon atoms, preferably alkyl containing 1 to 12 carbon atoms, more preferably alkyl containing 1 to 10 carbon atoms, and most preferably alkyl containing 1 to 6 carbon atoms. Non-limiting examples of alkyl include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, *n*-decyl, 3,3-diethylhexyl, 2,2-diethylhexyl, various branched isomers thereof, etc. More preferred is a lower alkyl having 1 to 6 carbon atoms, and non-limiting examples include methyl, ethyl, *n*-propyl, isopropyl, *n*-butyl, isobutyl, *tert*-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl may be substituted or unsubstituted. When substituted, the substituent may be substituted at any available connection site, wherein the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "heteroalkyl" refers to alkyl containing one or more heteroatoms selected from the group consisting of N, O and S, wherein the alkyl is as defined above.

The term "alkylene" refers to a saturated linear or branched aliphatic hydrocarbon group having residues derived from the parent alkane by removal of two hydrogen atoms from the same carbon atom or two different carbon atoms. It is a linear or branched group containing 1 to 20 carbon atoms, preferably alkylene containing 1 to 12 carbon atoms, more preferably alkylene containing 1 to 6 carbon atoms. Non-limiting examples of alkylene include, but are not limited to, methylene($-CH_2-$), 1,1-ethylidene($-CH(CH_3)-$), 1,2-ethylidene($-CH_2CH_2$)-, 1,1-propylidene($-CH(CH_2CH_3)-$), 1,2-propylidene($-CH_2CH(CH_3)-$), 1,3-propylidene($-CH_2CH_2CH_2-$), 1,4-butylidene($-CH_2CH_2CH_2CH_2-$), 1,5-butylidene($-CH_2CH_2CH_2CH_2CH_2-$), etc. The alkylene may be substituted or unsubstituted. When substituted, the substituent may be substituted at any available connection site with one or more substituents preferably independently optionally selected from the group consisting of alkyl, alkenyl, alkynyl, alkoxy, alkylthio, alkylamino, halogen, mercapto, hydroxy, nitro, cyano, cycloalkyl, heterocyclyl, aryl, heteroaryl, cycloalkoxy, heterocycloalkoxy, cycloalkylthio, heterocycloalkylthio

and oxo.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropyloxy, cyclobutoxy, cyclopentyloxy and cyclohexyloxy. The alkoxy may be optionally substituted or unsubstituted, and when it is substituted, the substituent is preferably one or more of the following groups independently selected from the group consisting of alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "cycloalkyl" refers to a saturated or partially unsaturated monocyclic or polycyclic hydrocarbon substituent. The cycloalkyl ring contains 3 to 20 carbon atoms, preferably 3 to 12 carbon atoms, more preferably 3 to 10 carbon atoms, and most preferably 3 to 7 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like. Polycyclic cycloalkyl includes spiro cycloalkyl, fused cycloalkyl, and bridged cycloalkyl.

The term "heterocyclyl" refers to a saturated or partially unsaturated monocyclic or polycyclic hydrocarbon substituent containing 3 to 20 ring atoms, wherein one or more of the ring atoms are heteroatoms selected from the group consisting of nitrogen, oxygen and $S(O)_m$ (where m is an integer from 0 to 2), excluding a cyclic portion of -O-O-, -O-S- or -S-S-, and the remaining ring atoms are carbon atoms. Heterocycloalkyl preferably contains 3 to 12 ring atoms, of which 1 to 4 are heteroatoms; more preferably, a cycloalkyl ring contains 3 to 10 ring atoms. Non-limiting examples of monocyclic heterocyclyl include pyrrolidinyl, piperidinyl, piperazinyl, morpholinyl, thiomorpholinyl, homopiperazinyl, etc. Non-limiting examples of polycyclic heterocyclyl include spiro heterocyclyl, fused heterocyclyl, and bridged heterocyclyl.

The term "spiro heterocyclyl" refers to a 5- to 20-membered polycyclic heterocyclyl group in which monocyclic rings share one atom (referred to as the spiro atom), wherein one or more ring atoms are heteroatoms selected from the group consisting of nitrogen, oxygen and $S(O)_m$ (where m is an integer from 0 to 2), and the remaining ring atoms are carbon atoms. These rings may contain one or more double bonds, but none of them has a fully conjugated π-electron system. Preferably, the spiro heterocyclyl is 6- to 14-membered, and more preferably 7- to 10-membered. According to the number of spiro atoms shared among the rings, the spiro heterocyclyl may be monospiro heterocyclyl, bispiro heterocyclyl or polyspiro heterocyclyl, preferably monospiro heterocyclyl and bispiro heterocyclyl, and more preferably 4-membered/4-membered, 4-membered/5-membered, 4-membered/6-membered, 5-membered/5-membered or 5-membered/6-membered monospiro heterocyclyl. Non-limiting examples of spiro heterocyclyl include:

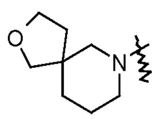 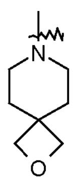 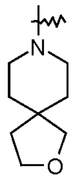 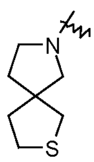 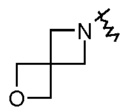 and 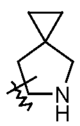 .

The term "fused heterocyclyl" refers to a 5- to 20-membered polycyclic heterocyclyl group in which each ring shares a pair of adjacent atoms with the other rings in the system, wherein one or more of the rings may contain one or more double bonds, but none of them has a fully conjugated π-electron system, wherein one or more of the ring atoms are heteroatoms selected from the group consisting of nitrogen, oxygen and $S(O)_m$ (where m is an integer from 0 to 2), and the remaining ring atoms are carbon atoms. Preferably, the fused heterocyclyl is 6- to 14-membered, and more preferably 7- to 10-membered. According to the number of the formed rings, the fused heterocyclyl may be bicyclic, tricyclic, tetracyclic or polycyclic fused heterocyclyl, preferably bicyclic or tricyclic fused heterocyclyl, and more preferably 5-membered/5-membered or 5-membered/6-membered bicyclic fused heterocyclyl. Non-limiting examples of fused heterocyclyl include:

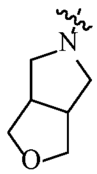 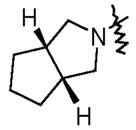 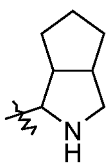 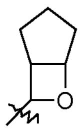 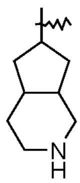 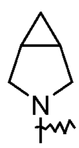 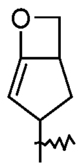 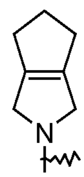

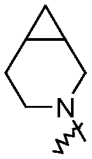 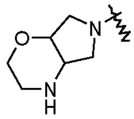 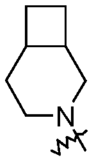 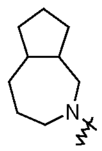 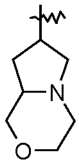 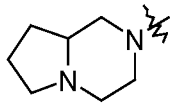 and 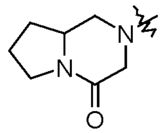 .

The term "bridged heterocyclyl" refers to a 5- to 14-membered polycyclic heterocyclyl group in which any two rings share two atoms that are not directly linked to each other, wherein these rings may contain one or more double bonds, but none of them has a fully conjugated π-electron system, wherein one or more of the ring atoms are heteroatoms selected from the group consisting of nitrogen, oxygen and $S(O)_m$ (where m is an integer from 0 to 2), and the remaining ring atoms are carbon atoms. Preferably, the bridged heterocyclyl is 6- to 14-membered, and more preferably 7- to 10-membered. According to the number of the formed rings, the bridged heterocyclyl may be bicyclic, tricyclic, tetracyclic or polycyclic bridged heterocyclyl, preferably bicyclic, tricyclic or tetracyclic bridged heterocyclyl, and more preferably bicyclic or tricyclic bridged heterocyclyl. Non-limiting examples of bridged heterocyclyl include:

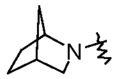 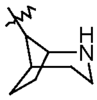 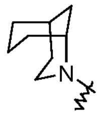 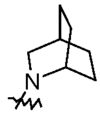

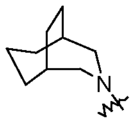 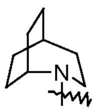 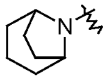 and 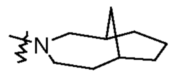 .

The heterocyclyl ring may be fused to an aryl, heteroaryl or cycloalkyl ring, wherein the ring linked to the parent structure is heterocyclyl. Non-limiting examples of the heterocyclyl ring include:

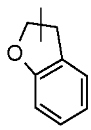 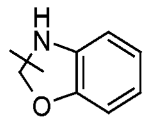 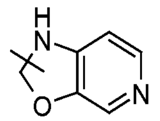 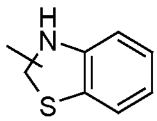 ,

etc.

The heterocyclyl may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio, heterocycloalkylthio and oxo.

The term "aryl" refers to a 6- to 14-membered, preferably 6- to 10-membered, carbon monocyclic or fused polycyclic (i.e., rings sharing a pair of adjacent carbon atoms) group having a conjugated π-electron system, such as phenyl and naphthyl, preferably phenyl. The aryl ring may be fused to a heteroaryl, heterocyclyl or cycloalkyl ring, wherein the ring linked to the parent structure is an aryl ring. Non-limiting examples of the aryl ring include:

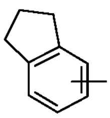 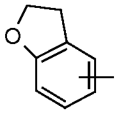 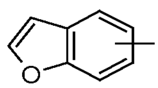 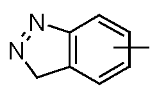 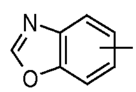 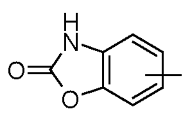

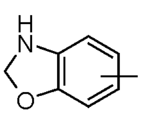 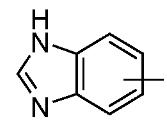 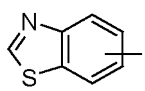 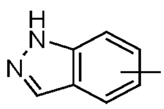 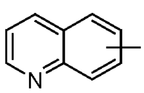 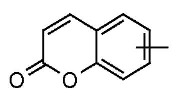

and

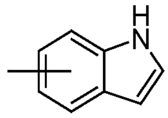.

The aryl may be substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "heteroaryl" refers to a heteroaromatic system containing 1 to 4 heteroatoms and 5 to 14 ring atoms, wherein the heteroatoms are selected from the group consisting of oxygen, sulfur and nitrogen. The heteroaryl is preferably 5- to 10-membered, more preferably 5- or 6-membered, such as furanyl, thienyl, pyridinyl, pyrrolyl, N-alkylpyrrolyl, pyrimidinyl, pyrazinyl, imidazolyl and tetrazolyl. The heteroaryl ring may be fused to an aryl, heterocyclyl or cycloalkyl ring, wherein the ring linked to the parent structure is a heteroaryl ring. Non-limiting examples of the heteroaryl ring include:

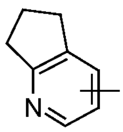 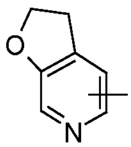 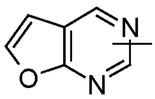 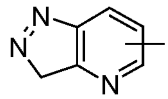 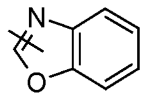 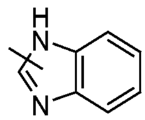

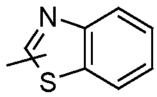 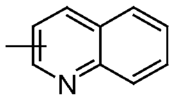 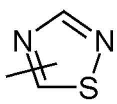 and 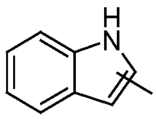.

The heteroaryl may be optionally substituted or unsubstituted, and when it is substituted, the substituent is preferably one or more of the following groups independently selected from the group consisting of: alkyl, alkenyl, alkynyl, alkoxy, alkylthio, alkylamino, halogen, mercapto, hydroxy, nitro, cyano, cycloalkyl, heterocycloalkyl, aryl, heteroaryl, cycloalkoxy, heterocycloalkoxy, cycloalkylthio and heterocycloalkylthio.

The term "amino protecting group" refers to a group that can be easily removed and is intended to protect an amino group from being changed when a reaction is conducted elsewhere in the molecule. Non-limiting examples of the amino protecting group include 9-fluorenylmethoxycarbonyl, *tert*-butoxycarbonyl, acetyl, benzyl, allyl, *p*-methoxybenzyl, etc. These groups may be optionally substituted with 1 to 3 substituents selected from the group consisting of halogen, alkoxy and nitro. The amino protecting group is preferably 9-fluorenylmethoxycarbonyl.

The term "cycloalkylalkyl" refers to alkyl substituted with one or more cycloalkyl groups, preferably one cycloalkyl group, wherein the alkyl is as defined above, and the cycloalkyl is as defined above.

The term "haloalkyl" refers to alkyl substituted with one or more halogens, wherein the alkyl is as defined above.

The term "deuterated alkyl" refers to alkyl substituted with one or more deuterium atoms, wherein the alkyl is as defined above.

The term "hydroxy" refers to -OH group.

The term "halogen" refers to fluorine, chlorine, bromine or iodine.

The term "amino" refers to $-NH_2$.

The term "nitro" refers to $-NO_2$.

The term "optional" or "optionally" means that the event or circumstance subsequently described may, but not necessarily, occur, and that the description includes instances where the event or circumstance occurs or does not occur. For example, "optionally comprising 1-3 antibody heavy chain variable regions" means that the antibody heavy chain variable region of a particular sequence may, but not necessarily, be present.

The term "substituted" means that one or more, preferably up to 5, more preferably 1 to 3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue efforts. For example, it may be unstable when amino or hydroxy having a free hydrogen is bound to a carbon atom having an unsaturated (e.g., olefinic) bond.

The term "drug loading" refers to the mean number of cytotoxic drugs loaded on each antibody or an antigen-binding fragment thereof in antibody drug conjugate molecules, and it may also be expressed in terms of a ratio of the number of drugs to the number of antibodies. The drug loading may range from 0-12, preferably 1-10, more preferably

3-8, and most preferably 5.3-6.1 cytotoxic drugs linked to each antibody or an antigen-binding fragment (Pc) thereof. In embodiments of the present disclosure, the drug loading is represented by n, which may also be referred to as a DAR (drug-antibody ratio) value, and, illustratively, may be a mean of 1, 2, 3, 4, 5, 6, 7, 8, 9 and 10. The mean number of drugs per ADC molecule upon coupling reactions can be characterized by conventional methods such as ultraviolet-visible spectroscopy (UV-Vis), hydrophobic interaction chromatography (HIC) mass spectrometry, ELISA assays and HPLC.

The loading of the cytotoxic drug can be controlled using the following non-limiting methods, including:

(1) controlling a molar ratio of a linking reagent to a monoclonal antibody,
(2) controlling reaction time and temperature, and
(3) selecting different reagents.

For preparation of conventional pharmaceutical compositions, reference is made to *Chinese Pharmacopoeia.*

The term "carrier" for the drug of the present disclosure refers to a system that can alter the manner in which the drug gets into a human body and the distribution of the drug in the human body, control the release rate of the drug, and deliver the drug to a targeted organ. The drug carrier release and targeted system can reduce drug degradation and loss, reduce side effects and improve bioavailability. For example, polymeric surfactants that can be used as carriers can self-assemble due to their unique amphiphilic structures to form various forms of aggregates, such as micelles, microemulsions, gels, liquid crystals and vesicles, as preferred examples. The aggregates have the capability of encapsulating drug molecules and have good permeability for membranes, and therefore can be used as excellent drug carriers.

"Giving" and "treating", when applied to animals, humans, experimental subjects, cells, tissues, organs or biological fluids, refer to contact of an exogenous drug, a therapeutic agent, a diagnostic agent or a composition with the animals, humans, subjects, cells, tissues, organs or biological fluids. "Giving" and "treating" can refer to, for example, therapeutic, pharmacokinetic, diagnostic, research and experimental methods. The treatment of cells comprises contacting a reagent with the cells and contacting the reagent with a fluid. "Giving" and "treating" also refer to treating, e.g., cells by reagents, diagnosis, binding compositions or by another cell *in vitro* and *ex vivo.* "Treating", when applied to human, veterinary or research subjects, refers to therapeutic treatment, preventive or prophylactic measures, and research and diagnostic applications.

"Treating" or "treatment" refers to administering a therapeutic agent, such as a composition comprising any one of the conjugation compounds of the present disclosure, either internally or externally to a patient with one or more symptoms of a disease on which the therapeutic agent is known to have a therapeutic effect. Typically, the therapeutic agent is administered in an amount effective to alleviate one or more symptoms of the disease in the patient or population being treated to induce regression of such symptoms or to inhibit the development of such symptoms to any clinically measurable degree. The amount of therapeutic agent effective to alleviate the symptoms of any particular disease (also referred to as a "therapeutically effective amount") may vary depending on a variety of factors, such as the disease state, age, and weight of the patient, and the ability of the drug to produce a desired therapeutic effect in the patient. Whether a symptom of a disease has been alleviated can be evaluated by any clinical testing methods commonly used by doctors or other health care professionals to evaluate the severity or progression of the symptom. Although the embodiments of the present disclosure (for example, treatment methods or products) may not be effective in alleviating the symptoms of each disease of interest, they shall reduce the symptoms of a disease of interest in a statistically significant number of patients, as determined according to any statistical testing methods known in the art, such as Student t-test, chi-square test, Mann and Whitney's U test, Kruskal-Wallis test (H test), Jonckheere-Terpstra test and Wilcoxon test.

"Effective amount" comprises an amount sufficient to ameliorate or prevent a symptom or condition of a medical disease. An effective amount also refers to an amount sufficient to allow or facilitate diagnosis. The effective amount for a particular patient or veterinary subject may vary depending on the factors such as the condition to be treated, the general health of the patient, the method and route and dosage of administration, and the severity of side effects. An effective amount can be the maximum dose or administration regimen to avoid significant side effects or toxic effects.

"Exchange" refers to the exchange of a solvent system that solubilizes an antibody protein. For example, a high-salt or hypertonic solvent system comprising the antibody protein is exchanged, by physical operations, with a buffer system of a stable formulation, such that the antibody protein is present in the stable formulation. The physical operations include, but are not limited to, ultrafiltration, dialysis or reconstitution following centrifugation.

## BRIEF DESCRIPTION OF THE DRAWINGS

FIG. 1A shows the results of plasma stability test for ADC-19 of the present disclosure.
FIG. 1B shows the results of plasma stability test for ADC-18 of the present disclosure.

FIG. 1C shows the results of plasma stability test for ADC-20 of the present disclosure.

FIG. 2 shows the evaluation of the efficacy of ADC-21 and ADC-24 of the present disclosure in JIMT-1 tumor-bearing mice.

FIG. 3 shows the evaluation of the therapeutic effect of ADCs on human breast cancer cell SK-BR-3 xenograft tumor nude mice.

FIG. 4 shows the results of plasma stability test for ADC-25 of the present disclosure.

FIG. 5 shows the efficacy of the ADCs of the present disclosure against human brain astrocytoma U87MG nude mouse xenograft tumors.

FIG. 6 shows the efficacy of the ADCs of the present disclosure against pleural effusion metastatic human pharyngeal cancer cell Detroit 562 nude mouse xenograft tumors.

FIG. 7 shows the efficacy of the ADCs of the present disclosure against human glioblastoma U87MG nude mouse xenograft tumors.

FIG. 8 shows fitted tendency graphs for a formula screening experiment, wherein the PS80 is in a unit of $10^{-4}$ g/mL, and the ADC-32 protein concentration (based on naked antibody) is in a unit of mg/mL.

## DETAILED DESCRIPTION

The present disclosure is further described below with reference to examples, which, however, are not intended to limit the scope of the present disclosure. The experimental methods in the examples of the present disclosure in which specific conditions are not specified are generally performed under conventional conditions, for example, by referring to Antibodies: A Laboratory Manual and Molecular Cloning: A Laboratory Manual by Cold Spring Harbor Laboratory, or under conditions recommended by the manufacturer of the raw material or the goods. Reagents without specific origins indicated are commercially available conventional reagents.

## I. Antibody drug conjugates

The structures of the compounds were determined by nuclear magnetic resonance (NMR) or mass spectrometry (MS). NMR spectra were measured using a Bruker AVANCE-400 nuclear magnetic resonance instrument, with deuterated dimethyl sulfoxide (*DMSO-d6*), deuterated chloroform ($CDCl_3$) and deuterated methanol ($CD_3OD$) as determination solvents and tetramethylsilane (TMS) as internal standard. Chemical shifts were given in unit of $10^{-6}$ (ppm).

MS analysis was performed using a FINNIGAN LCQAd (ESI) mass spectrometer (manufacturer: Thermo, model: Finnigan LCQ advantage MAX).

UPLC analysis was performed using a Waters Acquity UPLC SQD liquid chromatography-mass spectrometry system.

HPLC analysis was performed using an Agilent 1200DAD high pressure liquid chromatograph (Sunfire C18 150 × 4.6 mm chromatography column) and a Waters 2695-2996 high pressure liquid chromatograph (Gimini C18 150 × 4.6 mm chromatography column).

UV-HPLC analysis was performed using a Thermo nanodrop2000 ultraviolet spectrophotometer.

Proliferation inhibition rates and $IC_{50}$ values were determined using a PHERA starFS microplate reader (BMG, Germany).

Yantai Huanghai HSGF254 or Qingdao GF254 silica gel plates of specifications 0.15 mm to 0.2 mm were adopted for thin layer chromatography (TLC) analysis and 0.4 mm to 0.5 mm for TLC separation and purification.

Yantai Huanghai silica gel of 200-300 mesh is generally used as a carrier in column chromatography.

Known starting materials of the present disclosure may be synthesized using or according to methods known in the art, or may be purchased from ABCR GmbH & Co.KG, Acros Organnics, Aldrich Chemical Company, Accela ChemBio Inc, Chembee Chemicals, etc. In the Examples, the reactions were performed in an argon atmosphere or a nitrogen atmosphere unless otherwise stated.

The argon atmosphere or nitrogen atmosphere means that the reaction flask is linked to a balloon containing about 1 L of argon or nitrogen.

The hydrogen atmosphere means that the reaction flask is linked to a balloon containing about 1 L of hydrogen.

Parr 3916EKX hydrogenator, Qinglan QL-500 hydrogenator or HC2-SS hydrogenator was used in the pressurized hydrogenation reactions.

The hydrogenation reactions usually involve 3 cycles of vacuumization and hydrogen purge.

A CEM Discover-S 908860 microwave reactor was used in the microwave reactions.

In the Examples, the solution in the reaction refers to an aqueous solution unless otherwise stated.

In the Examples, the reaction temperature is room temperature unless otherwise stated.

The room temperature is the optimum reaction temperature, ranging from 20 °C to 30 °C. Preparation of PBS buffer at pH 6.5 in Examples: 8.5 g of $KH_2PO_4$, 8.56 g of $K_2HPO_4 \cdot 3H_2O$, 5.85 g of NaCl and 1.5 g of EDTA were added

to a flask, and the volume was brought to 2 L. The additions were all ultrasonically dissolved, and the solution was well mixed by shaking to give the desired buffer.

The eluent system for column chromatography and the developing solvent system for thin layer chromatography used for compound purification include: A: dichloromethane and isopropanol system, B: dichloromethane and methanol system, and C: petroleum ether and ethyl acetate system. The volume ratio of solvents was adjusted according to the polarity of the compound, or by adding a small amount of triethylamine and acidic or basic reagent.

Some of the compounds of the present disclosure are characterized by Q-TOF LC/MS. Q-TOF LC/MS analysis was performed using an Agilent 6530 accurate-mass quadrupole time-of-flight mass spectrometer and an Agilent 1290-Infinity ultra-high performance liquid chromatograph (Agilent Poroshell 300SB-C8 5 μm, 2.1 × 75 mm chromatography column).

**Example 1-1**

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-hydroxycyclopropane-1-carboxamide **1**

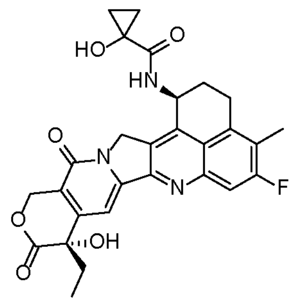

**1**

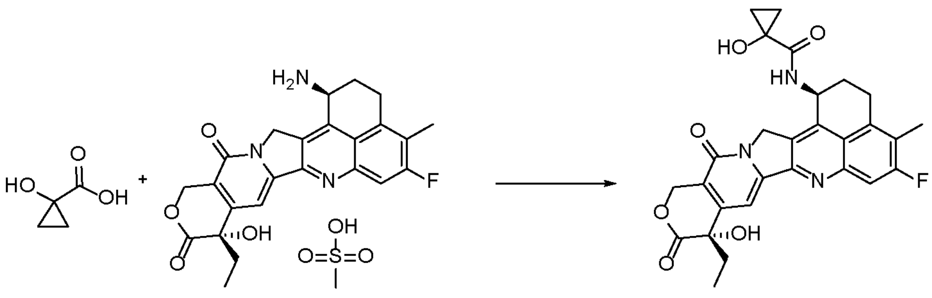

**1a** **1b** **1**

To exatecan mesylate **1b** (2.0 mg, 3.76 μmol, prepared as disclosed in Patent Application "EP0737686A1") was added 1 mL of N,N-dimethylformamide. The mixture was cooled to 0-5 °C in an ice-water bath, and a drop of triethylamine was added. The reaction mixture was stirred until it became clear. To the reaction mixture were successively added 1-hydroxycyclopropylcarboxylic acid 1a (1.4 mg, 3.7 μmol, prepared using known method "Tetrahedron Letters, 25(12), 1269-72; 1984") and 4-(4,6-dimethoxy-1,3,5-triazin-2-yl)-4-methylmorpholinium chloride (3.8 mg, 13.7 μmol). After addition, the reaction mixture was stirred at 0-5 °C for 2 h, quenched with 5 mL of water, and extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product 1 (1.6 mg, 82.1% yield).

MS m/z (ESI): 520.2 [M+1].

$^1H$ NMR (400 MHz, $CDCl_3$): δ 7.90-7.84 (m, 1H), 7.80-7.68(m, 1H), 5.80-5.70 (m, 1H), 5.62-5.54 (m, 2H), 5.44-5.32 (m, 2H), 5.28-5.10 (m, 2H), 3.40-3.15 (m, 3H), 2.44 (s, 3H), 2.23 (t, 1H), 2.06-1.75 (m, 2H), 1.68-1.56 (m, 1H), 1.22-1.18 (m, 2H), 1.04-0.98 (m, 2H), 0.89 (t, 3H).

**Example 1-2**

(*S*)-2-cyclopropyl-*N*- ((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **2-A**

(*R*)-2-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxyacetamide **2-B**

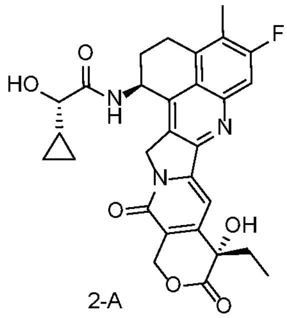
2-A

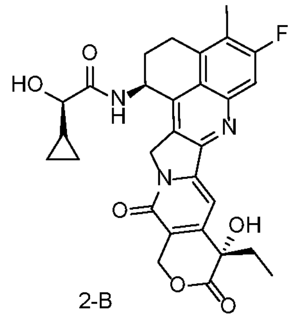
2-B

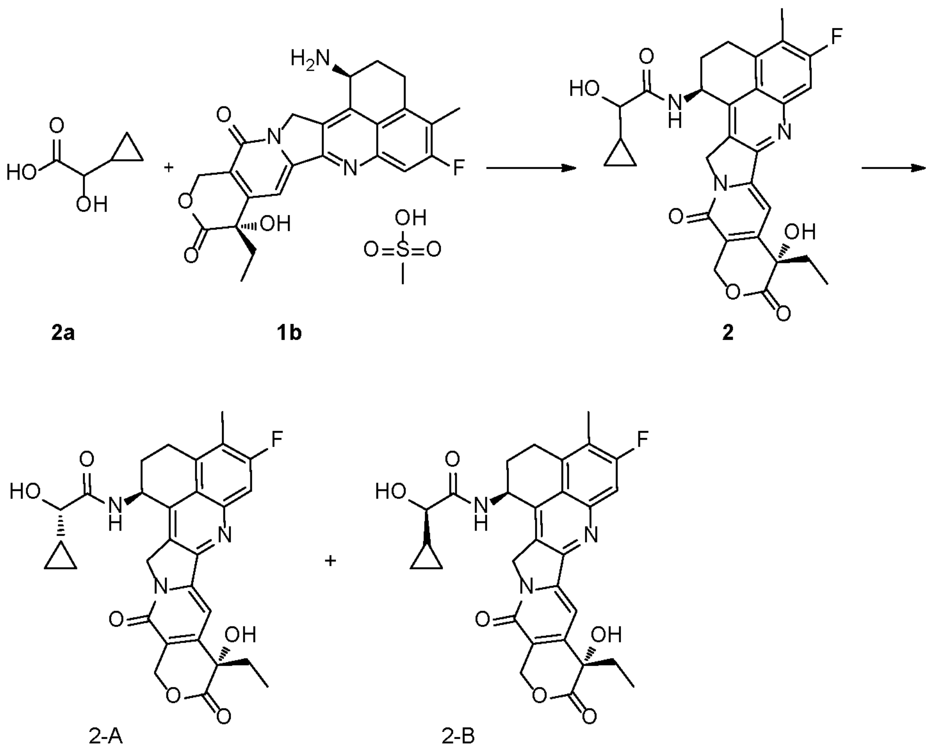
**2a** **1b** **2**

2-A 2-B

To compound **1b** (4 mg, 7.53 μmol) were added 2 mL of ethanol and 0.4 mL of *N,N*-dimethylformamide. The system was purged with argon three times, and the mixture was cooled to 0-5 °C in an ice-water bath, followed by dropwise addition of 0.3 mL of *N*-methylmorpholine. The reaction mixture was stirred until it became clear. To the reaction mixture were successively added 2-cyclopropyl-2-hydroxyacetic acid **2a** (2.3 mg, 19.8 μmol, prepared as disclosed in Patent Application "WO2013106717"), 1-hydroxybenzotriazole (3 mg, 22.4 μmol) and 1-(3-dimethylaminopropyl)-3-ethylcarbodiimide hydrochloride (4.3 mg, 22.4 μmol). After addition, the reaction mixture was stirred at 0-5 °C for 1 h. The ice-water bath was removed, and the reaction mixture was heated to 30 °C, stirred for 2 h, and concentrated under reduced pressure. The resulting crude compound **2** was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min), and the corresponding fractions were collected and concentrated under reduced pressure to give the title products (2-A: 1.5 mg, 2-B: 1.5 mg).

MS m/z (ESI): 534.0 [M+1].

Single-configuration compound 2-B (shorter retention time):
UPLC analysis: retention time: 1.06 min; purity: 88% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.37 (d, 1H), 7.76 (d, 1H), 7.30 (s, 1H), 6.51 (s, 1H), 5.58-5.56 (m, 1H), 5.48 (d, 1H), 5.41 (s, 2H), 5.32-5.29 (m, 2H), 3.60 (t, 1H), 3.19-3.13

(m, 1H), 2.38 (s, 3H), 2.20-2.14 (m, 1H), 1.98 (q, 2H), 1.87-1.83 (m, 1H), 1.50-1.40 (m, 1H), 1.34-1.28 (m, 1H), 0.86 (t, 3H), 0.50-0.39 (m, 4H).

Single-configuration compound 2-A (longer retention time):
UPLC analysis: retention time: 1.10 min; purity: 86% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.35 (d, 1H), 7.78 (d, 1H), 7.31 (s, 1H), 6.52 (s, 1H), 5.58-5.53 (m, 1H), 5.42 (s, 2H), 5.37 (d, 1H), 5.32 (t, 1H), 3.62 (t, 1H), 3.20-3.15 (m, 2H), 2.40 (s, 3H), 2.25-2.16 (m, 1H), 1.98 (q, 2H), 1.87-1.82 (m, 1H), 1.50-1.40 (m, 1H), 1.21-1.14 (m, 1H), 0.87 (t, 3H), 0.47-0.35 (m, 4H).

**Example 1-3**

(*S*)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-3,3,3-trifluoro-2-hydroxypropionamide **3-A**

(*R*)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-3,3,3-trifluoro-2-hydroxypropionamide **3-B**

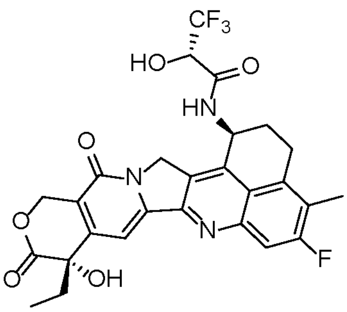

3-A

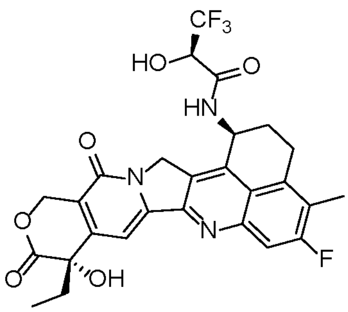

3-B

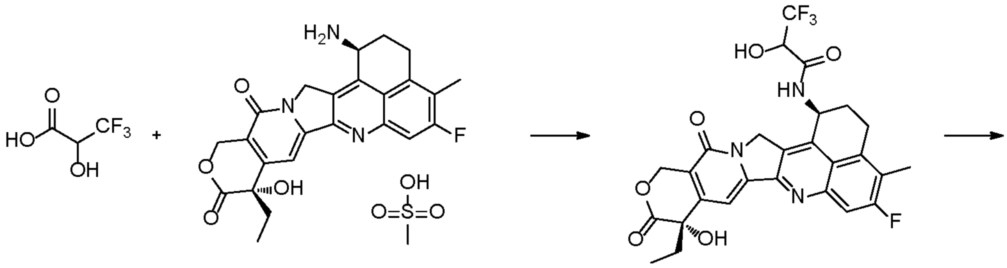

**3a** **1b** **3**

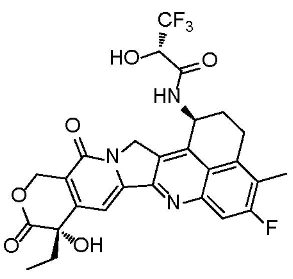

3-A

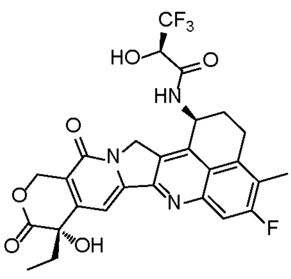

3-B

To compound **1b** (5.0 mg, 9.41 μmol) were added 2 mL of ethanol and 0.4 mL of *N,N*-dimethylformamide, and the mixture was cooled to 0-5 °C in an ice-water bath, followed by dropwise addition of 0.3 mL of N-methylmorpholine. The reaction mixture was stirred until it became clear. To the reaction mixture were successively added 3,3,3-trifluoro-2-hydroxypropionic acid **3a** (4.1 mg, 28.4 μmol, supplied by Alfa), 1-hydroxybenzotriazole (3.8 mg, 28.1 μmol) and 1-(3-dimethylaminopropyl)-3-ethylcarbodiimide hydrochloride (5.4 mg, 28.2 μmol). After addition, the reaction mixture was stirred at 0-5 °C for 10 min. The ice-water bath was removed, and the reaction mixture was heated to 30 °C, stirred for 8 h, and concentrated under reduced pressure. The resulting crude compound **3** was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min), and the corresponding fractions were collected and concentrated under reduced pressure to give the title products (1.5 mg, 1.5 mg).

MS m/z (ESI): 561.9 [M+1].

Single-configuration compound (shorter retention time):
UPLC analysis: retention time: 1.11 min; purity: 88% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.94 (d, 1H), 7.80 (d, 1H), 7.32 (s, 1H), 7.20 (d, 1H), 6.53 (s, 1H), 5.61-5.55 (m, 1H), 5.45-5.23 (m, 3H), 5.15-5.06 (m, 1H), 4.66-4.57 (m, 1H), 3.18-3.12 (m, 1H), 2.40 (s, 3H), 2.26-2.20 (m, 1H), 2.16-2.08 (m, 1H), 2.02-1.94 (m, 1H), 1.89-1.82 (m, 1H), 1.50-1.40 (m, 1H), 0.87 (t, 3H).

Single-configuration compound (longer retention time):
UPLC analysis: retention time: 1.19 min; purity: 90% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.97 (d, 1H), 7.80 (d, 1H), 7.31 (s, 1H), 7.16 (d, 1H), 6.53 (s, 1H), 5.63-5.55 (m, 1H), 5.45-5.20 (m, 3H), 5.16-5.07 (m, 1H), 4.66-4.57 (m, 1H), 3.18-3.12 (m, 1H), 2.40 (s, 3H), 2.22-2.14 (m, 1H), 2.04-1.95 (m, 2H), 1.89-1.82 (m, 1H), 1.50-1.40 (m, 1H), 0.87 (t, 3H).

## Example 1-4

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-hydroxycyclopentane-1-carboxamide **4**

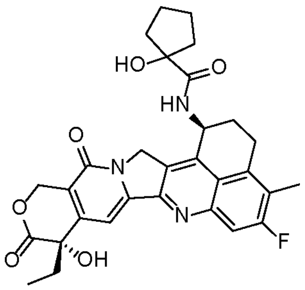

**4**

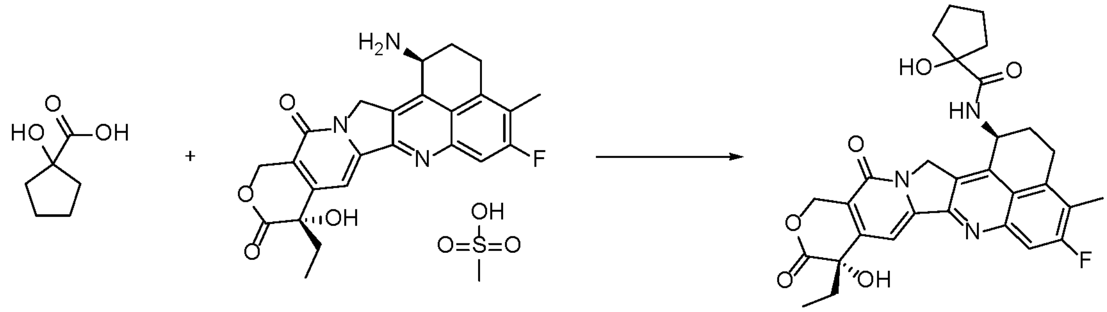

**4a** **1b** **4**

To compound **1b** (3.0 mg, 5.64 μmol) was added 1 mL of *N*,*N*-dimethylformamide. The mixture was cooled to 0-5 °C in an ice-water bath, and a drop of triethylamine was added. The reaction mixture was stirred until it became clear. To the reaction mixture were successively added 1-hydroxy-cyclopentanecarboxylic acid **4a** (2.2 mg, 16.9 μmol, prepared as disclosed in Patent Application "WO2013106717") and 4-(4,6-dimethoxy-1,3,5-triazin-2-yl)-4-methylmorpholinium chloride (4.7 mg, 16.9 μmol). After addition, the reaction mixture was stirred at 0-5 °C for 1 h, quenched with 5 mL of water, and extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **4** (2.5 mg, 80.9% yield).

MS m/z (ESI): 548.0 [M+1].

$^1$H NMR (400 MHz, $CDCl_3$): δ 7.73-7.62 (m, 2H), 5.75-5.62 (m, 1H), 5.46-5.32 (m, 2H), 5.26-5.10 (m, 1H), 3.30-3.10 (m, 1H), 2.43 (s, 3H), 2.28-2.20 (m, 2H), 2.08-1.84 (m, 8H), 1.69-1.58 (m, 2H), 1.04-1.00 (m, 2H), 0.89 (t, 3H).

**Example 1-5**

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-(hydroxymethyl)cyclopropane-1-carboxamide **5**

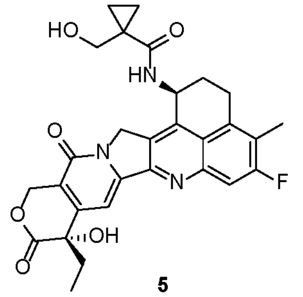

**5**

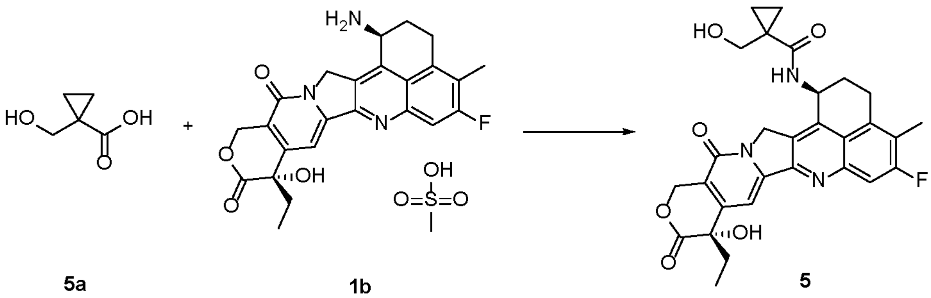

**5a** **1b** **5**

To compound **1b** (2.0 mg, 3.76 μmol) was added 1 mL of *N*,*N*-dimethylformamide. The mixture was cooled to 0-5 °C in an ice-water bath, and a drop of triethylamine was added. The reaction mixture was stirred until it became clear. To the reaction mixture were successively added 1-(hydroxymethyl)-cyclopentanecarboxylic acid **5a** (0.87 mg, 7.5 μmol, prepared as disclosed in Patent Application "WO201396771") and 4-(4,6-dimethoxy-1,3,5-triazin-2-yl)-4-methylmorpholinium chloride (2 mg, 7.24 μmol). After addition, the reaction mixture was stirred at 0-5 °C for 2 h, quenched with 5 mL of water, and extracted with ethyl acetate (8 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **5** (1.0 mg, 50% yield).

MS m/z (ESI): 533.9 [M+1].

$^1$H NMR (400 MHz, $CDCl_3$): δ 8.07 (s, 1H), 7.23-7.18 (m, 2H), 6.71-6.64 (m, 1H), 6.55-6.51 (m, 1H), 5.36-5.27 (m, 2H), 4.67-4.61 (m, 2H), 3.53-3.48 (m, 1H), 3.30-3.22 (m, 2H), 3.18-3.13 (m, 1H), 2.71-2.61 (m, 2H), 2.35-2.28 (m, 1H), 2.04-1.91 (m, 4H), 1.53-1.40 (m, 3H), 0.91-0.75 (m, 4H).

**Example 1-6**

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-(hydroxymethyl)cyclobutane-1-carboxamide **6**

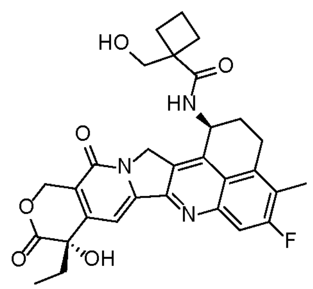

6

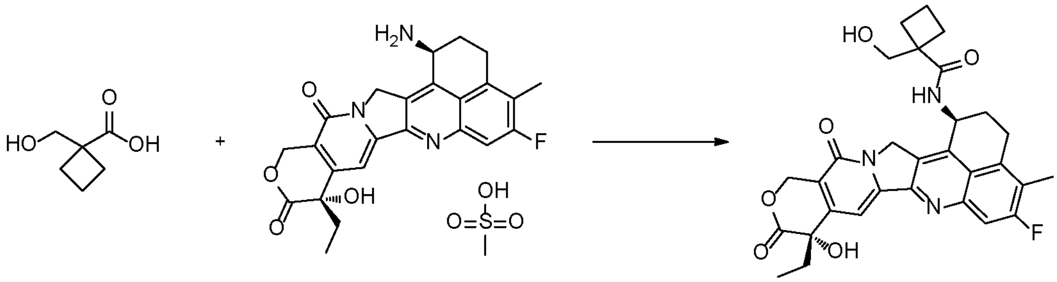

6a 1b 6

To compound **1b** (3.0 mg, 5.64 μmol) was added 1 mL of *N*,*N*-dimethylformamide. The mixture was cooled to 0-5 °C in an ice-water bath, and a drop of triethylamine was added. The reaction mixture was stirred until it became clear. To the reaction mixture were successively added 1-(hydroxymethyl)cyclobutane-1-carboxylic acid **6a** (2.2 mg, 16.9 μmol, prepared as disclosed in "Journal of the American Chemical Society, 2014, vol. 136, #22, p.8138-8142") and 4-(4,6-dimethoxy-1,3,5-triazin-2-yl)-4-methylmorpholinium chloride (4.7 mg, 16.9 μmol). After addition, the reaction mixture was stirred at 0-5 °C for 1 h, quenched with 5 mL of water, and extracted with ethyl acetate (10 mL × 3). The organic phases were combined, washed with saturated sodium chloride solution (5 mL × 2), dried over anhydrous sodium sulfate, and filtered. The filtrate was concentrated under reduced pressure, and the resulting residue was purified by thin layer chromatography with developing solvent system B to give the title product **6** (2.1 mg, 67.9% yield).

MS m/z (ESI): 548.0 [M+1].

$^1$H NMR (400 MHz, DMSO-$d_6$): δ 7.85-7.62 (m, 1H), 6.88 (br, 1H), 5.87-5.48 (m, 2H), 5.47-5.33 (m, 1H), 5.31-5.06 (m, 1H), 4.25-3.91 (m, 2H), 3.25 (br, 1H), 2.60-2.32 (m, 3H), 2.23 (t, 1H), 2.15-1.95 (m, 3H), 1.70-1.56 (m, 2H), 1.41-1.17 (m, 9H), 1.03 (s, 1H), 0.95-0.80 (m, 2H).

**Example 1-7**

*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-1-hydroxycyclobutane-1-carboxamide 7

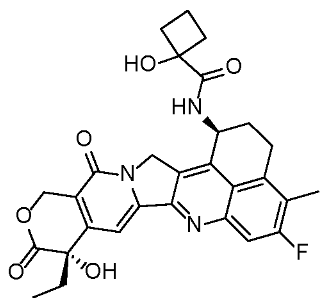

7

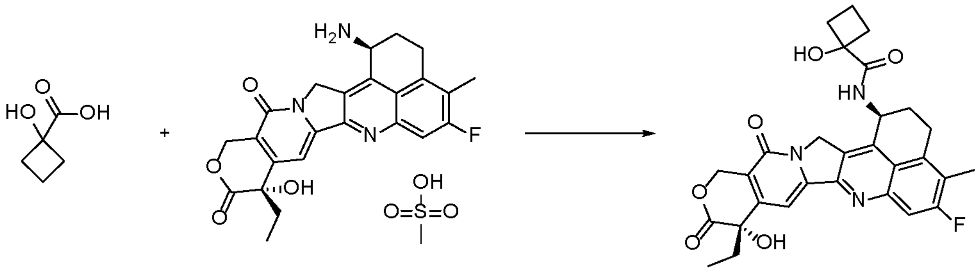

7a 1b 7

To compound **1b** (3.0 mg, 5.64 μmol) were added 2 mL of ethanol and 0.4 mL of *N,N*-dimethylformamide, and the mixture was cooled to 0-5 °C in an ice-water bath, followed by dropwise addition of 0.3 mL of N-methylmorpholine. The reaction mixture was stirred until it became clear. To the reaction mixture were successively added 1-hydroxycyclobutanecarboxylic acid **7a** (2.0 mg, 17.22 μmol, supplied by PharmaBlock), 1-hydroxybenzotriazole (2.3 mg, 17.0 μmol) and 1-(3-dimethylaminopropyl)-3-ethylcarbodiimide hydrochloride (3.2 mg, 16.7 μmol). After addition, the reaction mixture was stirred at 0-5 °C for 10 min. The ice-water bath was removed, and the reaction mixture was stirred at room temperature for 2 h, and concentrated under reduced pressure. The resulting residue was purified by thin layer chromatography with developing solvent system B to give the title product 7 (2.5 mg, 83.1% yield).

MS m/z (ESI): 534.0 [M+1].

$^1$H NMR (400 MHz, DMSO-$d_6$): δ 8.28 (d, 1H), 7.75 (d, 1H), 7.29 (s, 1H), 6.51 (s, 1H), 6.12 (s, 1H), 5.59-5.51 (m, 1H), 5.41 (s, 2H), 5.20-5.01 (m, 2H), 3.27-3.17 (m, 1H), 3.15-3.05 (m, 1H), 2.71-2.63 (m, 1H), 2.37 (s, 3H), 2.12-2.05 (m, 1H), 2.03-1.94 (m, 2H), 1.92-1.78 (m, 4H), 1.50-1.42 (m, 1H), 0.90-0.83 (m, 4H).

### Example 1-8

1-(((*S*)-7-benzyl-20-(2,5-dioxo-2,5-dihydro-1*H*-pyrrol-1-yl)-3,6,9,12,15-pentaoxo-2,5,8,11,14-pentaazaeicosyl)oxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclopropane-1-carboxamide **8**

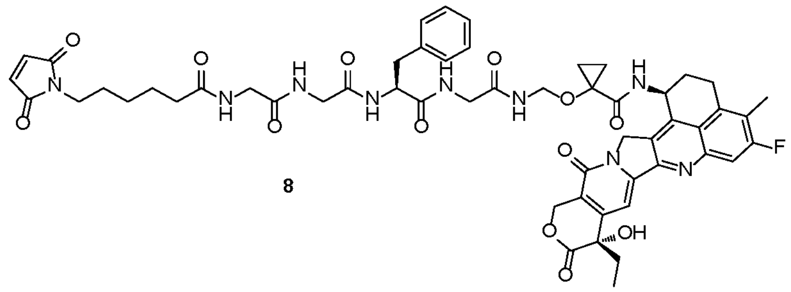

8

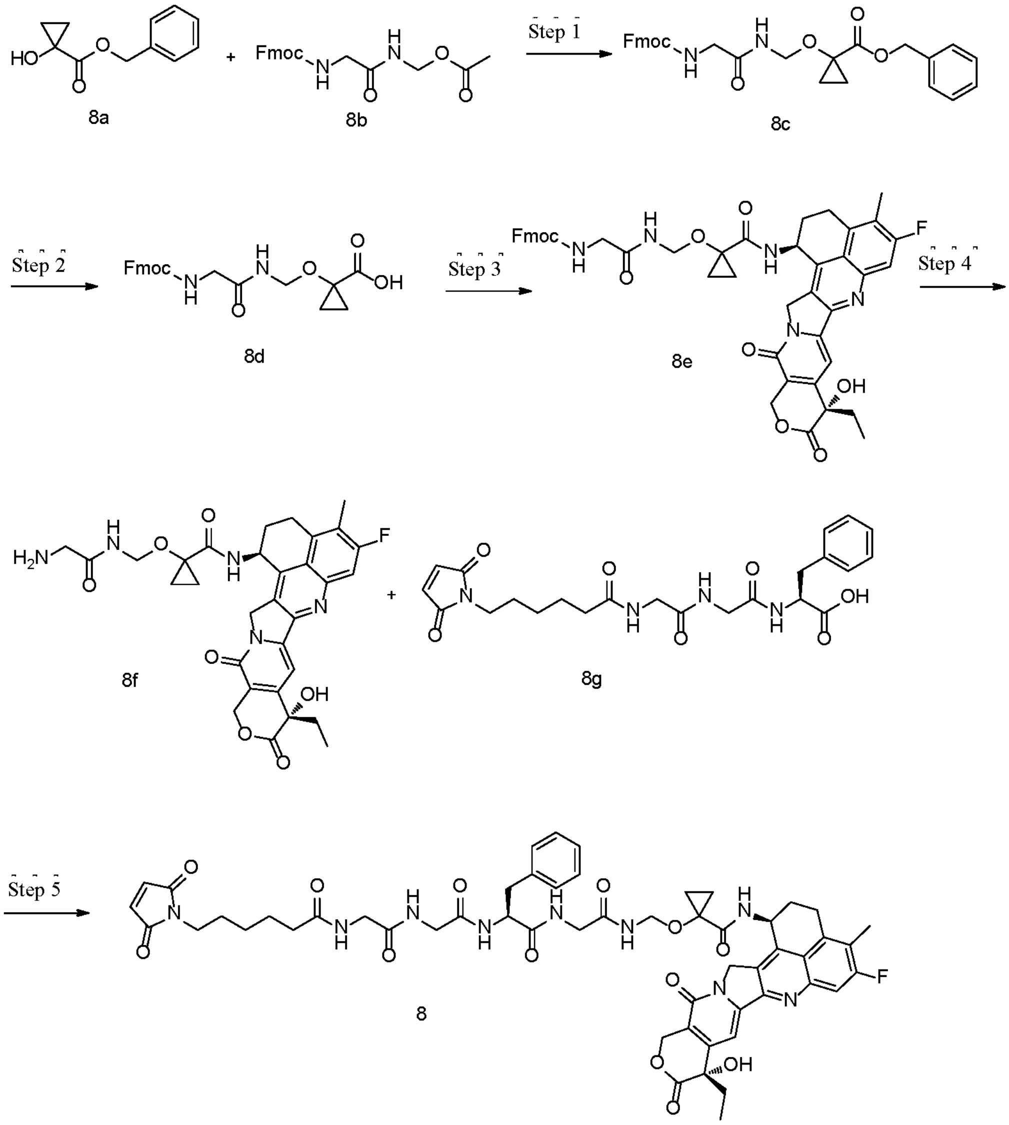


8a 8b 8c 8d 8e 8f 8g 8

Step 1

Benzyl 1-((2-((((9H-fluoren-9-yl)methoxy)carbonyl)amino)acetylamino)methoxy)cyclopropane-1-carboxylate **8c** Benzyl 1-hydroxycyclopropane-1-carboxylate **8a** (104 mg, 0.54 mmol; prepared as disclosed in Patent Application "US2005/20645") and 2-((((9*H*-fluoren-9-yl)methoxy)carbonyl)amino)acetylamino)methyl acetate **8b** (100 mg, 0.27 mmol; prepared as disclosed in Patent Application "CN105829346A") were added to a reaction flask, and 5 mL of tetrahydrofuran was added. The system was purged with argon three times, and the mixture was cooled to 0-5 °C in an ice-water bath, followed by addition of potassium tert-butoxide (61 mg, 0.54 mmol). The ice bath was removed, and the reaction mixture was warmed to room temperature and stirred for 10 min, followed by addition of 20 mL of ice water and by extraction with ethyl acetate (5 mL × 2) and chloroform (5 mL × 5). The organic phases were combined and concentrated. The resulting residue was dissolved in 3 mL of 1,4-dioxane, and 0.6 mL of water, sodium bicarbonate (27

mg, 0.32 mmol) and 9-fluorenylmethyl chloroformate (70 mg, 0.27 mmol) were added. The reaction mixture was stirred at room temperature for 1 h. 20 mL of water was added, followed by extraction with ethyl acetate (8 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system B to give the title product **8c** (100 mg, 73.6% yield).
MS m/z (ESI): 501.0 [M+1].

Step 2

1-((2-((((9H fluoren-9-yl)methoxy)carbonyl)amino)acetylamino)methoxy)cyclopropane-1-carboxylic acid **8d** Compound **8c** (50 mg, 0.10 mmol) was dissolved in 3 mL of a solvent mixture of tetrahydrofuran and ethyl acetate (V:V = 2:1), and palladium on carbon (25 mg, 10% loading) was added. The system was purged with hydrogen three times, and the reaction mixture was stirred at room temperature for 1 h. The reaction mixture was filtered through celite, and the filter cake was rinsed with tetrahydrofuran. The filtrate was concentrated to give the title product **8d** (41 mg, 100% yield).
MS m/z (ESI): 411.0 [M+1].

Step 3

(9*H*-fluoren-9-yl)methyl(2-(((1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)cyclopropoxy)methyl)amino)-2-oxoethyl)carbamate **8e** Compound **1b** (7 mg, 0.013 mmol) was added to a reaction flask, and 1 mL of *N,N*-dimethylformamide was added. The system was purged with argon three times, and the mixture was cooled to 0-5 °C in an ice-water bath, followed by addition of a drop of triethylamine, a solution of compound **8d** (7 mg, 0.017 mmol) in 0.5 mL of *N,N*-dimethylformamide, and 4-(4,6-dimethoxy-1,3,5-triazin-2-yl)-4-methylmorpholinium chloride (7 mg, 0.026 mmol). The reaction mixture was stirred in an ice bath for 35 min. 10 mL of water was added, followed by extraction with ethyl acetate (5 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **8e** (8.5 mg, 78.0% yield).
MS m/z (ESI): 828.0 [M+1].

Step 4

1-((2-Aminoacetylamino)methoxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclopropane-1-carboxamide **8f**

Compound **8e** (4 mg, 4.84 μmol) was dissolved in 0.2 mL of dichloromethane, and 0.1 mL of diethylamine was added. The reaction mixture was stirred at room temperature for 2 h and concentrated under reduced pressure. 2 mL of toluene was added, followed by concentration under reduced pressure; the procedures were repeated twice. The residue was slurried with 3 mL of n-hexane, and the upper n-hexane layer was removed; the procedures were repeated three times. The slurry was concentrated under reduced pressure to give the crude title product **8f** (2.9 mg), which was directly used in the next step without purification.
MS m/z (ESI): 606.0 [M+1].

Step 5

1-(((*S*)-7-benzyl-20-(2,5-dioxo-2,5-dihydro-1*H*-pyrrol-1-yl)-3,6,9,12,15-pentaoxo-2,5,8,11,14-pentaazaeicosyl)oxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclopropane-1-carboxamide **8** Crude **8f** (2.9 mg, 4.84 μmol) was dissolved in 0.5 mL of *N*,*N*-dimethylformamide. The system was purged with argon three times, and the solution was cooled to 0-5 °C in an ice-water bath. A solution of (*S*)-2(-2-(-2-(6-(2,5-dioxo-1*H*-pyrrol-1-yl)hexanamido)acetylamino)acetylamino)-3-phenylpropionic acid **8g** (2.7 mg, 5.80 μmol, prepared as disclosed in Patent Application "EP2907824") in 0.3 mL of *N,N*-dimethylformamide was added, followed by addition of 4-(4,6-dimethoxy-1,3,5-triazin-2-yl)-4-methylmorpholinium chloride (2.7 mg, 9.67 μmol). The reaction mixture was stirred in an ice bath for 30 min. Then, the ice bath was removed, and the reaction mixture was warmed to room temperature, stirred for 15 min, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **8** (2 mg, 39.0% yield).

MS m/z (ESI): 1060.0 [M+1].

$^{1}$H NMR (400 MHz, DMSO-$d_6$): δ 9.01 (d, 1H), 8.77 (t, 1H), 8.21 (t, 1H), 8.08-7.92 (m, 2H), 7.73 (d, 1H), 7.28 (s, 1H), 7.24-7.07 (m, 4H), 6.98 (s, 1H), 6.50 (s, 1H), 5.61 (q, 1H), 5.40 (s, 2H), 5.32 (t, 1H), 5.12 (q, 2H), 4.62 (t, 1H), 4.52 (t, 1H), 4.40-4.32 (m, 1H), 3.73-3.47 (m, 8H), 3.16-3.04 (m, 2H), 2.89 (dd, 1H), 2.69-2.55 (m, 2H), 2.37-2.23 (m, 4H), 2.12-1.93 (m, 4H), 1.90-1.74 (m, 2H), 1.52-1.38 (m, 4H), 1.33-1.11 (m, 5H), 0.91-0.81 (m, 4H).

**Example 1-9**

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5, 8,11,14-tetraazahexadec-16-yl)-6-(2,5-dioxo-2,5-dihydro-1*H*-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5, 8,11,14-tetraazahexadec-16-yl)-6-(2, 5-dioxo-2, 5-dihydro-1H-pyrrol-1-yl)hexanamide **9-B**

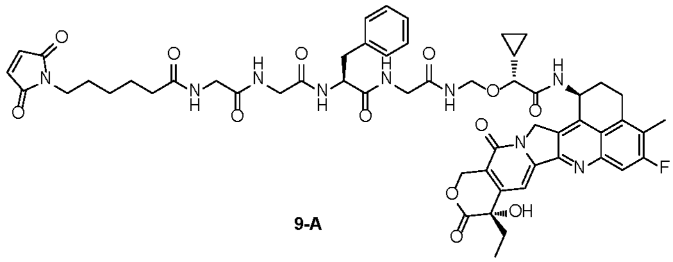

**9-A**

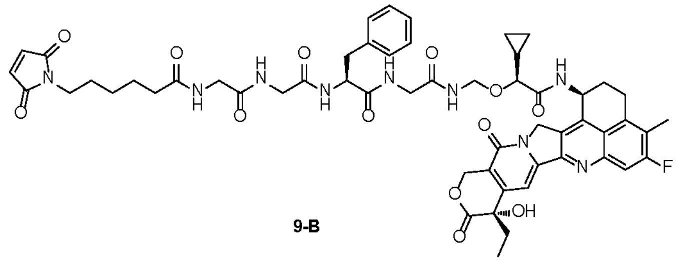

**9-B**

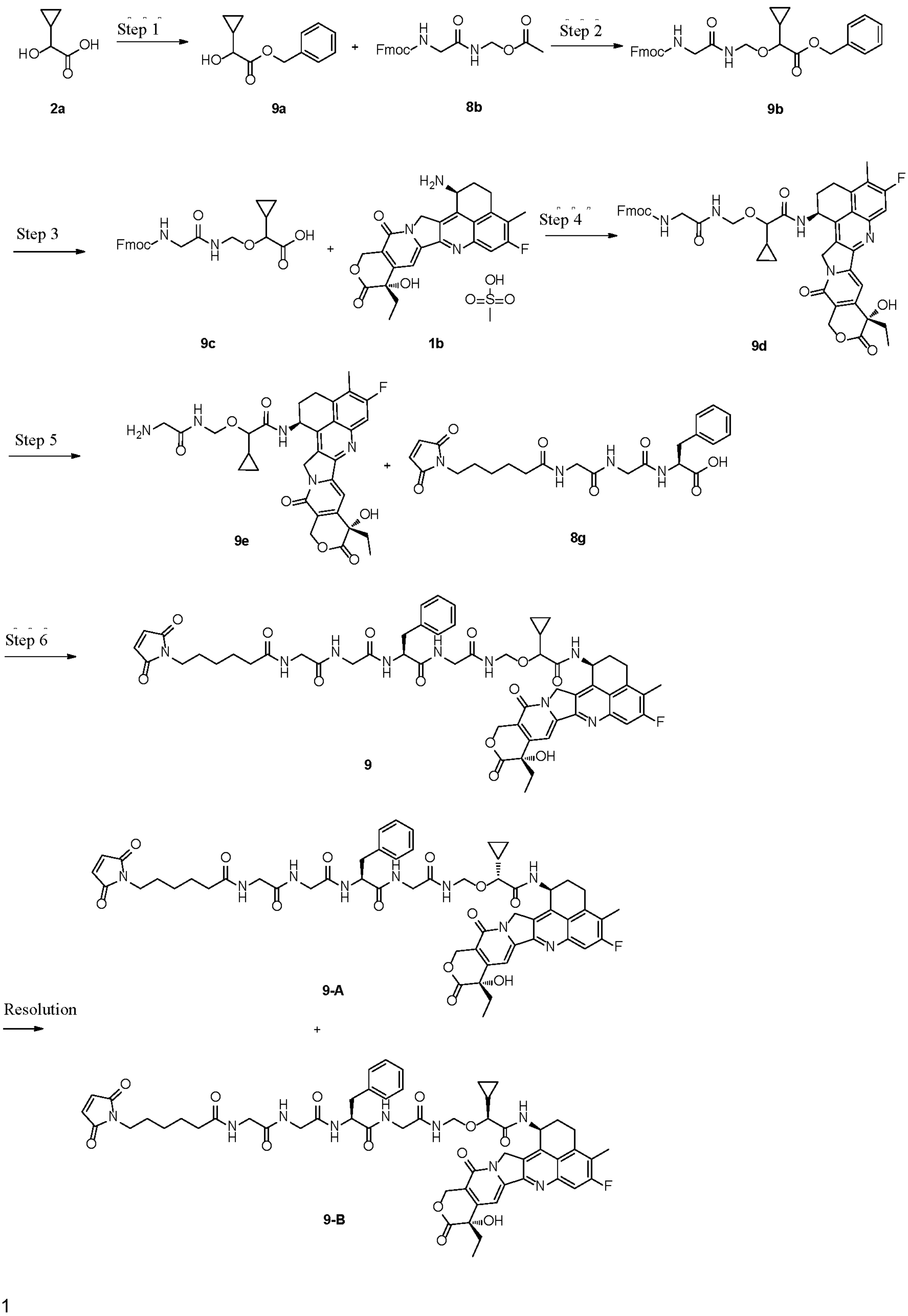


2a 9a 8b 9b

9c 1b 9d

9e 8g

9

9-A

+

9-B

Step 1

Benzyl 2-cyclopropyl-2-hydroxyacetate **9a**

Compound **2a** (1.3 g, 11.2 mmol; prepared as disclosed in Patent Application "WO2013/106717") was dissolved in 50 mL of acetonitrile, and potassium carbonate (6.18 g, 44.8 mmol), benzyl bromide (1.33 mL, 11.2 mmol) and

tetrabutylammonium iodide (413 mg, 1.1 mmol) were successively added. The reaction mixture was stirred at room temperature for 48 h and filtered through celite, and the filter cake was rinsed with ethyl acetate (10 mL). The filtrates were combined and concentrated under reduced pressure, and the resulting residue was purified by silica gel column chromatography with developing solvent system C to give the title product **9a** (2 g, 86.9% yield).

Step 2

Benzyl 10-cyclopropyl-1-(9*H*-fluoren-9-yl)-3,6-dioxo-2,9-dioxa-4,7-diazaundecan-11-oate **9b**

Compounds **9a** (120.9 mg, 0.586 mmol) and **8b** (180 mg, 0.489 mmol) were added to a reaction flask, and 4 mL of tetrahydrofuran was added. The system was purged with argon three times, and the reaction mixture was cooled to 0-5 °C in an ice-water bath, followed by addition of potassium tert-butoxide (109 mg, 0.98 mmol). The ice bath was removed, and the reaction mixture was warmed to room temperature and stirred for 40 min, followed by addition of 10 mL of ice water and by extraction with ethyl acetate (20 mL × 2) and chloroform (10 mL × 5). The organic phases were combined and concentrated. The resulting residue was dissolved in 4 mL of dioxane, and 2 mL of water, sodium bicarbonate (49.2 mg, 0.586 mmol) and 9-fluorenylmethyl chloroformate (126 mg, 0.49 mmol) were added. The reaction mixture was stirred at room temperature for 2 h. 20 mL of water was added, followed by extraction with ethyl acetate (10 mL × 3). The organic phase was washed with saturated sodium chloride solution (20 mL), dried over anhydrous sodium sulfate, and filtered. The filtrate was concentrated under reduced pressure. The resulting residue was purified by silica gel column chromatography with developing solvent system C to give the title product **9b** (48 mg, 19% yield).
MS m/z (ESI): 515.0 [M+1].

Step 3

10-cyclopropyl-1-(9H-fluoren-9-yl)-3,6-dioxo-2,9-dioxa-4,7-diazaundecan-11-oic acid **9c**

Compound **9b** (20 mg, 0.038 mmol) was dissolved in 4.5 mL of a solvent mixture of tetrahydrofuran and ethyl acetate (V:V = 2:1), and palladium on carbon (12 mg, 10% loading, dry basis) was added. The system was purged with hydrogen three times, and the reaction mixture was stirred at room temperature for 1 h. The reaction mixture was filtered through celite, and the filter cake was rinsed with ethyl acetate. The filtrate was concentrated to give the crude title product **9c** (13 mg), which was directly used in the next step without purification.
MS m/z (ESI): 424.9 [M+1].

Step 4

(9*H*-fluoren-9-yl)methyl(2-(((1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-oxoethoxy)methyl)amino)-2-oxoethyl)carbamate **9d**

Compound **1b** (10 mg, 18.8 μmol) was added to a reaction flask, and 1 mL of *N,N*-dimethylformamide was added. The system was purged with argon three times, and the mixture was cooled to 0-5 °C in an ice-water bath, followed by addition of a drop of triethylamine, crude **9c** (13 mg, 30.6 μmol), and 4-(4,6-dimethoxy-1,3,5-triazin-2-yl)-4-methylmorpholinium chloride (16.9 mg, 61.2 μmol). The reaction mixture was stirred in an ice bath for 40 min. 10 mL of water was added, followed by extraction with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **9d** (19 mg, 73.6% yield).
MS m/z (ESI): 842.1 [M+1].

Step 5

2-((2-Aminoacetylamino)methoxy)-2-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)acetamide **9e**

Compound **9d** (19 mg, 22.6 μmol) was dissolved in 2 mL of dichloromethane, and 1 mL of diethylamine was added. The reaction mixture was stirred at room temperature for 2 h and concentrated under reduced pressure. 1 mL of toluene was added, followed by concentration under reduced pressure; the procedures were repeated twice. The residue was slurried with 3 mL of *n*-hexane and let stand. Then, the supernatant was removed, and the solid was kept.

The solid residue was concentrated under reduced pressure and dried using an oil pump to give the crude title product **9e** (17 mg), which was directly used in the next step without purification.

MS m/z (ESI): 638.0 [M+18].

Step 6

*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11, 14-tetraazahexadec-16-yl)-6-(2,5-dioxo-2,5-dihydro-1H-pyrrol-1-yl)hexanamide **9-A**

*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8, 11, 14-tetraazahexadec-16-yl)-6-(2,5-dioxo-2,5-dihydro-1H-pyrrol-1-yl)hexanamide **9-B**

Crude **9e** (13.9 mg, 22.4 μmol) was dissolved in 0.6 mL of *N*,*N*-dimethylformamide. The system was purged with argon three times, and the solution was cooled to 0-5 °C in an ice-water bath. A solution of **8g** (21.2 mg, 44.8 μmol) in 0.3 mL of *N,N*-dimethylformamide was added, followed by addition of 4-(4,6-dimethoxy-1,3,5-triazin-2-yl)-4-methylmorpholinium chloride (18.5 mg, 67.3 μmol). The reaction mixture was stirred in an ice bath for 10 min. Then, the ice bath was removed, and the reaction mixture was warmed to room temperature and stirred for 1 h to produce compound **9.** The reaction mixture was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title products (9-A: 2.4 mg, 9-B: 1.7 mg).

MS m/z (ESI): 1074.4 [M+1].

Single-configuration compound 9-A (shorter retention time):
UPLC analysis: retention time: 1.14 min; purity: 85% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.60 (t, 1H), 8.51-8.49 (d, 1H), 8.32-8.24 (m, 1H), 8.13-8.02 (m, 2H), 8.02-7.96 (m, 1H), 7.82-7.75 (m, 1H), 7.31 (s, 1H), 7.26-7.15 (m, 4H), 6.99 (s, 1H), 6.55-6.48 (m, 1H), 5.65-5.54 (m, 1H), 5.41 (s, 2H), 5.35-5.15 (m, 3H), 4.74-4.62 (m, 1H), 4.54-4.40 (m, 2H), 3.76-3.64 (m, 4H), 3.62-3.48 (m, 2H), 3.20-3.07 (m, 2H), 3.04-2.94 (m, 1H), 2.80-2.62 (m, 1H), 2.45-2.30 (m, 3H), 2.25-2.15 (m, 2H), 2.15-2.04 (m, 2H), 1.93-1.78 (m, 2H), 1.52-1.39 (m, 3H), 1.34-1.12 (m, 5H), 0.87 (t, 3H), 0.64-0.38 (m, 4H).

Single-configuration compound 9-B (longer retention time):
UPLC analysis: retention time: 1.16 min; purity: 89% (chromatography column: ACQUITY UPLC BEHC18 1.7 μm 2.1 × 50 mm; mobile phase: A-water (5 mmol of $NH_4OAc$), B-acetonitrile).

$^1$H NMR (400 MHz, DMSO-$d_6$): δ 8.68-8.60 (m, 1H), 8.58-8.50 (m, 1H), 8.32-8.24 (m, 1H), 8.13-8.02 (m, 2H), 8.02-7.94 (m, 1H), 7.82-7.75 (m, 1H), 7.31 (s, 1H), 7.26-7.13 (m, 3H), 6.99 (s, 1H), 6.55-6.48 (m, 1H), 5.60-5.50 (m, 1H), 5.41 (s, 2H), 5.35-5.15 (m, 2H), 4.78-4.68 (m, 1H), 4.60-4.40 (m, 2H), 3.76-3.58 (m, 4H), 3.58-3.48 (m, 1H), 3.20-3.10 (m, 2H), 3.08-2.97 (m, 2H), 2.80-2.72 (m, 2H), 2.45-2.30 (m, 3H), 2.25-2.13 (m, 2H), 2.13-2.04 (m, 2H), 2.03-1.94 (m, 2H), 1.91-1.78 (m, 2H), 1.52-1.39 (m, 3H), 1.34-1.12 (m, 4H), 0.91-0.79 (m, 3H), 0.53-0.34 (m, 4H).

**Example 1-10**

*N*-((2*S,*10*S*)-10-benzyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)-1,1,1-trifluoro-6,9,12,15-tetraoxo-3-oxa-5,8,11,14-tetraazahexadec-16-yl)-6-(2,5-dioxo-2,5-dihydro-1*H*-pyrrol-1-yl)hexanamide **10-A**

*N*-((2*R*,10*S*)-10-benzyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)-1,1,1-trifluoro-6,9,12,15-tetraoxo-3-oxa-5,8,11,14-tetraazahexadec-16-yl)-6-(2,5-dioxo-2,5-dihydro-1*H*-pyrrol-1-yl)hexanamide **10-B**

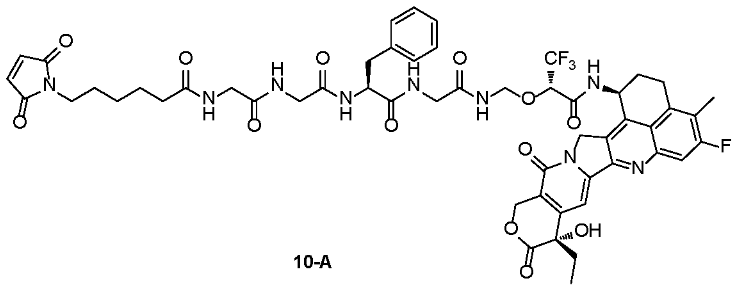

10-A

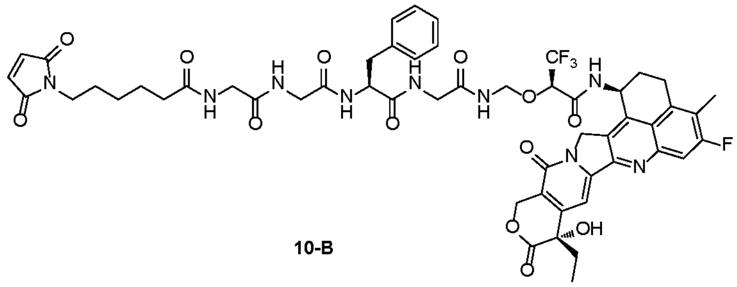

10-B

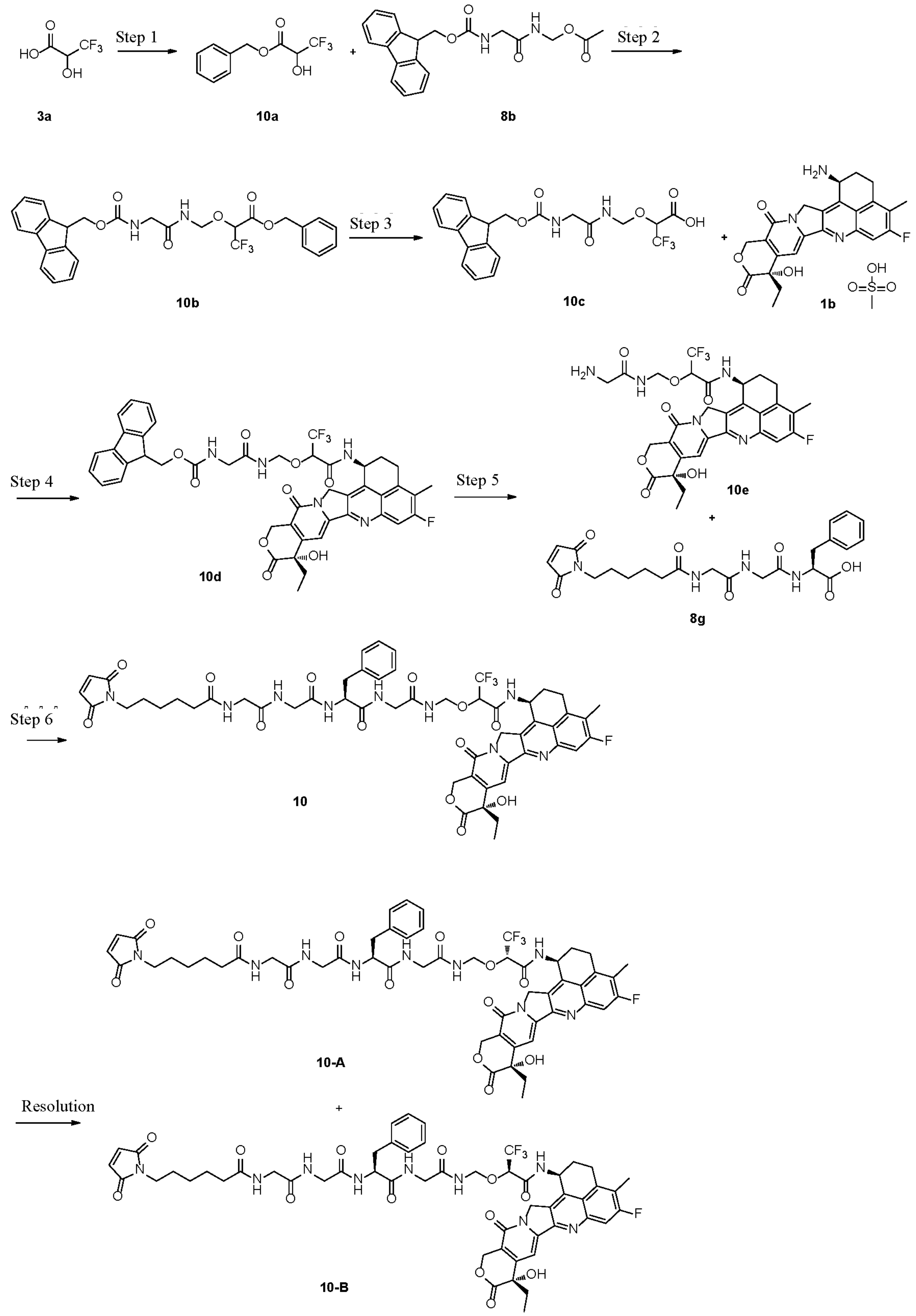


3a 10a 8b

10b 10c 1b

10d 10e 8g

10

10-A

10-B

Step 1

Benzyl 3,3,3-trifluoro-2-hydroxypropionate **10a**

Compound **3a** (1.80 g, 12.5 mmol) was dissolved in 100 mL of acetonitrile, and potassium carbonate (5.17 g, 37.5 mmol), benzyl bromide (4.48 mL, 37.5 mmol) and tetrabutylammonium iodide (231 mg, 0.63 mmol) were added successively. The reaction mixture was heated to 60 °C, stirred for 5 h, cooled to room temperature, and filtered. The filtrate was concentrated under reduced pressure, and the resulting residue was purified by silica gel column chromatography with developing solvent system C to give the title product **10a** (980 mg, 33.5% yield).

$^1$H NMR (400 MHz, $CDCl_3$): δ 7.43-7.36 (m, 5H), 5.34 (s, 2H), 4.53 (s, 1H), 3.44 (s, 1H).

Step 2

Benzyl 1-(9H-fluoren-9-yl)-3,6-dioxo-10-(trifluoromethyl)-2,9-dioxa-4,7-diazaundecan-11-oate **10b**

Compounds **8b** (63 mg, 0.17 mmol) and **10a** (80 mg, 0.34 mmol) were added to a reaction flask, and 3 mL of tetrahydrofuran was added. The system was purged with argon three times, and the reaction mixture was cooled to 0-5 °C in an ice-water bath, followed by addition of potassium *tert*-butoxide (38 mg, 0.34 mmol). The ice bath was removed, and the reaction mixture was warmed to room temperature and stirred for 20 min, followed by addition of 10 mL of ice water and by extraction with ethyl acetate (20 mL × 2) and chloroform (10 mL × 5). The organic phases were combined and concentrated. The resulting residue was dissolved in 2 mL of dioxane, and 0.4 mL of water, sodium bicarbonate (19 mg, 0.23 mmol) and 9-fluorenylmethyl chloroformate (49 mg, 0.19 mmol) were added. The mixture was stirred at room temperature for 1 h. 20 mL of water was added, followed by extraction with ethyl acetate (10 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system C to give the title product **10b** (51 mg, 55.3% yield).

MS m/z (ESI): 559.9 [M+18].

Step 3

1-(9*H*-fluoren-9-yl)-3,6-dioxo-1 0-(trifluoromethyl)-2,9-dioxa-4, 7 -diazaundecan-11-oic acid **10c**

Compound **10b** (15 mg, 0.28 mmol) was dissolved in 3 mL of a solvent mixture of tetrahydrofuran and ethyl acetate (V:V = 2:1), and palladium on carbon (15 mg, 10% loading) was added. The system was purged with hydrogen three times, and the reaction mixture was stirred at room temperature for 1 h. The reaction mixture was filtered through celite, and the filter cake was rinsed with tetrahydrofuran. The filtrate was concentrated to give the crude title product **10c** (13 mg).

MS m/z (ESI): 452.9 [M+1].

Step 4

(9*H*-fluoren-9-yl)methyl(2-((((3-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,1,1-trifluoro-3-oxoprop-2-yl)oxy)methyl)amino)-2-oxoethyl)carbamate **10d**

Compound **1b** (10 mg, 18.8 μmol) was added to a reaction flask, and 1 mL of *N,N*-dimethylformamide was added. The system was purged with argon three times, and the mixture was cooled to 0-5 °C in an ice-water bath, followed by addition of a drop of triethylamine, a solution of **10c** (13 mg, 28.7 μmol) in 0.5 mL of *N*,*N*-dimethylformamide, and 4-(4,6-dimethoxy-1,3,5-triazin-2-yl)-4-methylmorpholinium chloride (11 mg, 39.7 μmol). The reaction mixture was stirred in an ice bath for 30 min. 10 mL of water was added, followed by extraction with ethyl acetate (10 mL × 3). The organic phases were combined, washed with saturated sodium chloride solution (10 mL × 2), dried over anhydrous sodium sulfate, and filtered. The filtrate was concentrated under reduced pressure, and the resulting residue was purified by thin layer chromatography with developing solvent system B to give the title product **10d** (16 mg, 97.8% yield).

MS m/z (ESI): 870.0 [M+1].

Step 5

2-((2-Aminoacetylamino)methoxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-3,3,3-trifluoropropionamide **10e**

Compound **10d** (16 mg, 18.4 μmol) was dissolved in 0.6 mL of dichloromethane, and 0.3 mL of diethylamine was added. The reaction mixture was stirred at room temperature for 2 h and concentrated under reduced pressure. 2 mL of toluene was added, followed by concentration under reduced pressure; the procedures were repeated twice. The residue was slurried with 3 mL of *n*-hexane and let stand. Then, the supernatant was removed, and the solid was kept; the procedures were repeated three times. The solid residue was concentrated under reduced pressure and dried using an oil pump to give the crude title product **10e** (12 mg), which was directly used in the next step without purification.
MS m/z (ESI): 647.9 [M+1].

Step 6

*N*-((2*S*,10*S*)-10-benzyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)-1,1,1-trifluoro-6,9,12,15-tetraoxo-3-oxa-5,8,11,14-tetraazahexadec-16-yl)-6-(2,5-dioxo-2,5-dihydro-1*H*-pyrrol-1-yl)hexanamide **10-A**

*N*-((2*R*,10*S*)-10-benzyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)-1,1,1-trifluoro-6,9,12,15-tetraoxo-3-oxa-5,8,11,14-tetraazahexadec-16-yl)-6-(2,5-dioxo-2,5-dihydro-1*H*-pyrrol-1-yl)hexanamide **10-B**

Crude **10e** (12 mg, 18.5 μmol) was dissolved in 1.0 mL of *N*,*N*-dimethylformamide. The system was purged with argon three times, and the solution was cooled to 0-5 °C in an ice-water bath. A solution of **8g** (14 mg, 29.6 μmol) in 0.3 mL of *N*,*N*-dimethylformamide was added, followed by addition of 4-(4,6-dimethoxy-1,3,5-triazin-2-yl)-4-methylmorpholinium chloride (15 mg, 54.2 μmol). The reaction mixture was stirred in an ice bath for 30 min. Then, the ice bath was removed, and the reaction mixture was warmed to room temperature and stirred for 1 h to produce compound **10**. The reaction mixture was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title products (2.7 mg, 2.6 mg).
MS m/z (ESI): 1102.0 [M+1].
Single-configuration compound (shorter retention time):
UPLC analysis: retention time: 1.18 min; purity: 91% (chromatography column: ACQUITY UPLC BEHC18 1.7 μm 2.1 × 50 mm; mobile phase: A-water (5 mmol of $NH_4OAc$), B-acetonitrile).
$^1H$ NMR (400 MHz, DMSO-$d_6$): δ 8.97 (d, 1H), 8.85-8.76 (m, 1H), 8.37-8.27 (m, 1H), 8.12-8.02 (m, 1H), 8.02-7.95 (m, 1H), 7.80 (d, 1H), 7.31 (s, 1H), 7.26-7.10 (m, 4H), 6.99 (s, 1H), 6.66 (br, 1H), 6.52 (s, 1H), 5.65-5.54 (m, 1H), 5.41 (s, 1H), 5.37-5.25 (m, 3H), 5.23-5.13 (m, 1H), 4.81-4.68 (m, 2H), 4.51-4.41 (m, 1H), 3.78-3.45 (m, 6H), 3.21-3.13 (m, 1H), 3.02-2.93 (m, 1H), 2.77-2.63 (m, 2H), 2.45-2.29 (m, 3H), 2.24-2.05 (m, 3H), 2.04-1.93 (m, 5H), 1.90-1.75 (m, 2H), 1.52-1.38 (m, 4H), 0.90-0.78 (m, 5H).
Single-configuration compound (longer retention time):
UPLC analysis: retention time: 1.23 min; purity: 90% (chromatography column: ACQUITY UPLC BEHC18 1.7 μm 2.1 × 50 mm; mobile phase: A-water (5 mmol of $NH_4OAc$), B-acetonitrile).
$^1H$ NMR (400 MHz, DMSO-$d_6$): δ 9.05 (d, 1H), 8.97-8.88 (m, 1H), 8.35-8.27 (m, 1H), 8.11-8.03 (m, 1H), 8.02-7.95 (m, 1H), 7.80 (d, 1H), 7.34 (s, 1H), 7.29-7.13 (m, 4H), 6.99 (s, 1H), 6.66 (br, 1H), 6.54 (s, 1H), 5.64-5.55 (m, 1H), 5.43 (s, 1H), 5.36-5.20 (m, 3H), 4.92-4.85 (m, 1H), 4.82-4.72 (m, 2H), 4.52-4.42 (m, 1H), 3.77-3.48 (m, 6H), 3.21-3.14 (m, 1H), 3.03-2.95 (m, 1H), 2.79-2.65 (m, 2H), 2.47-2.28 (m, 3H), 2.25-2.05 (m, 3H), 2.05-1.94 (m, 5H), 1.91-1.76 (m, 2H), 1.52-1.37 (m, 4H), 0.92-0.77 (m, 5H).

**Example 1-11**

1-(((*S*)-7-benzyl-20-(2,5-dioxo-2,5-dihydro-1*H*-pyrrol-1-yl)-3,6,9,12,15-pentaoxo-2,5,8,11,14-pentaazaeicosyl)oxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclobutane-1-carboxamide **11**

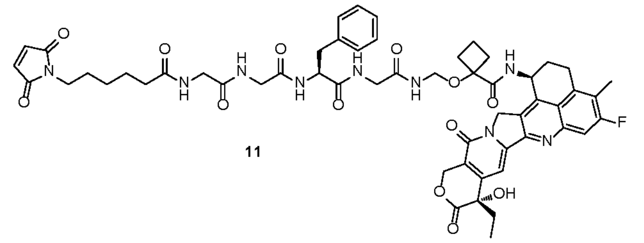

11

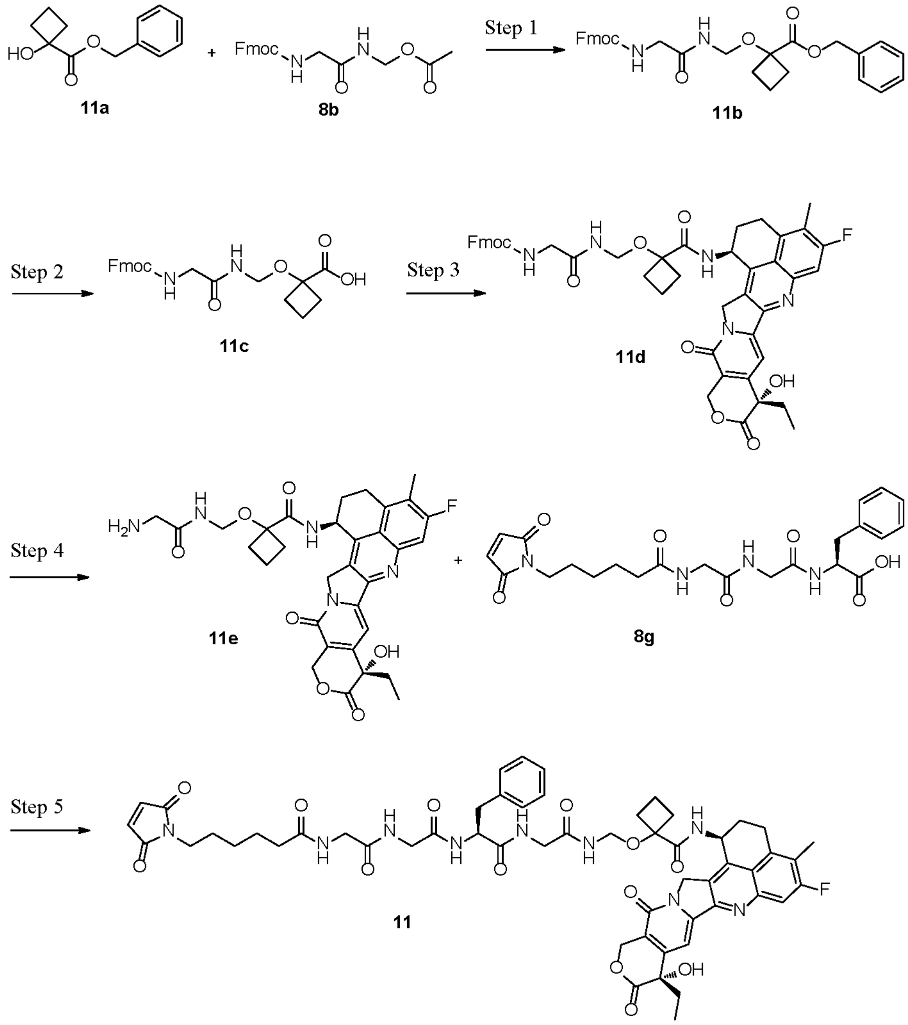


11a 8b 11b

11c 11d

11e 8g

11

Step 1

Benzyl 1-((2-((((9*H*-fluoren-9-yl)methoxy)carbonyl)amino)acetylamino)methoxy)cyclobutane-1-carboxylate **11b**

Benzyl 1-hydroxycyclobutane-carboxylate **11a** (167 mg, 0.81 mmol, prepared as disclosed in "Journal of Medicinal Chemistry, 2013, vol. 56, #13, p.5541-5552") and **8b** (150 mg, 0.41 mmol) were added to a reaction flask, and 5 mL of tetrahydrofuran was added. The system was purged with argon three times, and the reaction mixture was cooled to 0-5 °C in an ice-water bath, followed by addition of potassium tert-butoxide (92 mg, 0.82 mmol). The ice bath was removed, and the reaction mixture was warmed to room temperature and stirred for 10 min, followed by addition of 20 mL of ice water and by extraction with ethyl acetate (5 mL × 2) and chloroform (5 mL × 5). The organic phases were combined and concentrated. The resulting residue was dissolved in 3 mL of dioxane, and 0.6 mL of water, sodium bicarbonate (41 mg, 0.48 mmol) and 9-fluorenylmethyl chloroformate (105 mg, 0.41 mmol) were added. The mixture was stirred at room temperature for 1 h. 20 mL of water was added, followed by extraction with ethyl acetate (8 mL × 3). The organic phase was washed with saturated sodium chloride solution (20 mL), dried over anhydrous sodium sulfate, and filtered. The filtrate was concentrated under reduced pressure, and the resulting residue was purified by silica gel

column chromatography with developing solvent system C to give the title product **11b** (37 mg, 17.6% yield).

MS m/z (ESI): 514.6 [M+1].

Step 2

1-((2-((((9*H*-fluoren-9-yl)methoxy)carbonyl)amino)acetylamino)methoxy)cyclobutane-1-carboxylic acid **11c**

Compound **11b** (37 mg, 71.9 μmol) was dissolved in 3 mL of a solvent mixture of tetrahydrofuran and ethyl acetate (V:V = 2:1), and palladium on carbon (15 mg, 10% loading) was added. The system was purged with hydrogen three times, and the reaction mixture was stirred at room temperature for 2 h. The reaction mixture was filtered through celite, and the filter cake was rinsed with tetrahydrofuran. The filtrate was concentrated to give the title product **11c** (35 mg, 82% yield), which was directly used in the next step.

Step 3

(9*H*-fluoren-9-yl)methyl(2-(((1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-Z>]quinolin-1 -yl)aminocarbonyl)cyclobutoxy)methyl)amino)-2-oxoethyl)carbamate **11d** Compound **1b** (10 mg, 0.018 mmol) was added to a reaction flask, and 1 mL of *N,N*-dimethylformamide was added. The system was purged with argon three times, and the mixture was cooled to 0-5 °C in an ice-water bath, followed by addition of a drop of triethylamine, a solution of compound **11c** (13 mg, 0.031 mmol) in 0.5 mL of *N,N*-dimethylformamide, and 4-(4,6-dimethoxy-1,3,5-triazin-2-yl)-4-methylmorpholinium chloride (25 mg, 0.091 mmol). The reaction mixture was stirred in an ice bath for 40 min. 8 mL of water was added, followed by extraction with ethyl acetate (5 mL × 3). The organic phase was washed with saturated sodium chloride solution (8 mL), dried over anhydrous sodium sulfate, and filtered. The filtrate was concentrated under reduced pressure, and the resulting residue was purified by thin layer chromatography with developing solvent system A to give the title product **11d** (19 mg, 73.9% yield).

MS m/z (ESI): 842.3 [M+1].

Step 4

1-((2-Aminoacetylamino)methoxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclobutane-1-carboxamide **11e**

Compound **11d** (19 mg, 22.6 μmol) was dissolved in 2 mL of dichloromethane, and 1 mL of diethylamine was added. The reaction mixture was stirred at room temperature for 1.5 h and concentrated under reduced pressure. 1 mL of toluene was added, followed by concentration under reduced pressure; the procedures were repeated twice. The residue was slurried with 4 mL of *n*-hexane, and the upper *n*-hexane layer was removed; the procedures were repeated three times. The slurry was concentrated under reduced pressure to give the crude title product **11e** (15 mg), which was directly used in the next step without purification.

Step 5

1-(((*S*)-7-benzyl-20-(2,5-dioxo-2,5-dihydro-1*H*-pyrrol-1-yl)-3,6,9,12,15-pentaoxo-2,5,8,11,14-pentaazaeicosyl)oxy)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclobutane-1-carboxamide **11**

Crude **11e** (2 mg, 3.22 μmol) was dissolved in 0.5 mL of *N*,*N*-dimethylformamide. The system was purged with argon three times, and the solution was cooled to 0-5 °C in an ice-water bath. A solution of **8g** (1.5 mg, 3.17 μmol) in 0.3 mL of *N*,*N*-dimethylformamide was added, followed by addition of 4-(4,6-dimethoxy-1,3,5-triazin-2-yl)-4-methylmorpholinium chloride (2.7 mg, 9.67 μmol). The reaction mixture was stirred at room temperature for 30 min and concentrated to dryness with an oil pump to remove DMF, and the residue was dissolved in DCM and purified directly by thin layer chromatography twice (polarity of developing solvent: DCM/MeOH = 10/1) to give the title product **11** (1 mg, 28.8% yield).

MS m/z (ESI): 1073.6 [M+1].

$^1$H NMR (400 MHz, $CDCl_3$): δ 8.70-8.60 (m, 1H), 8.28-8.19 (m, 1H), 8.13-7.91 (m, 3H), 7.79-7.71 (d, 1H), 7.29 (s, 1H), 7.25-7.09 (m, 4H), 6.98 (s, 1H), 6.71-6.62 (m, 1H), 6.55-6.47 (m, 1H), 5.64-5.54 (m, 2H), 5.40 (s, 1H), 5.35-5.27 (t, 2H), 5.17-5.10 (m, 2H), 4.60-4.51 (m, 1H), 4.51-4.35 (m, 2H), 3.93-3.78 (m, 3H), 3.71-3.59 (m, 3H), 3.01-2.88 (m, 3H), 2.70-2.64 (m, 2H), 2.44-2.30 (m, 3H), 2.28-2.14 (m, 3H), 2.11-1.92 (m, 6H), 1.90-1.76 (m, 3H), 1.51-1.39 (m, 4H), 0.92-0.75 (m, 6H).

**Example 1-12**

(*S*)-3-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropionamide **12-A**

(*R*)-3-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropionamide **12-B**

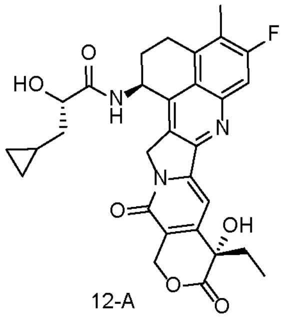
12-A

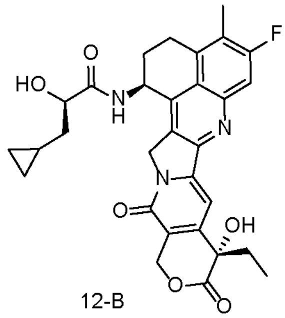
12-B

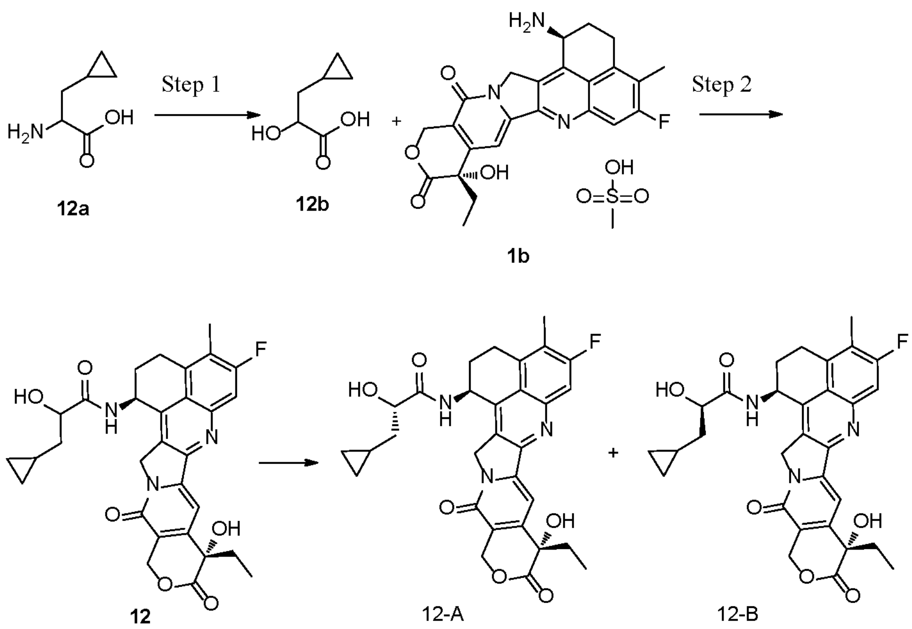

12a 12b 1b 12 12-A 12-B

Step 1

3-Cyclopropyl-2-hydroxypropionic acid **12b**

Compound **12a** (0.5 g, 3.87 mmol, supplied by Adamas) was dissolved in 35 mL of a solvent mixture of water and acetic acid (V:V = 4:1), and the solution was cooled to 0-5 °C in an ice-water bath, followed by dropwise addition of a 2 M aqueous solution of sodium nitrite (0.53 g, 7.74 mmol). The reaction mixture was warmed to room temperature and stirred for 3 h. Solid sodium chloride was added to the reaction mixture to saturate the aqueous phase. The reaction mixture was extracted with ethyl acetate (8 mL × 8), dried over anhydrous sodium sulfate, and filtered. The filtrate was concentrated to give the title product **12b** (0.45 g, 89.3% yield).

Step 2

(*S*)-3-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropionamide **12-A**

(*R*)-3-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)-2-hydroxypropionamide **12-B**

To compound **1b** (45 mg, 0.085 mmol) were added 1.5 mL of ethanol and 1.5 mL of *N,N*-dimethylformamide. The system was purged with argon three times, and 0.1 mL of *N*-methylmorpholine was added dropwise. The reaction mixture was stirred until it became clear. To the reaction mixture were successively added compound **12b** (90 mg, 0.691 mmol), 1-hydroxybenzotriazole (34 mg, 0.251 mmol) and 1-(3-dimethylaminopropyl)-3-ethylcarbodiimide hydrochloride (49 mg, 0.256 mmol). After addition, the reaction mixture was stirred at room temperature for 3 h and concentrated under reduced pressure, and the resulting crude compound **12** was purified by high performance liquid chromatography (separation conditions: chromatography column: Sharpsil-T C18 5 μm 21.2 × 250 mm; mobile phase: A-water (10 mmol of $NH_4OAc$), B-acetonitrile, gradient elution, flow rate: 18 mL/min) to give the title products (7 mg, 15 mg).

MS m/z (ESI): 547.9 [M+1].

Single-configuration compound (shorter retention time):

UPLC analysis: retention time: 1.345 min; purity: 72% (chromatography column: ZORBAX Ecliphase Plus C18 1.8 μm 2.1 × 50 mm; mobile phase: A-water (5 mmol of $NH_4OAc$), B-acetonitrile).

$^1$H NMR (400 MHz, DMSO-$d_6$): δ 8.42 (d, 1H), 7.78 (d, 1H), 7.30 (s, 1H), 6.51 (s, 1H), 5.60-5.50 (m, 2H), 5.42 (s, 1H), 5.19 (q, 2H), 4.02-4.00 (m, 1H), 3.21-3.11 (m, 2H), 2.39 (s, 3H), 2.21-2.07 (m, 2H), 2.05-1.95 (m, 1H), 1.92-1.68 (m, 4H), 1.53-1.41 (m, 1H),0.87 (t, 3H), 0.48-0.34 (m, 2H), 0.14-0.01 (m, 2H).

Single-configuration compound (longer retention time):

UPLC analysis: retention time: 1.399 min; purity: 88% (chromatography column: ZORBAX Ecliphase Plus C18 1.8 μm 2.1 × 50 mm; mobile phase: A-water (5 mmol of $NH_4OAc$), B-acetonitrile).

$^1$H NMR (400 MHz, DMSO-$d_6$): δ 8.36 (d, 1H), 7.77 (d, 1H), 7.31 (s, 1H), 6.51 (s, 1H), 5.58-5.51 (m, 1H), 5.48 (d, 1H), 5.42 (s, 1H),5.20 (q, 2H), 4.09-4.02 (m, 1H), 3.22-3.11 (m, 2H), 2.39 (s, 3H), 2.27-2.06 (m, 2H), 2.05-1.95 (m, 1H), 1.93-1.81 (m, 2H), 1.65-1.43 (m, 2H), 1.32-1.21 (m, 1H), 0.87 (t, 3H), 0.48-0.33 (m, 2H), 0.14-0.01 (m, 2H).

**Example 1-13 (reference example)**

*N*-((1*S*,9*S*)-9-ethyl-5-fluoro-9-hydroxy-4-methyl-10,13-dioxo-2,3,9,10,13,15- hexahydro-1*H*,12*H*-benzo[*de*]pyrano[3',4':6,7]indolizino[1,2-*b*]quinolin-1-yl)-2-

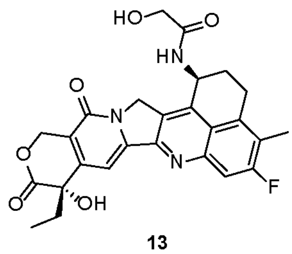

hydroxyacetamide

**13**

The title compound **13** was prepared as disclosed in "Example 76 on page 147 of the specification of Patent EP2907824A1".

**Example 1-14**

*N*-((2*R*,10*S*)-10-benzyl-2-(cyclopropylmethyl)-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5, 8,11,14-tetraazahexadec-16-yl)-6-(2, 5-dioxo-2, 5-dihydro-1*H*-pyrrol-1-yl)hexanamide **14-A**

*N*-((2*S*,10*S*)-10-benzyl-2-(cyclopropylmethyl)-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5, 8,11,14-tetraazahexadec-16-yl)-6-(2, 5-dioxo-2, 5-dihydro-1*H*-pyrrol-1-yl)hexanamide 14-B

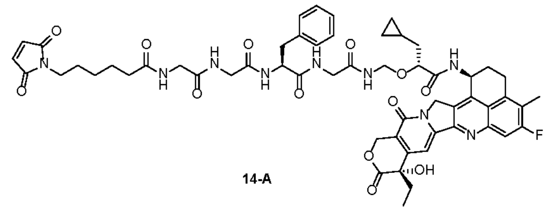

14-A

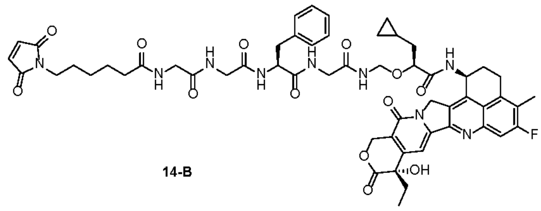

14-B

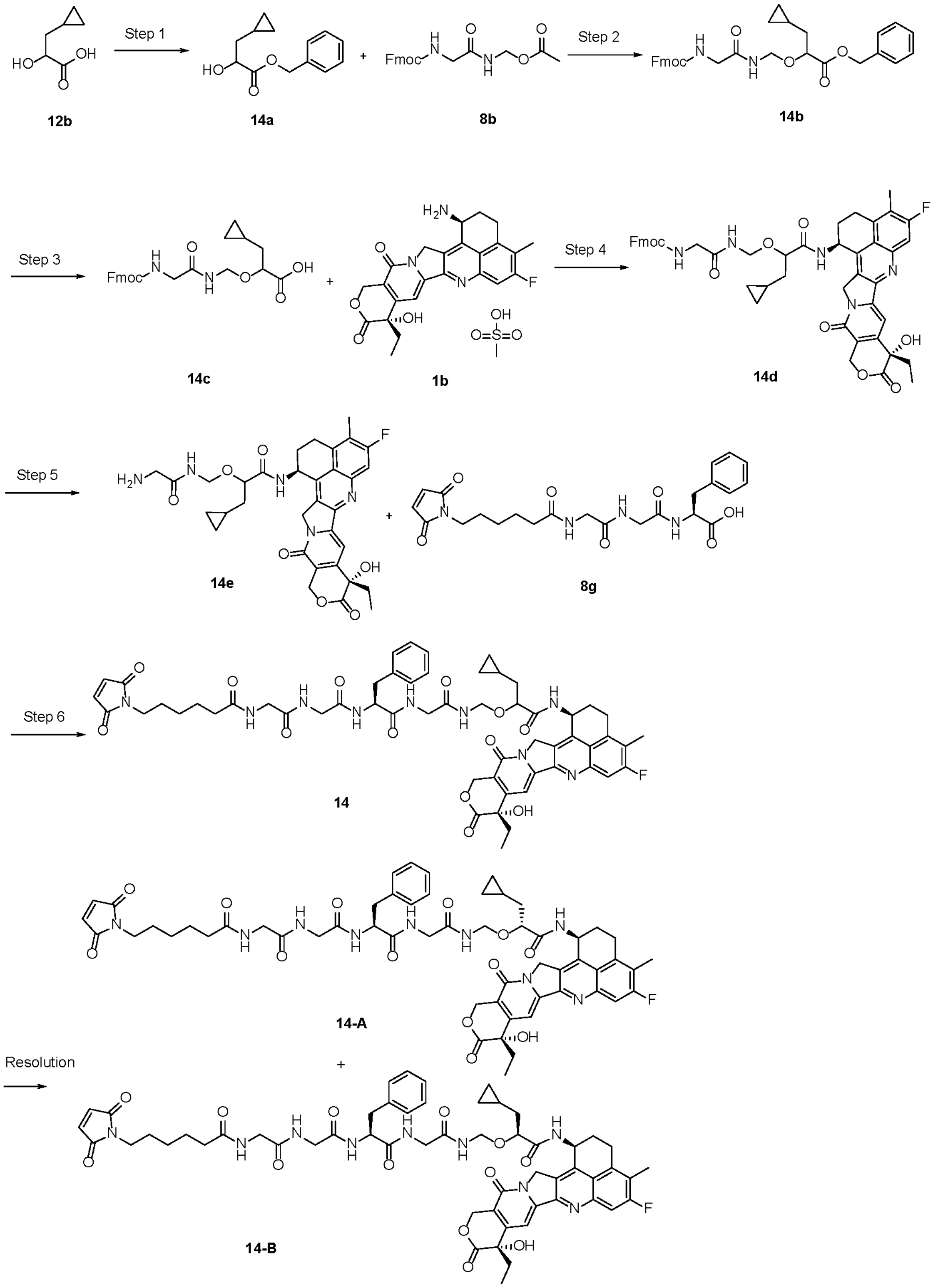


12b 14a 8b 14b

14c 1b 14d

14e 8g

14

14-A

+

14-B

Step 1

Benzyl 3-cyclopropyl-2-hydroxypropionate **14a**

Compound **12b** (200 mg, 1.54 mmol) was dissolved in 20 mL of acetonitrile, and potassium carbonate (1.06 g, 7.68 mmol), benzyl bromide (0.16 mL, 1.34 mmol) and tetrabutylammonium iodide (28 mg, 0.07 mmol) were added successively. The reaction mixture was stirred at room temperature for 48 h and filtered through celite, and the filter cake was rinsed with ethyl acetate (10 mL). The filtrates were combined and concentrated under reduced pressure, and the resulting residue was purified by silica gel column chromatography with developing solvent system C to give the title product **14a** (140 mg, 41.3% yield).

Step 2

Benzyl 10-(cyclopropylmethyl)-1-(9*H*-fluoren-9-yl)-3,6-dioxo-2,9-dioxa-4,7-diazaundecan-11-oate **14b**

Compounds **14a** (94 mg, 0.427 mmol) and **8b** (130 mg, 0.353 mmol) were added to a reaction flask, and 10 mL of tetrahydrofuran was added. The system was purged with argon three times, and the reaction mixture was cooled to 0-5 °C in an ice-water bath, followed by addition of potassium *tert*-butoxide (79 mg, 0.704 mmol). The ice bath was removed, and the reaction mixture was warmed to room temperature and stirred for 10 min, followed by addition of 20 mL of ice water and by extraction with ethyl acetate (10 mL × 4). The organic phase was washed with saturated sodium chloride solution (20 mL), dried over anhydrous sodium sulfate, and filtered, and the filtrate was concentrated under reduced pressure. The resulting residue was purified by silica gel column chromatography with developing solvent system C to give the title product **14b** (50 mg, 26.8% yield).
MS m/z (ESI): 529.2 [M+1].

Step 3

10-(Cyclopropylmethyl)-1-(9*H*-fluoren-9-yl)-3,6-dioxo-2,9-dioxa-4,7-diazaundecan-11-oic acid **14c**

Compound **14b** (27 mg, 0.051 mmol) was dissolved in 3 mL of ethyl acetate, and palladium on carbon (7 mg, 10% loading, dry basis) was added. The system was purged with hydrogen three times. The reaction mixture was stirred at room temperature for 1 h and filtered through celite, and the filter cake was rinsed with ethyl acetate. The filtrate was concentrated to give the crude title product **14c** (23 mg), which was directly used in the next step without purification.
MS m/z (ESI): 439.1 [M+1].

Step 4

(9*H*-fluoren-9-yl)methyl(2-((((3-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1-oxopropan-2-yl)oxy)methyl)amino)-2-oxoethyl)carbamate **14d**

Compound **1b** (22 mg, 42.38 μmol) was added to a reaction flask, and 3 mL of *N,N*-dimethylformamide was added. The system was purged with argon three times, and the mixture was cooled to 0-5 °C in an ice-water bath, followed by dropwise addition of triethylamine (4.3 mg, 42.49 μmol) and addition of crude **14c** (23 mg, 51.1 μmol) and 4-(4,6-dimethoxy-1,3,5-triazin-2-yl)-4-methylmorpholinium chloride (17.6 mg, 63.6 μmol). The reaction mixture was stirred in an ice bath for 40 min. 15 mL of water was added, followed by extraction with ethyl acetate (8 mL × 3). The organic phases were combined, washed with saturated sodium chloride solution (15 mL), dried over anhydrous sodium sulfate, and filtered. The filtrate was concentrated under reduced pressure. The resulting residue was purified by thin layer chromatography with developing solvent system B to give the title product **14d** (29 mg, 79.9% yield).
MS m/z (ESI): 856.1 [M+1].

Step 5

2-((2-Aminoacetylamino)methoxy)-3-cyclopropyl-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)propanamide **14e**

Compound **14d** (29 mg, 33.9 μmol) was dissolved in 0.8 mL of dichloromethane, and 0.4 mL of diethylamine was added. The reaction mixture was stirred at room temperature for 1.5 h and concentrated under reduced pressure.

1 mL of toluene was added, followed by concentration under reduced pressure; the procedures were repeated twice. The residue was slurried with 3 mL of *n*-hexane and let stand. Then, the supernatant was removed; and the procedures were repeated three times. The residue was concentrated under reduced pressure and dried using an oil pump to give the crude title product **14e** (22 mg), which was directly used in the next step without purification.

MS m/z (ESI): 634.1 [M+1].

Step 6

*N*-((2*R*,10*S*)-10-benzyl-2-(cyclopropylmethyl)-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8, 11, 14-tetraazahexadec-16-yl)-6-(2,5-dioxo-2,5-dihydro-1*H*-pyrrol-1-yl)hexanamide **14-A**

*N*-((2*S*,10*S*)-10-benzyl-2-(cyclopropylmethyl)-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8, 11, 14-tetraazahexadec-16-yl)-6-(2,5-dioxo-2,5-dihydro-1*H*-pyrrol-1-yl)hexanamide **14-B**

Crude **14e** (22 mg, 33.9 μmol) was dissolved in 2.5 mL of *N*,*N*-dimethylformamide. The system was purged with argon three times, and the solution was cooled to 0-5 °C in an ice-water bath, and **8g** (24 mg, 50.8 μmol) and 4-(4,6-dimethoxy-1,3,5-triazin-2-yl)-4-methylmorpholinium chloride (14 mg, 50.6 μmol) were added successively. Then, the ice bath was removed, and the reaction mixture was warmed to room temperature and stirred for 1 h to produce compound **14**. The reaction mixture was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min) to give the title products (2 mg, 2 mg).

MS m/z (ESI): 1088.4 [M+1].

Single-configuration compound (shorter retention time):
UPLC analysis: retention time: 1.18 min; purity: 88% (chromatography column: ACQUITY UPLC BEHC18 1.7 μm 2.1 × 50 mm; mobile phase: A-water (5 mmol of $NH_4OAc$), B-acetonitrile).

Single-configuration compound (longer retention time):
UPLC analysis: retention time: 1.23 min; purity: 96% (chromatography column: ACQUITY UPLC BEHC18 1.7 μm 2.1 × 50 mm; mobile phase: A-water (5 mmol of $NH_4OAc$), B-acetonitrile).

**Example 1-15**

1-((*S*)-9-benzyl-22-(2,5-dioxo-2,5-dihydro-1*H*-pyrrol-1-yl)-5,8,11,14,17-pentaoxo-2-oxa-4,7,10,13,16-pentaazadocosyl)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3,4':6,7]indolizino[1,2-*b*]quinolin-1-yl)cyclopropane-1-carboxamide **15**

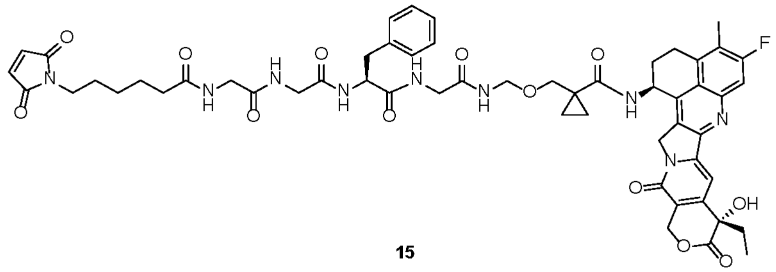

**15**

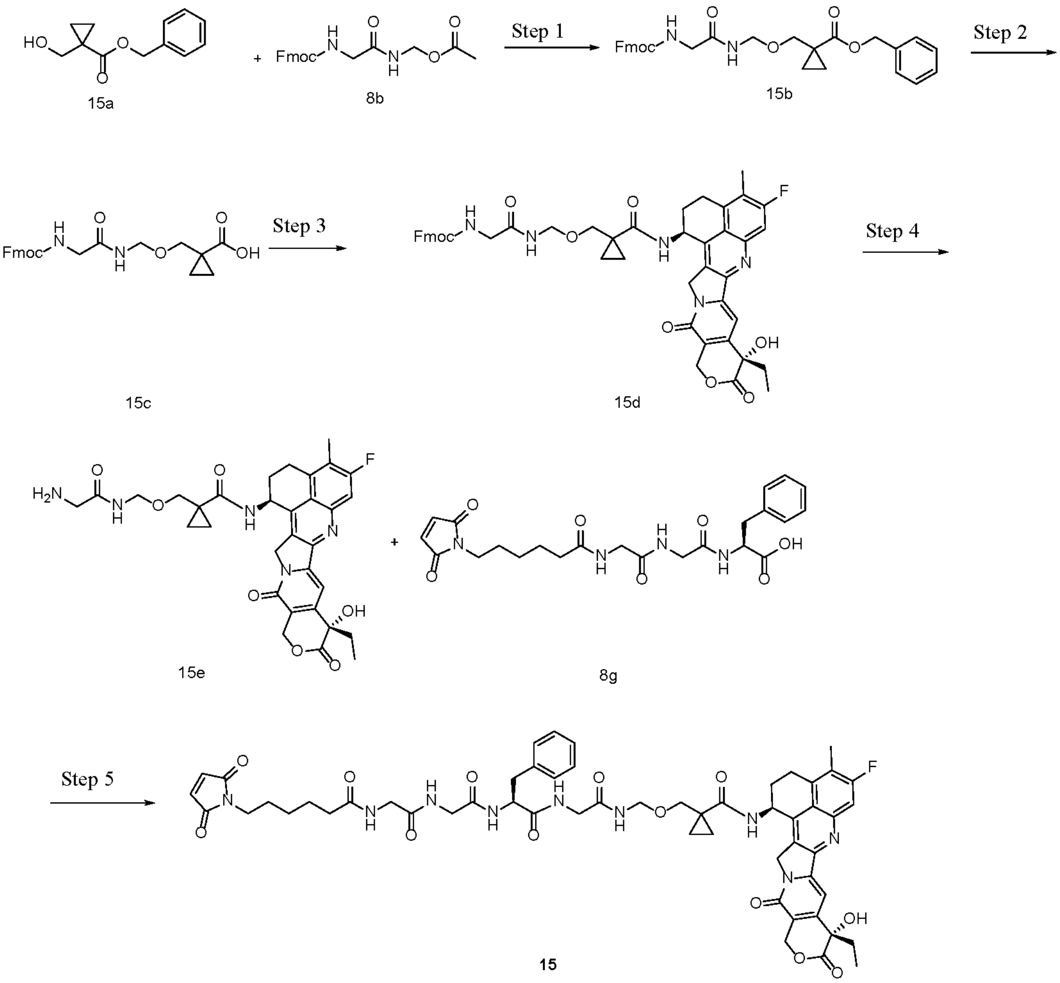


15a 8b 15b 15c 15d 15e 8g 15

Step 1

Benzyl 1-(10-(9*H*-fluoren-9-yl)-5,8-dioxo-2,9-dioxa-4,7-diazadecyl)cyclopropane-1-carboxylate **15b**

Compound **8b** (500 mg, 1.35 mmol) was added to a reaction flask, followed by addition of 6 mL of tetrahydrofuran, benzyl 1-hydroxymethylcyclopropane-1-carboxylate **15a** (233 mg, 1.13 mmol; prepared as disclosed in "Example 22-2 on page 262 of the specification of Patent Application EP2862856A1"). The system was purged with argon three times, and the reaction mixture was cooled to 0-5 °C in an ice-water bath, followed by addition of sodium hydride (54 mg, 1.35 mmol). Then, the ice-bath was removed, and the reaction mixture was warmed to room temperature, stirred for 40 min, and cooled to 0 °C, followed by addition of 20 mL of ice water and by extraction with ethyl acetate (5 mL × 2) and chloroform (5 mL × 5). The organic phases were combined, washed with saturated sodium chloride solution (20 mL), dried over anhydrous sodium sulfate, and filtered, and the filtrate was concentrated under reduced pressure. The resulting residue was purified by silica gel column chromatography with developing solvent system B to give the title product **15b** (15 mg, 2.5% yield).

MS m/z (ESI): 515.2 [M+1].

Step 2

1-(10-(9*H*-fluoren-9-yl)-5,8-dioxo-2,9-dioxa-4,7-diazadecyl)cyclopropane-1-carboxylic acid **15c**

Compound **15b** (15 mg, 0.029 mmol) was dissolved in 2 mL of ethyl acetate, and palladium on carbon (3 mg, 10% loading, dry basis) was added. The system was purged with hydrogen three times, and the reaction mixture was stirred at room temperature for 4.5 h and filtered through celite, and the filter cake was rinsed with ethyl acetate. The filtrate was concentrated to give the title product **15c** (11 mg, 89% yield).

MS m/z (ESI): 425.2 [M+1].

Step 3

(9*H*-fluoren-9-yl)methyl(2-((((1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)cyclopropyl)methoxy)methyl)amino)2-oxoethyl)carbamate **15d**

Compound **1b** (10 mg, 0.021 mmol) was added to a reaction flask, and 1 mL of *N,N*-dimethylformamide was added. The system was purged with argon three times, and the mixture was cooled to 0-5 °C in an ice-water bath, followed by addition of a drop of triethylamine, compound **15c** (11 mg, 0.026 mmol) and 4-(4,6-dimethoxy-1,3,5-triazin-2-yl)-4-methylmorpholinium chloride (10.7 mg, 0.039 mmol). After addition, the reaction mixture was stirred at room temperature for 60 min. 10 mL of water was added, followed by extraction with ethyl acetate (5 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **15d** (19 mg, 87.0% yield).

MS m/z (ESI): 842.2 [M+1].

Step 4

1-(((2-Aminoacetylamino)methoxy)methyl)-*N*-((1*S*,9*S*)-9-ethyl-5-fluoro-9-hydroxy-4-methyl-10,13-dioxo-2,3,9,10,13,15-hexahydro-1*H*,12*H* benzo[*de*]pyrano[3',4':6,7]indolizino[1,2-*b*]quinolin-1-yl)cyclopropane-1-carboxamide **15e**

Compound **15d** (19 mg, 22.56 μmol) was dissolved in 2 mL of dichloromethane, and 1 mL of diethylamine was added. The reaction mixture was stirred at room temperature for 1.5 h and concentrated at 0 °C under reduced pressure. 1 mL of toluene was added, followed by concentration under reduced pressure; the procedures were repeated twice. The residue was slurried with 3 mL of n-hexane, and the upper n-hexane layer was removed; the procedures were repeated three times. The slurry was concentrated under reduced pressure to give the crude title product **15e** (13.9 mg), which was directly used in the next step without purification.

MS m/z (ESI): 620.1 [M+1].

Step 5

1-((*S*)-9-benzyl-22-(2,5-dioxo-2,5-dihydro-1*H*-pyrrol-1-yl)-5,8,11,14,17-pentaoxo-2-oxa-4,7,10,13,16-pentaazadocosyl)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclopropane-1-carboxamide **15**

Crude **15e** (13.9 mg, 22.4 μmol) was dissolved in 1 mL of *N,N*-dimethylformamide. The system was purged with argon three times, and the solution was cooled to 0-5 °C in an ice-water bath, followed by addition **of 8g** (15.8 mg, 33.4 μmol) and 4-(4,6-dimethoxy-1,3,5-triazin-2-yl)-4-methylmorpholinium chloride (9.3 mg, 33.6 μmol). The reaction mixture was warmed to room temperature, stirred for 60 min, and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **15** (2.5 mg, 10.3% yield).

MS m/z (ESI): 1074.2 [M+1].

$^1H$ NMR (400 MHz, DMSO-$d_6$): δ 8.51-8.37 (m, 1H), 8.22 (t, 1H), 8.14-8.02 (m, 2H), 8.011-7.94 (m, 1H), 7.82-7.73 (m, 1H), 7.29 (s, 1H), 7.26-7.10 (m, 3H), 6.98 (s, 1H), 6.53-6.47 (m, 1H), 5.62-5.50 (m, 1H), 5.45-5.36 (m, 1H), 5.35-5.23 (m, 2H), 5.13-5.02 (m, 2H), 4.61-4.50 (m, 2H), 4.42-4.28 (m, 2H), 3.76-3.61 (m, 3H), 3.60-3.45 (m, 3H), 3.27-3.23 (m, 1H), 3.20-2.81 (m,7H), 2.75-2.61 (m, 3H), 241-2.28 (m, 3H), 2.23-2.13 (m, 2H), 2.11-2.01 (m, 1H), 2.03-1.94 (m, 1H), 1.90 (s, 1H), 1.87-1.74 (m, 2H), 1.53-1.36 (m, 3H), 1.29-1.08 (m, 4H), 0.90-0.68 (m, 4H).

**Example 1-16**

1-((*S*)-9-benzyl-22-(2,5-dioxo-2,5-dihydro-1*H*-pyrrol-1-yl)-5,8,11,14,17-pentaoxo-2-oxa-4,7,10,13,16-pentaazadocosyl)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclobutane-1-carboxamide **16**

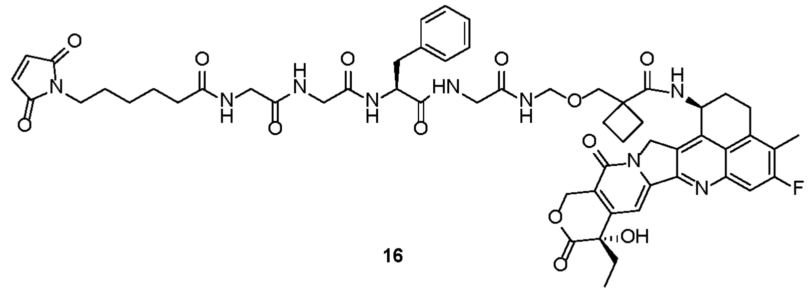

16

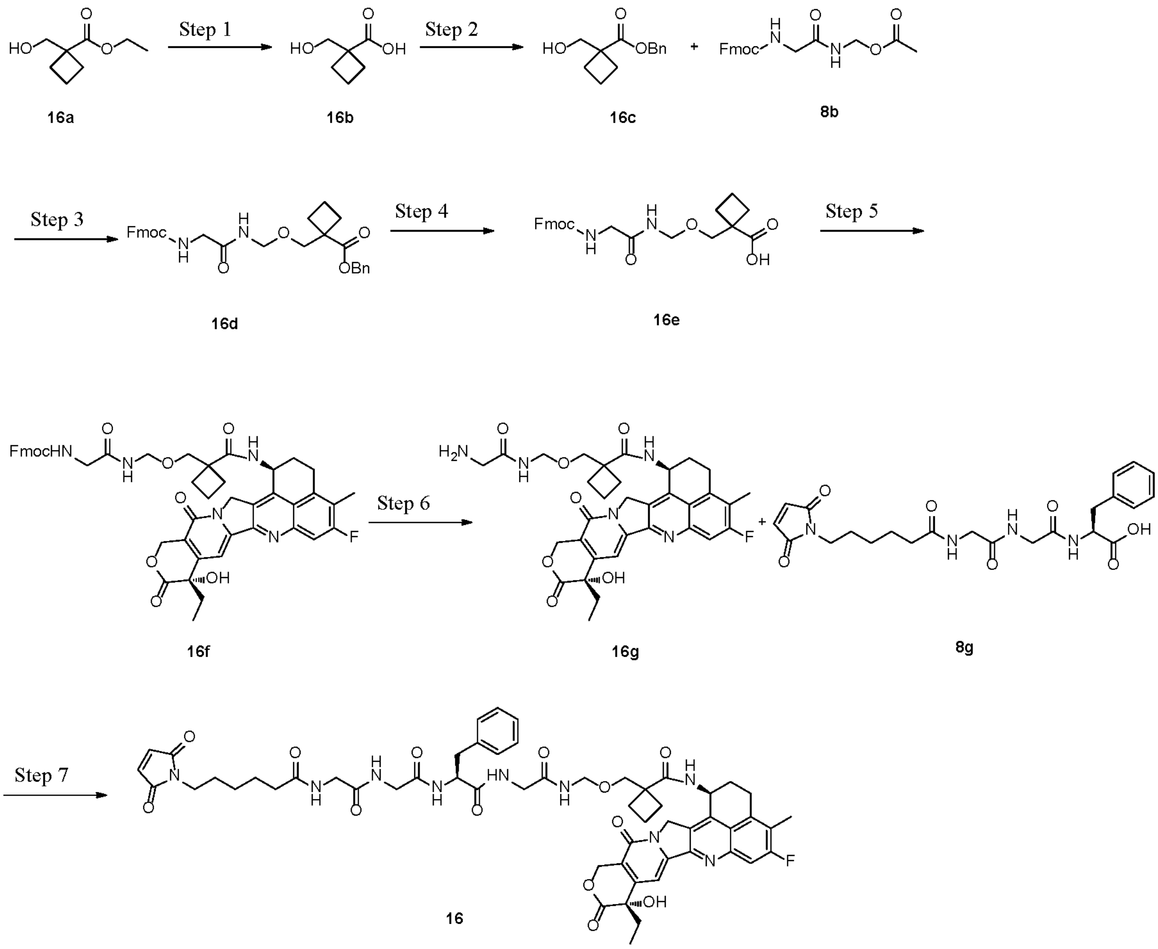


16a 16b 16c 8b

16d 16e

16f 16g 8g

16

Step 1

1-(Hydroxymethyl)cyclobutane-1-carboxylic acid **16b**

Ethyl 1-(hydroxymethyl)cyclobutanecarboxylate **16a** (250 mg, 1.58 mmol, supplied by Alfa) was dissolved in methanol (2 mL) and water (1 mL), and sodium hydroxide (126 mg, 3.15 mmol) was added. The reaction mixture was warmed to 40 °C, stirred for 3 h, cooled to room temperature, and concentrated under reduced pressure to remove the organic solvent. The reaction mixture was reversely extracted with diethyl ether (10 mL) to collect the aqueous phase. The aqueous phase was adjusted to pH 3-4 with 6 N aqueous hydrochloric acid and concentrated under reduced pressure to give a solid. 3 mL of toluene was added, followed by concentration under reduced pressure to dryness; the procedures were repeated three times. The residue was dried using an oil pump to give the crude title product **16b** (206 mg), which was directly used in the next step without purification.

MS m/z (ESI, NEG): 129.2 [M-1].

Step 2

Benzyl 1-(hydroxymethyl)cyclobutane-1-carboxylate **16c**

Crude **16b** (206 mg, 1.58 mmol) was dissolved in acetonitrile (15 mL), and anhydrous potassium carbonate (1.09 g, 7.90 mmol), tetrabutylammonium iodide (29 mg, 78.51 μmol) and benzyl bromide (216 mg, 1.26 mmol) were added. The reaction mixture was stirred at room temperature overnight and filtered. The filtrate was concentrated under reduced pressure, and the resulting residue was purified by silica gel column chromatography with developing solvent system C to give the title product **16c** (112 mg, 32.1% yield).
MS m/z (ESI): 221.1 [M+1].

Step 3

Benzyl 1-(10-(9*H*-fluoren-9-yl)-5,8-dioxo-2,9-dioxa-4,7-diazadecyl)cyclobutane-1-carboxylate **16d**

Compounds **16c** (77 mg, 0.35 mmol) and **8b** (100 mg, 0.27 mmol) were added to a reaction flask, and 3 mL of tetrahydrofuran was added. The system was purged with argon three times, and the reaction mixture was cooled to 0-5 °C in an ice-water bath, followed by addition of potassium *tert*-butoxide (61 mg, 0.54 mmol). The reaction mixture was stirred in the ice bath for 10 min. 20 mL of ice water was added, followed by extraction with ethyl acetate (5 mL) and chloroform (5 mL × 5). The organic phases were combined and concentrated. The resulting residue was dissolved in 3 mL of 1,4-dioxane, and 0.5 mL of water, sodium bicarbonate (27 mg, 0.32 mmol) and 9-fluorenylmethyl chloroformate (71 mg, 0.27 mmol) were added. The reaction mixture was stirred at room temperature for 1 h. 20 mL of water was added, followed by extraction with ethyl acetate (10 mL × 3). The organic phase was washed with saturated sodium chloride solution (20 mL), dried over anhydrous sodium sulfate, and filtered. The filtrate was concentrated under reduced pressure, and the resulting residue was purified by silica gel column chromatography with developing solvent system C to give the title product **16d** (24 mg, 16.7% yield).
MS m/z (ESI): 551.3 [M+23].

Step 4

1-(10-(9*H*-fluoren-9-yl)-5,8-dioxo-2,9-dioxa-4,7-diazadecyl)cyclobutane-1-carboxylic acid **16e**

Compound **16d** (12 mg, 22.7 μmol) was dissolved in 1.5 mL of a solvent mixture of tetrahydrofuran and ethyl acetate (V:V = 2:1), and palladium on carbon (5 mg, 10% loading) was added. The system was purged with hydrogen three times, and the reaction mixture was stirred at room temperature for 2 h. The reaction mixture was filtered through celite, and the filter cake was rinsed with ethyl acetate. The filtrate was concentrated under reduced pressure to give the crude title product **16e** (10 mg), which was directly used in the next step without purification.

Step 5

(9*H*-fluoren-9-yl)methyl(2-((((1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)cyclobutyl)methoxy)methyl)amino)-2-oxoethyl)carbamate **16f**

Compound **1b** (7.5 mg, 0.014 mmol) was added to a reaction flask, and 1 mL of *N,N*-dimethylformamide was added. The system was purged with argon three times, and the mixture was cooled to 0-5 °C in an ice-water bath, followed by addition of a drop of triethylamine, a solution of crude **16e** (10 mg) in 0.5 mL of *N,N*-dimethylformamide, and 4-(4,6-dimethoxy-1,3,5-triazin-2-yl)-4-methylmorpholinium chloride (6 mg, 0.026 mmol). The reaction mixture was stirred in an ice bath for 30 min. 10 mL of water was added, followed by extraction with ethyl acetate (10 mL × 3). The organic phase was washed with saturated sodium chloride solution (10 mL), dried over anhydrous sodium sulfate, and filtered. The filtrate was concentrated under reduced pressure, and the resulting residue was purified by thin layer chromatography with developing solvent system B to give the title product **16f** (10.6 mg, 87.8% yield).
MS m/z (ESI): 856.2 [M+1].

Step 6

1-(((2-Aminoacetylamino)methoxy)methyl)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclobutane-1-carboxamide 16g

Compound **16f** (10.6 mg, 12.4 μmol) was dissolved in 0.6 mL of dichloromethane, and 0.3 mL of diethylamine was added. The reaction mixture was stirred at room temperature for 2 h and concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The residue was concentrated under reduced pressure to give the crude title product **16g** (8 mg), which was directly used in the next step without purification.
MS m/z (ESI): 634.1 [M+1].

Step 7

1-((*S*)-9-benzyl-22-(2,5-dioxo-2,5-dihydro-1*H*-pyrrol-1-yl)-5,8,11,14,17-pentaoxo-2-oxa-4,7,10,13,16-pentaazadocosyl)-*N*-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)cyclobutane-1-carboxamide **16**

Crude **16g** (8 mg) was dissolved in 1 mL of *N*,*N*-dimethylformamide, and **8g** (8.8 mg, 18.6 μmol) and 4-(4,6-dimethoxy-1,3,5-triazin-2-yl)-4-methylmorpholinium chloride (5.2 mg, 18.8 μmol) were added. The reaction mixture was stirred at room temperature for 30 min and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min) to give the title product **16** (1.0 mg, 7.2% yield).
MS m/z (ESI): 1088.0 [M+1].

**Example 1-17**

(1*r*,4*r*)-*N*((*S*)-7-benzyl-1-(1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)cyclopropoxy)-3,6,9,12,15-pentaoxo-17,20,23,26,29,32,35,38,41-nonaoxa-2,5,8, 11, 14-pentaazatetratridec-43-yl)-4-((2,5-dioxo-2,5-dihydro-1H-pyrrol-1-yl)methyl)cyclohexane-1-carboxamide 17

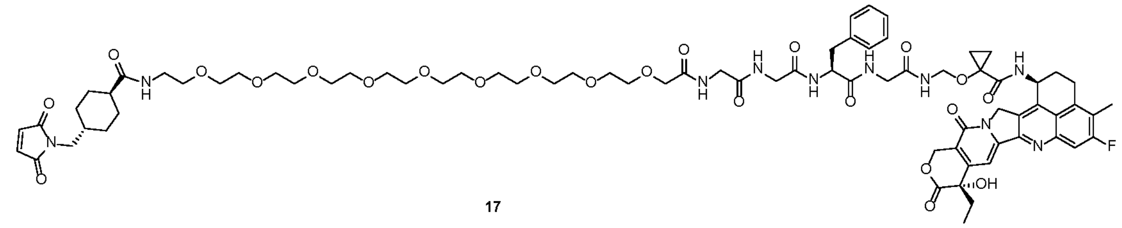

17

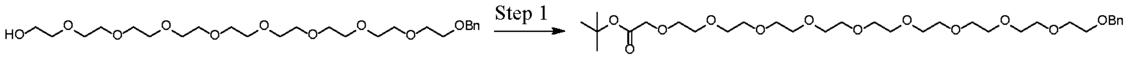


17a 17b

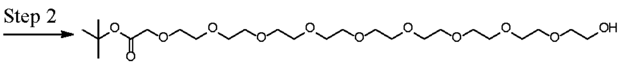


17c

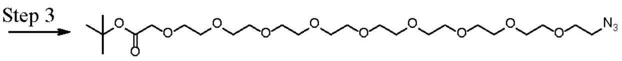


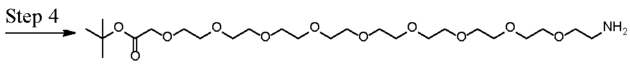


17d

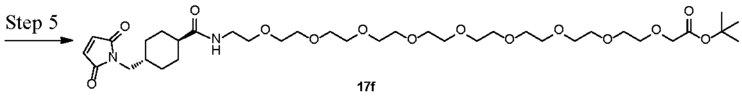


17f

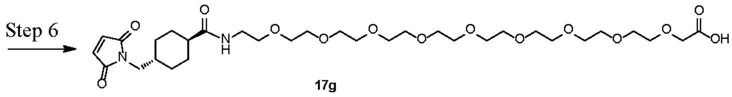


17g

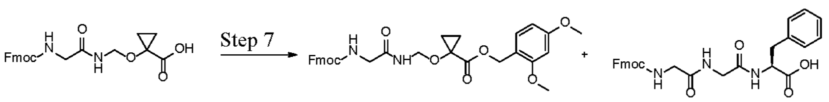


8d 17h 17i

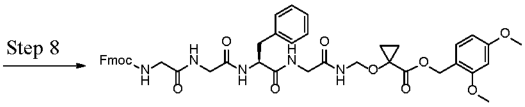


17j

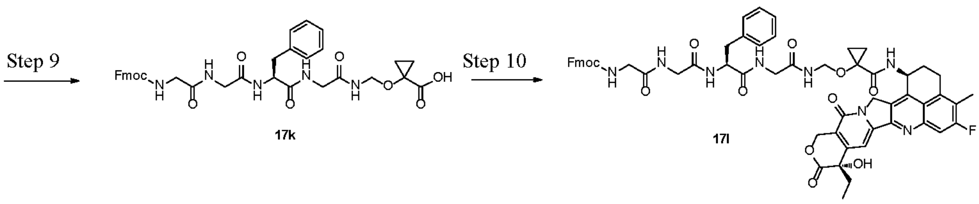


17k 17l

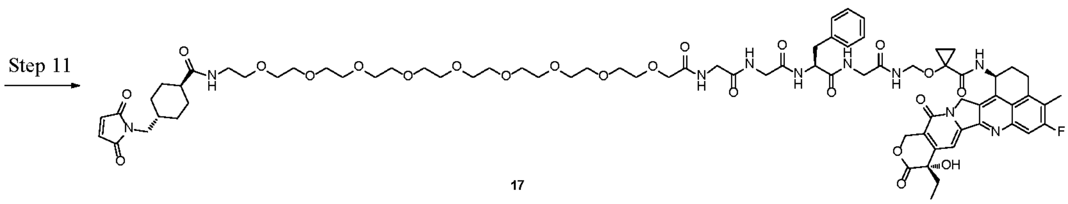


17

Step 1

tert-Butyl 1-phenyl-2,5,8,11,14,17,20,23,26,29-decaoxahentriacontane-31-oate 17b

1-Phenyl-2,5,8,11,14,17,20,23,26-nonaoxaoctacosane-28-ol 17a (0.34 g, 0.67 mmol, supplied by Bide) was

dissolved in 10 mL of dichloromethane, and silver oxide (0.24 g, 1.01 mmol), *tert*-butyl bromoacetate (0.16 g, 0.81 mmol) and potassium iodide (0.07 g, 0.40 mmol) were successively added. The reaction mixture was stirred at room temperature for 3 h, and filtered. The filtrate was concentrated under reduced pressure, and the resulting residue was purified by silica gel column chromatography with developing solvent system B to give the title product **17b** (0.42 g, 100% yield).

MS m/z (ESI): 636.3 [M+18].

Step 2

*tert*-Butyl 29-hydroxy-3,6,9,12,15,18,21,24,27-nonaoxanonacosane-1-oate **17c**

Compound **17b** (417 mg, 0.67 mmol) was dissolved in 15 mL of tetrahydrofuran, and palladium on carbon (110 mg, 10% loading, dry basis) was added. The system was purged with hydrogen three times, and the reaction mixture was warmed to 60 °C, stirred for 3 h, filtered through celite, and the filter cake was rinsed with tetrahydrofuran. The filtrate was concentrated to give the crude title product **17c** (357 mg), which was directly used in the next step without purification.

MS m/z (ESI): 546.2 [M+18].

Step 3

*tert*-Butyl 29-azido-3,6,9,12,15,18,21,24,27-nonaoxanonacosane-1-oate **17d**

Compound **17c** (357 mg, 0.675 mmol) was dissolved in 10 mL of toluene, and diphenyl phosphoryl azide (279 mg, 1.014 mmol) and 1,8-diazabicycloundec-7-ene (206 mg, 1.353 mmol) were added. The system was purged with argon three times, and the reaction mixture was stirred at room temperature for 2 h, then warmed to 105 °C, reacted for 19 h, cooled to room temperature and concentrated. 20 mL of water was added, followed by extraction with ethyl acetate (10 mL × 4). The organic phase was washed with saturated sodium chloride solution (20 mL), dried over anhydrous sodium sulfate, and filtered. The filtrate was concentrated under reduced pressure. The resulting residue was purified by silica gel column chromatography with developing solvent system B to give the crude title product **17d** (412 mg).

MS m/z (ESI): 571.3 [M+18].

Step 4

*tert*-Butyl 29-amino-3,6,9,12,15,18,21,24,27-nonaoxanonacosane-1-oate **17e**

Compound **17d** (230 mg, 0.415 mmol) was dissolved in 8 mL of tetrahydrofuran, and palladium on carbon (58 mg, 10% loading, dry basis) was added. The system was purged with hydrogen three times. The reaction mixture was stirred at room temperature for 2 h and filtered through celite, and the filter cake was rinsed with tetrahydrofuran. The filtrate was concentrated to give the crude title product **17e** (220 mg), which was directly used in the next step without purification.

MS m/z (ESI): 528.2 [M+1].

Step 5

*tert*-Butyl 1-((1*r*,4*r*)-4-((2,5-dioxo-2,5-dihydro-1*H*-pyrrol-1-yl)methyl)cyclohexyl)-1-oxo-5,8,11,14,17,20,23,26,29-nonaoxa-2-azatriundec-31-oate **17f**

(*1*r,4*r*)-4-((2,5-dioxo-2,5-dihydro-1*H*-pyrrol-1-yl)methyl)cyclohexane-1-carboxylic acid (98.5 mg, 0.415 mmol) was dissolved in 10 mL of dichloromethane, and 2-(7-benzotriazol oxide)-*N*,*N*,*N*',*N*'-tetramethyluronium hexafluorophosphate (190 mg, 0.500 mmol) and *N*,*N*-diisopropylethylamine (162 mg, 1.253 mmol) were added. The system was purged with argon three times, and crude **17e** (220 mg, 0.417 mmol) was added. The reaction mixture was stirred at room temperature for 1 h. 15 mL of water was added, followed by extraction with dichloromethane (8 mL × 3). The organic phases were combined, washed with saturated sodium chloride solution (15 mL), dried over anhydrous sodium sulfate, and filtered. The filtrate was concentrated under reduced pressure. The resulting residue was purified by silica gel column chromatography with developing solvent system B to give the title product **17f** (122 mg, 39.2% yield).

MS m/z (ESI): 747.2 [M+1].

Step 6

1-((1*r*,4*r*)-4-((2,5-dioxo-2,5-dihydro-1*H*-pyrrol-1-yl)methyl)cyclohexyl)-1-oxo-5,8,11,14, 17,20,23,26,29-nonaoxa-2-azatriundec-31-oic acid **17g**

Compound **17f** (122 mg, 0.163 mmol) was dissolved in 0.8 mL of dichloromethane, and 0.4 mL of trifluoroacetic acid was added. The reaction mixture was stirred at room temperature for 1 h, diluted with 15 mL of dichloromethane, and concentrated under reduced pressure; 10 mL of *n*-hexane was added, followed by concentration under reduced pressure; the procedures were repeated twice. Then 10 mL of toluene was added, and the resulting mixture was concentrated under reduced pressure, slurried three times with 10 mL of a solvent mixture of *n*-hexane:diethyl ether = 5:1 until the pH reached 7, concentrated, and dried with an oil pump to give the title product **17g** (98 mg, 86.8% yield).
MS m/z (ESI): 691.2 [M+1].

Step 7

2,4-Dimethoxybenzyl 1-((2-((((9*H*-fluoren-9-yl)methoxy)carbonyl)amino)acetylamino)methoxy)cyclopropyl-1-carboxylate **17h**

Compound **8d** (164 mg, 0.40 mmol) was dissolved in dichloromethane (5 mL), and 2,4-dimethoxybenzyl alcohol (81 mg, 0.48 mmol), 1-ethyl-(3-dimethylaminopropyl)carbonyldiimine hydrochloride (115 mg, 0.60 mmol) and 4-dimethylaminopyridine (5 mg, 0.041 mmol) were added successively. After addition, the reaction mixture was stirred at room temperature for 1 h. 20 mL of water was added, and layers formed after shaking. The aqueous phase was extracted with dichloromethane (8 mL × 3). The organic phases were combined, washed with saturated sodium chloride solution (20 mL), dried over anhydrous sodium sulfate, and filtered. The filtrate was concentrated under reduced pressure. The resulting residue was purified by silica gel column chromatography with developing solvent system C to give the title product **17h** (124 mg, 55.4% yield).
MS m/z (ESI): 583.1 [M+23].

Step 8

2,4-Dimethoxybenzyl (*S*)-1-((11-benzyl-1-(9*H*-fluoren-9-yl)-3,6,9,12,15-pentaoxo-2-oxa-4,7,10,13,16-pentaazaheptadecane-17-yl)oxy)cyclopropyl-1 -carboxylate **17j**

Compound **17h** (39 mg, 69.6 μmol) was dissolved in 0.6 mL of dichloromethane, and 0.3 mL of diethylamine was added. The reaction mixture was stirred at room temperature for 1 h and concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times, followed by concentration under reduced pressure. The resulting crude product was dissolved in 2 mL of *N*,*N*-dimethylformamide, and (((9*H*-fluoren-9-yl)methoxy)carbonyl)glycyl-L-phenylalanine **17i** (35 mg, 69.8 μmol, prepared as disclosed in "Example 7-12 on page 13 of the specification of Patent Application CN108853514A") and 4-(4,6-dimethoxy-1,3,5-triazin-2-yl)-4-methylmorpholinium chloride (23 mg, 83.1 μmol) were added. The reaction mixture was stirred at room temperature for 1 h. 10 mL of water was added, followed by extraction with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **17j** (48 mg, 83.9% yield).
MS m/z (ESI): 822.0 [M+1].

Step 9

(*S*)-1-((11-benzyl-1-(9*H*-fluoren-9-yl)-3,6,9,12,15-pentaoxo-2-oxa-4,7,10,13,16-pentaazaheptadecane-17-yl)oxy)cyclopropane-1-carboxylic acid **17k**

Compound **17j** (48 mg, 58.4 μmol) was dissolved in 1.4 mL of a 3% (v/v) solution of dichloroacetic acid in dichloromethane, and the solution was cooled to 0-5 °C in an ice-water bath, followed by addition of triethylsilane (21 mg, 180.6 μmol). The reaction mixture was stirred in the ice bath for 3 h. The reaction mixture was concentrated under reduced pressure in the ice bath to remove half of the organic solvent. 5 mL of diethyl ether was added, and the resulting mixture was warmed to room temperature naturally and slurried to precipitate a white solid, and filtered. The filter cake was collected and dried with an oil pump to give the title product **17k** (33 mg, 84.1% yield).

Step 10

(9*H*-fluoren-9-yl)methyl((*S*)-7-benzyl-1-(1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)cyclopropoxy)-3,6,9,12-tetraoxo-2,5,8,11-tetraazatridecan-13-yl)carbamate **17l**

Compound **1b** (20 mg, 42.4 μmol) was added to a reaction flask, and 1 mL of a 10% (v/v) solution of methanol in dichloromethane was added. The system was purged with argon three times, and the mixture was cooled to 0-5 °C in an ice-water bath, followed by addition of a drop of triethylamine. The mixture was stirred until compound **1b** was dissolved. Compound **17k** (33 mg, 49.1 μmol) was dissolved in 1 mL of a 10% (v/v) solution of methanol in dichloromethane, and then the above reaction mixture was added dropwise, followed by addition of 4-(4,6-dimethoxy-1,3,5-triazin-2-yl)-4-methylmorpholinium chloride (17.6 mg, 63.6 μmol). The reaction mixture was warmed to room temperature and stirred for 1 h. 10 mL of dichloromethane and 5 mL of water were added, and the resulting mixture was stirred for 5 min, and let stand to form layers. The organic phase was collected, and the aqueous phase was extracted with dichloromethane (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **17l** (37 mg, 80.2% yield).

MS m/z (ESI): 1090.1 [M+1].

Step 11

(1*r*,4*r*)-*N*-((*S*)-7-benzyl-1-(1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)aminocarbonyl)cyclopropoxy)-3,6,9,12,15-pentaoxo-17,20,23,26,29,32,35,38,41-nonaoxa-2,5,8,11,14-pentaazatetratridec-43-yl)-4-((2,5-dioxo-2,5-dihydro-1*H*-pyrrol-1-yl)methyl)cyclohexane-1-carboxamide **17**

Compound **17l** (15.5 mg, 14.23 μmol) was dissolved in 0.6 mL of dichloromethane, and 0.3 mL of diethylamine was added. The reaction mixture was stirred at room temperature for 1.5 h and concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The reaction mixture was concentrated under reduced pressure and then dried with an oil pump. The resulting crude product was dissolved in 1 mL of *N*,*N*-dimethylformamide, and compound **17g** (11 mg, 15.92 μmol) and 4-(4,6-dimethoxy-1,3,5-triazin-2-yl)-4-methylmorpholinium chloride (6.0 mg, 21.68 μmol) were added. The system was purged with argon three times, and the reaction mixture was stirred at room temperature for 30 min and purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **17** (6 mg, 27.4% yield).

MS m/z (ESI): 1556.4 [M+18].

$^1H$ NMR (400 MHz, DMSO-$d_6$): δ 8.98 (d, 1H), 8.76 (s, 1H), 8.20 (br, 1H), 8.12-7.95 (m, 3H), 7.93-7.76 (m, 2H), 7.75-7.66 (m, 2H), 7.24 (s, 1H), 7.20-7.05 (m, 6H), 6.97 (s, 1H), 6.64 (br, 1H), 6.55 (d, 1H), 6.47 (s, 1H), 5.61-5.52 (m, 2H), 5.37 (s, 1H), 5.33-5.23 (m, 2H), 5.18 (s, 1H), 5.13 (s, 1H), 5.05 (s, 1H), 5.00 (s, 1H), 4.65-4.55 (m, 2H), 4.53-4.45 (m, 1H), 4.38-4.28 (m, 2H), 3.84 (s, 2H), 3.67 (d, 3H), 3.60-3.40 (m, 33H), 3.18 (d, 1H), 3.15-3.08 (m, 3H), 2.28 (s, 3H), 2.00-1.92 (m, 3H), 1.85 (s, 2H), 1.82-1.73 (m, 2H), 1.68-1.52 (m, 4H), 1.29-1.15 (m, 3H), 0.86-0.76 (m, 5H).

**Example 1-18**

(1*r*,4*r*)-*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15,18-hexaoxo-3,20,23,26,29,32,35,38,41,44-decaoxa-5,8,11,14,17-pentaaza-tetrahexadec-46-yl)-4-((2,5-dioxo-2,5-dihydro-1*H*-pyrrol-1-yl)methyl)cyclohexane-1-carboxamide **18**

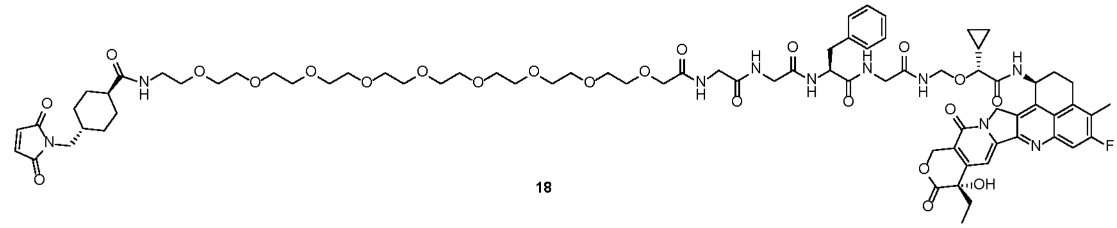

18

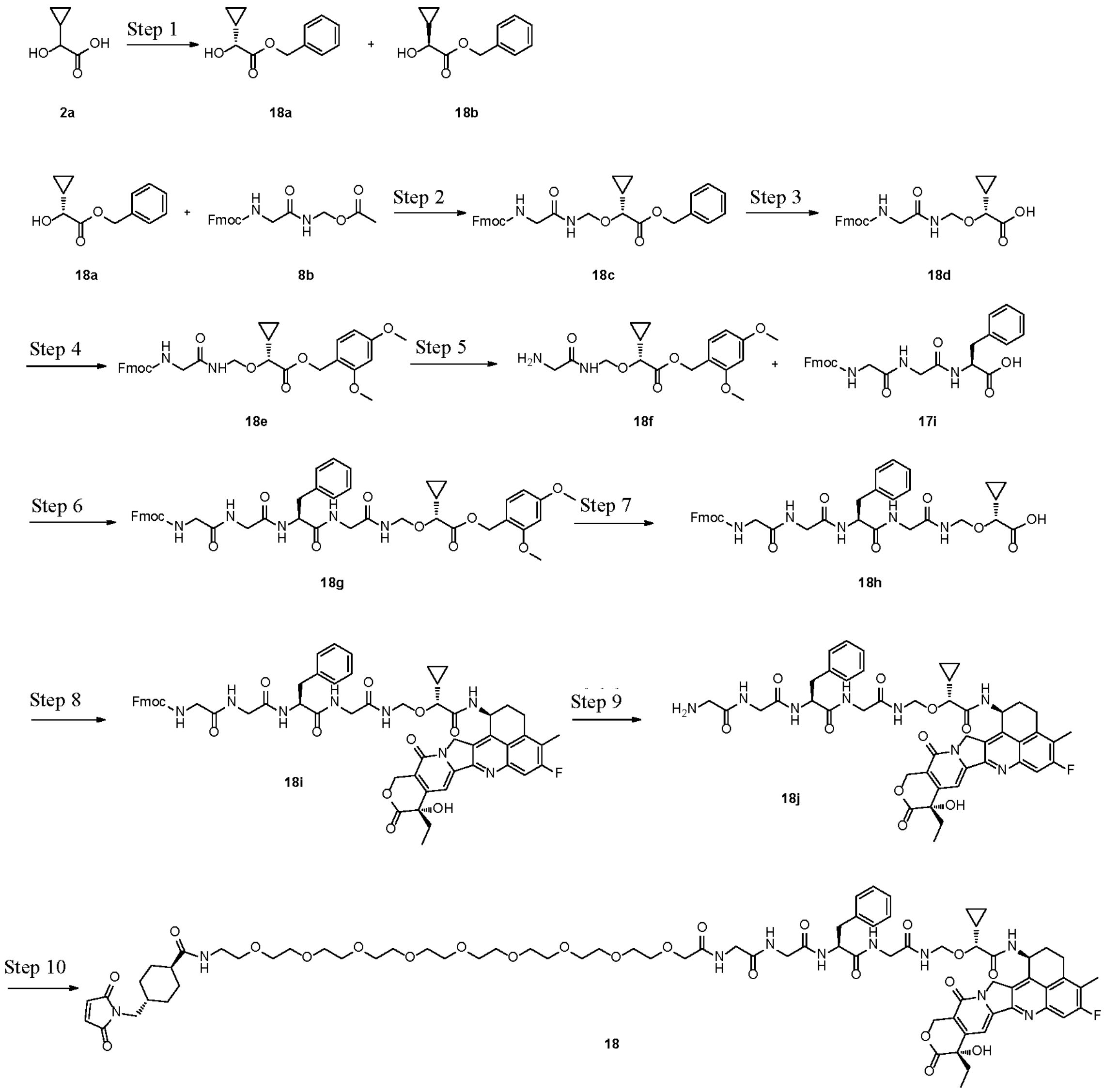


2a 18a 18b

18a 8b 18c 18d

18e 18f 17i

18g 18h

18i 18j

18

Step 1

Benzyl (R)-2-cyclopropyl-2-hydroxyacetate **18a**

Benzyl (S)-2-cyclopropyl-2-hydroxyacetate **18b**

Compound **2a** (7.4 g, 63.7 mmol) was dissolved in 200 mL of acetonitrile, and potassium carbonate (35 g, 253.6 mmol), benzyl bromide (9.3 g, 54.4 mmol) and tetrabutylammonium iodide (500 mg, 1.36 mmol) were added successively. The reaction mixture was stirred at room temperature for 16 h and filtered through celite, and the filter cake was rinsed with ethyl acetate (10 mL). The filtrates were combined and concentrated under reduced pressure, and the resulting residue (4.1 g) was purified by silica gel column chromatography with developing solvent system C and further by chiral resolution to give the title products **18a** (1.1 g) and **18b** (1.2 g).

Step 2

Benzyl (R)-10-cyclopropyl-1-(9*H*-fluoren-9-yl)-3,6-dioxo-2,9-dioxa-4,7-diazaundecan-11-oate **18c**

Compound **8b** (3.1 g, 8.41 mmol) was dissolved in tetrahydrofuran (55 mL), and compound **18a** (2.0 g, 9.70 mmol) was added. The mixture was cooled to 0-5 °C in an ice-water bath, and potassium *tert*-butoxide (1.89 g, 16.84 mmol) was added. The mixture was stirred in the ice-water bath for 10 min. Ethyl acetate (30 mL) and water (20 mL) were added, and the resulting mixture was let stand to form layers. The aqueous phase was extracted with chloroform (30 mL × 5), and the organic phases were combined, and concentrated under reduced pressure. The resulting residue was dissolved in 1,4-dioxane (32 mL) and water (8 mL), and sodium carbonate (1.78 g, 16.79 mmol) and 9-fluorenylmethyl chloroformate (2.18 g, 8.42 mmol) were added. The resulting mixture was stirred at room temperature for 2 h. Water (30 mL) was added, followed by extraction with ethyl acetate (50 mL × 3). The organic phases were combined, washed with saturated sodium chloride solution (30 mL × 2), dried over anhydrous sodium sulfate, and filtered. The filtrate was concentrated under reduced pressure. The resulting residue was purified by column chromatography with developing solvent system C to give the title product **18c** (1.3 g, 30.0% yield).
MS m/z (ESI): 515.2 [M+1].

Step 3

(*R*)-10-cyclopropyl-1-(9*H*-fluoren-9-yl)-3,6-dioxo-2,9-dioxa-4,7-diazaundecan-11-oic acid **18d**

Compound **18c** (1.29 g, 2.51 mmol) was dissolved in ethyl acetate (15 mL), and palladium on carbon (260 mg, 10% loading, dry basis) was added. The system was purged with hydrogen three times, and the reaction mixture was stirred at room temperature for 5 h and filtered through celite, and the filter cake was rinsed with ethyl acetate (20 mL) and methanol (20 mL). The filtrate was concentrated to give the crude title product **18d** (980 mg), which was directly used in the next step without purification.
MS m/z (ESI): 425.1 [M+1].

Step 4

2,4-Dimethoxybenzyl(R)-10-cyclopropyl-1-(9*H*-fluoren-9-yl)-3,6-dioxo-2,9-dioxa-4,7-diazaundecan-11-ester **18e**

Crude **18d** (980 mg, 2.31 mmol) was dissolved in dichloromethane (15 mL), and 2,4-dimethoxybenzyl alcohol (777 mg, 4.62 mmol), 1-ethyl-(3-dimethylaminopropyl) carbonyldiimine hydrochloride (664 mg, 3.46 mmol) and 4-dimethylaminopyridine (28 mg, 0.23 mmol) were added. The reaction mixture was stirred at room temperature for 1 h, and concentrated under reduced pressure to remove the organic solvent. 20 mL of water was added, followed by extraction with ethyl acetate (50 mL × 3). The organic phases were combined, washed with saturated sodium chloride solution (30 mL × 2), dried over anhydrous sodium sulfate, and filtered. The filtrate was concentrated under reduced pressure. The resulting residue was purified by column chromatography with developing solvent system C to give the title product **18e** (810 mg, 61.1% yield).
MS m/z (ESI): 575.0 [M+1].

Step 5

2,4-Dimethoxybenzyl(*R*)-2-((2-aminoacetylamino)methoxy)-2-cyclopropylacetate **18f**

Compound **18e** (33 mg, 57.4 μmol) was dissolved in 0.6 mL of dichloromethane, and 0.3 mL of diethylamine was added. The reaction mixture was stirred at room temperature for 1 h and concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **18f** (21 mg), which was directly used in the next step without purification.

Step 6

2,4-Dimethoxybenzyl(11*S*,19*R*)-11-benzyl-19-cyclopropyl-1-(9*H*-fluoren-9-yl)-3,6,9,12,15-pentaoxo-2,18-dioxa-4,7,10,13,16-pentaazaicosan-20-oate **18g**

Crude **18f** (21 mg, 57.4 μmol) was dissolved in 3 mL of *N*,*N*-dimethylformamide, and compound **17i** (29 mg, 57.8 μmol) and 4-(4,6-dimethoxy-1,3,5-triazin-2-yl)-4-methylmorpholinium chloride (19 mg, 68.7 μmol) were added. The reaction mixture was stirred at room temperature for 1 h. 10 mL of water was added, followed by extraction with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **18g** (37 mg, 77.1% yield).

MS m/z (ESI): 853.0 [M+18].

Step 7

(11*S*,19*R*)-11-benzyl-19-cyclopropyl-1-(9*H*-fluoren-9-yl)-3,6,9,12,15-pentaoxo-2,18-dioxa-4,7,10,13,16-pentaazaicosan-20-oic acid **18h**

Compound **18g** (37 mg, 44.3 μmol) was dissolved in 1.4 mL of a 3% (v/v) solution of dichloroacetic acid in dichloromethane, and the solution was cooled to 0-5 °C in an ice-water bath, followed by addition of triethylsilane (15.4 mg, 132.4 μmol). The reaction mixture was stirred in the ice bath for 3 h. The reaction mixture was concentrated under reduced pressure in the ice bath to remove half of the organic solvent, and 5 mL of diethyl ether was added. The resulting mixture was warmed to room temperature naturally and slurried to precipitate a white solid, and filtered. The filter cake was collected and dried with an oil pump to give the title product **18h** (24 mg, 79.1% yield).

MS m/z (ESI): 708.2 [M+23].

Step 8

(9*H*-fluoren-9-yl)methyl((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadec-16-yl)carbamate **18i**

Compound **1b** (30 mg, 63.6 μmol) was added to a reaction flask, and 1 mL of a 10% (v/v) solution of methanol in dichloromethane was added. The system was purged with argon three times, and the mixture was cooled to 0-5 °C in an ice-water bath, followed by addition of a drop of triethylamine. The mixture was stirred until compound **1b** was dissolved. Compound **18h** (65 mg, 94.8 μmol) was dissolved in 1 mL of a 10% (v/v) solution of methanol in dichloromethane, and then the above reaction mixture was added dropwise, followed by addition of 4-(4,6-dimethoxy-1,3,5-triazin-2-yl)-4-methylmorpholinium chloride (27 mg, 97.6 μmol). The reaction mixture was warmed to room temperature and stirred for 1 h. 10 mL of dichloromethane and 5 mL of water were added, and the resulting mixture was stirred for 5 min, and let stand to form layers. The organic phase was collected, and the aqueous phase was extracted with dichloromethane (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **18i** (25 mg, 35.6% yield).

MS m/z (ESI): 1104.4 [M+1].

Step 9

(*S*)-2-(2-(2-aminoacetylamino)acetylamino)-*N*-(2-((((*R*)-1-cyclopropyl-2-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-oxoethoxy)methyl)amino)-2-oxoethoxy)-3-phenylpropanamide **18j**

Compound **18i** (12 mg, 10.9 μmol) was dissolved in 0.6 mL of dichloromethane, and 0.3 mL of diethylamine was added. The reaction mixture was stirred at room temperature for 1.5 h and concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **18j** (10 mg),

which was directly used in the next step without purification.

MS m/z (ESI): 881.0 [M+1].

Step 10

(1*r*,4*r*)-*N*-((2*R*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15,18-hexaoxo-3,20,23,26,29,32,3 5,3 8,41,44-decaoxa-5, 8,11,14,17-pentaaza-tetrahexadec-46-yl)-4-((2,5-dioxo-2,5-dihydro-1*H*-pyrrol-1-yl)methyl)cyclohexane-1-carboxamide **18**

Crude **18j** (10 mg) was dissolved in 1 mL of *N*,*N*-dimethylformamide, and compound **17g** (8.5 mg, 12.3 μmol) and 4-(4,6-dimethoxy-1,3,5-triazin-2-yl)-4-methylmorpholinium chloride (4.6 mg, 16.6 μmol) were added. The reaction mixture was stirred at room temperature for 30 min and filtered. The filtrate was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **18** (9.5 mg, 56.2% yield).

MS m/z (ESI): 1570.2 [M+18].

$^1H$ NMR (400 MHz, DMSO-$d_6$): δ 8.77 (d, 1H), 8.59-8.55 (m, 1H), 8.42 (d, 1H), 8.37-8.28 (m, 1H), 8.25-8.06 (m, 2H), 7.96-7.86 (m, 1H), 7.86-7.70 (m, 2H), 7.32-7.28 (m, 1H), 7.25-7.14 (m, 3H), 6.67 (m, 1H), 5.96 (s, 1H), 5.80-5.72 (m, 1H), 5.62-5.52 (m, 2H), 5.43-5.30 (m, 3H), 5.28-5.17 (m, 2H), 5.12-5.08 (m, 1H), 4.72-4.35 (m, 8H), 3.95-3.70 (m, 13H), 3.35-3.22 (m, 14H), 2.42-2.32 (m, 3H), 2.05-1.98 (m, 4H), 1.88-1.82 (m, 12H), 1.47-1.39 (m, 3H), 1.32-1.18 (m, 11H), 0.90-0.80 (m, 4H), 0.52-0.37 (m, 3H), 0.32-0.18 (m, 2H).

**Example 1-19**

(1*r*,4*r*)-N-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15,18-hexaoxo-3,20,23,26,29,32,35,38,41,44-decaoxa-5,8,11,14,17-pentaaza-tetrahexadec-46-yl)-4-((2,5-dioxo-2,5-dihydro-1*H*-pyrrol-1-yl)methyl)cyclohexane-1-carboxamide **19**

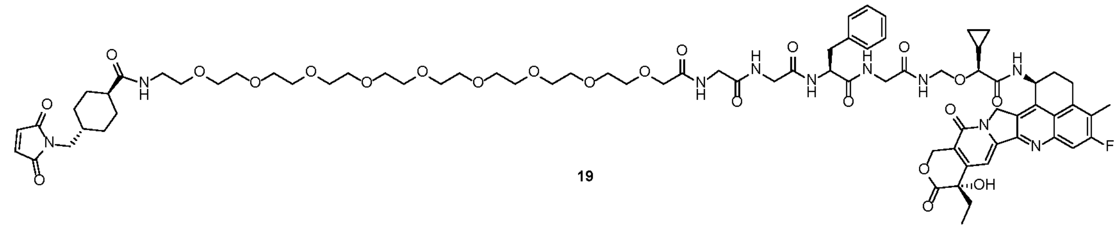

19

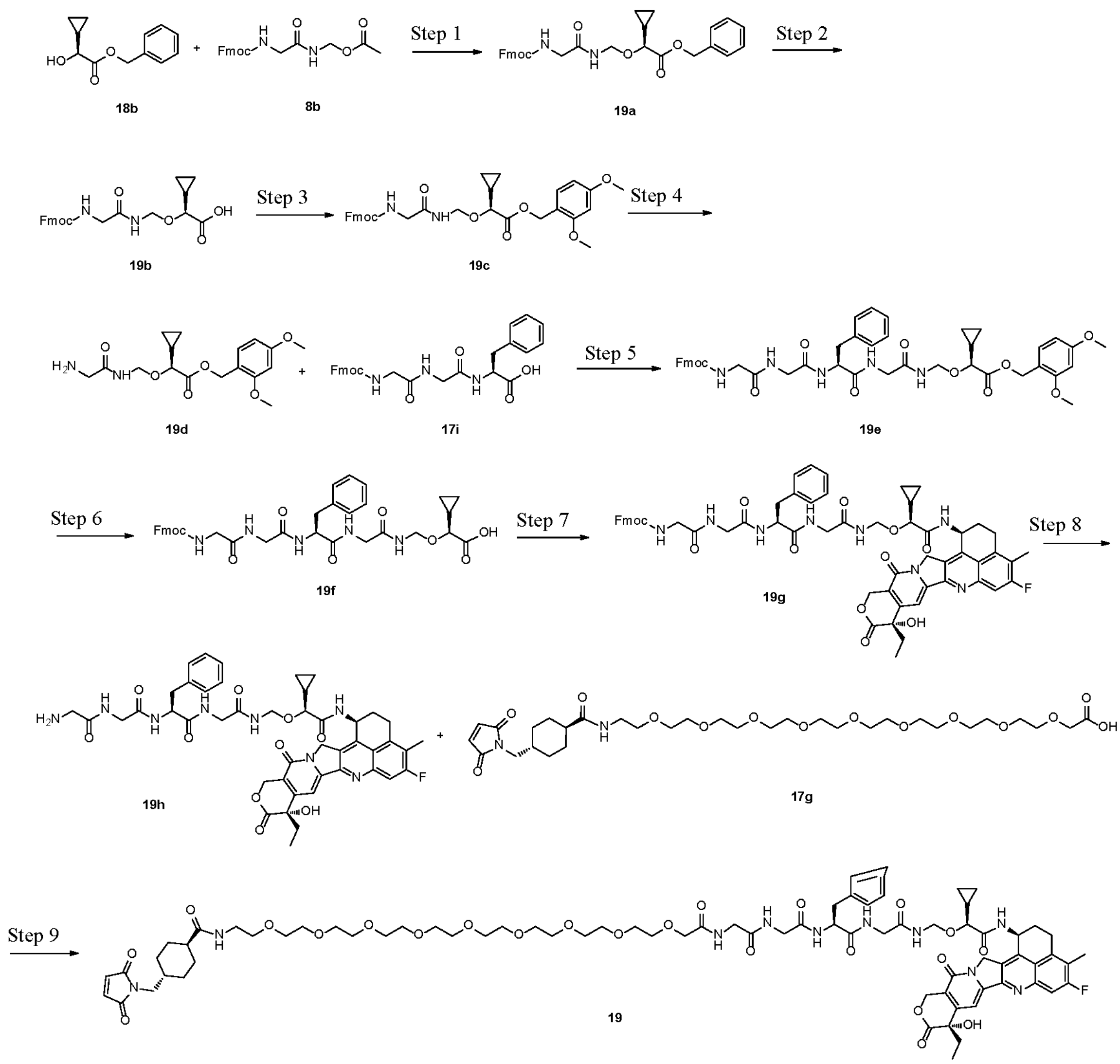


18b 8b 19a 19b 19c 19d 17i 19e 19f 19g 19h 17g 19

Step 1

Benzyl (*S*)-10-cyclopropyl-1-(9*H*-fluoren-9-yl)-3,6-dioxo-2,9-dioxa-4,7-diazaundecan-11-oate **19a**

Compound **18b** (252 mg, 1.22 mmol) was added to a reaction flask, and 4 mL of dichloromethane was added. The system was purged with argon three times, and the mixture was cooled to 0-5 °C in an ice water bath, followed by addition of lithium *tert*-butoxide (98 mg, 1.22 mmol). The reaction mixture was stirred in the ice water bath for 15 min and became clear, and **8b** (300 mg, 814.3 μmol) was added. The reaction mixture was stirred in the ice-water bath for 2.5 h. Water (10 mL) was added for liquid separation. The aqueous phase was extracted with dichloromethane (8 mL × 2), and the organic phases were combined and washed with water (10 mL × 1) and saturated brine (10 mL × 2), dried over anhydrous sodium sulfate, and filtered and concentrated to give the crude product. The resulting residue was purified by silica gel column chromatography with developing solvent system C to give the title product **19a** (282 mg, 67.2% yield).

Step 2

(S)-10-cyclopropyl-1-(9*H*-fluoren-9-yl)-3,6-dioxo-2,9-dioxa-4,7-diazaundecan-11-oic acid **19b**

Compound **19a** (280 mg, 0.554 mmol) was dissolved in 8 mL of ethyl acetate, and palladium on carbon (84 mg, 10% loading, dry basis) was added. The system was purged with hydrogen three times, and the reaction mixture was stirred at room temperature for 3 h and filtered through celite, and the filter cake was rinsed with ethyl acetate. The filtrate was concentrated to give the crude title product **19b** (230 mg), which was directly used in the next step without

purification.

Step 3

2,4-Dimethoxybenzyl(*S*)-10-cyclopropyl-1-(9*H*-fluoren-9-yl)-3,6-dioxo-2,9-dioxa-4,7-diazaundecan-11-oate **19c**

Crude **19b** (230 mg, 541.8 μmol) was dissolved in 7 mL of dichloromethane, and 2,4-dimethoxybenzyl alcohol (136.7 mg, 812.7 μmol), 1-ethyl-(3-dimethylaminopropyl)carbonyldiimine hydrochloride (155 mg, 808.5 μmol) and 4-dimethylaminopyridine (6.6 mg, 53.5 μmol) were added successively. The reaction mixture was stirred at room temperature for 16 h, diluted with 10 mL of dichloromethane, washed with water (10 mL × 1) and saturated brine (10 mL × 2), dried over anhydrous sodium sulfate, and filtered and concentrated to give the crude product. The resulting residue was purified by thin layer chromatography with developing solvent system B to give the title product **19c** (159 mg, 51.0% yield).

Step 4

2,4-Dimethoxybenzyl(*S*)-2-((2-aminoacetylamino)methoxy)-2-cyclopropylacetate **19d**

Compound **19c** (60 mg, 104.4 μmol) was dissolved in 1 mL of dichloromethane, and 0.5 mL of diethylamine was added. The reaction mixture was stirred at room temperature for 1 h and concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **19d** (21 mg), which was directly used in the next step without purification.

Step 5

2,4-Dimethoxybenzyl(11*S*,19*S*)-11-benzyl-19-cyclopropyl-1-(9*H*-fluoren-9-yl)-3,6,9,12,15-pentaoxo-2,18-dioxa-4,7,10,13,16-pentaazaeicosa-20-oate **19e**

Crude **19d** (36 mg, 102.2 μmol) was dissolved in 4 mL of *N*,*N*-dimethylformamide, and compound **17i** (52 mg, 103.6 μmol) and 4-(4,6-dimethoxy-1,3,5-triazin-2-yl)-4-methylmorpholinium chloride (34.6 mg, 125.0 μmol) were added. The reaction mixture was stirred at room temperature for 1 h. 10 mL of water was added, followed by extraction with ethyl acetate (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **19e** (70 mg, 80.2% yield).

Step 6

(11*S*,19*S*)-11-benzyl-19-cyclopropyl-1-(9*H*-fluoren-9-yl)-3,6,9,12,15-pentaoxo-2,18-dioxa-4,7,10,13,16-pentaazaeicosa-20-oic acid **19f**

Compound **19e** (70 mg, 83.7 μmol) was dissolved in 2.5 mL of a 3% (v/v) solution of dichloroacetic acid in dichloromethane, and the solution was cooled to 0-5 °C in an ice-water bath, followed by addition of triethylsilane (29 mg, 249.4 μmol). The reaction mixture was stirred in the ice bath for 3 h. The reaction mixture was concentrated under reduced pressure in an ice bath to remove half of the organic solvent, and 5 mL of diethyl ether was added. The resulting mixture was warmed to room temperature naturally and slurried to precipitate a white solid, and filtered. The filter cake was collected and dried with an oil pump to give the title product **19f** (57 mg, 99.2% yield).

Step 7

(9*H*-fluoren-9-yl)methyl((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15-pentaoxo-3-oxa-5,8,11,14-tetraazahexadec-16-yl)carbamate **19g**

Compound **1b** (30 mg, 63.6 μmol) was added to a reaction flask, and 1 mL of a 10% (v/v) solution of methanol in dichloromethane was added. The system was purged with argon three times, and the mixture was cooled to 0-5 °C in an ice-water bath, followed by addition of a drop of triethylamine. The mixture was stirred until compound **1b** was

dissolved. Compound **19f** (57 mg, 83.1 μmol) was dissolved in 1 mL of a 10% (v/v) solution of methanol in dichloromethane, and then the above reaction mixture was added dropwise, followed by addition of 4-(4,6-dimethoxy-1,3,5-triazin-2-yl)-4-methylmorpholinium chloride (26 mg, 93.9 μmol). The reaction mixture was warmed to room temperature and stirred for 1 h. 10 mL of dichloromethane and 5 mL of water were added, and the resulting mixture was stirred for 5 min, and let stand to form layers. The organic phase was collected, and the aqueous phase was extracted with dichloromethane (10 mL × 3). The organic phases were combined, washed with saturated sodium chloride solution (10 mL × 2), dried over anhydrous sodium sulfate, and filtered. The filtrate was concentrated under reduced pressure. The resulting residue was purified by thin layer chromatography with developing solvent system B to give the title product **19g** (56 mg, 79.8% yield).

MS m/z (ESI): 1103.1 [M+1].

Step 8

(*S*)-2-(2-(2-aminoacetylamino)acetylamino)-*N*-(2-((((*S*)-1-cyclopropyl-2-((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-2-oxoethoxy)methyl)amino)-2-oxoethyl)-3-phenylpropanamide **19h**

Compound **19g** (4.6 mg, 4.16 μmol) was dissolved in 1.5 mL of dichloromethane, and 0.75 mL of diethylamine was added. The reaction mixture was stirred at room temperature for 1.6 h and concentrated under reduced pressure. 2 mL of toluene was added, followed by concentration under reduced pressure; the procedures were repeated twice. The residue was slurried with 3 mL of *n*-hexane, and the upper *n*-hexane layer was removed; the procedures were repeated three times. The slurry was concentrated under reduced pressure to give the crude title product **19h** (4.0 mg), which was directly used in the next step without purification.

Step 9

(1*r*,4*r*)-*N*-((2*S*,10*S*)-10-benzyl-2-cyclopropyl-1-(((1*S*,9*S*)-9-ethyl-5-fluoro-9-hydroxy-4-methyl-10,13-dioxo-2,3,9,10,13,15-hexahydro-1*H*,12*H*-benzo[*de*]pyrano[3',4':6,7]indolizino[1,2-*b*]quinolin-1-yl)amino)-1,6,9,12,15,18-hexaoxo-3,20,23,26,29,32,35,38,41,44-decaoxa-5,8,11,14,17-pentaaza-tetrahexadec-46-yl)-4-((2,5-dioxo-2,5-dihydro-1*H*-pyrrol-1-yl)methyl)cyclohexane-1-carboxamide **19**

Crude **19h** (4.0 mg) was dissolved in 1 mL of *N*,*N*-dimethylformamide, and compound **17g** (2.9 mg, 4.2 μmol) and 4-(4,6-dimethoxy-1,3,5-triazin-2-yl)-4-methylmorpholinium chloride (1.5 mg, 5.4 μmol) were added. The reaction mixture was stirred at room temperature for 40 min and filtered. The filtrate was purified by high performance liquid chromatography (separation conditions: chromatography column: XBridge Prep C18 OBD 5 μm 19 × 250 mm; mobile phase: A-water (10 mmol of $NH_4OAc$), B-acetonitrile, gradient elution, flow rate: 18 mL/min). The corresponding fractions were collected and concentrated under reduced pressure to give the title product **19** (2.1 mg, 32.4% yield).

$^1H$ NMR (400 MHz, DMSO-$d_6$): δ 8.71-8.62 (m, 1H), 8.59-8.51 (m, 1H), 8.34-8.26 (m, 1H), 8.14-8.02 (m, 2H), 7.95-7.86 (m, 1H), 7.83-7.69 (m, 2H), 7.35-7.31 (m, 1H), 7.29-7.11 (m, 3H), 7.01 (s, 1H), 6.72-6.50 (m, 3H), 5.59-5.50 (m, 2H), 5.42 (s, 2H), 5.38-5.18 (m, 3H), 4.79-4.69 (m, 2H), 4.61-4.42 (m, 3H), 3.91 (s, 2H), 3.79-3.65 (m, 4H), 3.63-3.44 (m, 13H), 3.41-3.30 (m, 2H), 3.26-3.09 (m, 5H), 3.08-2.84 (m, 4H), 2.81-2.64 (m, 3H), 2.42-2.28 (m, 3H), 2.24-2.12 (m, 2H), 2.05-1.93 (m, 4H), 1.89-1.77 (m, 2H), 1.72-1.56 (m, 3H), 1.53-1.38 (m, 3H), 1.34-1.10 (m, 11H), 0.94-0.78 (m, 5H), 0.52-0.35 (m, 3H).

**Example 1-20 (reference example)**

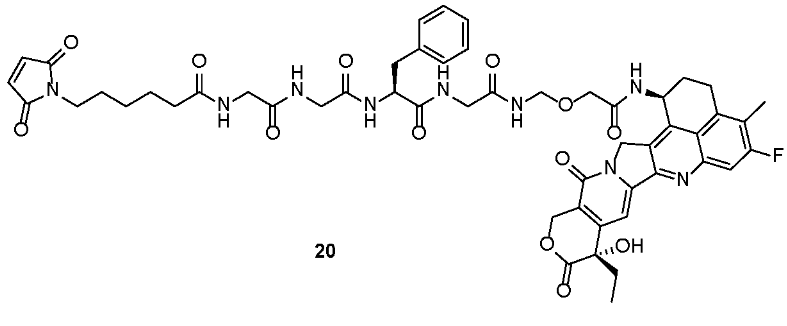

**20**

The title compound **20** was prepared as disclosed in "Example 58 on page 163 of the specification of Patent CN104755494A".

The following antibodies can be prepared using a conventional preparation method for antibodies, and can be obtained by, for example, vector construction, transfection of eukaryotic cells such as HEK293 cells (Life Technologies Cat. No. 11625019), and expression purification.

The following is the sequence of trastuzumab:

Light chain

DIQMTQSPSSLSASVGDRVTITCRASQDVNTAVAWYQQKPGKAPKLLIYSASFLY
SGVPSRFSGSRSGTDFTLTISSLQPEDFATYYCQQHYTTPPTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 1

Heavy chain

EVQLVESGGGLVQPGGSLRLSCAASGFNIKDTYIHWVRQAPGKGLEWVARIYPT
NGYTRYADSVKGRFTISADTSKNTAYLQMNSLRAEDTAVYYCSRWGGDGFYAM
DYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM
HEALHNHYTQKSLSLSPGK

SEQ ID NO: 2

The following is the sequence of pertuzumab:

Light chain

DIQMTQSPSSLSASVGDRVTITCKASQDVSIGVAWYQQKPGKAPKLLIYSASYRY
TGVPSRFSGSGSGTDFTLTISSLQPEDFATYYCQQYYIYPYTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC

SEQ ID NO: 3

Heavy chain

EVQLVESGGGLVQPGGSLRLSCAASGFTFTDYTMDWVRQAPGKGLEWVADVN
PNSGGSIYNQRFKGRFTLSVDRSKNTLYLQMNSLRAEDTAVYYCARNLGPSFYF
DYWGQGTLVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSREEMTKNQVSLTCLVKGFYP
SDIAVEWESNGQPENNYKTTPPVLDSDGSFFLYSKLTVDKSRWQQGNVFSCSVM
HEALHNHYTQKSLSLSPGK

SEQ ID NO: 4

The following is the sequence of B7H3 antibody 1F9DS:

Light chain

DTVVTQEPSFSVSPGGTVTLTCGLSSGSVSTSHYPSWYQQTPGQAPRMLIYNTN
TRSSGVPDRFSGSILGNKAALTITGAQADDESDYYCAIHVDRDIWVFGGGTKLT
VLGQPKANPTVTLFPPSSEELQANKATLVCLISDFYPGAVTVAWKADGSPVKAG
VETTKPSKQSNNKYAASSYLSLTPEQWKSHRSYSCQVTHEGSTVEKTVAPTEC

SEQ ID NO: 5

Heavy chain

QVQLVQSGGGVVQPGTSLRLSCAASGFIFSSSAMHWVRQAPGKGLEWVAVISY
DGSNKYYVDSVKGRFTISRDNSKNTLYLQMNSLRAEDTAVYYCARSARLYASF
DYWGQGALVTVSSASTKGPSVFPLAPSSKSTSGGTAALGCLVKDYFPEPVTVSW
NSGALTSGVHTFPAVLQSSGLYSLSSVVTVPSSSLGTQTYICNVNHKPSNTKVDK
KVEPKSCDKTHTCPPCPAPELLGGPSVFLFPPKPKDTLMISRTPEVTCVVVDVSH
EDPEVKFNWYVDGVEVHNAKTKPREEQYNSTYRVVSVLTVLHQDWLNGKEY
KCKVSNKALPAPIEKTISKAKGQPREPQVYTLPPSRDELTKNQVSLTCLVKGFYP
SDIAVEWESNGQPENNYKTTPPVLDSDGSFFLYSKLTVDKSRWQQGNVFSCSVM
HEALHNHYTQKSLSLSPGK

SEQ ID NO: 6

**Example 1-21. ADC-1**

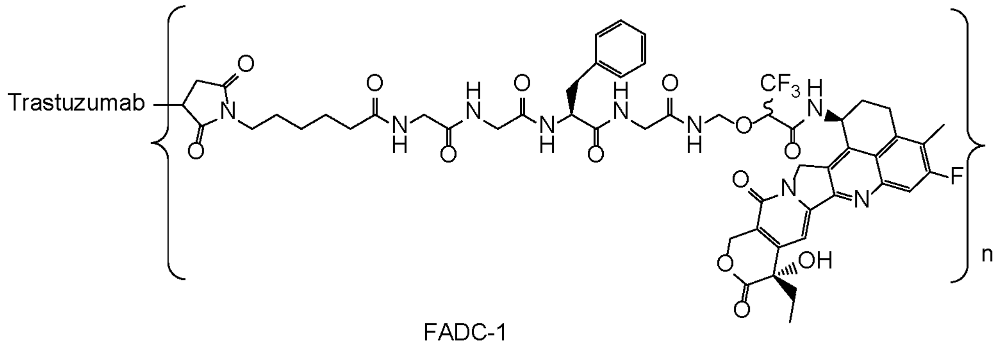

FADC-1

To antibody trastuzumab in aqueous PBS buffer (0.05 M aqueous PBS buffer at pH 6.5, 2.5 mL, 9.96 mg/mL, 0.168 μmol) was added at 37 °C a prepared aqueous solution of tris(2-carboxyethyl)phosphine (10 mM, 0.082 mL, 0.82 μmol). The reaction mixture was shaken on a water bath shaker at 37 °C for 3 h before the reaction was terminated. The reaction mixture was cooled to 25 °C in a water bath and diluted to 5.0 mg/mL, and 2.0 mL of the solution was collected for use in the next step.

Compound **10**-shorter retention time compound (2.1 mg, 2.02 μmol) was dissolved in 0.10 mL of DMSO, and the solution was added to the above 2.0 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-1** of FADC-1 general formula in PBS buffer (5.0 mg/mL, 1.1 mL), which was then stored at 4 °C.

Mean calculated by UV-HPLC: n = 5.09.

**Example 1-22. ADC-2**

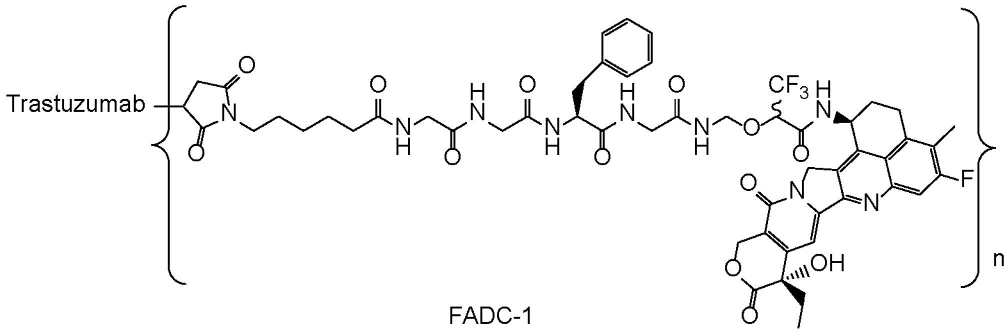

FADC-1

To antibody trastuzumab in aqueous PBS buffer (0.05 M aqueous PBS buffer at pH 6.5, 2.5 mL, 9.96 mg/mL, 0.168 μmol) was added at 37 °C a prepared aqueous solution of tris(2-carboxyethyl)phosphine (10 mM, 0.082 mL, 0.82 μmol). The reaction mixture was shaken on a water bath shaker at 37 °C for 3 h before the reaction was terminated. The reaction mixture was cooled to 25 °C in a water bath and diluted to 5.0 mg/mL, and 2.0 mL of the solution was collected for use in the next step.

Compound **10**-longer retention time compound (2.1 mg, 2.02 μmol) was dissolved in 0.10 mL of DMSO, and the solution was added to the above 2.0 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-2** of FADC-1 general formula in PBS buffer (4.95 mg/mL, 1.1 mL), which was then stored at 4 °C.

Mean calculated by UV-HPLC: n = 7.39.

**Example 1-23. ADC-3**

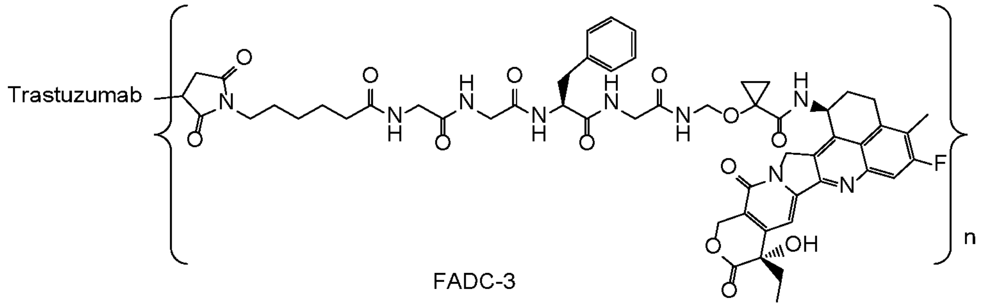

FADC-3

To antibody trastuzumab in aqueous PBS buffer (0.05 M aqueous PBS buffer at pH 6.5, 2.5 mL, 9.96 mg/mL, 0.168 μmol) was added at 37 °C a prepared aqueous solution of tris(2-carboxyethyl)phosphine (10 mM, 0.082 mL, 0.82 μmol). The reaction mixture was shaken on a water bath shaker at 37 °C for 3 h before the reaction was terminated. The reaction mixture was cooled to 25 °C in a water bath and diluted to 5.0 mg/mL, and 2.0 mL of the solution was collected for use in the next step.

Compound **8** (2.1 mg, 2.02 μmol) was dissolved in 0.10 mL of DMSO, and the solution was added to the above 2.0 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-3** of FADC-3 general formula in PBS buffer (5.24 mg/mL, 1.1 mL), which was then stored at 4 °C.

Mean calculated by UV-HPLC: n = 7.36.

**Example 1-24. ADC-4**

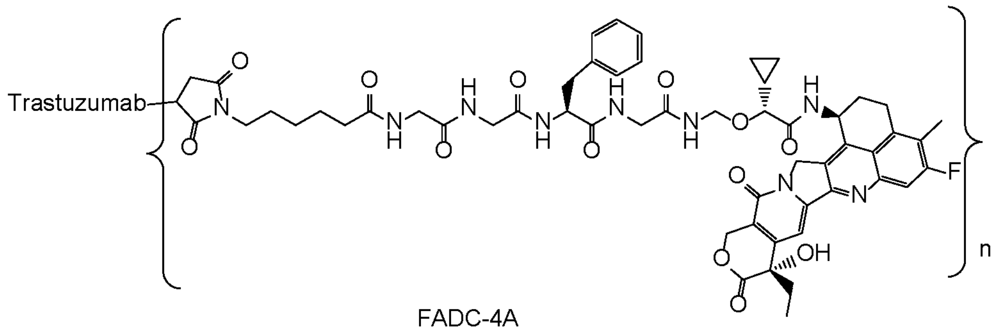

FADC-4A

To antibody trastuzumab in aqueous PBS buffer (0.05 M aqueous PBS buffer at pH 6.5, 3.74 mL, 13.38 mg/mL, 0.338 μmol) was added at 37 °C a prepared aqueous solution of tris(2-carboxyethyl)phosphine (10 mM, 0.173 mL, 1.73 μmol). The reaction mixture was shaken on a water bath shaker at 37 °C for 3 h before the reaction was terminated. The reaction mixture was cooled to 25 °C in a water bath and diluted to 6.7 mg/mL, and 1.3 mL of the solution was collected for use in the next step.

Compound **9**-shorter retention time compound 9-A (1.0 mg, 0.93 μmol) was dissolved in 0.10 mL of DMSO, and the solution was added to the above 1.3 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-4** of FADC-4A general formula in PBS buffer (1.72 mg/mL, 2.36 mL), which was then stored at 4 °C.

Mean calculated by UV-HPLC: n = 7.39.

**Example 1-25. ADC-5**

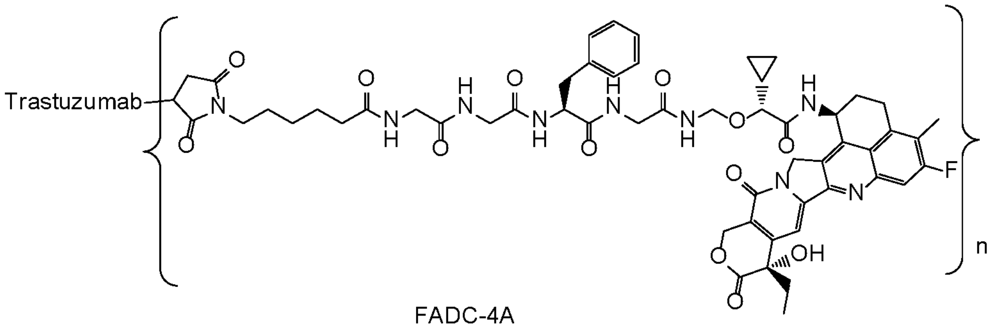

FADC-4A

To antibody trastuzumab in aqueous PBS buffer (0.05 M aqueous PBS buffer at pH 6.5, 3.0 mL, 6.70 mg/mL, 0.136 μmol) was added at 37 °C a prepared aqueous solution of tris(2-carboxyethyl)phosphine (10 mM, 0.067 mL, 0.67 μmol). The reaction mixture was shaken on a water bath shaker at 37 °C for 3 h before the reaction was terminated. The reaction mixture was cooled to 25 °C in a water bath, and 0.614 mL of the solution was collected for use in the next step.

Compound **9**-shorter retention time compound 9-A (0.5 mg, 0.42 μmol) was dissolved in 0.031 mL of DMSO, and the solution was added to the above 0.614 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-5** of FADC-4A general formula in PBS buffer (3.08 mg/mL, 0.82 mL), which was then stored at 4 °C.

Mean calculated by UV-HPLC: n = 3.16.

## Example 1-26. ADC-6

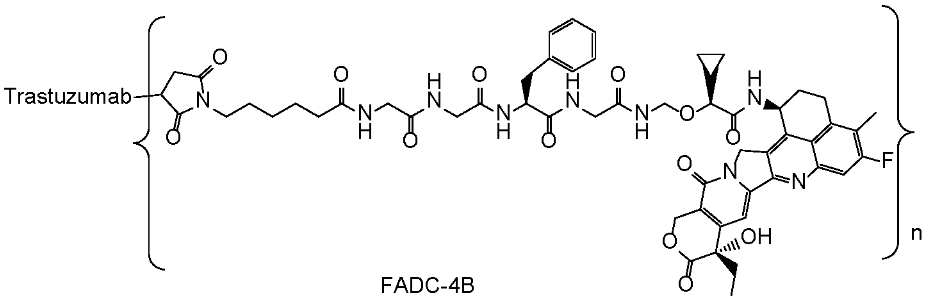

FADC-4B

To antibody trastuzumab in aqueous PBS buffer (0.05 M aqueous PBS buffer at pH 6.5, 3.74 mL, 13.38 mg/mL, 0.338 μmol) was added at 37 °C a prepared aqueous solution of tris(2-carboxyethyl)phosphine (10 mM, 0.173 mL, 1.73 μmol). The reaction mixture was shaken on a water bath shaker at 37 °C for 3 h before the reaction was terminated. The reaction mixture was cooled to 25 °C in a water bath and diluted to 6.7 mg/mL, and 0.75 mL of the solution was collected for use in the next step.

Compound 9-longer retention time compound 9-B (0.68 mg, 0.63 μmol) was dissolved in 0.10 mL of DMSO, and the solution was added to the above 0.75 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-6** of FADC-4B general formula in PBS buffer (1.78 mg/mL, 1.78 mL), which was then stored at 4 °C.

Mean calculated by UV-HPLC: n = 3.94.

## Example 1-27. ADC-7

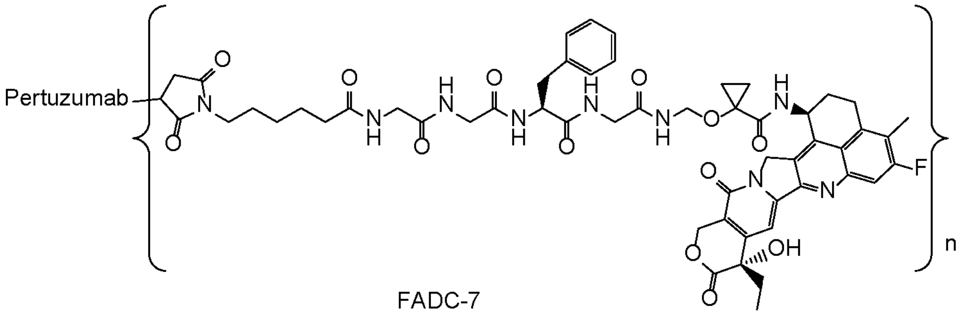

FADC-7

To antibody pertuzumab in aqueous PBS buffer (0.05 M aqueous PBS buffer at pH 6.5, 5.0 mL, 10 mg/mL, 0.338 μmol) was added at 37 °C a prepared aqueous solution of tris(2-carboxyethyl)phosphine (10 mM, 0.173 mL, 1.73 μmol). The reaction mixture was shaken on a water bath shaker at 37 °C for 3 h before the reaction was terminated. The reaction mixture was cooled to 25 °C in a water bath and diluted to 5.0 mg/mL, and 1.0 mL of the solution was collected for use in the next step.

Compound **8** (0.65 mg, 0.6 μmol) was dissolved in 0.1 mL of DMSO, and the solution was added to the above 1.0 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-7** of FADC-7 general formula in PBS buffer (1.42 mg/mL, 2.15 mL), which was then stored at 4 °C.

Mean calculated by UV-HPLC: n = 6.91.

**Example 1-28. ADC-8**

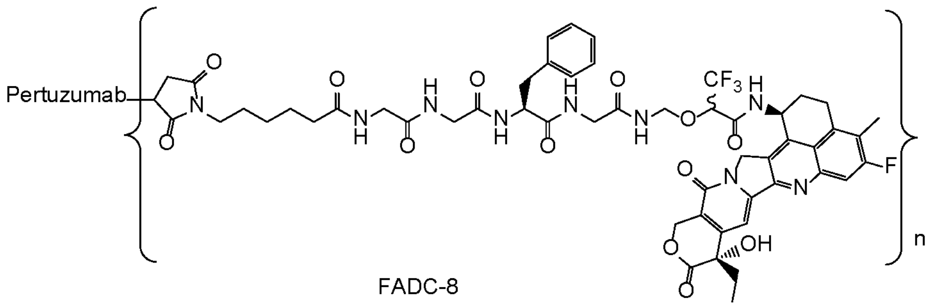

FADC-8

To antibody pertuzumab in aqueous PBS buffer (0.05 M aqueous PBS buffer at pH 6.5, 5.0 mL, 10 mg/mL, 0.338 μmol) was added at 37 °C a prepared aqueous solution of tris(2-carboxyethyl)phosphine (10 mM, 0.173 mL, 1.73 μmol). The reaction mixture was shaken on a water bath shaker at 37 °C for 3 h before the reaction was terminated. The reaction mixture was cooled to 25 °C in a water bath and diluted to 5.0 mg/mL, and 1.6 mL of the solution was collected for use in the next step.

Compound **10**-shorter retention time compound (1.04 mg, 1.0 μmol) was dissolved in 0.1 mL of DMSO, and the solution was added to the above 1.6 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-8** of FADC-8 general formula in PBS buffer (2.14 mg/mL, 2.31 mL), which was then stored at 4 °C.

Mean calculated by UV-HPLC: n = 6.58.

**Example 1-29. ADC-9**

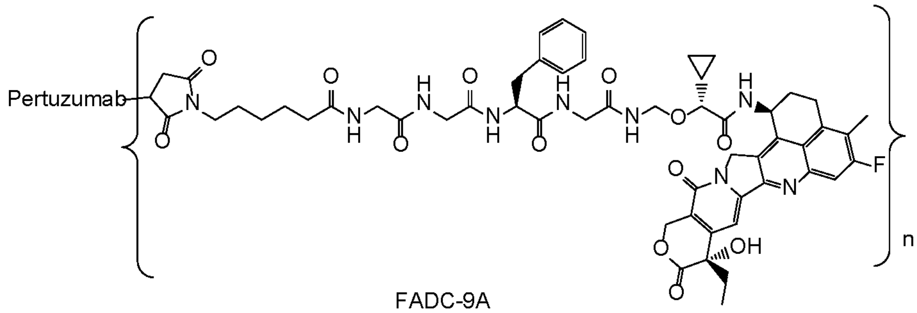

FADC-9A

To antibody pertuzumab in aqueous PBS buffer (0.05 M aqueous PBS buffer at pH 6.5, 5.0 mL, 10 mg/mL, 0.338 μmol) was added at 37 °C a prepared aqueous solution of tris(2-carboxyethyl)phosphine (10 mM, 0.173 mL, 1.73 μmol). The reaction mixture was shaken on a water bath shaker at 37 °C for 3 h before the reaction was terminated. The reaction mixture was cooled to 25 °C in a water bath and diluted to 5.0 mg/mL, and 0.8 mL of the solution was collected for use in the next step.

Compound **9**-shorter retention time compound 9-A (0.55 mg, 0.5 μmol) was dissolved in 0.1 mL of DMSO, and the solution was added to the above 0.8 mL of the solution. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-9** of FADC-9A general formula in PBS buffer (2.27 mg/mL, 1.11 mL), which was then stored at 4 °C.

Mean calculated by UV-HPLC: n = 3.16.

**Example 1-30. ADC-10**

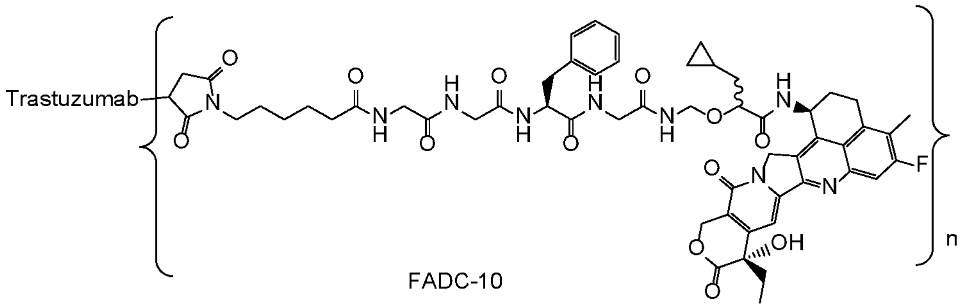

FADC-10

To antibody trastuzumab in aqueous PBS buffer (0.05 M aqueous PBS buffer at pH 6.5, 10.0 mg/mL, 0.574 mL, 38.78 nmol) was added at 37 °C a prepared aqueous solution of tris(2-carboxyethyl)phosphine (TCEP) (10 mM, 19.76 μL, 197.6 nmol). The reaction mixture was shaken on a water bath shaker at 37 °C for 3 h before the reaction was terminated. The reaction mixture was cooled to 25 °C in a water bath.

Compound **14**-shorter retention time compound (0.64 mg, 588 nmol) was dissolved in 40 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-10** of FADC-10 general formula in PBS buffer (5.48 mg/mL, 1.03 mL), which was then stored at 4 °C.

Mean calculated by UV-Vis: n = 6.25.

**Example 1-31. ADC-11**

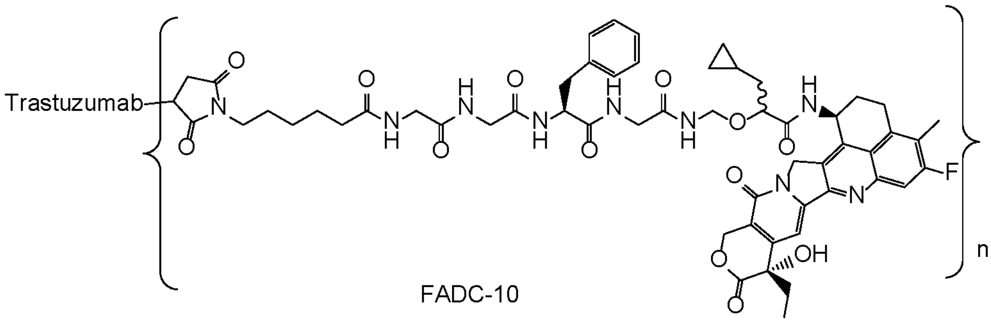

FADC-10

To antibody trastuzumab in aqueous PBS buffer (0.05 M aqueous PBS buffer at pH 6.5, 10.0 mg/mL, 0.646 mL, 43.64 nmol) was added at 37 °C a prepared aqueous solution of tris(2-carboxyethyl)phosphine (TCEP) (10 mM, 22.24 μL, 222.4 nmol). The reaction mixture was shaken on a water bath shaker at 37 °C for 3 h before the reaction was terminated. The reaction mixture was cooled to 25 °C in a water bath.

Compound **14**-longer retention time compound (0.72 mg, 662 nmol) was dissolved in 40 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-11** of FADC-10 general formula in PBS buffer (2.13 mg/mL, 1.87 mL), which was then stored at 4 °C.

Mean calculated by UV-Vis: n = 7.03.

**Example 1-32. ADC-12**

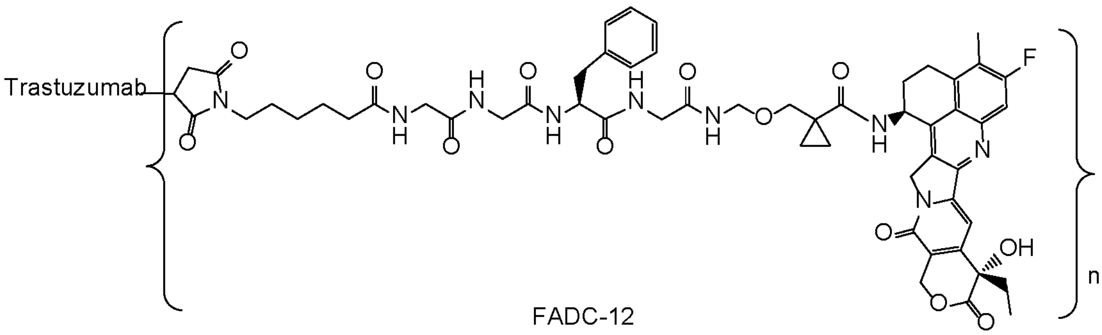

FADC-12

To antibody trastuzumab in aqueous PBS buffer (0.05 M aqueous PBS buffer at pH 6.5, 10.0 mg/mL, 0.726 mL, 49.05 nmol) was added at 37 °C a prepared aqueous solution of tris(2-carboxyethyl)phosphine (TCEP) (10 mM, 25.0 μL, 250.0 nmol). The reaction mixture was shaken on a water bath shaker at 37 °C for 3 h before the reaction was terminated. The reaction mixture was cooled to 25 °C in a water bath.

Compound **15** (0.81 mg, 754 nmol) was dissolved in 40 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-12** of FADC-12 general formula in PBS buffer (3.34 mg/mL, 1.45 mL), which was then stored at 4 °C.

Mean calculated by UV-Vis: n = 6.93.

**Example 1-33. ADC-13**

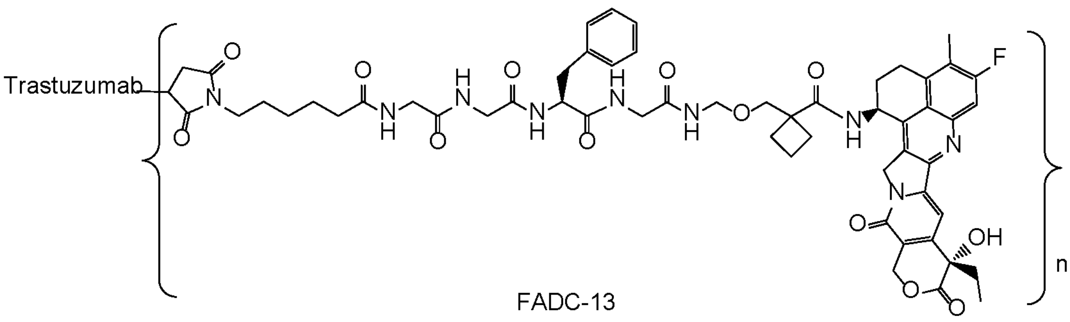

FADC-13

To antibody trastuzumab in aqueous PBS buffer (0.05 M aqueous PBS buffer at pH 6.5, 10.0 mg/mL, 0.287 mL, 19.39 nmol) was added at 37 °C a prepared aqueous solution of tris(2-carboxyethyl)phosphine (TCEP) (10 mM, 9.88 μL, 98.8 nmol). The reaction mixture was shaken on a water bath shaker at 37 °C for 3 h before the reaction was terminated. The reaction mixture was cooled to 25 °C in a water bath.

Compound **16** (0.32 mg, 294 nmol) was dissolved in 20 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 Maqueous PBS buffer at pH 6.5, containing 0.001 M EDTA) to give exemplary product **ADC-13** of FADC-13 general formula in PBS buffer (2.37 mg/mL, 0.88 mL), which was then stored at 4 °C.

Mean calculated by UV-Vis: n = 6.53.

**Example 1-34. ADC-14**

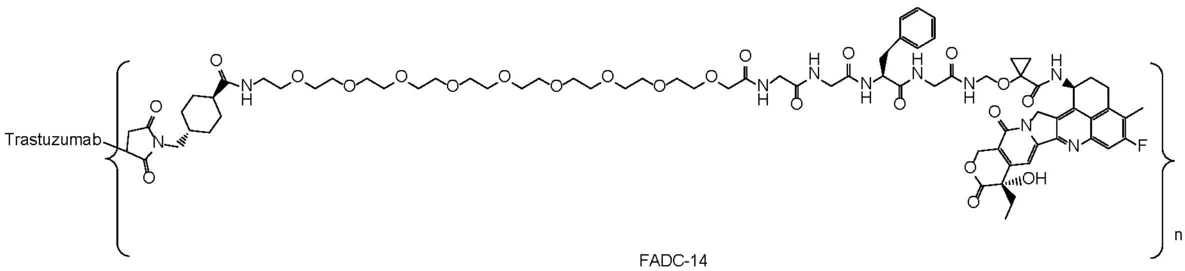

FADC-14

To antibody trastuzumab in aqueous PBS buffer (0.05 M aqueous PBS buffer at pH 6.5, 10.0 mg/mL, 0.592 mL, 40.0 nmol) was added at 37 °C a prepared aqueous solution of tris(2-carboxyethyl)phosphine (TCEP) (10 mM, 20.38 μL, 203.8 nmol). The reaction mixture was shaken on a water bath shaker at 37 °C for 3 h before the reaction was terminated. The reaction mixture was cooled to 25 °C in a water bath.

Compound **17** (0.92 mg, 598 nmol) was dissolved in 40 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-14** of FADC-14 general formula in PBS buffer (0.30 mg/mL, 12.0 mL), which was then stored at 4 °C.

Mean calculated by UV-Vis: n = 7.61.

**Example 1-35. ADC-15**

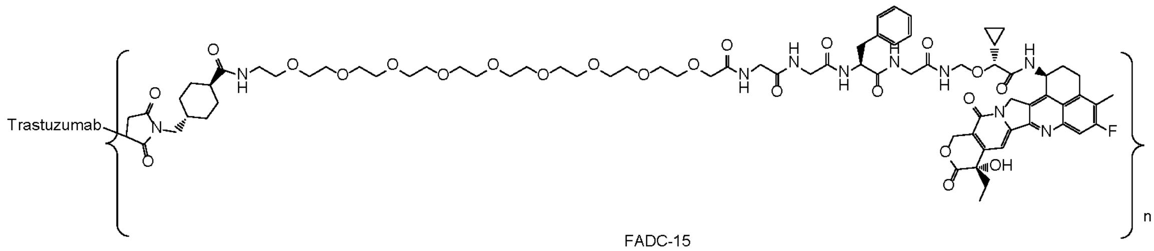

FADC-15

To antibody trastuzumab in aqueous PBS buffer (0.05 M aqueous PBS buffer at pH 6.5, 10.0 mg/mL, 0.592 mL, 40.0 nmol) was added at 37 °C a prepared aqueous solution of tris(2-carboxyethyl)phosphine (TCEP) (10 mM, 20.38 μL, 203.8 nmol). The reaction mixture was shaken on a water bath shaker at 37 °C for 3 h before the reaction was terminated. The reaction mixture was cooled to 25 °C in a water bath.

Compound **18** (0.93 mg, 599 nmol) was dissolved in 40 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-15** of FADC-15 general formula in PBS buffer (0.32 mg/mL, 11.8 mL), which was then stored at 4 °C.

Mean calculated by UV-Vis: n = 7.89.

**Example 1-36. ADC-16**

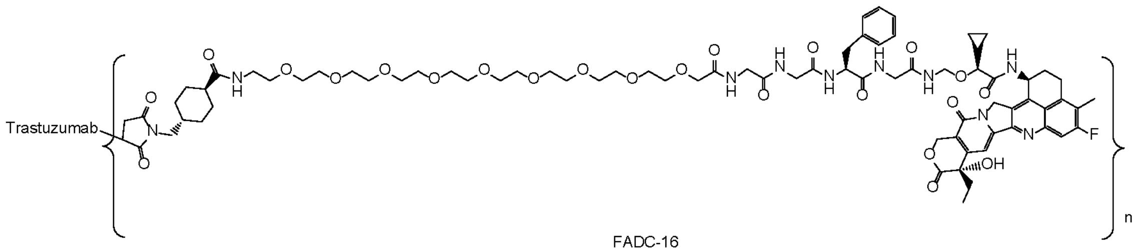

FADC-16

To antibody trastuzumab in aqueous PBS buffer (0.05 M aqueous PBS buffer at pH 6.5, 10.0 mg/mL, 0.53 mL, 35.8 nmol) was added at 37 °C a prepared aqueous solution of tris(2-carboxyethyl)phosphine (TCEP) (10 mM, 18.25 μL, 182.5 nmol). The reaction mixture was shaken on a water bath shaker at 37 °C for 3 h before the reaction was terminated. The reaction mixture was cooled to 25 °C in a water bath.

Compound 19 (0.83 mg, 534 nmol) was dissolved in 35 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-16** of FADC-16 general formula in PBS buffer (0.32 mg/mL, 12.0 mL), which was then stored at 4 °C.

Mean calculated by UV-Vis: n = 7.43.

**Example 1-37. ADC-17**

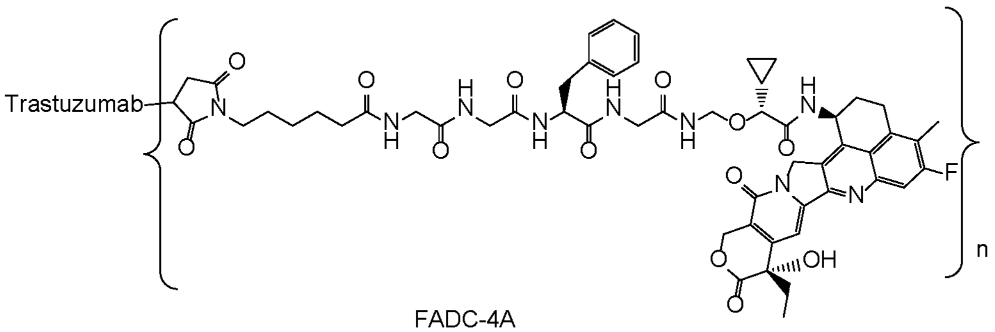

FADC-4A

To antibody trastuzumab in aqueous PBS buffer (0.05 M aqueous PBS buffer at pH 6.5, 10.0 mg/mL, 2.0 mL, 135.12 nmol) was added at 37 °C a prepared aqueous solution of tris(2-carboxyethyl)phosphine (TCEP) (10 mM, 43.2 μL, 432 nmol). The reaction mixture was shaken on a water bath shaker at 37 °C for 3 h before the reaction was terminated. The reaction mixture was cooled to 25 °C in a water bath.

Compound **9**-shorter retention time compound **9-A** (2.22 mg, 2067 nmol) was dissolved in 175 μL, of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-17** of FADC-4A general formula in PBS buffer (1.32 mg/mL, 12.0 mL), which was then stored at 4 °C.

Mean calculated by UV-Vis: n = 5.42.

**Example 1-38. ADC-18 (reference example)**

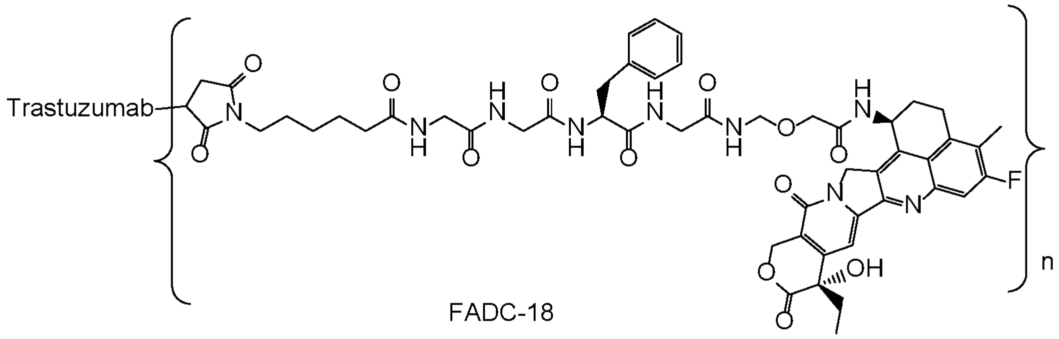

FADC-18

To antibody trastuzumab in aqueous PBS buffer (0.05 M aqueous PBS buffer at pH 6.5, 10.0 mg/mL, 1.5 mL, 101.3 nmol) was added at 37 °C a prepared aqueous solution of tris(2-carboxyethyl)phosphine (TCEP) (10 mM, 51.7 μL, 517 nmol). The reaction mixture was shaken on a water bath shaker at 37 °C for 3 h before the reaction was terminated. The reaction mixture was cooled to 25 °C in a water bath.

Compound **20** (2.0 mg, 1934 nmol) was dissolved in 100 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-18** of FADC-18 general formula in PBS buffer (0.79 mg/mL, 13.0 mL), which was then stored at 4 °C.

Mean calculated by UV-Vis: n = 7.23.

**Example 1-39. ADC-19**

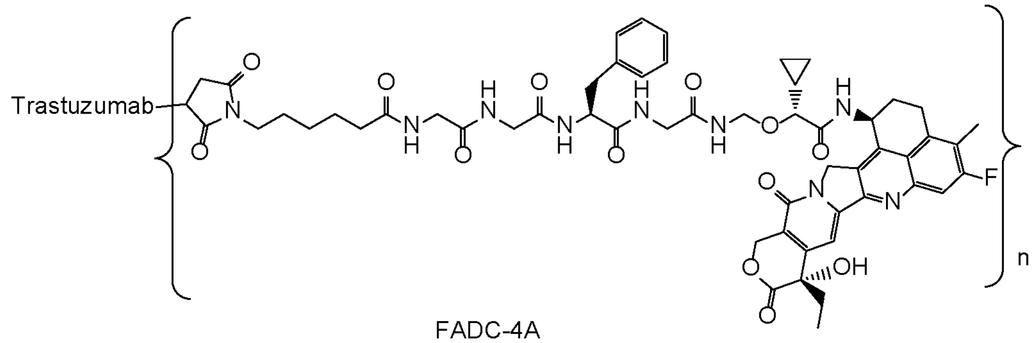

FADC-4A

To antibody trastuzumab in aqueous PBS buffer (0.05 M aqueous PBS buffer at pH 6.5, 10.0 mg/mL, 1.36 mL, 91.9 nmol) was added at 37 °C a prepared aqueous solution of tris(2-carboxyethyl)phosphine (TCEP) (10 mM, 46.9 μL, 469 nmol). The reaction mixture was shaken on a water bath shaker at 37 °C for 3 h before the reaction was terminated. The reaction mixture was cooled to 25 °C in a water bath.

Compound **9**-shorter retention time compound **9-A** (2.0 mg, 1862 nmol) was dissolved in 100 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-19** of FADC-4A general formula in PBS buffer (0.73 mg/mL, 13.0 mL), which was then stored at 4 °C.

Mean calculated by UV-Vis: n = 6.26.

**Example 1-40. ADC-20**

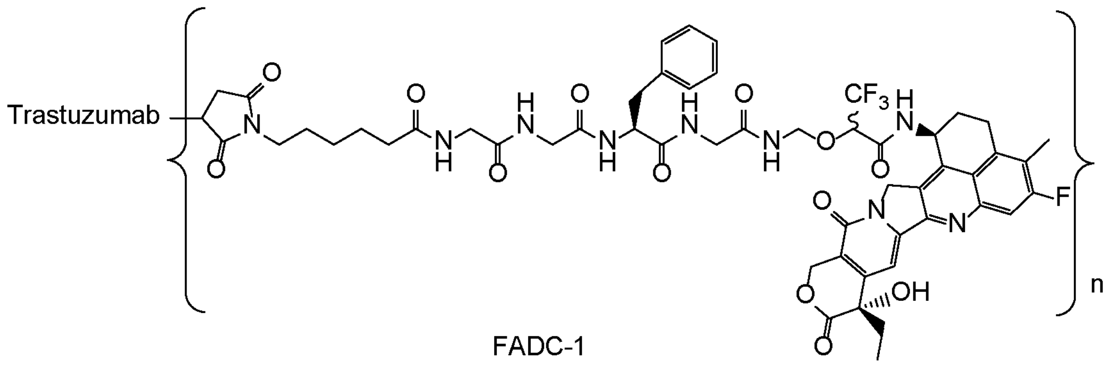

FADC-1

To antibody trastuzumab in aqueous PBS buffer (0.05 M aqueous PBS buffer at pH 6.5, 10.0 mg/mL, 1.5 mL, 101.3 nmol) was added at 37 °C a prepared aqueous solution of tris(2-carboxyethyl)phosphine (TCEP) (10 mM, 51.7 μL, 517 nmol). The reaction mixture was shaken on a water bath shaker at 37 °C for 3 h before the reaction was terminated. The reaction mixture was cooled to 25 °C in a water bath.

Compound 10-longer retention time compound (2.0 mg, 1815 nmol) was dissolved in 100 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-20** of FADC-1 general formula in PBS buffer (0.73 mg/mL, 13.0 mL), which was then stored at 4 °C.

Mean calculated by UV-Vis: n = 7.43.

**Example 1-41. ADC-21 (reference example)**

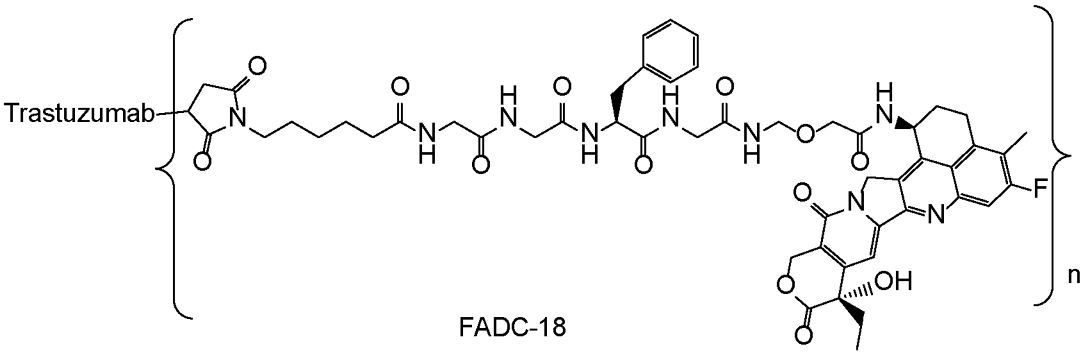

FADC-18

To antibody trastuzumab in aqueous PBS buffer (0.05 M aqueous PBS buffer at pH 6.5, 10.0 mg/mL, 1.86 mL, 125.4 nmol) was added at 37 °C a prepared aqueous solution of tris(2-carboxyethyl)phosphine (TCEP) (10 mM, 63.9 μL, 639 nmol). The reaction mixture was shaken on a water bath shaker at 37 °C for 3 h before the reaction was terminated. The reaction mixture was cooled to 25 °C in a water bath.

Compound **20** (2.07 mg, 2001 nmol) was dissolved in 150 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-21** of FADC-18 general formula in PBS buffer (2.91 mg/mL, 4.44 mL), which was then stored at 4 °C.

Mean calculated by UV-Vis: n = 7.23.

**Example 1-42. ADC-22**

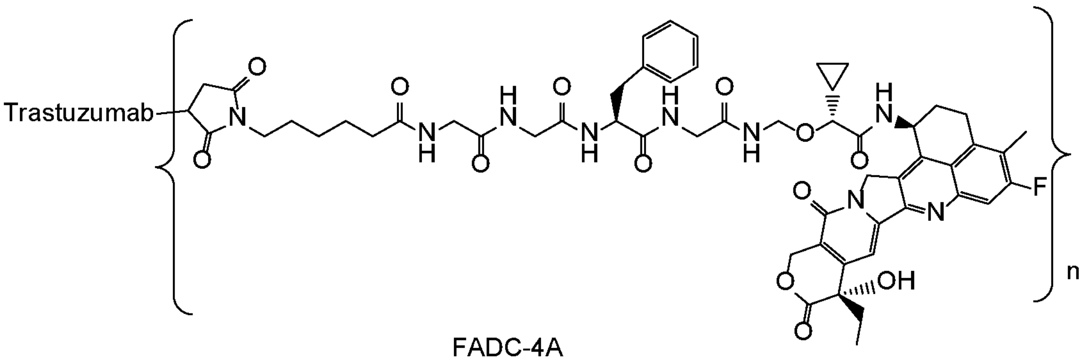

FADC-4A

To antibody trastuzumab in aqueous PBS buffer (0.05 M aqueous PBS buffer at pH 6.5, 10.0 mg/mL, 1.88 mL, 127.2 nmol) was added at 37 °C a prepared aqueous solution of tris(2-carboxyethyl)phosphine (TCEP) (10 mM, 64.9 μL, 649 nmol). The reaction mixture was shaken on a water bath shaker at 37 °C for 3 h before the reaction was terminated. The reaction mixture was cooled to 25 °C in a water bath.

Compound **9**-shorter retention time compound **9-A** (2.1 mg, 1955 nmol) was dissolved in 150 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-22** of FADC-4A general formula in PBS buffer (3.56 mg/mL, 3.98 mL), which was then stored at 4 °C.

Mean calculated by UV-Vis: n = 6.79.

**Example 1-43. ADC-23 (reference example)**

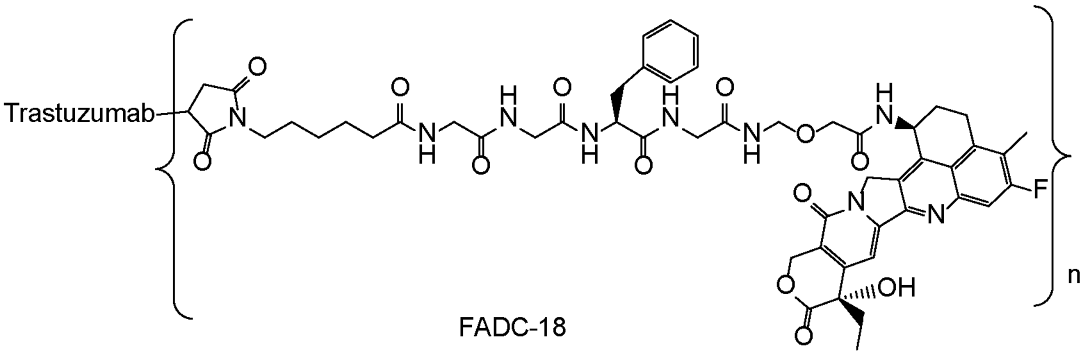

FADC-18

To antibody trastuzumab in aqueous PBS buffer (0.05 M aqueous PBS buffer at pH 6.5, 10.0 mg/mL, 345 mL, 23.31 μmol) was added at 37 °C a prepared aqueous solution of tris(2-carboxyethyl)phosphine (TCEP) (10 mM, 11.89 mL, 118.9 μmol). The reaction mixture was shaken on a water bath shaker at 37 °C for 3.5 h before the reaction was terminated. The reaction mixture was cooled to 25 °C in a water bath.

Compound **20** (362 mg, 350 μmol) was dissolved in 7.12 mL of MeCN and 3.56 mL of DMSO, and the solution was added to the above reaction mixture. The reaction mixture was shaken on a water bath shaker at 25 °C for 3 h before the reaction was terminated. The reaction mixture was purified by desalting through ultrafiltration membranes with 2% (v/v) MeCN and 1% (v/v) DMSO-containing aqueous PBS buffer (0.05 M aqueous PBS buffer at pH 6.5) and aqueous succinic acid buffer (0.01 M aqueous succinic acid buffer at pH 5.3) successively. Then sucrose was added at 60 mg/mL, and tween 20 at 0.2 mg/mL. The mixture was bottled and lyophilized to give a lyophilized powder sample of exemplary product **ADC-23** of FADC-18 general formula, which was then stored at 4 °C. Mean calculated by UV-Vis: n = 7.05.

**Example 1-44. ADC-24**

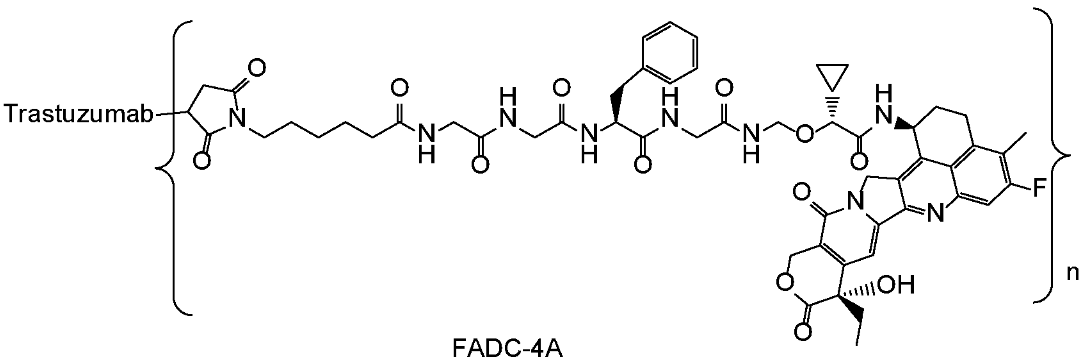

FADC-4A

To antibody trastuzumab in aqueous PBS buffer (0.05 M aqueous PBS buffer at pH 6.5, 10.0 mg/mL, 332 mL, 22.43 μmol) was added at 37 °C a prepared aqueous solution of tris(2-carboxyethyl)phosphine (TCEP) (10 mM, 11.44 mL, 114.4 μmol). The reaction mixture was shaken on a water bath shaker at 37 °C for 3.5 h before the reaction was terminated. The reaction mixture was cooled to 25 °C in a water bath.

Compound **9**-shorter retention time compound **9-A** (241 mg, 224 μmol) was dissolved in 13.76 mL of MeCN and 6.88 mL of DMSO, and the solution was added to the above reaction mixture. The reaction mixture was shaken on a water bath shaker at 25 °C for 3 h before the reaction was terminated. The reaction mixture was purified by desalting through ultrafiltration membranes with 4% (v/v) MeCN and 2% (v/v) DMSO-containing aqueous PBS buffer (0.05 M aqueous PBS buffer at pH 6.5) and aqueous succinic acid buffer (0.01 M aqueous succinic acid buffer at pH 5.3) successively. Then sucrose was added at 60 mg/mL, and tween 20 at 0.2 mg/mL. The mixture was bottled and lyophilized to give a lyophilized powder sample of exemplary product **ADC-24** of FADC-4A general formula, which was then stored at 4 °C.

Mean calculated by UV-Vis: n = 7.07.

**Example 1-45. ADC-25**

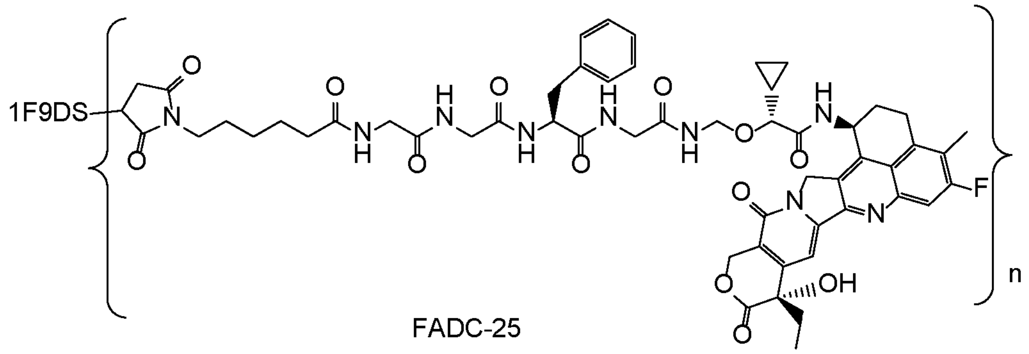

FADC-25

To antibody B7H3 antibody 1F9DS in aqueous PBS buffer (0.05 M aqueous PBS buffer at pH 6.5, 10.0 mg/mL, 2.14 mL, 144.60 nmol) was added at 37 °C a prepared aqueous solution of tris(2-carboxyethyl)phosphine (TCEP) (10 mM, 73.7 μL, 740 nmol). The reaction mixture was shaken on a water bath shaker at 37 °C for 3 h before the reaction was terminated. The reaction mixture was cooled to 25 °C in a water bath.

Compound **9**-shorter retention time compound **9-A** (3.0 mg, 2793 nmol) was dissolved in 150 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-25** of FADC-25 general formula in PBS buffer (1.28 mg/mL, 13.0 mL), which was then stored at 4 °C.

Mean calculated by UV-Vis: n = 6.87.

**Example 1-46. ADC-26 (reference example)**

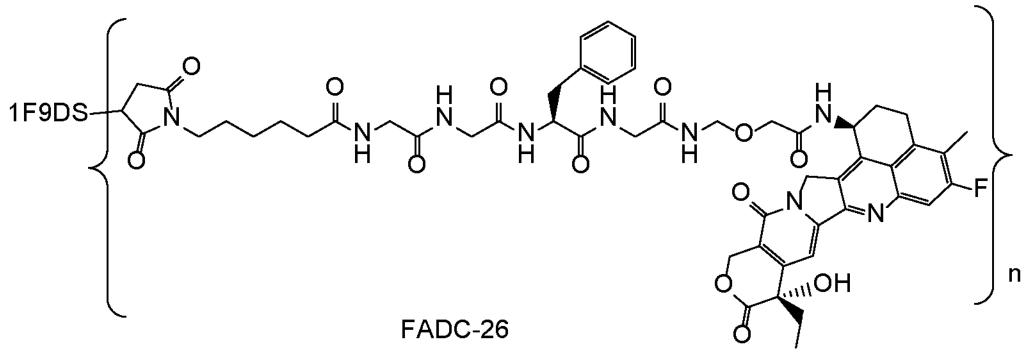

FADC-26

To antibody B7H3 antibody 1F9DS in aqueous PBS buffer (0.05 M aqueous PBS buffer at pH 6.5, 10.0 mg/mL, 0.89 mL, 60.14 nmol) was added at 37 °C a prepared aqueous solution of tris(2-carboxyethyl)phosphine (TCEP) (10 mM, 30.1 μL, 300 nmol). The reaction mixture was shaken on a water bath shaker at 37 °C for 3 h before the reaction was terminated. The reaction mixture was cooled to 25 °C in a water bath.

Compound **20** (1.0 mg, 967 nmol) was dissolved in 100 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-26** of FADC-26 general formula in PBS buffer (1.61 mg/mL, 4.0 mL), which was then stored at 4 °C.

Mean calculated by UV-Vis: n = 6.15.

**Example 1-47. ADC-27**

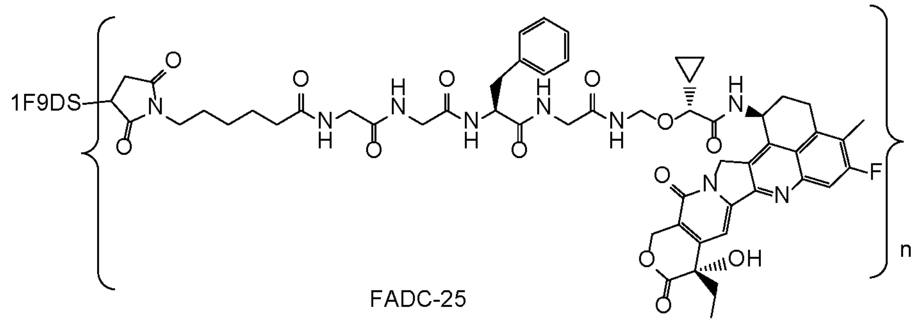

FADC-25

To antibody B7H3 antibody 1F9DS in aqueous PBS buffer (0.05 M aqueous PBS buffer at pH 6.5, 10.0 mg/mL, 0.89 mL, 60.14 nmol) was added at 37 °C a prepared aqueous solution of tris(2-carboxyethyl)phosphine (TCEP) (10 mM, 30.1 μL, 300 nmol). The reaction mixture was shaken on a water bath shaker at 37 °C for 3 h before the reaction was terminated. The reaction mixture was cooled to 25 °C in a water bath.

Compound **9**-shorter retention time compound **9-A** (1.02 mg, 950 nmol) was dissolved in 100 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-27** of FADC-25 general formula in PBS buffer (1.94 mg/mL, 3.5 mL), which was then stored at 4 °C.

Mean calculated by UV-Vis: n = 6.11.

**Example 1-48. ADC-28 (reference example)**

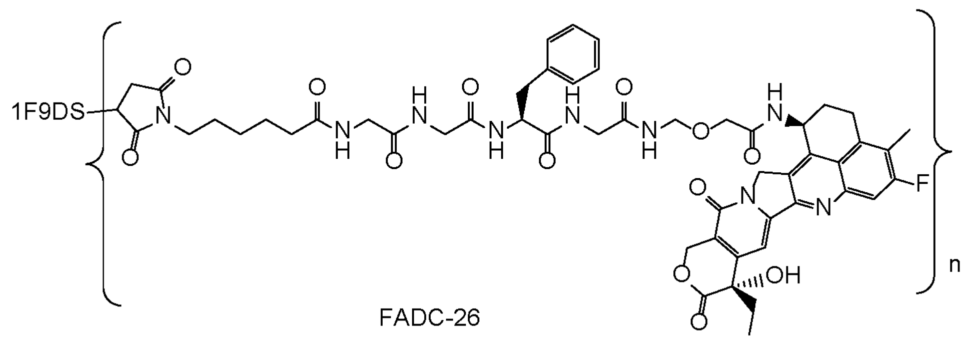

FADC-26

To antibody B7H3 antibody 1F9DS in aqueous PBS buffer (0.05 M aqueous PBS buffer at pH 6.5, 10.0 mg/mL, 2.36 mL, 159.47 nmol) was added at 37 °C a prepared aqueous solution of tris(2-carboxyethyl)phosphine (TCEP) (10 mM, 81.3 μL, 810 nmol). The reaction mixture was shaken on a water bath shaker at 37 °C for 3 h before the reaction was terminated. The reaction mixture was cooled to 25 °C in a water bath.

Compound **20** (3.0 mg, 2901 nmol) was dissolved in 150 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-28** of FADC-26 general formula in PBS buffer (1.29 mg/mL, 13.0 mL), which was then stored at 4 °C.

Mean calculated by UV-Vis: n = 7.46.

**Example 1-49. ADC-29**

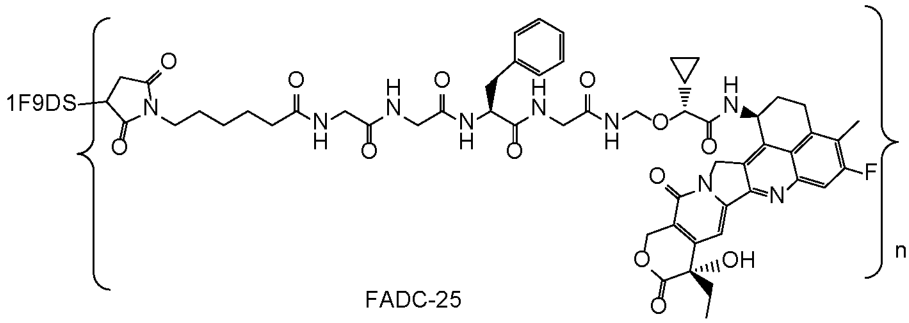

FADC-25

To antibody B7H3 antibody 1F9DS in aqueous PBS buffer (0.05 M aqueous PBS buffer at pH 6.5, 10.0 mg/mL, 0.80 mL, 50.06 nmol) was added at 37 °C a prepared aqueous solution of tris(2-carboxyethyl)phosphine (TCEP) (10 mM, 28.6 μL, 290 nmol). The reaction mixture was shaken on a water bath shaker at 37 °C for 3 h before the reaction was terminated. The reaction mixture was cooled to 25 °C in a water bath.

Compound **9**-shorter retention time compound **9-A** (1.29 mg, 1201 nmol) was dissolved in 100 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 M aqueous PBS buffer at pH 6.5, containing 0.001 M EDTA) to give exemplary product **ADC-29** of FADC-25 general formula in PBS buffer (2.63 mg/mL, 2.4 mL), which was then stored at 4 °C.

Mean calculated by UV-Vis: n = 7.24.

**Example 1-50. ADC-30 (reference example)**

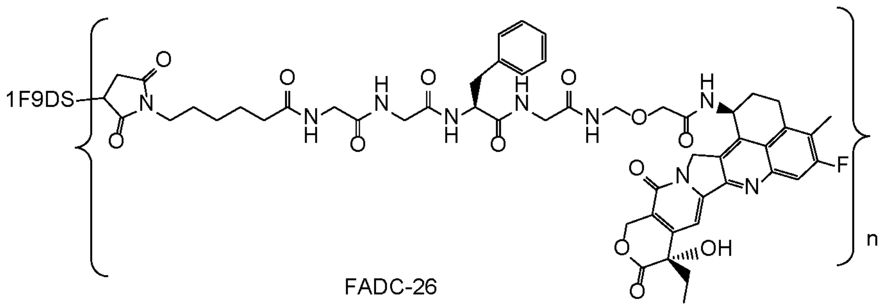

FADC-26

To antibody B7H3 antibody 1F9DS in aqueous PBS buffer (0.05 M aqueous PBS buffer at pH 6.5, 10.0 mg/mL, 0.86 mL, 58.4 nmol) was added at 37 °C a prepared aqueous solution of tris(2-carboxyethyl)phosphine (TCEP) (10 mM, 29.1 μL, 290 nmol). The reaction mixture was shaken on a water bath shaker at 37 °C for 3 h before the reaction was terminated. The reaction mixture was cooled to 25 °C in a water bath.

Compound **20** (1.0 mg, 967 nmol) was dissolved in 100 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-30** of FADC-26 general formula in PBS buffer (1.61 mg/mL, 4.0 mL), which was then stored at 4 °C.

Mean calculated by UV-Vis: n = 6.15.

**Example 1-51. ADC-31**

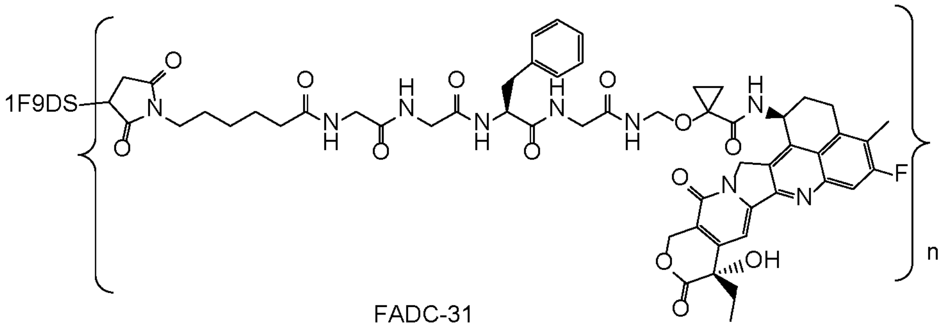

FADC-31

To antibody B7H3 antibody 1F9DS in aqueous PBS buffer (0.05 M aqueous PBS buffer at pH 6.5, 10.0 mg/mL, 0.89 mL, 60.14 nmol) was added at 37 °C a prepared aqueous solution of tris(2-carboxyethyl)phosphine (TCEP) (10 mM, 30.1 μL, 300 nmol). The reaction mixture was shaken on a water bath shaker at 37 °C for 3 h before the reaction was terminated. The reaction mixture was cooled to 25 °C in a water bath.

Compound **8** (1.0 mg, 943 nmol) was dissolved in 100 μL of DMSO, and the solution was added to the above reaction mixture. The reaction mixture was shaken on a water bath shaker at 25 °C for 3 h before the reaction was terminated. The reaction mixture was desalted and purified through a Sephadex G25 gel column (elution phase: 0.05 *M* aqueous PBS buffer at pH 6.5, containing 0.001 M EDTA) to give exemplary product **ADC-31** of FADC-31 general formula in PBS buffer (1.47 mg/mL, 4.5 mL), which was then stored at 4 °C.

Mean calculated by UV-Vis: n = 6.33.

**Example 1-52. ADC-32**

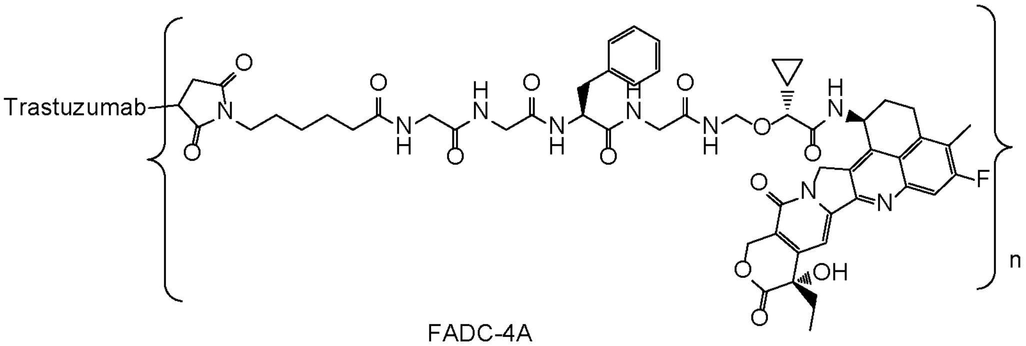

FADC-4A

At 25 °C, in a buffer containing 20 mM histidine-hydrochloric acid and 2.5 mM EDTA (pH 5.6), 5.0 g of trastuzumab stock solution (34.44 μmol; trastuzumab was diluted with 20 mM histidine-hydrochloric acid buffer to a final concentration of 15 mg/mL) and 34.64 mg of tris(2-carboxyethyl)phosphine hydrochloride (reducing agent TCEP, Sigma, 120.84 μmol) were stirred in a water bath at a constant temperature for 3 h to give a solution of intermediate I.

Compound **9**-shorter retention time compound **9-A** (406.2 mg, 378.17 μmol) was dissolved in 9.98 mL of DMSO to give a solution of compound **9-A** in DMSO. 23.42 mL of DMSO was added to the above solution of intermediate I in advance, and then the above solution of compound **9-A** in DMSO was added to the solution of intermediate I to which DMSO had been added in advance. The reaction mixture was stirred in a water bath at 25 °C for 1 h, quenched with cysteine, and filtered. At 25 °C, the reaction mixture was subjected to buffer exchange by 10-fold and 16-fold volume equal-volume ultrafiltration through an ultrafiltration membrane (30 kd) with a 10% (v/v) DMSO-containing aqueous buffer of 20 mM histidine-hydrochloric acid and 2.5 mM EDTA (pH = 6.0), and an aqueous buffer of 10 mM histidine-hydrochloric acid (pH = 5.5), successively, to remove small molecules and residual solvents, and exemplary product **ADC-32** of FADC-4A general formula was obtained.

Mean calculated by HIC: n = 6.0.

**Example 1-53. ADC-33**

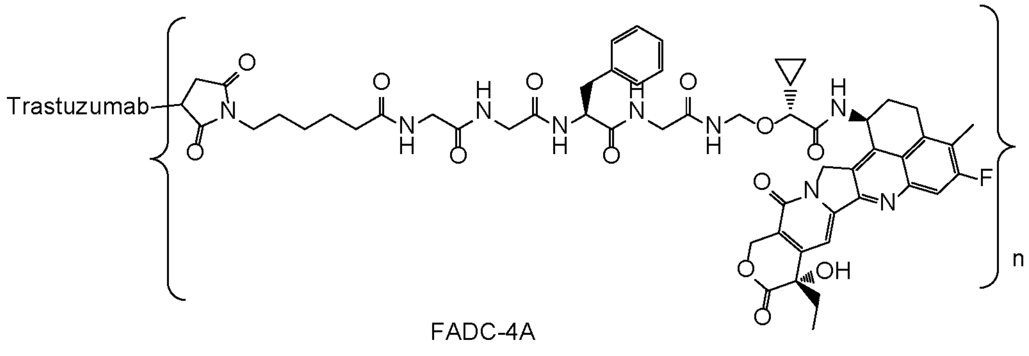

FADC-4A

At 37 °C, in a buffer containing 20 mM histidine-hydrochloric acid and 2.5 mM EDTA (pH 6.0), 8.0 g of trastuzumab stock solution (55.11 μmol; trastuzumab was diluted with 20 mM histidine buffer to a final concentration of 15 mg/mL) and 123.95 mg of tris(2-carboxyethyl)phosphine hydrochloride (reducing agent TCEP, Sigma, 432.41 μmol) were stirred in a water bath at a constant temperature for 5 h to give a solution of intermediate I.

Compound **9**-shorter retention time compound **9-A** (754.7 mg, 702.63 μmol) was dissolved in 15.99 mL of DMSO to give a solution of compound **9-A** in DMSO. 37.34 mL of DMSO was added to the above solution of intermediate I in advance, and then the above solution of compound **9-A** in DMSO was added to the solution of intermediate I to which DMSO had been added in advance. The reaction mixture was stirred in a water bath at 37 °C for 1 h before the reaction was terminated, and the reaction mixture was filtered. At 25 °C, the reaction mixture was subjected to buffer exchange by 8-fold and 16-fold volume equal-volume ultrafiltration through an ultrafiltration membrane (30 kd) with a 10% (v/v) DMSO-containing aqueous buffer of 20 mM histidine-hydrochloric acid and 2.5 mM EDTA (pH = 6.0), and an aqueous buffer of 10 mM histidine-hydrochloric acid (pH = 6.0), successively, to remove small molecules and residual solvents, and exemplary product **ADC-33** of FADC-4A general formula was obtained.

Mean calculated by HIC: n = 7.15.

**Drug loading analysis of ADC stock solution**

Purpose and principle of experiment

An ADC is an antibody cross-linked drug. Its mechanism for treating diseases is to transport toxin molecules into cells depending on the targeting ability of the antibody and kill the cells. The drug loading plays a decisive role in the drug efficacy. The drug loading in the ADC stock solution was determined by ultraviolet-visible spectrophotometry (UV-Vis) or hydrophobic interaction chromatography (HIC).

1. Ultraviolet-visible spectrophotometry

Cuvettes containing sodium succinate buffer were placed into the reference cell and sample cell, and the absorbance of the solvent blank was subtracted. Then, a cuvette containing test solution was placed into the sample cell, and the absorbances at 280 nm and 370 nm were determined.

Calculation: the loading in the ADC stock solution was determined by ultraviolet spectrophotometry (instrument: Thermo nanodrop 2000 ultraviolet spectrophotometer), based on the principle that the total absorbance value of the ADC stock solution at a certain wavelength was the sum of the absorbance values of the cytotoxic drug and the monoclonal antibody at that wavelength, namely:

$$(1)\ A_{280\ nm} = \varepsilon_{mab\text{-}280} b C_{mab} + \varepsilon_{Drug\text{-}280} b C_{Drug}$$

$\varepsilon_{Drug\text{-}280}$: the mean molar extinction coefficient of the drug at 280 nm was 5100;
$C_{Drug}$: the concentration of the drug;
$\varepsilon_{mab\text{-}280}$: the mean molar extinction coefficient of the trastuzumab stock solution or the pertuzumab stock solution at 280 nm was 214,600;
$C_{mab}$: the concentration of the monoclonal antibody stock solution of trastuzumab or pertuzumab;
b: the optical path length was 1 cm.

Similarly, an equation for the total absorbance value of the sample at 370 nm can be given as:

$$(2)\ A_{370\ nm} = \varepsilon_{mab\text{-}370} b C_{mab} + \varepsilon_{Drug\text{-}370} b C_{Drug}$$

$\varepsilon_{Drug\text{-}370}$: the mean molar extinction coefficient of the drug at 370 nm was 19,000;
$C_{Drug}$: the concentration of the drug;
$\varepsilon_{mab\text{-}370}$: the extinction coefficient of the monoclonal antibody stock solution of trastuzumab or pertuzumab at 370 nm was 0;
$C_{mab}$: the concentration of the monoclonal antibody stock solution of trastuzumab;
b: the optical path length was 1 cm.

The drug loading can be calculated using both equations (1) and (2) as well as the extinction coefficients of the monoclonal antibody and the drug at both wavelengths and their concentrations.

$$\text{Drug loading n} = C_{Drug}/C_{mab}.$$

2. Hydrophobic interaction chromatography

2.1 HPLC analysis was carried out under the following measuring conditions:

HPLC system: Agilent high performance liquid chromatograph HPLC system Detector: DAD detector (measurement wavelength: 280 nm)
Chromatography column: TSKgel Butyl-NPR (4.6 mm × 100 mm, 2.5 μm)
Column temperature: 30 °C
Flow rate: 0.5 mL/min
Sample chamber temperature: 4 °C
Mobile phase A: a pH 7.00 aqueous solution containing 1.5 M ammonium sulfate ($(NH_4)_2SO_4$) and 20 mM disodium phosphate ($Na_2HPO_4$).
Mobile phase B: a pH 7.00 mixed solution containing 75% 20 mM disodium phosphate ($Na_2HPO_4$) and 25% isopropanol.
Gradient program: 0.0% B to 0.0% B (0 min to 3.0 min), 0.0% B to 100.0% B (3.0 min to 30.0 min), 100.0% B to 100.0% B (30.0 min to 33.0 min), 0.0% B to 0.0% B (33.1 min to 40.0 min),
Sample injection amount: 50 μg

2.2 Data analysis

Based on the current data, because of the characteristics of the column, based on the difference in salt concentrations, the antibody drug conjugate was eluted in ascending order according to the number of bound drugs. Therefore, the distribution of the number of conjugated drugs was obtained by measuring the area of each peak. According to the elution order, the peaks were D0 (antibody with no drug linker bound), D2, D4, D6 and D8. The drug loading DAR (n) was calculated as:

Table 1. Drug loading calculation by hydrophobic interaction chromatography

| Name | Number of linked drugs | Weighted peak area percentage |
|---|---|---|
| D0 | 0 | Peak area of D0 × number of linked drugs |
| D2 | 2 | Peak area of D2 × number of linked drugs |
| D4 | 4 | Peak area of D4 × number of linked drugs |
| D6 | 6 | Peak area of D6 × number of linked drugs |
| D8 | 8 | Peak area of D8 × number of linked drugs |

$$\text{Drug loading n} = \Sigma(\text{weighted peak areas})/100$$

## Biological Evaluation

### Test Example 1-1: Test for Inhibition of *In Vitro* Proliferation of Tumor Cells by Compounds Disclosed Herein

I. Purpose

This experiment is intended to determine the inhibitory activity of the pharmaceutical compounds of the present disclosure against the *in vitro* proliferation of U87MG cells (Cell Bank, Chinese Academy of Sciences, Cat # TCHu138) and SK-BR-3 tumor cells (human breast cancer cells, ATCC, Cat # HTB-30). The cells were treated *in vitro* with a compound at different concentrations. After 6 days of culture, the proliferation of the cells was determined using a CTG (CellTiter-Glo® Luminescent Cell Viability Assay, Promega, Cat # G7573) reagent, and the *in vitro* activity of the compound was evaluated according to the $IC_{50}$ value.

II. Method

The method for testing the inhibition of the *in vitro* proliferation of U87MG cells was described below as an example for the method for assaying for the inhibitory activity of the compounds of the present disclosure against the *in vitro* proliferation of tumor cells. The method was also applicable to, but not limited to, the test for the inhibitory activity against the *in vitro* proliferation of other tumor cells.

1. Cell culture: U87MG and SK-BR-3 cells were cultured in a 10% FBS-containing EMEM medium (GE, Cat # SH30024.01) and a 10% FBS-containing McCoy's 5A medium (Gibco, Cat # 16600-108), respectively.
2. Cell preparation: U87MG and SK-BR-3 cells growing at log phase were collected, washed once with PBS (phosphate buffered saline, Shanghai BasalMedia Technologies Co., Ltd.), and then digested with 2-3 mL of trypsin (0.25% Trypsin-EDTA (1×), Gibico, Life Technologies) for 2-3 min; after being fully digested, the cells were eluted with 10-15 mL of a cell culture medium; the eluate was centrifuged at 1000 rpm for 5 min, and the supernatant was discarded; then the cells were resuspended in 10-20 mL of a cell culture medium to form single-cell suspensions.
3. Cell plating: the U87MG and SK-BR-3 single-cell suspensions were each well mixed, and their cell densities were adjusted with a cell culture medium to $2.75 \times 10^3$ cells/mL and $8.25 \times 10^3$ cells/mL, respectively; the density-adjusted cell suspensions were each well mixed and added to a 96-well cell culture plate at 180 μL/well. To each of the peripheral wells of the 96-well plates was added 200 μL of media only. The plates were incubated in an incubator (37 °C, 5% $CO_2$) for 24 h.
4. Compound preparation: the compound was dissolved in DMSO (dimethyl sulfoxide, Shanghai Titan Scientific Co., Ltd.) to prepare a stock solution at an initial concentration of 10 mM.

Small-molecule compounds were at an initial concentration of 500 nM. Drug formulation was performed as follows.

Different test samples at concentration 100 μM (30 μL) were added to the first column of a 96-well U-bottom plate, and 20 μL of DMSO was added to each well of the second column through the eleventh column. The samples in the first column (10 μL) were added to the 20 μL of DMSO in the second column, and the mixtures were well mixed. The mixtures (10 μL) were added to the third column, and so on to the tenth column. The drugs in the plate (5 μL per well) were transferred to EMEM media (95 μL), and the mixtures were well mixed for later use.

ADCs were prepared at an initial concentration of 10 nM or 500 nM as follows.

Different test samples at concentration 100 nM or 5 μM (100 μL) were added to the first column of a 96-well plate, and 100 μL of PBS was added to each well of the second column through the eleventh column. The samples in the first column (50 μL) were added to the 100 μL of PBS in the second column, and the mixtures were well mixed. The mixtures (50 μL) were added to the third column, and so on, by 3-fold dilution, to the tenth column.

5. Sample adding: the test samples prepared at different concentrations (20 μL) were added to the culture plate, with two duplicate wells set for each sample. The plate was incubated in an incubator (37 °C, 5% $CO_2$) for 6 days.
6. Color developing: the 96-well cell culture plate was taken out, and 90 μL of CTG solution was added to each well, followed by 10 min of incubation at room temperature.
7. Plate reading: the 96-well cell culture plate was taken out and tested in a microplate reader (BMG labtech, PHERAstar FS) for chemiluminescence.

III. Data analysis

Data were processed and analyzed using Microsoft Excel and Graphpad Prism 5. The experimental results

are shown in the Table 2 below.

Table 2. $IC_{50}$ values of the small-molecule fragments of the present disclosure in inhibiting *in vitro* proliferation of SK-BR-3 cells and U87 cells

| Compound No. | $IC_{50}$ (nM) | |
|---|---|---|
| | SK-BR-3 | U87 |
| 1 | 0.12 | 0.23 |
| 2-shorter retention time 2-B | 0.33 | 0.86 |
| 2-longer retention time 2-A | 8.11 | 2.31 |
| 3-shorter retention time | 0.36 | 0.83 |
| 3-longer retention time | 1.67 | 2.98 |
| 4 | 1.9 | / |
| 5 | / | 4.81 |
| 6 | / | 1.83 |
| 7 | / | 1.95 |

Conclusion: The small-molecule fragments of the present disclosure have significant inhibitory activity against the proliferation of SK-BR-3 cells and U87 cells, and the chiral centers have certain influence on the inhibitory activity of the compounds.

**Test Example 1-2: Test for Inhibition of *In Vitro* Proliferation of HER2-targeted Tumor Cells by Antibody Drug Conjugates of the Present Disclosure**

This experiment was intended to determine the inhibitory activity of the HER2-targeting antibody drug conjugates of the present disclosure against the *in vitro* proliferation of SK-BR-3 (human breast cancer cells, ATCC, Cat # HTB-30) and MDA-MB-468 (human breast cancer cells, ATCC, Cat # HTB-132). The cells were treated *in vitro* with a compound at different concentrations. After 6 days of culture, the proliferation of cells was determined using CTG reagents, and the *in vitro* activity of the compound was evaluated according to $IC_{50}$ value.

The test method of Test Example 1 was followed. SK-BR-3 and MDA-MB-468 cells were used in the tests, and the cell culture media were a 10% FBS-containing McCoy's 5A medium (Gibco, Cat # 16600-108), a 10% FBS-containing EMEM medium (GE, Cat # SH30024.01), and a 10% FBS-containing L-15 medium (ThermoFisher, Cat # 11415-114). The cell densities of the three strains of cells were adjusted to $8.33 \times 10^3$ cells/mL, $8.33 \times 10^3$ cells/mL and $1.39 \times 10^4$ cells/mL with cell culture media, respectively, and the density-adjusted cell suspensions were well mixed and added to 96-well cell culture plates at 180 $\mu$L/well. The related compounds were tested, and the results are shown in Table 3 below.

Table 3. $IC_{50}$ values of the antibody drug conjugates of the present disclosure in inhibiting *in vitro* proliferation of tumor cells with HER2 targets

| Compound No. | $IC_{50}$ (nM) | |
|---|---|---|
| | SK-BR-3 | MDA-MB-468 |
| ADC-3 | 0.43 | >50 |
| ADC-4 | 0.30 | >50 |
| ADC-6 | 0.48 | >50 |
| ADC-7 | 0.14 | >50 |
| ADC-9 | 0.95 | >50 |
| ADC-10 | 1.36 | >50 |
| ADC-11 | 0.73 | >50 |
| ADC-12 | 0.82 | >50 |

(continued)

| Compound No. | $IC_{50}$ (nM) | |
|---|---|---|
| | SK-BR-3 | MDA-MB-468 |
| ADC-13 | 0.47 | >50 |
| ADC-14 | 0.53 | >50 |
| ADC-15 | 0.38 | >50 |
| ADC-16 | 0.49 | >50 |
| ADC-17 | 0.37 | >50 |

Conclusion: the HER2-targeting antibody drug conjugates of the present disclosure showed significant inhibitory activity against the proliferation of HER2-positive SK-BR-3 cells while showed weak inhibitory activity against the proliferation of HER2-negative MDA-MB-468 cells, exhibiting good selectivity.

**Test Example 1-3: Plasma Stability Test for Her2-ADC**

Samples ADC-19, ADC-18 and ADC-20, human plasma, monkey plasma ( Shanghai Medicilon Inc.) and a solution of 1% BSA (Sigma) in PBS (Sangon Biotech (Shanghai)) were each filtered through a 0.22 μm filter for sterilization. ADC-19, ADC-18 and ADC-20 were each added to the sterile plasma or 1% BSA solution in PBS at a final concentration of 200 μg/mL. The resulting mixture was incubated in a cell incubator at 37 °C. The day of incubation was defined as day 0. Samples were taken on days 7, 14 and 21 and tested for the free toxin.

25 μL of samples were taken out to a 96-well plate; 50 μL of an internal standard working solution (100 ng/mL camptothecin solution in acetonitrile) and 150 μL of acetonitrile were added; the mixture was vortexed for 5 min and centrifuged for 10 min (4000 rpm), and 5 μL of the resulting samples were used for LC/MS/MS (Applied Biosystems, USA) analysis.

The results showed that: ADC-19 was fairly stable in both human and monkey plasma, as well as a solution of 1% BSA in PBS, with a release rate of free toxin of no more than 2.1% at the highest, and tended to be stable on day 14, see FIG. 1A.

ADC-18 was poorly stable in human and monkey plasma with release rates of free toxin of 14.5% and 8.10% at the highest, respectively. It was relatively stable in 1% BSA solution in PBS, see FIG. 1B.

ADC-20 was poorly stable in human plasma, monkey plasma, and a solution of 1% BSA in PBS, with release rates of free toxin of 21.7%, 29.7% and 21.7% at the highest, respectively. It was in a degraded state in the solution of 1% BSA in PBS, see FIG. 1C.

**Test Example 1-4: Evaluation of Drug Efficacy on JIMT-1 Tumor-Bearing Mice**

I. Purpose

With nu/nu nude mice used as test animals, Her2-ADC antibodies T-DM1, ADC-21 and ADC-24 were administered by intraperitoneal injection, and then their efficacy was evaluated in trastuzumab (Herceptin)-resistant human breast cancer cell strain JIMT-1 xenograft tumor nude mice.

II. Test drugs and materials

1. Test drugs

T-DM1 (prepared by reference US20050169933)
ADC-21: 3 mg/kg
ADC-21: 10 mg/kg
ADC-24: 3 mg/kg
ADC-24: 10 mg/kg
Blank control (Blank): PBS

2. Preparation method: all prepared by dilution in PBS.
3. Test animals

nu/nu nude mice, purchased from Beijing Vital River.

III. Method

Mice were inoculated subcutaneously on the right flank with JIMT-1 cells (Nanjing Cobioer) ($5 \times 10^6$ cells/mouse, with 50% artificial basement membrane). After 8 days, the tumors grew to a size of $203.09 \pm 11.94$ $mm^3$, and the animals were randomized into 6 groups of 8 (d1).

The drug was administrated by intraperitoneal injection for a total of 2 times. The tumor volumes and body weights were measured twice a week and the results were recorded. Excel 2003 statistical software was used. The mean values were calculated as avg; the SD values were calculated as STDEV; the SEM values were calculated as STDEV/SQRT; and the inter-group difference P-values were calculated as TTEST.

Tumor volume (V) was calculated as:

$$V = 1/2 \times L_{long} \times L_{short}^2$$

$$\text{Relative volume (RTV)} = V_T/V_0$$

$$\text{Tumor inhibition rate (\%)} = (C_{RTV} - T_{RTV})/C_{RTV}\ (\%)$$

Where $V_0$ and $V_T$ are the tumor volumes at the beginning and end of the experiment, respectively. $C_{RTV}$ and $T_{RTV}$ are the relative tumor volumes of the blank group (Vehicle, PBS) and the experimental groups, respectively, at the end of the experiment.

IV Results

The experimental results are shown in FIG. 2. The experiment ended after two intraperitoneal injections were performed and then observation was performed for 34 days. T-DM1 (10 mg/kg) had no inhibition effect on tumors; 3 mg/kg of ADC-21 had the tumor inhibition rate of 46.22% ($P < 0.01$); 10 mg/kg of ADC-21 had the tumor inhibition rate of 56.77% ($P < 0.001$); 3 mg/kg of ADC-24 had the tumor inhibition rate of 62.77% ($P < 0.001$); 10 mg/kg of ADC-24 had the tumor inhibition rate of 76.32% ($P < 0.001$). Under the condition of the same dosage, the tumor inhibition effect of the ADC-24 was significantly better than that of the ADC-21.

**Test Example 1-5: Evaluation of Drug Efficacy on SK-BR-3 Tumor-Bearing Mice**

I. Purpose

With nu/nu nude mice used as test animals, Her2-ADC antibodies ADC-21 and ADC-22 were administered by intraperitoneal injection, and then their efficacy was evaluated in human breast cancer cell SK-BR-3 xenograft tumor nude mice.

II. Test drugs and materials

1. Test drugs

ADC-21: 1 mg/kg
ADC-21: 6 mg/kg
ADC-22: 1 mg/kg
ADC-22: 6 mg/kg
Blank control (Blank): PBS.

2. Preparation method: all prepared by dilution in PBS.
3. Test animals

nu/nu nude mice, purchased from Beijing Vital River.

III. Method

Mice were inoculated subcutaneously on the right flank with SK-BR-3 cells (ATCC) ($5 \times 10^6$ cells/mouse, with 50% artificial basement membrane). After 20 days, the tumors grew to a size of $153.34 \pm 11.73$ $mm^3$, and the animals were randomized into 5 groups of 8 (d0).

The drug was administrated by intraperitoneal injection for a total of 1 time. The tumor volumes and body weights were measured twice a week and the results were recorded. Excel 2003 statistical software was used. The mean values were calculated as avg; the SD values were calculated as STDEV; the SEM values were calculated as STDEV/SQRT; and the inter-group difference P-values were calculated as TTEST.

Tumor volume (V) was calculated as:

$$V = 1/2 \times L_{long} \times L_{short}^2$$

$$\text{Relative volume (RTV)} = V_T/V_0$$

$$\text{Tumor inhibition rate (\%)} = (C_{RTV} - T_{RTV})/C_{RTV}\ (\%)$$

Where $V_0$ and $V_T$ are the tumor volumes at the beginning and end of the experiment, respectively. $C_{RTV}$ and $T_{RTV}$ are the relative tumor volumes of the blank control group and the experimental groups, respectively, at the end of the experiment.

IV Results

The experimental results are shown in FIG. 3. The experiment ended after one intraperitoneal injection was performed and then observation was performed for 28 days. 1 mg/kg of ADC-21 had the tumor inhibition rate of 15.01%; 6 mg/kg of ADC-21 had the tumor inhibition rate of 77.4%, and had significant difference compared with that of the blank control ($P < 0.001$). 1 mg/kg of ADC-22 had the tumor inhibition rate of 19.82%; 6 mg/kg of ADC-22 had the tumor inhibition rate of 98.38% ($P < 0.001$). Under the condition of the same dosage of 6 mg/kg, the tumor inhibition effect of the ADC-22 was significantly better than that of the ADC-21.

**Test Example 1-6: Plasma Stability**

Sample ADC-25 was well mixed with each of human plasma, monkey plasma and 1% BSA solution in PBS at a final concentration of 100 μg/mL, and then the mixtures were sterilized by filtration and incubated in a 37 °C water bath. The day of incubation was defined as day 0. Samples were taken on days 7, 14 and 21 and tested for the free toxin. Samples at different time points were taken out, left to cool to room temperature, and well mixed by vortexing. 25 μL of each of the samples was added to a 96-well plate, followed by the addition of 50 μL of an internal standard working solution (100 ng/mL camptothecin solution in acetonitrile) and 150 μL of acetonitrile. The resulting mixtures were vortexed for 5 min and centrifuged at 4000 rpm for 10 min. 5 μL of the supernatant was collected for LC/MS/MS analysis.

The results showed that: ADC-25 was fairly stable in both human and monkey plasma, as well as a solution of 1% BSA in PBS, with a release rate of free toxin of no more than 2% at the highest, and tended to be stable on day 14, see FIG. 4.

**Test Example 1-7: Evaluation of Efficacy of ADCs Against Human Brain Astrocytoma U87MG Nude Mouse Xenograft Tumor**

I. Purpose

With BALB/cA-nude mice used as test animals, the efficacy of the ADC compounds of the present disclosure against human brain astrocytoma U87MG nude mouse xenograft tumors was evaluated in this experiment.

II. Test drugs and materials

1. Test drugs

ADC-27 (3 mg/kg)
ADC-26 (3 mg/kg)
Blank control (Blank): PBS buffer at pH 7.4.

2. Preparation method: PBS buffer at pH 7.4.
3. Test animals

BALB/cA-nude mice: purchased from Shanghai Jiesijie Laboratory Animal Co., Ltd.

III. Method

Six to seven-week-old laboratory female BALB/cA-nude mice were inoculated subcutaneously with human brain astrocytoma U87MG cells (human brain astrocytoma, Cell Bank, Chinese Academy of Sciences, Cat # TCHu138). On the tenth day after inoculation of the cells, the animals were randomly grouped (D0), 8 animals per group, administered by intraperitoneal injection once a week for a total of 3 times, and tumor volumes and body weights were measured 2-3 times per week and the data were recorded. Tumor volume (V) was calculated as follows:

$$V = 1/2 \times a \times b^2$$

where a and b represent length and width, respectively.

$$\text{Relative volume (RTV)} = V_T/V_0$$

$$\text{Tumor inhibition rate (\%)} = (C_{RTV} - T_{RTV})/C_{RTV} \text{ (\%)}$$

Where $V_0$ and $V_T$ are the tumor volumes at the beginning and end of the experiment, respectively. $C_{RTV}$ and $T_{RTV}$ are the relative tumor volumes of the control group (Blank) and the experimental groups, respectively, at the end of the experiment.

IV Results

Intraperitoneal injection (i.p.) administration was given once a week for a total of 3 times; after observation for 22 days, 3 mg/kg of ADC-27 had the tumor inhibition rate of 63.3% ($P < 0.0001$), and 3 mg/kg of ADC-26 had the inhibition rate of 49.1%. ADC-27 showed a stronger anti-tumor efficacy than ADC-26.

The weights of all animals in each group were normal in the administration process, and the ADCs had no obvious toxic or side effect. The results are shown in Table 4 and FIG. 5. The detected antibodies could effectively inhibit the growth of U87MG xenograft tumor in tumor-bearing nude mice, and showed dose dependency.

Table 4. Efficacy of administered antibodies against human brain astrocytoma U87MG nude mouse xenograft tumors (D22)

| Group | Mean tumor volume ($mm^3$) | | | | Relative tumor volume | | Tumor inhibition rate (%) |
|---|---|---|---|---|---|---|---|
| | Day 0 | SEM | Day 22 | SEM | Day 22 | SEM | Day 22 |
| Blank control | 167.06 | 17.74 | 2906.96 | 327.6 | 17.76 | 1.63 | - |
| ADC-27 3mpk | 167.07 | 16.06 | 1172.48 | 80.27 | 7.55 | 0.95 | 63.3*** |

(continued)

| Group | Mean tumor volume ($mm^3$) | | | | Relative tumor volume | | Tumor inhibition rate (%) |
|---|---|---|---|---|---|---|---|
| | Day 0 | SEM | Day 22 | SEM | Day 22 | SEM | Day 22 |
| ADC-26 3mpk | 167.73 | 17.63 | 1561.03 | 303.37 | 8.83 | 1.17 | 49.1*** |
| *** indicates P < 0.001 | | | | | | | |

**Test Example 1-8: Evaluation of Efficacy of ADCs Against Pleural Effusion Metastatic Human Pharyngeal Cancer Cell Detroit 562 Nude Mouse Xenograft Tumors**

I. Purpose

With BALB/cA-nude mice used as test animals, and the efficacy of the ADC compounds of the present disclosure against pleural effusion metastatic human pharyngeal cancer cell Detroit 562 nude mouse xenograft tumors was evaluated in this experiment.

II. Test drugs and materials

1. Test drugs

    ADC-29 (3 mg/kg)
    ADC-28 (3 mg/kg)
    Negative control ADC (3 mg/kg): antibody drug conjugate formed by coupling non-B7H3-targeted antibodies to compound 20.

2. Preparation method: all prepared by dilution in PBS.
3. Test animals

BALB/cA-nude mice: purchased from Changzhou Cavens Laboratory Animal Co., Ltd.

III. Method

Six to seven-week-old laboratory female BALB/cA-nude mice were inoculated subcutaneously with pleural effusion metastatic human pharyngeal cancer cells Detroit 562 cells (ATCC, Cat # ATCC® CCL-138™). On the tenth day after inoculation of the cells, the animals were randomly grouped (D0), 8 animals per group, administered by intraperitoneal injection once a week for a total of 3 times, and tumor volumes and body weights were measured 2-3 times per week and the data were recorded. Tumor volume (V) was calculated as follows:

$$V = 1/2 \times a \times b^2$$

where a and b represent length and width, respectively.

$$\text{Relative volume (RTV)} = V_T/V_0$$

$$\text{Tumor inhibition rate (\%)} = (C_{RTV} - T_{RTV})/C_{RTV}\ (\%)$$

Where $V_0$ and $V_T$ are the tumor volumes at the beginning and end of the experiment, respectively. $C_{RTV}$ and $T_{RTV}$ are the relative tumor volumes of the control group (negative control) and the experimental groups, respectively, at the end of the experiment.

IV Results

The drugs were administered by intraperitoneal injection once a week, and a total of 3 injections were administered. On day 28 of observation, tumor inhibition rates of the test ADCs ADC-29 3 mg/kg (3 mpk) and ADC-28 3 mg/kg mg/kg (3 mpk) reached 72.27% (P < 0.001) and 56.2% (P < 0.001), respectively. ADC-29 showed a stronger anti-tumor efficacy than ADC-28.

The weights of all animals in each group were normal in the administration process, and the ADCs had no obvious toxic or side effect. The results are shown in Table 5 and FIG. 6. The detected antibodies could effectively inhibit the growth of Detroit 562 xenograft tumor in tumor-bearing nude mice, and showed dose dependency.

Table 5. Efficacy of administered antibodies against Detroit 562 xenograft tumors in tumor-bearing nude mice (D28)

| Group | Mean tumor volume ($mm^3$) | | Mean tumor volume ($mm^3$) | | Relative tumor volume | | Tumor inhibition rate (%) Day 28 |
|---|---|---|---|---|---|---|---|
| | Day 0 | SEM | Day 28 | SEM | Day 28 | SEM | |
| Negative control | 182.70 | 6.79 | 1317.99 | 223.20 | 7.47 | 1.46 | - |
| ADC-29 3mpk | 182.59 | 6.50 | 381.48 | 105.76 | 2.07 | 0.58 | 72.27*** |
| ADC-28 3mpk | 182.57 | 6.92 | 578.07 | 160.13 | 3.43 | 1.09 | 56.2*** |
| *** indicates P < 0.001 | | | | | | | |

**Test Example 1-9: Evaluation of Drug Efficacy on U87-MG Tumor-Bearing Mice**

I. Purpose

With Balb/c nude mice used as test animals, the efficacy of the B7H3-antibody drug conjugate after intraperitoneal injection was evaluated on a human glioblastoma cell U87MG xenograft tumor model of the mice.

II. Test drugs and materials

1. Test drugs

   ADC-30 1 mg/kg
   ADC-30 3 mg/kg
   ADC-31 1 mg/kg
   ADC-31 3 mg/kg
   Blank control (Blank): PBS

2. Preparation method: all prepared by dilution in PBS.
3. Test animals

BALB/cA-nude mice: purchased from Shanghai Slac Laboratory Animal Co., Ltd.

III. Method

Mice were inoculated subcutaneously on the right flank with U87MG cells (human brain astrocytoma, Cell Bank, Chinese Academy of Sciences, Cat # TCHu138) ($2.5 \times 10^6$ cells/mouse). After 14 days, the tumors grew to a size of 167.49 $mm^3$, and the animals were randomized into 5 groups of 8 (d1).

The drug was administrated by intraperitoneal injection once a week, and a total of 3 injections were given. The tumor volumes and body weights were measured twice a week and the results were recorded.

Excel 2003 statistical software was used. The mean values were calculated as avg; the SD values were calculated as STDEV; the SEM values were calculated as STDEV/SQRT; and the inter-group difference P-values were calculated as TTEST.

Tumor volume (V) was calculated as:

$$V = 1/2 \times L_{long} \times L_{short}^2$$

$$\text{Relative volume (RTV)} = V_T/V_0$$

$$\text{Tumor inhibition rate (\%)} = (C_{RTV} - T_{RTV})/C_{RTV}\ (\%)$$

Where $V_0$ and $V_T$ are the tumor volumes at the beginning and end of the experiment, respectively. $C_{RTV}$ and $T_{RTV}$ are the relative tumor volumes of the blank control group (Vehicle) and the experimental groups, respectively, at the end of the experiment.

IV Results

The experimental results are shown in FIG. 7. The drugs were administrated by intraperitoneal injection once a week, and a total of 3 injections were administered. On day 18 of observation, tumor inhibition rates of test ADCs ADC-30 1 mg/kg, ADC-30 3 mg/kg, ADC-31 1 mg/kg and ADC-31 3 mg/kg reached 0.31%, 45.23% ($P < 0.0001$), 39.22% ($P < 0.01$) and 80.24% ($P < 0.0001$), respectively. Under the condition of the same dosage, the tumor inhibition effect of the ADC-31 was significantly better than that of the ADC-30.

**II. Formulation**

**The equipment used in the formulation preparation and determination and the calculation method for results are shown below.**

**SEC molecular exclusion chromatography:**

This is a method for analyzing the separation of asolute by the relative relationship between the pore size of the gel pores and the size of the polymer sample molecule coil. SEC% (SEC monomer content percentage) = A monomer/A total × 100% (A monomer represents the peak area of the main peak monomer in the sample, and A total represents the sum of all peak areas).

Instrument for SEC measurement: Agilent 1260; column: waters, XBrige BEH200Å SEC (300 × 7.8 mm 3.5 μm).

**CE capillary gel electrophoresis:**

This is a method of moving the gel into a capillary as a supporting medium for electrophoresis and separating according to the molecular weight of the sample under a certain voltage.

Reduced CE purity percentage = A main peak/A total × 100% (A main peak represents the light chain main peak area + the heavy chain main peak area, and A total represents the sum of all peak areas).

Instrument for CE measurement: Beckman model plus800

**Turbidity measurement:**

The degree to which light is hindered from passing through a water layer indicates the ability of the water layer to scatter and absorb light. It is related not only to the suspended matter content but also to the particle composition, size and shape as well as the reflection performance of its surface. In comparing the absorption values of the same protein sample at the same concentration at the same wavelength (within near ultraviolet and visible wavelength regions), the greater the absorption value is, the greater the turbidity is, the more the protein molecules in the sample tend to aggregate. The instrument for measurement was a multifunctional microplate reader (Molecular Devices M5). Samples in the same volume were added to a 96-well plate and the absorbance values were read.

**Osmotic pressure measurement:**

The freezing point method was used for measuring the osmotic pressure. The freezing point of a solution is measured by using a high-sensitivity temperature-sensing element on the basis of the proportional relation between the

freezing point depression value and the molar concentration of the solution, and then converted into the osmotic pressure through electric quantity. Manufacturer of instrument: Loser, model: OM815.

**Protein concentration determination:**

Because the toxin in the antibody drug conjugate absorbed light at 280 nm, the protein concentration was corrected using the following formulas:

$$A_{280} = C_d \times \varepsilon_{280d} + C_{mab} \times \varepsilon_{280mab}$$

$$A_{370} = C_d \times \varepsilon_{370d}$$

where Cd represents the toxin concentration, Cmab represents the protein concentration, ε280d represents the extinction coefficient of the toxin at 280 nm, ε280mab represents the extinction coefficient of the protein at 280 nm, ε370d represents the extinction coefficient of the toxin at 370 nm, ε280mab = 1.49 mg-1*cm-1*mL, ε280d = 5000 (molar extinction coefficient of toxin at 280 nm)/1074.13 (molecular weight of toxin) = 4.65 mg-1*cm-1*mL, and ε370d = 19000 (molar extinction coefficient of toxin at 370 nm)/1074.13 (molecular weight of toxin) = 17.69 mg-1*cm-1*mL; the above extinction coefficients were mass extinction coefficients.

Instrument for protein concentration measurement: ultraviolet-visible spectrophotometer; model: Nano Drop oneC; optical path length: 1 mm.

Illustratively, a molecular weight of the naked antibody is 145.181 kDa, and a molecular weight of the toxin is 1074 Da; if the mean DAR value is 5.7, the calculated ADC molecular weight will be 151.317 kDa, which is 1.042 times that of the naked antibody. Illustratively, if the ADC DAR value is 5.7 and the concentration of the naked antibody protein is 20.00 mg/mL, the ADC concentration will be 20.84 mg/mL.

**Example 2-1. Screening for Buffer Systems and pH Values for Formulations**

20 mg/mL (protein concentration based on naked antibody) ADC-33 formulations (containing no saccharide, surfactant and other buffers except for the following buffer systems) were prepared using the following buffer systems:

1) 10 mM histidine-sodium acetate, pH 5.0
2) 10 mM histidine-sodium acetate, pH 5.5
3) 10 mM histidine-sodium acetate, pH 6.0
4) 10 mM histidine-sodium acetate, pH 6.5
5) 10 mM histidine-histidine hydrochloride, pH 5.5
6) 10 mM succinic acid-sodium succinate, pH 5.5
7) 10 mM citric acid-sodium citrate, pH 5.5

Samples were taken for a high-temperature stability study (40 °C) and a shaking study (25 °C, 300 rpm). The shaken sample contained 0.1 mg/mL polysorbate 80 (PS80), and the other samples contained 0.4 mg/mL PS80. Each formulation was filtered, and a container was filled with it, plugged and capped. The samples were subjected to the forced degradation experiments described above and examined for appearance, SEC and turbidity.

The results are shown in Table 6. The results show that where histidine-sodium acetate was used as the buffer, the appearances of the samples gradually worsened, the turbidities increased, and the SEC monomer proportion decreased, as the pH value increased within pH 5.0-6.5. At pH 5.5, the sample containing 10 mM histidine-sodium acetate and the sample containing 10 mM histidine hydrochloride were similar in appearance and SEC%, and were superior to those containing 10 mM succinic acid-sodium succinate or 10 mM citric acid-sodium citrate. Surprisingly, the formulation containing 10 mM succinic acid-sodium succinate was significantly superior in turbidity to the other formulations after the shaking study.

Table 6. pH and buffer system stability results

| No. | Conditions | Appearance | Turbidity A550 | SEC% |
|---|---|---|---|---|
| 1 | D0 | Clear and transparent | 0.051 | 95.9 |
| | 40°C D12 | Slightly opalescent | 0.058 | 92.0 |
| | Shaking D12 | Slightly opalescent | 0.055 | 95.0 |
| 2 | D0 | Clear and transparent | 0.050 | 95.8 |
| | 40°C D12 | Slightly opalescent | 0.061 | 91.6 |
| | Shaking D12 | Slightly opalescent | 0.056 | 93.3 |
| 3 | D0 | Clear and transparent | 0.051 | 95.4 |
| | 40°C D12 | Slightly opalescent | 0.061 | 91.0 |
| | Shaking D12 | Slightly opalescent | 0.060 | 91.3 |
| 4 | D0 | Clear and transparent | 0.050 | 94.4 |
| | 40°C D12 | A large amount of particles like a haze of smoke | 0.085 | 89.3 |
| | Shaking D12 | Slightly opalescent | 0.058 | 87.4 |
| 5 | D0 | Clear and transparent | 0.051 | 95.5 |
| | 40°C D12 | Slightly opalescent | 0.065 | 90.9 |
| | Shaking D12 | Slightly opalescent | 0.060 | 93.1 |
| 6 | D0 | Clear and transparent | 0.053 | 95.7 |
| | 40°C D12 | Slightly opalescent, fine particles | 0.055 | 89.7 |
| | Shaking D12 | Slightly opalescent | 0.053 | 91.5 |
| 7 | D0 | Clear and transparent | 0.057 | 95.5 |
| | 40°C D12 | Opalescent | 0.063 | 88.8 |
| | Shaking D12 | Opalescent | 0.059 | 89.3 |
| Note: in the table, "D" indicates day; for example, D12 indicates 12 days, and D0 indicates the start of the experiment; the same applies below. | | | | |

**Example 2-2. Screening for Surfactant Species**

20 mg/mL (protein concentration based on naked antibody) ADC-32 formulations containing different polysorbate surfactants and each containing 10 mM succinic acid-sodium succinate (pH 5.0) buffer and 80 mg/mL sucrose were prepared. Each formulation was filtered, and a container was filled with it, plugged and capped. The samples were subjected to a high-temperature stability study (40 °C) and a shaking study (25 °C, 300 rpm) and examined for appearance, SEC and reduced CE.

The results are shown in Table 7. There were no significant differences in appearance, SEC and reduced CE between the PS20 (polysorbate 20)-containing formulations and the PS80-containing formulations under different conditions, which indicates that both PS20 and PS80 were able to stabilize ADC-32 effectively.

Table 7. Results of the screening for polysorbate species

| Polysorbate species | Conditions | Appearance | SEC% | Reduced CE% |
|---|---|---|---|---|
| PS80 0.2 mg/mL | D0 | Clear and transparent | 99.1 | 98.7 |
| | Shaking D15 | Clear and transparent | 97.2 | 98.4 |
| | 40°C D15 | Clear and transparent | 95.4 | 98.3 |

(continued)

| Polysorbate species | Conditions | Appearance | SEC% | Reduced CE% |
|---|---|---|---|---|
| PS20 0.2 mg/mL | D0 | Clear and transparent | 99.0 | 98.7 |
| | Shaking D15 | Clear and transparent | 97.7 | 98.7 |
| | 40°C D15 | Clear and transparent | 95.4 | 98.2 |

**Examples 2-3. Screening for Surfactant Concentrations**

Samples containing 0, 0.1, 0.2, 0.4 and 0.6 mg/mL PS80 were prepared. Each sample also contained 10 mM succinic acid-sodium succinate (pH 5.0), 80 mg/mL sucrose and 20 mg/mL (protein concentration based on naked antibody) ADC-32. The samples were filtered to remove bacteria and particles and diluted with 0.9% NaCl such that the ADC-32 concentration was 0.2 mg/mL. The dilutions were examined for appearance and insoluble microparticles.

The results are shown in Table 8. Where the PS80 concentration in the formulation was at 0-0.2 mg/mL, as the concentration increased, the increase in the insoluble microparticles in the dilution became smaller and there were fewer and fewer visible particles. Where the concentration was at 0.2-0.6 mg/mL, there were no differences in insoluble microparticles between the dilutions and the appearances were good.

Table 8. Results of the screening for PS80 concentrations

| PS80 concentration | Time | Appearance | Insoluble microparticles/100 mL | | |
|---|---|---|---|---|---|
| | | | ≧2μm | ≧10μm | ≧25μm |
| 0mg/mL | 0h | Clear and transparent, occasional particles | 8892 | 458 | 33 |
| | 4°C 24h | Distinct particles | 39408 | 4317 | 100 |
| 0.1mg/mL PS80 | 0h | Clear and transparent, occasional particles | 5267 | 525 | 17 |
| | 4°C 24h | Clear and transparent, occasional particles | 13342 | 508 | 0 |
| 0.2mg/mL PS80 | 0h | Clear and transparent | 2733 | 308 | 17 |
| | 4°C 24h | Clear and transparent | 4050 | 283 | 17 |
| 0.4mg/mL PS80 | 0h | Clear and transparent | 1367 | 175 | 17 |
| | 4°C 24h | Clear and transparent | 3267 | 433 | 8 |
| 0.6mg/mL PS80 | 0h | Clear and transparent | 2933 | 267 | 0 |
| | 4°C 24h | Clear and transparent | 4167 | 192 | 0 |

**Examples 2-4. Screening for Sugar Species**

Different samples containing 80 mg/mL sucrose, trehalose and mannitol were prepared. Each sample also contained 10 mM succinic acid-sodium succinate (pH 5.0), 0.2 mg/mL PS80 and 20 mg/mL (protein concentration based on naked antibody) ADC-32. The samples were filtered to remove bacteria and particles. Each formulation was filtered, and a container was filled with it, plugged and capped. The samples were subjected to a high-temperature stability study (40 °C) and a -35 °C/4 °C freezing/thawing cycle study and examined for appearance, SEC and reduced CE.

The results are shown in Table 9. Under freezing/thawing conditions, sucrose was superior to trehalose and mannitol in appearance. Sucrose and trehalose were superior to mannitol in SEC results. At 40 °C, sucrose was slightly superior to trehalose in reduced CE results.

Table 9. Results of the screening for saccharide species

| Saccharide species | Conditions | Appearance | SEC% | Reduced CE% |
|---|---|---|---|---|
| 80 mg/mL sucrose | D0 | Clear and transparent | 99.23 | 97.44 |
| | 5 freezing/ thawing cycles | Clear and transparent | 98.88 | 97.54 |
| | 40°C D17 | Clear and transparent | 95.05 | 98.04 |

(continued)

| Saccharide species | Conditions | Appearance | SEC% | Reduced CE% |
|---|---|---|---|---|
| 80 mg/mL trehalose | D0 | Clear and transparent | 99.23 | 97.33 |
| | 5 freezing/thawing cycles | A small amount of fine particles | 98.83 | 98.36 |
| | 40°C D17 | Clear and transparent | 95.38 | 96.50 |
| 80 mg/mL mannitol | D0 | Clear and transparent | 99.20 | 97.55 |
| | 5 freezing/thawing cycles | Distinct fine particles | 90.24 | 96.72 |
| | 40°C D17 | Clear and transparent | 95.29 | 97.28 |

**Examples 2-5. Screening for pH, Antibody Drug Conjugate Concentrations and Polysorbates**

With 10 mM succinic acid-sodium succinate as the buffer and 80 mg/mL sucrose as the stabilizer, formulas were designed with respect to the pH, ADC-32 protein concentration (based on the naked antibody) and the polysorbate concentration, as shown in Table 10. A high-temperature stability study (40 °C), a lighting study (4 °C, 4500 Lx) and a - 35 °C/4 °C freezing/thawing cycle study were conducted, and the results were statistically analyzed using the least square method with SEC and reduced CE as evaluation indexes. The results are shown in Table 11 and FIG. 8. The formulations of the present disclosure are shown to be highly pH-dependent. Under lighting, shaking and 40 °C conditions, as the pH increased, there was a greater decrease in SEC; similarly, under lighting conditions, as the pH increased, reduced CE increased, and there was a greater decrease in the monomer peak, which suggests that the formulations of the present disclosure should be at low pH values to have improved stability. Under shaking conditions, as the PS80 concentration increased, there was a downward trend toward SEC. There were no differences in the effects PS80 had on SEC and reduced CE under the other conditions. As the ADC-32 protein concentration increased, there was a greater decrease in the SEC monomer peak at 40 °C. There were no differences in the effects the ADC-32 protein concentration had on SEC and reduced CE under the other conditions.

Table 10. Formula designs for screening experiments

| Formula No. | pH | PS80 ($10^{-4}$g/mL) | ADC-32 protein concentration (mg/mL, based on naked antibody) |
|---|---|---|---|
| 1 | 5.0 | 1.7 | 10 |
| 2 | 5.2 | 1 | 30 |
| 3 | 5.5 | 3 | 10 |
| 4 | 4.8 | 2 | 30 |
| 5 | 4.8 | 1 | 20 |
| 6 | 5.5 | 1 | 10 |
| 7 | 5.5 | 2 | 30 |
| 8 | 4.8 | 3 | 16 |
| 9 | 5.5 | 2 | 20 |
| 10 | 5.2 | 3 | 30 |
| 11 | 5.2 | 2 | 20 |
| 12 | 5.2 | 2 | 20 |

Table 11. Results of the screening for formulas

| No. | Conditions | Appearance | SEC% | △SEC% | Reduced CE% | △Reduced CE% |
|---|---|---|---|---|---|---|
| 1 | D0 | Clear and transparent | 99.2 | 0.0 | 99.0 | 0.0 |
| | Lighting D10 | Clear and transparent, yellowish | 88.9 | 10.3 | 91.5 | 7.5 |
| | Shaking D15 | Clear and transparent | 97.8 | 1.4 | 99.0 | 0.0 |
| | 40 °C D15 | Clear and transparent | 96.0 | 3.2 | 97.9 | 1.1 |
| 2 | D0 | Clear and transparent | 98.9 | 0.0 | 98.7 | 0.0 |
| | Lighting D10 | Clear and transparent, yellowish | 79.4 | 19.5 | 90.5 | 8.2 |
| | Shaking D15 | Clear and transparent | 96.3 | 2.6 | 99.1 | -0.4 |
| | 40 °C D15 | Clear and transparent | 94.7 | 4.2 | 97.9 | 0.7 |
| 3 | D0 | Clear and transparent | 98.9 | 0.0 | 98.7 | 0.0 |
| | Lighting D10 | Clear and transparent, yellowish | 77.2 | 21.7 | 87.2 | 11.5 |
| | Shaking D15 | Clear and transparent | 96.9 | 2.0 | 97.5 | 1.2 |
| | 40 °C D15 | Clear and transparent | 95.1 | 3.8 | 98.2 | 0.5 |
| 4 | D0 | Clear and transparent | 99.0 | 0.0 | 98.7 | 0.0 |
| | Lighting D10 | Clear and transparent, yellowish | 86.3 | 12.7 | 91.4 | 7.3 |
| | Shaking D15 | Clear and transparent | 97.7 | 1.3 | 98.8 | -0.1 |
| | 40 °C D15 | Clear and transparent | 95.3 | 3.7 | 98.1 | 0.6 |
| 5 | D0 | Clear and transparent | 99.1 | 0.0 | 98.7 | 0.0 |
| | Lighting D10 | Clear and transparent, yellowish | 90.0 | 9.1 | 92.1 | 6.7 |
| | Shaking D15 | Clear and transparent | 97.5 | 1.6 | 98.4 | 0.4 |
| | 40 °C D15 | Clear and transparent | 95.7 | 3.4 | 98.0 | 0.7 |
| 6 | D0 | Clear and transparent | 98.9 | 0.0 | 98.9 | 0.0 |
| | Lighting D10 | Clear and transparent, yellowish | 76.2 | 22.8 | 86.9 | 12.0 |
| | Shaking D15 | Clear and transparent | 95.0 | 4.0 | 98.5 | 0.4 |
| | 40 °C D15 | Clear and transparent | 95.2 | 3.7 | 98.1 | 0.8 |
| 7 | D0 | Clear and transparent | 98.7 | 0.0 | 98.3 | 0.0 |
| | Lighting D10 | Clear and transparent, yellowish | 68.2 | 30.5 | 86.8 | 11.4 |
| | Shaking D15 | Clear and transparent | 96.1 | 2.6 | 98.9 | -0.6 |
| | 40 °C D15 | Clear and transparent | 94.3 | 4.4 | 97.7 | 0.6 |
| 8 | D0 | Clear and transparent | 99.2 | 0.0 | 99.0 | 0.0 |
| | Lighting D10 | Clear and transparent, yellowish | 88.3 | 10.9 | 91.8 | 7.2 |
| | Shaking D15 | Clear and transparent | 98.1 | 1.0 | 98.9 | 0.1 |
| | 40 °C D15 | A small amount of small particles | 95.8 | 3.4 | 98.5 | 0.5 |
| 9 | D0 | Clear and transparent | 98.9 | 0.0 | 97.6 | 0.0 |
| | Lighting D10 | Clear and transparent, yellowish | 73.8 | 25.1 | 87.1 | 10.5 |
| | Shaking D15 | Clear and transparent | 96.3 | 2.6 | 97.7 | -0.1 |
| | 40 °C D15 | Clear and transparent | 94.7 | 4.1 | 97.1 | 0.5 |

(continued)

| No. | Conditions | Appearance | SEC% | △SEC% | Reduced CE% | △Reduced CE% |
|---|---|---|---|---|---|---|
| 10 | D0 | Clear and transparent | 98.9 | 0.0 | 98.1 | 0.0 |
| | Lighting D10 | Clear and transparent, yellowish | 80.9 | 18.0 | 90.4 | 7.7 |
| | Shaking D15 | Clear and transparent | 97.3 | 1.6 | 99.0 | -1.0 |
| | 40 °C D15 | Clear and transparent | 94.8 | 4.2 | 98.5 | -0.5 |
| 11 | D0 | Clear and transparent | 99.1 | 0.0 | 98.7 | 0.0 |
| | Lighting D10 | Clear and transparent, yellowish | 80.5 | 18.6 | 88.7 | 10.0 |
| | Shaking D15 | Clear and transparent | 97.2 | 1.9 | 98.4 | 0.3 |
| | 40 °C D15 | Clear and transparent | 95.4 | 3.7 | 98.3 | 0.3 |
| 12 | D0 | Clear and transparent | 99.0 | 0.0 | 98.7 | 0.0 |
| | Lighting D10 | Clear and transparent, yellowish | 78.2 | 20.8 | 88.4 | 10.3 |
| | Shaking D15 | Clear and transparent | 97.1 | 1.9 | 99.1 | -0.4 |
| | 40 °C D15 | Clear and transparent | 95.4 | 3.6 | 97.9 | 0.8 |

**Examples 2-6. Lyophilization Process Development**

A 20 mg/mL (protein concentration based on naked antibody) ADC-33 stock solution containing 10 mM succinic acid-sodium succinate pH 5.0, 80 mg/mL sucrose and 0.2 mg/mL PS80 was prepared, filtered, bottled and then lyophilized according to the lyophilization process parameters 1 (see Table 12). The lyophilization sample was in the form of a white powder cake with a flat surface and contained 1.05% water. The bottom of the powder cake was slightly depressed in the center. The sample was reconstituted with water for injection, and the pH value was measured. The results are shown in Table 13. The ionic strength (≥ 10 mM) was high enough for buffering. The formula included 80 mg/mL sucrose, which could meet the iso-osmotic requirement.

Table 12. Lyophilization process parameters 1

| Lyophilization process parameter | Temperature setting (°C) | Time setting (min) | Retention time (h) | Degree of vacuum (Pa) |
|---|---|---|---|---|
| Pre-freezing | 5 | 10 | 1 | N/A |
| Pre-freezing | -45 | 50 | 2 | N/A |
| Primary drying | -25 | 120 | 40 | 10 |
| Secondary drying | 25 | 60 | 8 | 1 |

Table 13. Final formula pH and osmolality results

| pH of buffer | Osmotic pressure of stock solution | pH of stock solution | pH after reconstitution |
|---|---|---|---|
| 5.00 | 298mosm | 5.06 | 5.06 |

A 20 mg/mL (protein concentration based on naked antibody) ADC-33 stock solution containing 10 mM succinic acid-sodium succinate pH 5.0, 80 mg/mL sucrose and 0.2 mg/mL PS80 was prepared, filtered, bottled and then lyophilized according to the lyophilization process parameters 2 (see Table 14). The surface of the powder cake was flat and showed no wrinkles and depressions. 0.89% water was contained. The lyophilization process could meet the requirement for product quality.

Table 14. Lyophilization process parameters 2

| Lyophilization process parameter | Temperature setting (°C) | Time setting (min) | Retention time (h) | Degree of vacuum (Pa) |
|---|---|---|---|---|
| Pre-freezing | -5 | 10 | 1 | N/A |
| Pre-freezing | -45 | 40 | 2 | N/A |
| Primary drying | -20 | 120 | 35 | 10 |
| Secondary drying | 25 | 60 | 8 | 1 |

A 20 mg/mL (protein concentration based on naked antibody) ADC-32 stock solution containing 10 mM succinic acid-sodium succinate pH 5.0, 80 mg/mL sucrose and 0.2 mg/mL PS80 was prepared, filtered, bottled and then lyophilized according to the lyophilization process parameters 3 (see Table 15). The surface of the powder cake was flat and showed no depressions. The bottom edge of the powder cake slightly shrank. 1.17% water was contained. The lyophilization process could substantially meet the requirement for product quality.

Table 15. Lyophilization process parameters 3

| Lyophilization process parameter | Temperature setting (°C) | Time setting (min) | Retention time (h) | Degree of vacuum (Pa) |
|---|---|---|---|---|
| Pre-freezing | -5 | 10 | 1 | N/A |
| Pre-freezing | -45 | 40 | 2 | N/A |
| Primary drying | -15 | 120 | 30 | 10 |
| Secondary drying | 25 | 60 | 9 | 1 |

**Examples 2-7. Long-term stability data**

A 20 mg/mL (protein concentration based on naked antibody) ADC-32 stock solution containing 10 mM succinic acid-sodium succinate pH 5.0, 80 mg/mL sucrose and 0.2 mg/mL PS80 was prepared, filtered and bottled, and then pilot batches of finished products of the stock solution were formulated according to the lyophilization process parameters 2, subjected to long-term storage at 2-8 °C, reconstituted and then tested. The results are shown in Table 16. Compared to the products on D0, those under 2-8 °C M3 conditions showed SEC, reduced CE and free toxin levels within acceptable ranges (SEC% ≥ 93%; reduced CE% ≥ 95%; free toxin level ≤ 330 ppm). The SEC slightly decreased by 0.5%, while both the reduced CE and free toxin level remained unchanged.

Table 16. Long-term stability data results

| Batch No. | Conditions | SEC% | Reduced CE% | Free toxin |
|---|---|---|---|---|
| P1927 | D0 | 97.6 | 99.3 | <5ppm |
| | 2~8 °C M3 | 97.2 | 99.2 | <5ppm |
| P1929 | D0 | 97.8 | 99.3 | <5ppm |
| | 2~8°C M3 | 97.2 | 99.2 | <5ppm |
| Note: in the table, "M" represents month; for example, M3 represents 3 months. | | | | |

## Claims

1. A pharmaceutical composition, comprising an antibody drug conjugate and a buffer, wherein the antibody drug conjugate has a structure of general formula (Pc-L-Y-D):

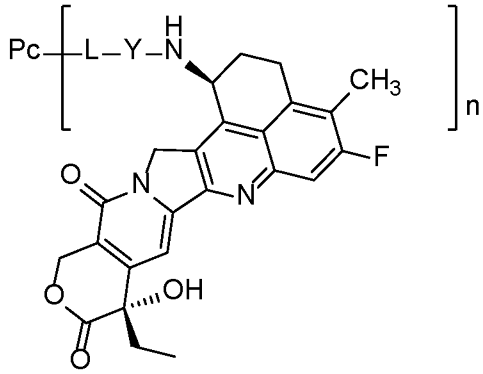

(Pc-L-Y-D) ;

wherein:

Y is selected from the group consisting of -O-$(CR^aR^b)_m$-$CR^1R^2$-C(O)-, -O-$CR^1R^2$-$(CR^aR^b)_m$-, -O-$CR^1R^2$-, -NH-$(CR^aR^b)_m$-$CR^1R^2$-C(O)- and -S-$(CR^aR^b)_m$-$CR^1R^2$-C(O)-;
$R^a$ and $R^b$ are identical or different and are each independently selected from the group consisting of hydrogen, deuterium, halogen, alkyl, haloalkyl, deuterated alkyl, alkoxy, hydroxy, amino, cyano, nitro, hydroxyalkyl, cycloalkyl and heterocyclyl;
or, $R^a$ and $R^b$, together with carbon atoms linked thereto, form cycloalkyl or heterocyclyl;
$R^1$ is selected from the group consisting of halogen, haloalkyl, deuterated alkyl, cycloalkyl, cycloalkylalkyl, alkoxyalkyl, heterocyclyl, aryl and heteroaryl;
$R^2$ is selected from the group consisting of hydrogen, halogen, haloalkyl, deuterated alkyl, cycloalkyl, cycloalkylalkyl, alkoxyalkyl, heterocyclyl, aryl and heteroaryl;
or, $R^1$ and $R^2$, together with carbon atoms linked thereto, form cycloalkyl or heterocyclyl;
or, $R^a$ and $R^2$, together with carbon atoms connected thereto, form cycloalkyl or heterocyclyl;
m is an integer from 0 to 4;
n is a decimal or an integer from 1 to 10;
L is a linker unit;
Pc is an antibody or an antigen-binding fragment thereof;
the pharmaceutical composition is at a pH of 4.5 to 5.2, preferably a pH of 4.8 to 5.2, and more preferably a pH of 5.0 to 5.1.

2. The pharmaceutical composition according to claim 1, wherein the pharmaceutical composition further comprises a surfactant, the surfactant being preferably a polysorbate, more preferably polysorbate 80 or polysorbate 20, and most preferably polysorbate 80.

3. The pharmaceutical composition according to claim 2, wherein the surfactant is at a concentration of about 0.01 mg/mL to about 1.0 mg/mL, preferably about 0.05 mg/mL to about 0.5 mg/mL, and more preferably about 0.2 mg/mL.

4. The pharmaceutical composition according to any one of claims 1 to 3, wherein the composition further comprises a saccharide, the saccharide being preferably selected from the group consisting of sucrose, mannitol, sorbitol and trehalose, and more preferably being sucrose.

5. The pharmaceutical composition according to claim 4, wherein the saccharide is at a concentration of about 60 mg/mL to about 90 mg/mL, preferably about 80 mg/mL.

6. The pharmaceutical composition according to any one of claims 1 to 5, wherein the antibody drug conjugate is at a concentration of about 1 mg/mL to about 100 mg/mL, preferably about 10 mg/mL to about 30 mg/mL, and more preferably about 20 mg/mL to about 22 mg/mL.

7. The pharmaceutical composition according to any one of claims 1 to 6, wherein the buffer is selected from the group consisting of a histidine salt buffer, a succinate buffer and a citrate buffer, preferably a succinate buffer, and more preferably a succinic acid-sodium succinate buffer.

**8.** The pharmaceutical composition according to any one of claims 1 to 7, wherein the buffer is at a concentration of about 5 mM to about 50 mM, preferably about 5 mM to about 20 mM, and more preferably about 10 mM.

**9.** The pharmaceutical composition according to any one of claims 1 to 8, comprising the following components:

(a) the antibody drug conjugate at about 10 mg/mL to about 30 mg/mL, (b) a polysorbate at about 0.05 mg/mL to about 0.5 mg/mL, (c) a saccharide at about 60 mg/mL to about 90 mg/mL, and (d) a buffer at about 5 mM to about 20 mM; the pharmaceutical composition being at a pH of 4.8-5.2;

preferably comprising the following components:

(a) the antibody drug conjugate at about 20 mg/mL to about 22 mg/mL, (b) polysorbate 80 at about 0.2 mg/mL, (c) sucrose at about 80 mg/mL, and (d) a succinate buffer at about 10 mM; the composition being at a pH of 5.0-5.1.

**10.** The pharmaceutical composition according to any one of claims 1 to 9, wherein the antibody drug conjugate has the following structure:

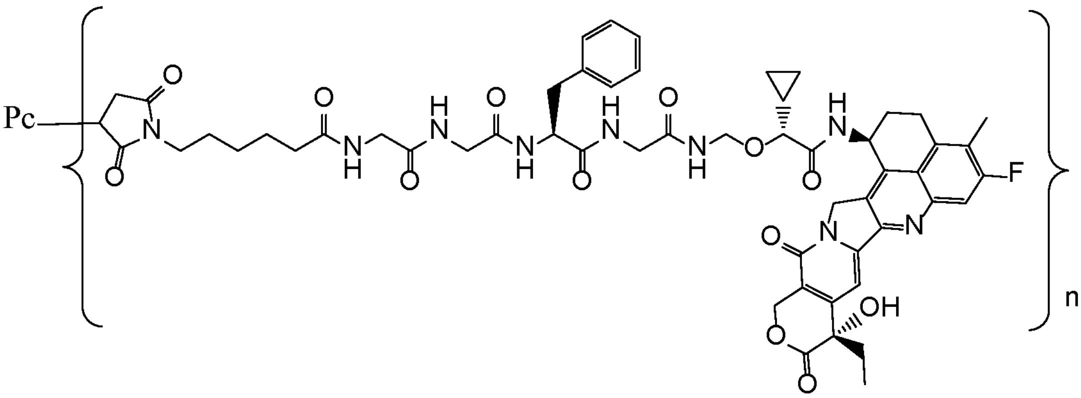

;

wherein:

n is a decimal or an integer from 3 to 8;
Pc is an antibody or an antigen-binding fragment thereof.

**11.** The pharmaceutical composition according to any one of claims 1 to 10, wherein the antibody or the antigen-binding fragment thereof is selected from the group consisting of an anti-HER2 (ErbB2) antibody, an anti-EGFR antibody, an anti-B7-H3 antibody, an antic-Met antibody, an anti-HER3 (ErbB3) antibody, an anti-HER4 (ErbB4) antibody, an anti-CD20 antibody, an anti-CD22 antibody, an anti-CD30 antibody, an anti-CD33 antibody, an anti-CD44 antibody, an anti-CD56 antibody, an anti-CD70 antibody, an anti-CD73 antibody, an anti-CD105 antibody, an anti-CEA antibody, an anti-A33 antibody, an anti-Cripto antibody, an anti-EphA2 antibody, an anti-G250 antibody, an anti-MUCl antibody, an anti-Lewis Y antibody, an anti-VEGFR antibody, an anti-GPNMB antibody, an anti-integrin antibody, an anti-PSMA antibody, an anti-tenascin-C antibody, an anti-SLC44A4 antibody and an anti-mesothelin antibody, or antigen-binding fragments thereof; preferably, the antibody or the antigen-binding fragment thereof is selected from the group consisting of trastuzumab, pertuzumab, nimotuzumab, enoblituzumab, emibetuzumab, inotuzumab, pinatuzumab, brentuximab, gemtuzumab, bivatuzumab, lorvotuzumab, cBR96 and glematumamab, or antigen-binding fragments thereof.

**12.** The pharmaceutical composition according to any one of claims 1 to 11, wherein the antibody conjugate has the following structure:

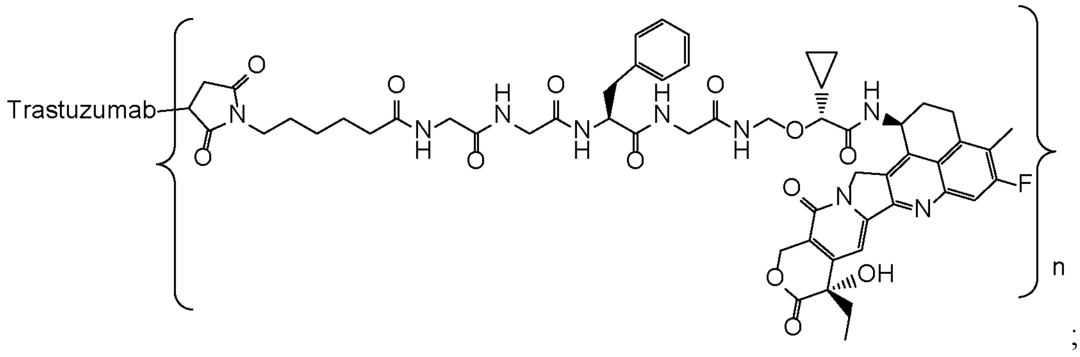

wherein n is a decimal or an integer from 3 to 8.

**13.** A pharmaceutical composition, comprising an antibody drug conjugate having the following structure:

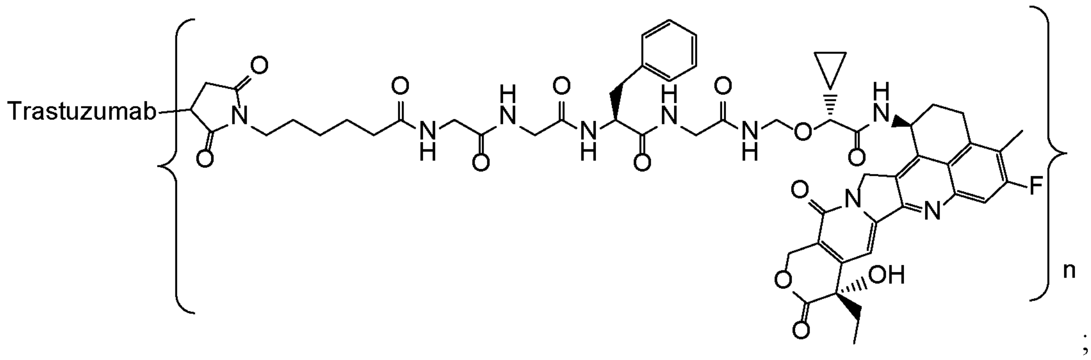

wherein n is a decimal or an integer from 3 to 8;

and the pharmaceutical composition comprises:

(a) the antibody drug conjugate at about 10 mg/mL to about 30 mg/mL, (b) a polysorbate at about 0.05 mg/mL to about 0.5 mg/mL, (c) a saccharide at about 60 mg/mL to about 90 mg/mL, and (d) a buffer at about 5 mM to about 20 mM; the pharmaceutical composition being at a pH of 4.8 to 5.2;

preferably, the pharmaceutical composition comprises: (a) the antibody drug conjugate at about 20 mg/mL to about 22 mg/mL, (b) polysorbate 80 at about 0.2 mg/mL, (c) sucrose at about 80 mg/mL, and (d) a succinate buffer at about 10 mM; the pharmaceutical composition being at a pH of 5.0 to 5.1.

**14.** A lyophilized formulation comprising an antibody drug conjugate, wherein the formulation can be reconstituted to form the pharmaceutical composition according to any one of claims 1 to 13.

**15.** A method for preparing a lyophilized formulation comprising an antibody drug conjugate, comprising a step of lyophilizing the pharmaceutical composition according to any one of claims 1 to 13.

**16.** A lyophilized formulation comprising an antibody drug conjugate, obtained by lyophilizing the pharmaceutical composition according to any one of claims 1 to 13.

**17.** A reconstituted solution comprising an antibody drug conjugate, obtained by reconstituting the lyophilized formulation according to claim 14 or 16;

wherein preferably, the reconstituted solution comprises the following components:

(a) the antibody drug conjugate at about 10 mg/mL to about 30 mg/mL, (b) a polysorbate at about 0.05 mg/mL to about 0.5 mg/mL, (c) a saccharide at about 60 mg/mL to about 90 mg/mL, and (d) a buffer at

about 5 mM to about 20 mM; the reconstituted solution being at a pH of 4.8 to 5.2;

more preferably, the reconstituted solution comprises the following components:

(a) an antibody drug conjugate at about 20 mg/mL to about 22 mg/mL, (b) polysorbate 80 at about 0.2 mg/mL, (c) sucrose at about 80 mg/mL, and (d) a succinate buffer at about 10 mM; the reconstituted solution being at a pH of 5.0 to 5.1.

**18.** An article of manufacture, comprising a container containing the pharmaceutical composition according to any one of claims 1 to 13, the lyophilized formulation according to claim 14 or 16, or the reconstituted solution according to claim 17.

**19.** A method for treating a disease, comprising administering to a patient an effective amount of the pharmaceutical composition according to any one of claims 1 to 13, the lyophilized formulation according to claim 14 or 16, the reconstituted solution according to claim 17, or the article of manufacture according to claim 18, wherein preferably, the disease is cancer associated with HER2, HER3, B7H3 or EGFR expression; more preferably, the cancer is selected from the group consisting of breast cancer, ovarian cancer, cervical cancer, uterine cancer, prostate cancer, kidney cancer, urinary tract cancer, bladder cancer, liver cancer, stomach cancer, endometrial cancer, salivary gland carcinoma, esophageal cancer, melanoma, neuroglioma, neuroblastoma, sarcoma, lung cancer, colon cancer, rectal cancer, colorectal cancer, leukemia, bone cancer, skin cancer, thyroid cancer, pancreatic cancer and lymphoma.

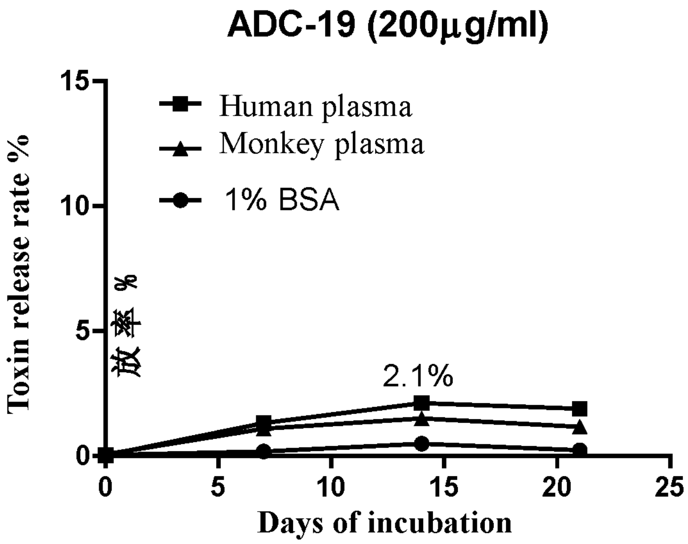
ADC-19 (200μg/ml)
Human plasma
Monkey plasma
1% BSA
Toxin release rate %
放率%
2.1%
Days of incubation

FIG. 1A

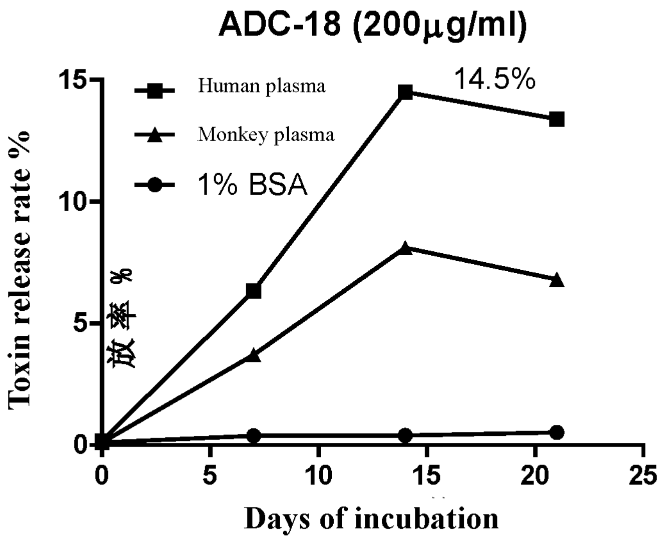
ADC-18 (200μg/ml)
Human plasma
Monkey plasma
1% BSA
14.5%
Toxin release rate %
放率%
Days of incubation

FIG. 1B

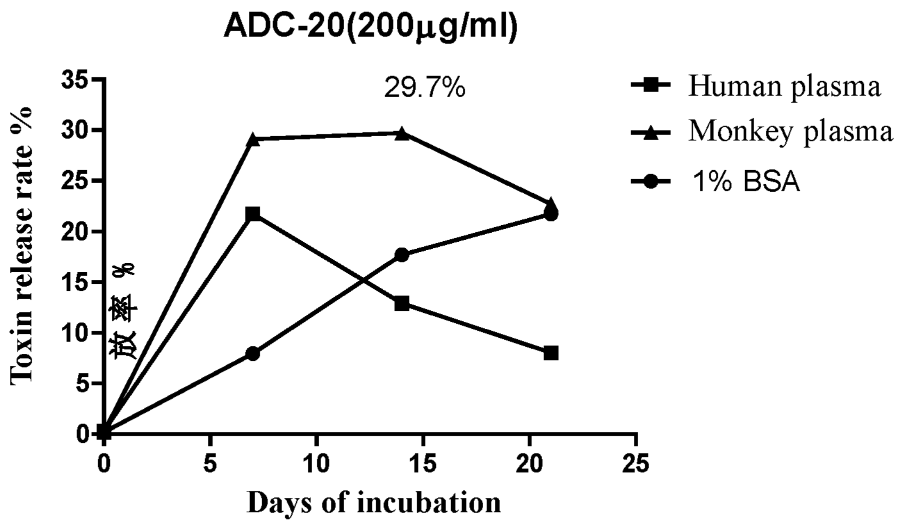
ADC-20(200μg/ml)
29.7%
Human plasma
Monkey plasma
1% BSA
Toxin release rate %
放率%
Days of incubation
0
5
10
15
20
25
30
35

FIG. 1C

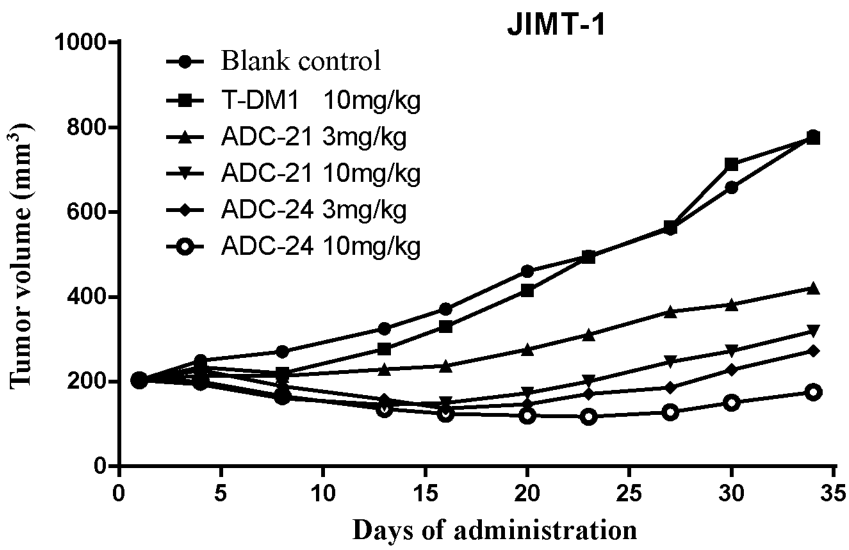
JIMT-1
Blank control
T-DM1 10mg/kg
ADC-21 3mg/kg
ADC-21 10mg/kg
ADC-24 3mg/kg
ADC-24 10mg/kg
Tumor volume (mm3)
Days of administration
0
200
400
600
800
1000

FIG. 2

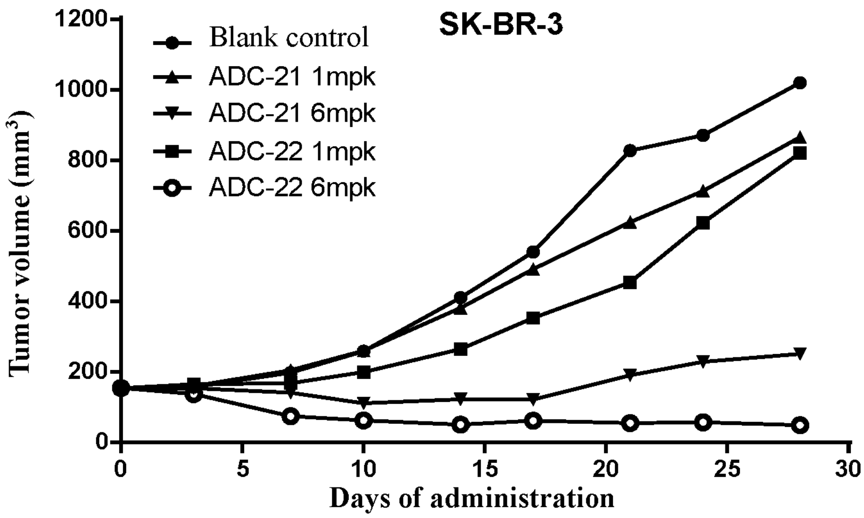
SK-BR-3
Blank control
ADC-21 1mpk
ADC-21 6mpk
ADC-22 1mpk
ADC-22 6mpk
Tumor volume (mm3)
1200
1000
800
600
400
200
0
0
5
10
15
20
25
30
Days of administration

FIG. 3

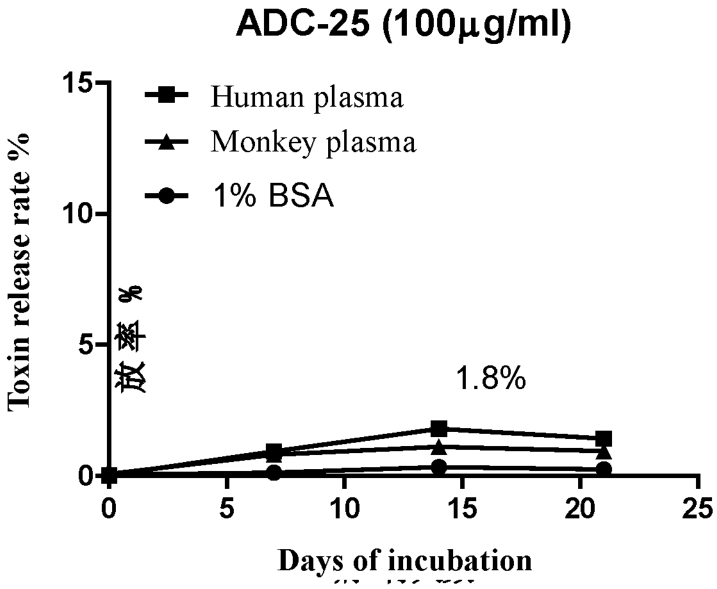
ADC-25 (100µg/ml)
Human plasma
Monkey plasma
1% BSA
Toxin release rate %
15
10
5
0
1.8%
0
5
10
15
20
25
Days of incubation

FIG. 4

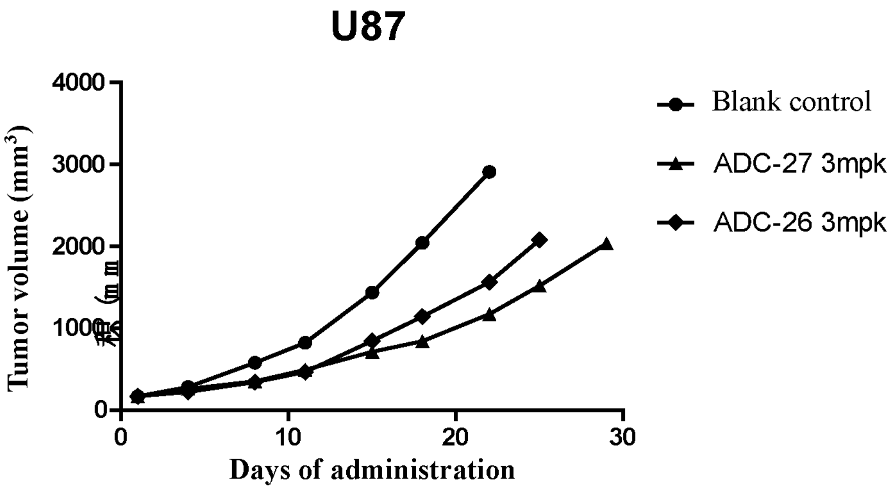
U87
Tumor volume (mm³)
4000
3000
2000
1000
0
0
10
20
30
Days of administration
Blank control
ADC-27 3mpk
ADC-26 3mpk

FIG. 5

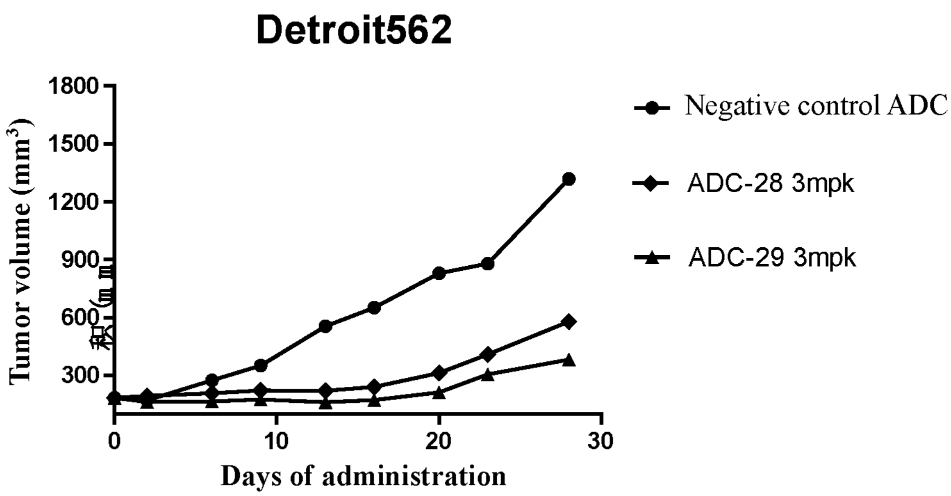
Detroit562
Tumor volume (mm³)
1800
1500
1200
900
600
300
0
10
20
30
Days of administration
Negative control ADC
ADC-28 3mpk
ADC-29 3mpk

FIG. 6

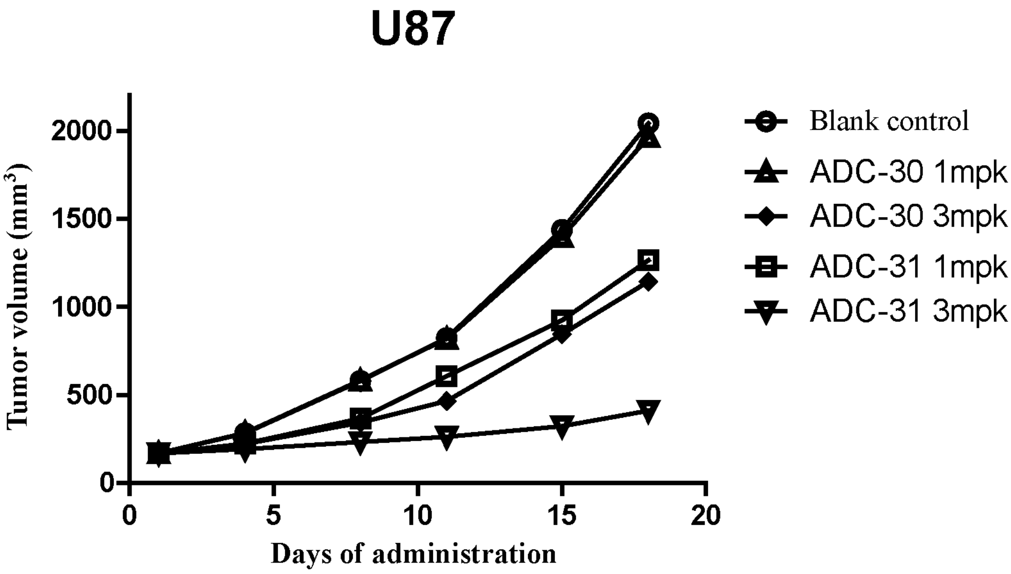
U87
Tumor volume (mm$^3$)
2000
1500
1000
500
0
0
5
10
15
20
Days of administration
Blank control
ADC-30 1mpk
ADC-30 3mpk
ADC-31 1mpk
ADC-31 3mpk

FIG. 7

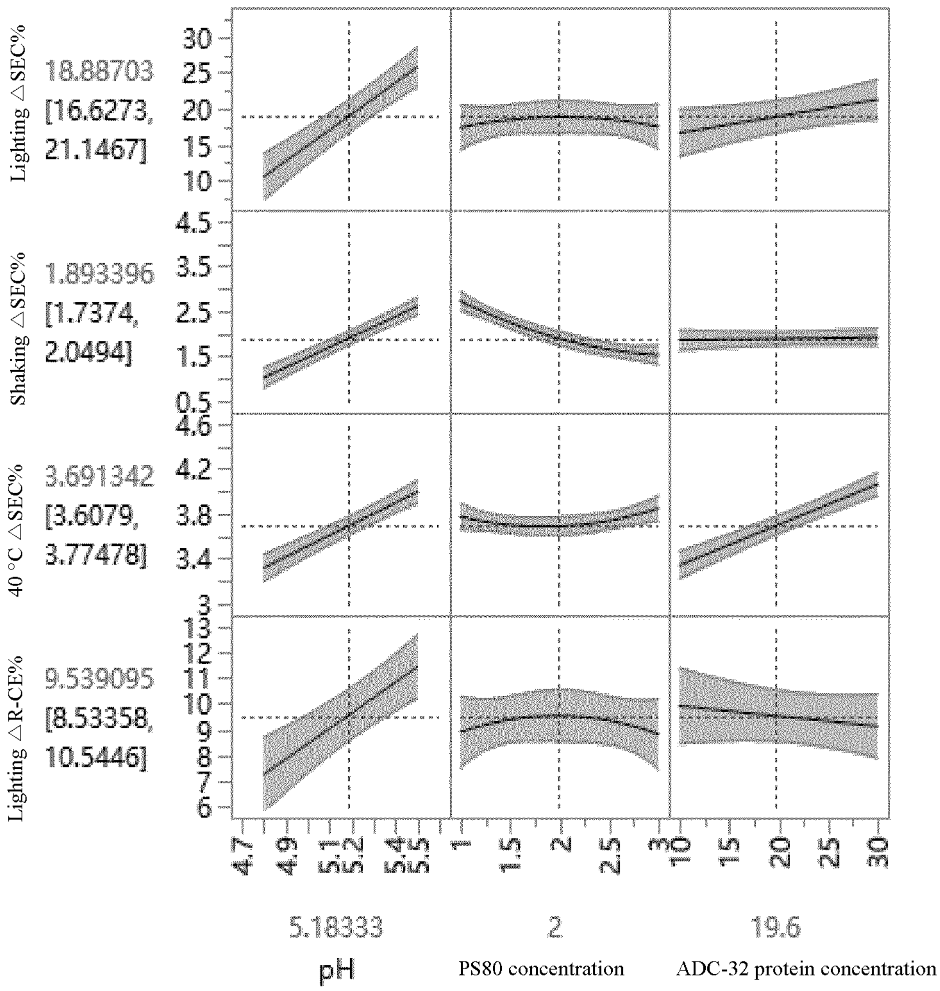
Lighting △SEC%
18.88703
[16.6273,
21.1467]
Shaking △SEC%
1.893396
[1.7374,
2.0494]
40 °C △SEC%
3.691342
[3.6079,
3.77478]
Lighting △R-CE%
9.539095
[8.53358,
10.5446]
5.18333
pH
2
PS80 concentration
19.6
ADC-32 protein concentration

FIG. 8

## INTERNATIONAL SEARCH REPORT

International application No. **PCT/CN2021/082854**

### A. CLASSIFICATION OF SUBJECT MATTER

A61K 47/68(2017.01)i; A61K 47/65(2017.01)i; A61K 31/4745(2006.01)i; A61P 35/00(2006.01)i; A61P 35/02(2006.01)i; C07D 491/22(2006.01)i

According to International Patent Classification (IPC) or to both national classification and IPC

### B. FIELDS SEARCHED

Minimum documentation searched (classification system followed by classification symbols)

A61K, A61P, C07D

Documentation searched other than minimum documentation to the extent that such documents are included in the fields searched

Electronic data base consulted during the international search (name of data base and, where practicable, search terms used)

VEN; CNABS; CNTXT; WOTXT, USTXT; EPTXT; CNKI; web of science; stn; 抗体; 依沙替康, 曲妥珠; 偶联; 偶联物; 冻干; 缓冲剂; 肿瘤; antibody; antibody-drug; trastuzumab; deruxtecan; conjugates; stn权利要求1结构式检索 structural search on claim 1

### C. DOCUMENTS CONSIDERED TO BE RELEVANT

| Category* | Citation of document, with indication, where appropriate, of the relevant passages | Relevant to claim No. |
|---|---|---|
| X | CN 110464847 A (DAIICHI SANKYO COMPANY, LIMITED) 19 November 2019 (2019-11-19) claims 23 and 24, description, paragraphs [0799], [0971] and [0972], and embodiment 16 | 1, 11, 18, 19 |
| X | 苏铮等 (SU, Zheng et al.). "人表皮生长因子受体2阳性乳腺癌在研靶向新药—trastuzumab deruxtecan (Novel Drug for Breast Cancer——Trastuzumab Deruxtecan)" *临床药物治疗杂志 (Clinical Medication Journal),* Vol. 17, No. 1, 15 January 2019 (2019-01-15), ISSN: 1672-3384, p. 1, the abstract, and p. 2, right column, lines 1-11, and figure 1 | 1, 11, 18, 19 |
| Y | CN 110464847 A (DAIICHI SANKYO COMPANY, LIMITED) 19 November 2019 (2019-11-19) claims 23 and 24, description, paragraphs [0799], [0971] and [0972], and embodiment 16 | 2-9, 11, 14-19 |
| Y | CN 105163763 A (ABBVIE DEUTSCHLAND GMBH & CO. KG; ABBVIE INC.) 16 December 2015 (2015-12-16) claims 1-21, and description, paragraph [0016] | 2-9, 11, 14-19 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 June 2021** | **29 June 2021** |

| Name and mailing address of the ISA/CN | Authorized officer |
|---|---|
| **China National Intellectual Property Administration (ISA/CN)** **No. 6, Xitucheng Road, Jimenqiao, Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2021/082854**

**C. DOCUMENTS CONSIDERED TO BE RELEVANT**

| Category* | Citation of document, with indication, where appropriate, of the relevant passages | Relevant to claim No. |
|---|---|---|
| A | CN 104755494 A (DAIICHI SANKYO COMPANY, LIMITED) 01 July 2015 (2015-07-01) entire document | 10, 12, 13 |

Form PCT/ISA/210 (second sheet) (January 2015)

INTERNATIONAL SEARCH REPORT

International application No.
PCT/CN2021/082854

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

International application No.
**PCT/CN2021/082854**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **19**
because they relate to subject matter not required to be searched by this Authority, namely:

[1] Claim 19 relates to methods of treatment and is a subject matter that does not warrant an international search according to the criteria set out in PCT Rule 39.1(iv). However, it is expected that the applicant may modify claim 19 to a corresponding pharmaceutical use claim, so an international search report is made on this basis.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2021/082854**

| Patent document cited in search report | Publication date (day/month/year) | Patent family member(s) | Publication date (day/month/year) |
|---|---|---|---|
| CN 110464847 A | 19 November 2019 | CN 105829346 A | 03 August 2016 |
| | | RU 2683780 C2 | 02 April 2019 |
| | | AU 2015212265 B2 | 02 January 2020 |
| | | EP 3101032 A4 | 06 September 2017 |
| | | PH 12016500904 A1 | 11 July 2016 |
| | | JP 2017095457 A | 01 June 2017 |
| | | CN 105829346 B | 23 August 2019 |
| | | JP 2017105789 A | 15 June 2017 |
| | | KR 101916553 B1 | 07 November 2018 |
| | | TW 201613649 A | 16 April 2016 |
| | | RU 2016123597 A | 02 March 2018 |
| | | SG 10201800210T A | 27 February 2018 |
| | | SG 11201603960X A | 28 July 2016 |
| | | IL 245252 D0 | 30 June 2016 |
| | | JP 2020097617 A | 25 June 2020 |
| | | WO 2015115091 A1 | 06 August 2015 |
| | | US 2020385486 A1 | 10 December 2020 |
| | | JP 2019112403 A | 11 July 2019 |
| | | KR 102190548 B1 | 14 December 2020 |
| | | EP 3101032 B1 | 16 January 2019 |
| | | LT 3101032 T | 25 March 2019 |
| | | AU 2020200596 A1 | 20 February 2020 |
| | | CA 2928794 C | 13 August 2019 |
| | | US 2019077880 A1 | 14 March 2019 |
| | | IL 278891 D0 | 31 January 2021 |
| | | JP 6665325 B2 | 13 March 2020 |
| | | KR 20180122038 A | 09 November 2018 |
| | | US 10155821 B2 | 18 December 2018 |
| | | PL 3101032 T3 | 31 July 2019 |
| | | CA 2928794 A1 | 06 August 2015 |
| | | ES 2727351 T3 | 15 October 2019 |
| | | JP 5998289 B2 | 28 September 2016 |
| | | MX 2016006498 A | 04 August 2016 |
| | | HU E044389 T2 | 28 October 2019 |
| | | IL 245252 A | 30 June 2019 |
| | | IL 266790 D0 | 31 July 2019 |
| | | JP 2017036285 A | 16 February 2017 |
| | | JP 6466895 B2 | 06 February 2019 |
| | | ZA 201602802 B | 27 September 2017 |
| | | PT 3101032 T | 03 April 2019 |
| | | EP 3466976 A1 | 10 April 2019 |
| | | US 2016333112 A1 | 17 November 2016 |
| | | DK 3101032 T3 | 29 April 2019 |
| | | KR 20200140932 A | 16 December 2020 |
| | | RS 58415 B1 | 30 April 2019 |
| | | JP WO2015115091 A1 | 23 March 2017 |
| | | HR P20190431 T1 | 19 April 2019 |
| | | TW I661839 B | 11 June 2019 |
| | | JP 6046301 B1 | 14 December 2016 |
| | | SI 3101032 T1 | 28 February 2019 |

Form PCT/ISA/210 (patent family annex) (January 2015)

**INTERNATIONAL SEARCH REPORT**
**Information on patent family members**

International application No.
**PCT/CN2021/082854**

| Patent document cited in search report | Publication date (day/month/year) | Patent family member(s) | Publication date (day/month/year) |
|---|---|---|---|
| CN 105163763 A | 16 December 2015 | DK 2968588 T3 | 23 April 2019 |
| | | RU 2015144287 A | 24 April 2017 |
| | | AU 2018267662 A1 | 13 December 2018 |
| | | HU E044071 T2 | 30 September 2019 |
| | | NZ 630826 A | 30 June 2017 |
| | | EP 2968588 B1 | 09 January 2019 |
| | | CN 110433139 A | 12 November 2019 |
| | | JP 2020143152 A | 10 September 2020 |
| | | EP 3517132 A1 | 31 July 2019 |
| | | WO 2014143765 A1 | 18 September 2014 |
| | | SI 2968588 T1 | 31 May 2019 |
| | | BR 112015023391 A8 | 30 January 2018 |
| | | ME 03457 B | 20 January 2020 |
| | | KR 20150131366 A | 24 November 2015 |
| | | HR P20190672 T1 | 23 August 2019 |
| | | IL 274881 D0 | 30 July 2020 |
| | | CA 2906130 A1 | 18 September 2014 |
| | | RU 2015144287 A3 | 19 March 2018 |
| | | MX 2015012549 A | 21 June 2016 |
| | | AU 2014228172 B2 | 06 December 2018 |
| | | RU 2020125266 A | 24 September 2020 |
| | | HK 1217644 A1 | 20 January 2017 |
| | | JP 2016515511 A | 30 May 2016 |
| | | TR 201905313 T4 | 21 May 2019 |
| | | LT 2968588 T | 10 May 2019 |
| | | PT 2968588 T | 08 May 2019 |
| | | US 2020282072 A1 | 10 September 2020 |
| | | ES 2727134 T3 | 14 October 2019 |
| | | SG 10201707633V A | 29 November 2017 |
| | | IL 241009 D0 | 30 November 2015 |
| | | EP 2968588 A1 | 20 January 2016 |
| | | TW I689313 B | 01 April 2020 |
| | | WO 2014143765 A8 | 11 September 2015 |
| | | JP 6382935 B2 | 29 August 2018 |
| | | TW 201519903 A | 01 June 2015 |
| | | BR 112015023391 A2 | 28 November 2017 |
| | | CN 105163763 B | 02 July 2019 |
| | | JP 2018184468 A | 22 November 2018 |
| | | RS 58666 B1 | 28 June 2019 |
| | | HK 1219044 A1 | 24 March 2017 |
| | | TW 201945032 A | 01 December 2019 |
| | | PL 2968588 T3 | 30 August 2019 |
| | | SG 11201507430R A | 29 October 2015 |
| | | US 2014286969 A1 | 25 September 2014 |
| | | AU 2014228172 A1 | 24 September 2015 |
| | | AU 2020277164 A1 | 24 December 2020 |
| CN 104755494 A | 01 July 2015 | TW 202033499 A | 16 September 2020 |
| | | HR P20200353 T1 | 12 June 2020 |
| | | LT 2907824 T | 11 June 2018 |
| | | KR 20200026323 A | 10 March 2020 |

Form PCT/ISA/210 (patent family annex) (January 2015)

**INTERNATIONAL SEARCH REPORT**
**Information on patent family members**

International application No.
**PCT/CN2021/082854**

| Patent document cited in search report | Publication date (day/month/year) | Patent family member(s) | | | Publication date (day/month/year) |
|---|---|---|---|---|---|
| | | SG | 10201804788X | A | 30 July 2018 |
| | | KR | 101841818 | B1 | 26 March 2018 |
| | | AU | 2013328111 | A1 | 12 March 2015 |
| | | TW | 201811746 | A | 01 April 2018 |
| | | TW | 201420117 | A | 01 June 2014 |
| | | HU | E039000 | T2 | 28 December 2018 |
| | | PH | 12015500667 | B1 | 18 May 2015 |
| | | JP | 2020180124 | A | 05 November 2020 |
| | | JP | 6030267 | B1 | 24 November 2016 |
| | | EP | 3342785 | A1 | 04 July 2018 |
| | | JP | 2018188455 | A | 29 November 2018 |
| | | CA | 2885800 | A1 | 17 April 2014 |
| | | HR | P20180870 | T1 | 13 July 2018 |
| | | MX | 2019004502 | A | 21 August 2019 |
| | | JP | 6715888 | B2 | 01 July 2020 |
| | | PL | 3342785 | T3 | 07 September 2020 |
| | | KR | 102087017 | B1 | 10 March 2020 |
| | | ES | 2773710 | T3 | 14 July 2020 |
| | | LT | 3342785 | T | 10 February 2020 |
| | | JP | 2016196484 | A | 24 November 2016 |
| | | HU | E048748 | T2 | 28 August 2020 |
| | | MX | 2015003903 | A | 17 July 2015 |
| | | NZ | 705394 | A | 26 October 2018 |
| | | CY | 1120363 | T1 | 10 July 2019 |
| | | TW | 201920098 | A | 01 June 2019 |
| | | PL | 2907824 | T3 | 31 August 2018 |
| | | JP | 5953378 | B2 | 20 July 2016 |
| | | MX | 364484 | B | 29 April 2019 |
| | | AU | 2013328111 | B2 | 02 November 2017 |
| | | CN | 104755494 | B | 07 September 2018 |
| | | TW | I615152 | B | 21 February 2018 |
| | | DK | 2907824 | T3 | 23 July 2018 |
| | | EP | 2907824 | B1 | 11 April 2018 |
| | | KR | 20190135559 | A | 06 December 2019 |
| | | IL | 271760 | D0 | 27 February 2020 |
| | | JP | 6371452 | B2 | 08 August 2018 |
| | | WO | 2014057687 | A1 | 17 April 2014 |
| | | CA | 3021435 | A1 | 17 April 2014 |
| | | US | 2016279259 | A1 | 29 September 2016 |
| | | RU | 2018128384 | A | 02 October 2018 |
| | | JP | 2018008982 | A | 18 January 2018 |
| | | JP | WO2014057687 | A1 | 05 September 2016 |
| | | US | 2019008981 | A1 | 10 January 2019 |
| | | NZ | 746440 | A | 29 November 2019 |
| | | US | 10195288 | B2 | 05 February 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202010219601
- CN 202110287012
- US 4970198 A
- US 5079233 A
- US 5585089 A
- US 5606040 A
- US 5693762 A
- US 5739116 A
- US 5767285 A
- US 5773001 A
- WO 2004010957 A
- WO 2005001038 A
- US 7090843 A
- US 7659241 B
- WO 2008025020 A
- WO 2005037992 A
- US 8088387 B
- WO 2014057687 A
- CN 2019107873 W
- WO 2020063676 A
- US 20050238649 A1
- US 5208020 A
- EP 0737686 A1
- WO 2013106717 A
- WO 201396771 A
- US 200520645 B
- CN 105829346 A
- EP 2907824 A
- EP 2907824 A1
- EP 2862856 A1
- CN 108853514 A
- CN 104755494 A
- US 20050169933 A

### Non-patent literature cited in the description

- **MULLARD A.** *Nature Reviews Drug Discovery,* 2013, vol. 12, 329-332
- **DIJOSEPH JF ; ARMELLINO DC.** *Blood,* 2004, vol. 103, 1807-1814
- *Drugs of the Future,* 2000, vol. 25 (7), 686
- *Nat. Biotechnol,* 2003, vol. 21 (7), 778-784
- *Clinical Cancer Research,* 2016, vol. 22 (20), 5097-5108
- *Cancer Sci,* 2016, vol. 107, 1039-1046
- *J. Biol. Chem.,* 1968, vol. 243, 3558
- **KABAT, E. A. et al.** Sequences of Proteins of Immunological Interest. 1991
- **WATSON et al.** Molecular Biology of the Gene. The Benjamin/Cummings Pub. Co, 1987, 224
- **CHARI et al.** *Cancer Research,* 1992, vol. 52, 127-131
- Antibodies: A Laboratory Manual and Molecular Cloning: A Laboratory Manual. Cold Spring Harbor Laboratory
- *Tetrahedron Letters,* 1984, vol. 25 (12), 1269-72
- *Journal of the American Chemical Society,* 2014, vol. 136 (22), 8138-8142
- *Journal of Medicinal Chemistry,* 2013, vol. 56 (13), 5541-5552